# EUROPEAN PATENT APPLICATION

(11) **EP 4 019 495 A1**
(43) Date of publication of application: **29.06.2022**
(21) Application number: 20853761.3
(22) Date of filing: 18.08.2020
(51) Int. Cl.: C07C 235/84, A61K 31/166, A61K 31/341, A61P 35/00

(54) **NOVEL COMPOUND HAVING CANCER METASTASIS INHIBITORY ACTIVITY, PREPARATION METHOD THEREFOR, AND PHARMACEUTICAL COMPOSITION FOR INHIBITING CANCER METASTASIS AND INVASION OR TREATING COLORECTAL CANCER, COMPRISING COMPOUND**

(30) Priority: 19.08.2019 KR 20190101284
(71) Applicant: Cellgentek Co., Ltd, Cheongju-si, Chungcheongbuk-do 28161 (KR)
(72) Inventor: KIM, Kyung Keun, Gwangju 61402 (KR); HA, Hyung Ho, Suncheon-si Jeollanam-do 57930 (KR); KIM, Hangun, Gwangju 61946 (KR); BAE, Woo Kyun, Gwangju 61684 (KR); BAE, Jung A, Gwangju 61766 (KR)
(74) Representative: Mewburn Ellis LLP
(86) International application number: PCT/KR2020/010999
(87) International publication number: WO 2021/034087

(57) **Abstract**

The present invention provides a novel compound that increases KAI1 promoter activity, a pharmaceutically acceptable salt thereof, and a composition for inhibiting cancer metastasis and invasion or treating colorectal cancer containing the same.

## Description

### Technical Field

The present invention provides a novel compound having cancer metastasis inhibitory activity, and a composition for inhibiting cancer metastasis and invasion or treating colorectal cancer containing the same.

### Background Art

The number of cancer (malignant neoplasm) deaths in Korea in 2002 was 62,887, which accounts for 25.5% (29.6% of male deaths, and 20.5% of female deaths) of the total number of deaths (246,515) in Korea in 2002, and cancer is the first leading cause of death in Korea. This means that about 131 people per 100,000 people in Korea die from cancer (2002 Statistics Korea). From the viewpoint of cancer treatment, one of the biggest reasons that the mortality rate of cancer patients cannot be reduced is that the invasion and metastasis of cancer cells cannot be suppressed in the process of malignancy. Recent studies have merely elucidated the molecular mechanism of the cancer metastasis process by identifying cancer metastasis-related genes (e.g., Twist, KAI1, etc.). Therefore, drugs or treatment methods capable of effectively inhibiting or preventing cancer metastasis using these cancer metastasis-related genes have not yet been reported. In the case of the United States, recent statistical analysis results show that the five-year survival rate for metastatic cancer has not improved for the past 20-30 years.

In this regard, KAI1 (Kangai 1) was initially identified as a metastatic suppressor in prostate carcinoma. The KAI1 protein is located on human chromosome 11p11 and belongs to the transmembrane 4 superfamily (TM4SF). KAI1 can regulate signal transduction from cell to cell and from cell to extracellular matrix (ECM), and may be involved in several basic biological processes such as fusion, migration, adhesion, mutation and invasion. Reduced or deleted expression of KAI1 has been demonstrated to be associated with metastasis and prognosis of various carcinomas, including laryngeal, prostate, breast, lung, gastric, colorectal and hepatocellular carcinomas.

Currently, as a method of treating cancer, surgery, chemotherapy, radiation therapy, etc. are used for cancer detected at an early stage, but the side effects thereof are also emerging as a big problem, and at the time when primary tumors are detected, metastases thereof are found in most cases. In addition, in the case of terminal cancer or metastatic cancer, there is no special treatment, and thus the patient dies within a limited period of time. Accordingly, to develop a new approach for cancer treatment, studies have been conducted to develop carcinogenic inhibitors and cancer treatment agents, which have few side effects and are highly effective in inhibiting or reducing metastasis of cancer, from natural products with relatively low toxicity.

### DISCLOSURE

### Technical Problem

Under this background, the present inventors have made extensive research efforts to discover novel small molecule compounds capable of inhibiting cancer metastasis, particularly colorectal cancer metastasis, and as a result, have found that novel compounds according to the present invention may exhibit excellent cancer metastasis inhibitory activity by efficiently increasing the activity of the KAI1 promoter gene, thereby completing the present invention.

### Technical Solution

To achieve the above, one embodiment of the present invention provides a compound represented by the following Formula 1 or a pharmaceutically acceptable salt thereof: 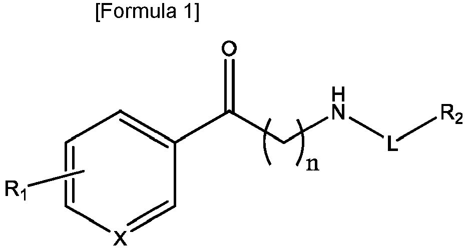 wherein
X is C or N;
L is C1 to C4 alkylene or a bond;
R₁ is hydrogen, halogen, substituted or unsubstituted C1-C4 alkyl, substituted or unsubstituted C1-C4 alkoxy, di(C1-C4 alkyl)amino, mono(C1-C4 alkyl)amino, amino, or substituted or unsubstituted 5- to 12-membered heterocycle,
wherein the substituent of the substituted alkyl may be: amino unsubstituted or substituted with one or two substituents selected from the group consisting of C1-C6 alkyl, C2-C6 alkenyl, C2-C6 alkynyl, C6-C9 aryl, benzyl, hydroxy-C1-C6 alkyl and C3-C8 cycloalkyl; or a 5- to 12-membered heterocycle or heteroaryl unsubstituted or substituted with one or two substituents selected from the group consisting of C1-C4 alkyl, C1-C4 alkoxycarbonyl, C6-C9 aryl unsubstituted or substituted with one or two substituents selected from the group consisting of C1-C4 alkyl, C1-C4 alkoxy, halogen and nitro, and 5- to 10-membered heterocycles or heteroaryls;
R₂ is 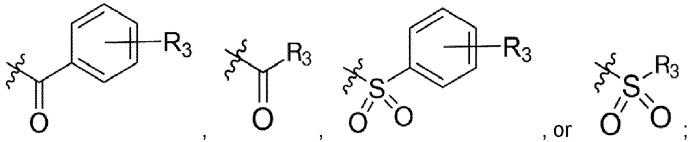
R₃ is at least one selected from the group consisting of hydrogen, halogen, substituted or unsubstituted C1-C6 alkyl, substituted or unsubstituted C2-C6 alkenyl, substituted or unsubstituted C3-C8 cycloalkyl, substituted or unsubstituted C1-C6 alkoxy, substituted or unsubstituted 5- to 9-membered heteroaryl, substituted or unsubstituted C6-C10 aryl, substituted or unsubstituted C6-C10 aryloxy, substituted or unsubstituted 5- to 9-membered heterocyclyl, substituted or unsubstituted 5- to 9-membered heterocyclyloxy, nitro, 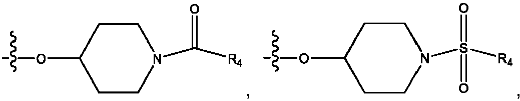 and -O-CH₂-C(O)-NH-R₅;
R₄ hydrogen; halogen; C1-C6 alkyl unsubstituted or substituted with C1-C4 alkoxy, phenoxy or phenyl, wherein the phenyl may be unsubstituted or substituted with halogen; C2-C6 alkenyl; amino; di(C1-C4 alkyl)amino; C6-C9 aryl unsubstituted or substituted with at least one selected from the group consisting of halogen, C1-C4 alkoxy, C1-C4 alkyl unsubstituted or substituted with at least one halogen, nitro, C1-C4 alkylcarbonyloxy, and -SO₂; C3-C8 cycloalkyl; or 5- to 9-membered heterocyclyl;
R₅ is at least one (one or two) selected from the group consisting of hydrogen, halogen, hydroxy, substituted or unsubstituted C1-C4 alkyl, nitro, and C6-C10 aryloxy; and
n is an integer ranging from 0 to 2, preferably an integer ranging from 0 to 1.

Preferably, the substituent of the substituted C1-C6 alkyl, substituted C2-C6 alkenyl, substituted C3-C8 cycloalkyl, substituted C1-C6 alkoxy, substituted 5- to 9-membered heteroaryl, substituted C6-C10 aryl, substituted C6-C10 aryloxy, substituted 5- to 9-membered heterocyclyl, or substituted 5- to 9-membered heterocyclyloxy of R₃ may be at least one selected from the group consisting of C1-C4 alkoxy, C6-C9 aryl and C6-C9 aryloxy, and more preferably, may be at least one selected from the group consisting of phenyl, methoxy and phenoxy.

### Advantageous Effects

The compound or pharmaceutically acceptable salt thereof according to the present invention may efficiently increase the activity of the KAI1 promoter gene by significantly increasing the activity of the KAI1 promoter, thereby exhibiting an excellent activity of inhibiting cancer metastasis and invasion or treating colorectal cancer. Thus, it is useful as an inhibitor of cancer metastasis and invasion and as a cancer therapeutic agent.

### Best Mode

Hereinafter, the present invention will be described in more detail.

The definition of each substituent used herein will be described in detail. Unless otherwise specified, each substituent has the following definition.

In the present specification, examples of "halogen" include fluoro, chloro, bromo and iodo.

As used herein, the term "alkyl" refers to a linear or branched aliphatic saturated hydrocarbon group, preferably an alkyl having 1 to 6 carbon atoms, more preferably an alkyl having 1 to 4 carbon atoms. Examples of such alkyl include methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, and 2-ethylbutyl.

As used herein, the term "heterocycle" refers to a non-aromatic ring containing, a ring constituent atom, a heteroatom selected from a nitrogen atom, a sulfur atom and an oxygen atom other than a carbon atom, and preferably includes a 5- to 12-membered non-aromatic ring containing 1 to 4 heteroatoms. Examples of this non-aromatic ring include tetrahydrothienyl, tetrahydrofuranyl, pyrrolinyl, pyrrolidinyl, imidazolinyl, imidazolidinyl, oxazolinyl, oxazolidinyl, pyrazolinyl, pyrazolidinyl, thiazolinyl, thiazolidinyl, tetrahydroisothiazolyl, tetrahydrooxazolyl, tetrahydroisoxazolyl, piperidinyl, piperazinyl, tetrahydropyridinyl, dihydropyridinyl, dihydrothiopyranyl, tetrahydropyrimidinyl, tetrahydropyridazinyl, dihydropyranyl, tetrahydropyranyl, tetrahydrothiopyranyl, morpholinyl, thiomorpholinyl, azepanyl, diazepanyl, and azepinyl.

As used herein, the term "heteroaryl" refers to an aromatic ring containing, as a ring constituent atom, a heteroatom selected from among a nitrogen atom, a sulfur atom and an oxygen atom other than a carbon atom, and preferably includes a 5- to 12-membered aromatic ring containing 1 to 4 hetero atoms. Examples of this aromatic ring include thienyl, furyl, pyrrolyl, imidazolyl, pyrazolyl, thiazolyl, isothiazolyl, oxazolyl, isoxazolyl, pyridyl, pyrazinyl, pyrimidinyl, pyridazinyl, 1,2,4-oxadiazolyl, 1,3,4-oxadiazolyl, 1,2,4-thiadiazolyl, 1,3,4-thiadiazolyl, triazolyl, tetrazolyl, indenyl, triazinyl, and dihydroisoquinolinyl.

In a preferred embodiment, in the compound of Formula 1,
X is C or N;
L is C1 to C4 alkylene or a bond;
R₁ is hydrogen, F, Cl, Br, substituted or unsubstituted C1-C4 alkyl, methoxy, N(CH₃)₂, NH(CH₃), amino, or a substituted or unsubstituted 5 to 12-membered heterocycle,
wherein the substituent of the substituted alkyl may be unsubstituted or substituted with dimethylamino, diethylamino, dipropylamino, dibutylamino, hydroxyethyl(ethyl)amino, ethyl(butyl)amino, dipentylamino, methyl(ethynyl)amino, ethyl(phenyl)amino, diallylamino, methyl(benzyl)amino, methyl(hydroxyethyl)amino, phenyl(methyl)amino, ethyl(phenyl)amino, butyl(hydroxyethyl)amino, butyl(methyl)amino, dicyclohexylamino, ethoxycarbonyl-piperazine, t-butoxycarbonyl-piperazine, fluorophenyl-piperidine, pyridinyl-piperazine, pyrimidinyl-piperazine, hydroxyethylpiperazine, methoxyphenyl-piperazine, nitrophenyl-piperazine, dimethylmorpholine, morpholine, methylpiperazine, dihydroxyisoquinoline, cyclohexyl(methyl)amino, isopropyl(methyl)amino, fluorophenyl-piperazine, or piperidinylpiperidine;
R₂ is 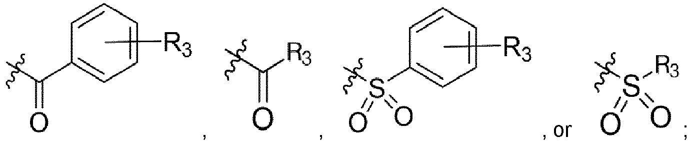
R₃ is at least one selected from the group consisting of hydrogen, F, Cl, Br, substituted or unsubstituted C1-C4 alkyl, substituted or unsubstituted C2-C4 alkenyl, substituted or unsubstituted C3-C8 cycloalkyl, substituted or unsubstituted C1-C4 alkoxy, substituted or unsubstituted 5- to 9-membered heteroaryl, substituted or unsubstituted C6-C10 aryl, substituted or unsubstituted C6-C10 aryloxy, substituted or unsubstituted 5- to 9-membered heterocyclyl, substituted or unsubstituted 5- to 9-membered heterocyclyloxy, nitro, 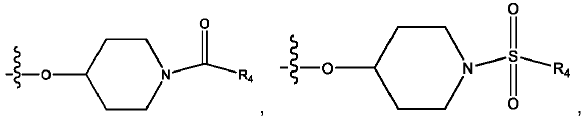 and -O-CH₂-C(O)-NH-R₅;
R₄ is hydrogen; F, Cl, Br; C1-C4 unsubstituted or substituted with methoxy, ethoxy, phenoxy or phenyl, wherein the phenyl may be unsubstituted or substituted with halogen; C2-C4 alkenyl; amino; di(C1-C4 alkyl)amino; C6-C9 aryl unsubstituted or substituted with at least one selected from the group consisting of halogen, C1-C3 alkoxy, C1-C4 alkyl unsubstituted or substituted with at least one halogen, nitro, C1-C3 alkylcarbonyloxy, and -SO₂; C3-C8 cycloalkyl; or 5- to 9-membered heterocyclyl;
R₅ is at least one (one or two) selected from the group consisting of hydrogen, halogen, hydroxy, substituted or unsubstituted C1-C4 alkyl, nitro, and C6-C10 aryloxy; and
n is an integer ranging from 0 to 2, preferably an integer ranging from 0 to 1.

Preferably, the substituent of the substituted C1-C4 alkyl, substituted C2-C4 alkenyl, substituted C3-C8 cycloalkyl, substituted C1-C4 alkoxy, substituted 5- to 9-membered heteroaryl, substituted C6-C10 aryl, substituted C6-C10 aryloxy, substituted 5- to 9-membered heterocyclyl, or substituted 5- to 9-membered heterocyclyloxy of R₃ may be at least one selected from the group consisting of C1-C4 alkoxy, C6-C9 aryl and C6-C9 aryloxy, and more preferably, may be at least one selected from the group consisting of phenyl, methoxy and phenoxy.

In a specific embodiment, examples of the compound include compounds 1 to 305 shown in Table 1 below.

**[Table 1]**

| **Comp ound No.** | **Code** | **Structure** | **MW** | **Quantity (mg)** | **DMSO (ul)** |
|---|---|---|---|---|---|
| 1 | DKC25a01 | 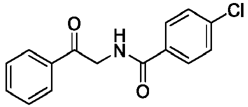 | 273.72 | 1.1mg | 402 |
| 2 | DKC25a02 | 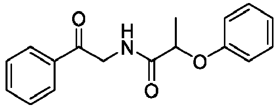 | 283.33 | 0.9mg | 318 |
| 3 | DKC25a03 | 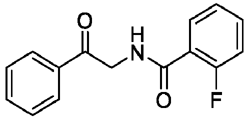 | 257.26 | 1.3mg | 505 |
| 4 | DKC25a04 | 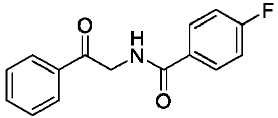 | 257.26 | 1.5mg | 583 |
| 5 | DKC25a05 | 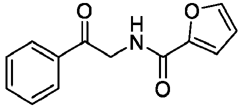 | 229.24 | 1.3mg | 567 |
| 6 | DKC25a06 | 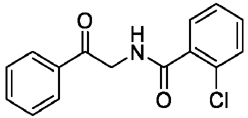 | 273.72 | 1.4mg | 511 |
| 7 | DKC25a07 | 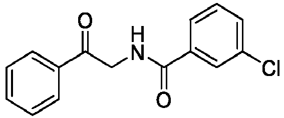 | 273.72 | 1.2mg | 438 |
| 8 | DKC25a08 | 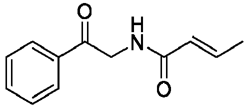 | 203.24 | 1.2mg | 590 |
| 9 | DKC25a09 | 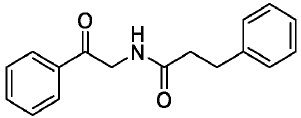 | 267.33 | 1.0mg | 374 |
| 10 | DKC25a10 | 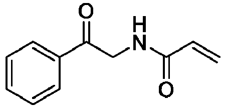 | 189.21 | 1.2mg | 634 |
| 11 | DKC25a11 | 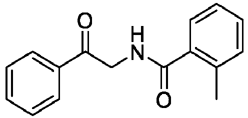 | 253.30 | 0.8mg | 316 |
| 12 | DKC25a12 | 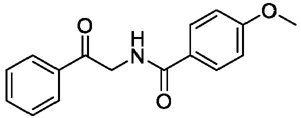 | 269.30 | 1.5mg | 557 |
| 13 | DKC25a13 | 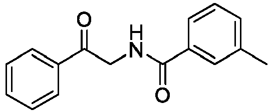 | 253.30 | 1.2mg | 474 |
| 14 | DKC25a14 | 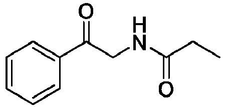 | 191.23 | 1.4mg | 732 |
| 15 | DKC25a15 | 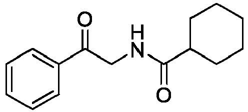 | 245.32 | 0.8mg | 326 |
| 16 | DKC25a16 | 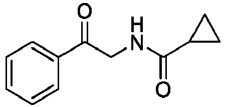 | 203.24 | 1.7mg | 836 |
| 17 | DKC25a17 | 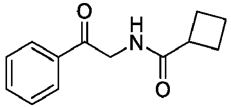 | 217.27 | 1.7mg | 782 |
| 18 | DKC25a18 | 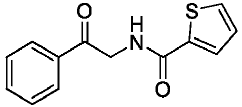 | 245.30 | 1.4mg | 571 |
| 19 | DKC25a19 | 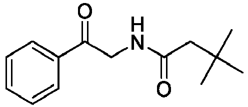 | 233.31 | 1.0mg | 429 |
| 20 | DKC25a20 | 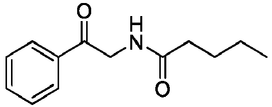 | 219.28 | 1.0mg | 456 |
| 21 | DKC25a21 | 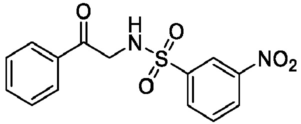 | 320.32 | 1.3mg | 406 |
| 22 | DKC25a22 | 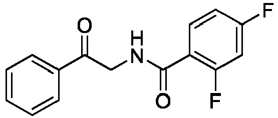 | 275.25 | 0.9mg | 327 |
| 23 | DKC25a23 | 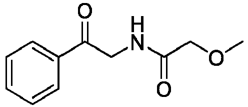 | 207.23 | 1.2mg | 579 |
| 24 | DKC25a24 | 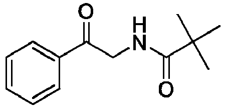 | 219.28 | 1.2mg | 547 |
| 25 | DKC25a25 | 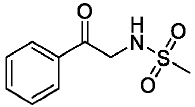 | 213.25 | 0.9mg | 422 |
| 26 | DKC25a26 | 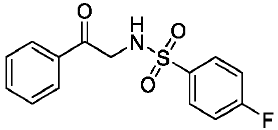 | 293.31 | 1.3mg | 443 |
| 27 | DKC25a27 | 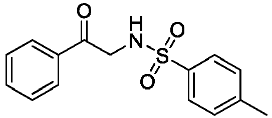 | 289.35 | 1.1mg | 380 |
| 28 | DKC25a28 | 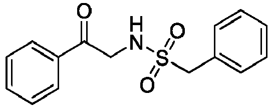 | 289.35 | 1.7mg | 588 |
| 29 | DKC25a29 | 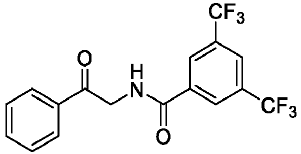 | 375.27 | 1.1mg | 293 |
| 30 | DKC25a30 | 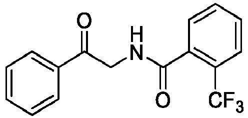 | 307.27 | 1.4mg | 456 |
| 31 | DKC25a31 | 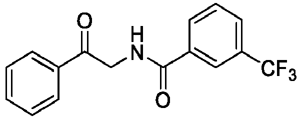 | 307.27 | 1.4mg | 456 |
| 32 | DKC25a32 | 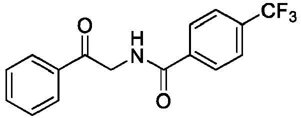 | 307.27 | 1.1mg | 358 |
| 33 | DKC25a33 | 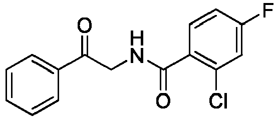 | 291.71 | 1.2mg | 411 |
| 34 | DKC25a34 | 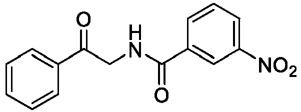 | 284.27 | 1.0mg | 352 |
| 35 | DKC25a35 | 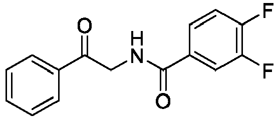 | 275.25 | 1.5mg | 545 |
| 36 | DKC25a36 | 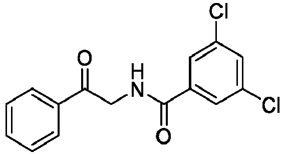 | 308.16 | 0.9mg | 292 |
| 37 | DKC25a37 | 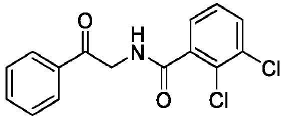 | 308.16 | 1.5mg | 487 |
| 38 | DKC25a38 | 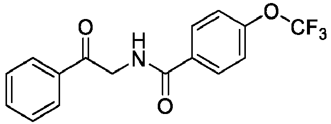 | 323.27 | 1.4mg | 433 |
| 39 | DKC25a39 | 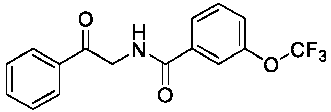 | 323.27 | 1.7mg | 526 |
| 40 | DKC25a40 | 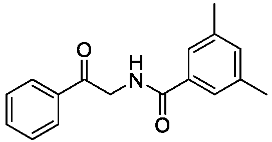 | 267.33 | 1.4mg | 524 |
| 41 | DKC25a41 | 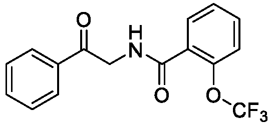 | 323.27 | 1.4mg | 433 |
| 42 | DKC25a42 | 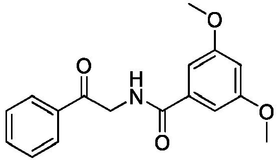 | 299.33 | 1.4mg | 468 |
| 43 | DKC25a43 | 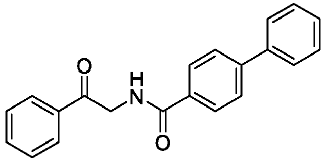 | 315.37 | 1.4mg | 444 |
| 44 | DKC25b01 | 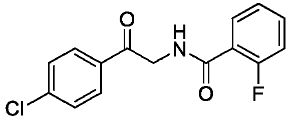 | 291.71 | 1.3mg | 446 |
| 45 | DKC25b02 | 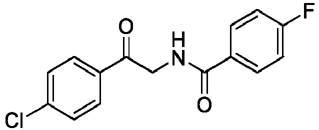 | 291.71 | 1.0mg | 343 |
| 46 | DKC25b03 | 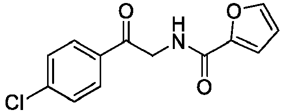 | 263.68 | 1.1mg | 417 |
| 47 | DKC25b04 | 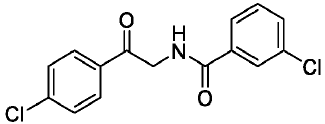 | 308.16 | 0.9mg | 292 |
| 48 | DKC25b05 | 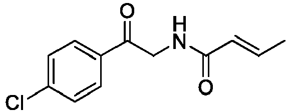 | 237.68 | 1.0mg | 421 |
| 49 | DKC25b06 | 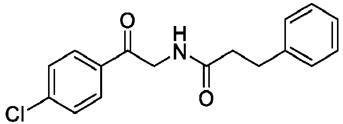 | 301.77 | 1.5mg | 497 |
| 50 | DKC25b07 | 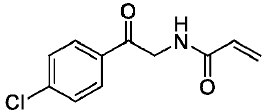 | 223.66 | 1.7mg | 760 |
| 51 | DKC25b08 | 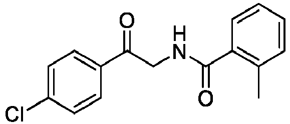 | 287.74 | 1.5mg | 521 |
| 52 | DKC25b09 | 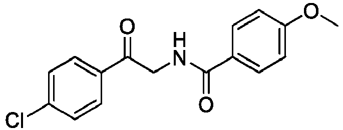 | 303.74 | 1.4mg | 461 |
| 53 | DKC25b10 | 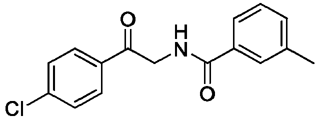 | 287.74 | 1.2mg | 417 |
| 54 | DKC25b11 | 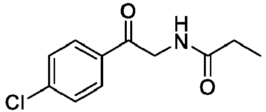 | 225.67 | 1.3mg | 576 |
| 55 | DKC25b12 | 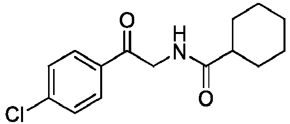 | 279.76 | 0.9mg | 322 |
| 56 | DKC25b13 | 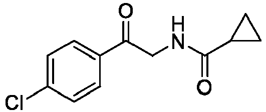 | 237.68 | 0.9mg | 379 |
| 57 | DKC25b14 | 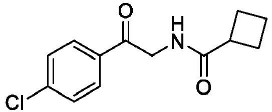 | 251.71 | 1.0mg | 397 |
| 58 | DKC25b15 | 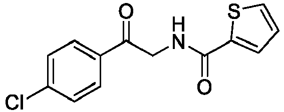 | 279.74 | 1.5mg | 536 |
| 59 | DKC25b16 | 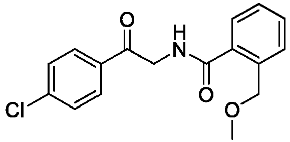 | 317.77 | 1.0mg | 315 |
| 60 | DKC25b17 | 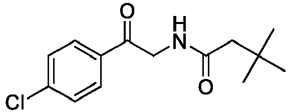 | 267.75 | 1.2mg | 448 |
| 61 | DKC25b18 | 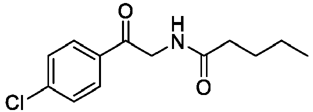 | 253.73 | 1.1mg | 434 |
| 62 | DKC25b19 | 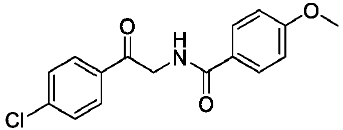 | 303.74 | 1.0mg | 329 |
| 63 | DKC25b20 | 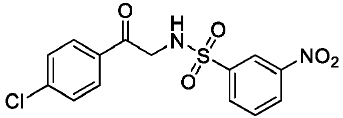 | 354.76 | 1.0mg | 282 |
| 64 | DKC25b21 | 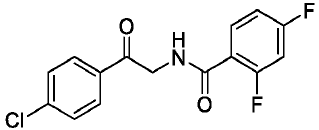 | 309.70 | 1.6mg | 517 |
| 65 | DKC25b22 | 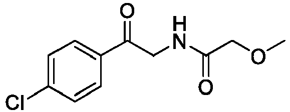 | 241.67 | 1.0mg | 414 |
| 66 | DKC25b23 | 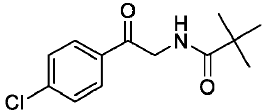 | 253.73 | 1.2mg | 473 |
| 67 | DKC25b24 | 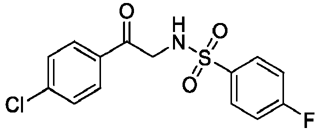 | 327.75 | 1.4mg | 427 |
| 68 | DKC25b25 | 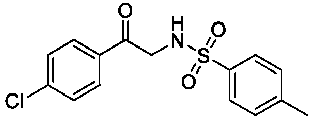 | 323.79 | 1.1mg | 340 |
| 69 | DKC25b26 | 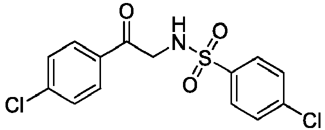 | 344.21 | 1.7mg | 494 |
| 70 | DKC25b27 | 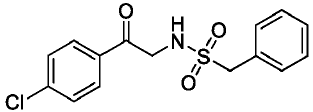 | 323.79 | 1.6mg | 494 |
| 71 | DKC25b28 | 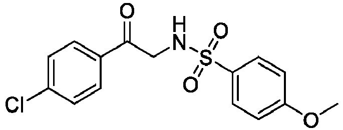 | 339.79 | 1.6mg | 471 |
| 72 | DKC25b29 | 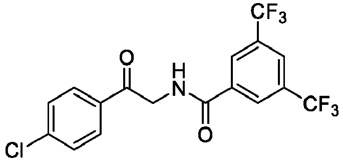 | 409.71 | 1.7mg | 415 |
| 73 | DKC25b30 | 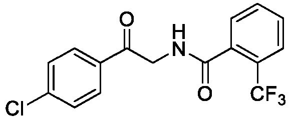 | 341.71 | 1.5mg | 439 |
| 74 | DKC25b31 | 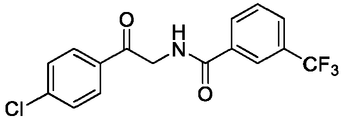 | 341.71 | 1.1mg | 322 |
| 75 | DKC25b32 | 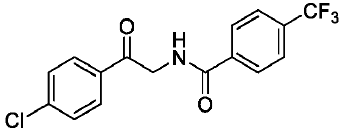 | 341.71 | 1.2mg | 351 |
| 76 | DKC25b33 | 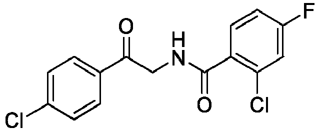 | 326.15 | 1.5mg | 460 |
| 77 | DKC25b34 | 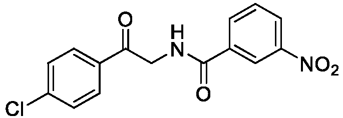 | 318.71 | 1.7mg | 533 |
| 78 | DKC25b35 | 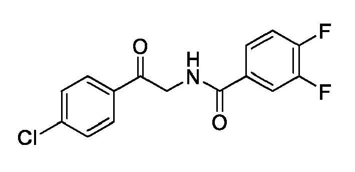 | 309.70 | 0.8mg | 258 |
| 79 | DKC25b36 | 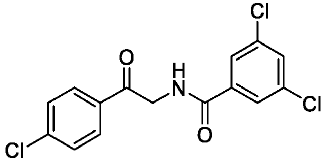 | 342.60 | 1.2mg | 350 |
| 80 | DKC25b37 | 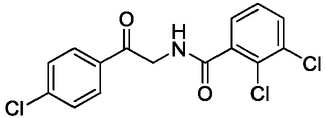 | 342.60 | 1.5mg | 438 |
| 81 | DKC25b38 | 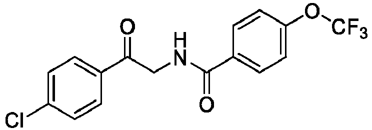 | 357.71 | 1.1mg | 308 |
| 82 | DKC25b39 | 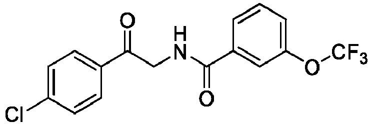 | 357.71 | 1.5mg | 419 |
| 83 | DKC25b40 | 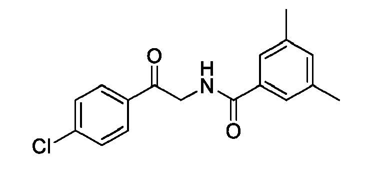 | 301.77 | 1.0mg | 331 |
| 84 | DKC25b41 | 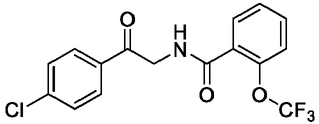 | 357.71 | 1.5mg | 419 |
| 85 | DKC25b42 | 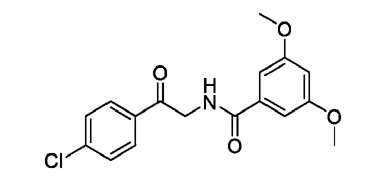 | 333.77 | 0.8mg | 240 |
| 86 | DKC25b43 | 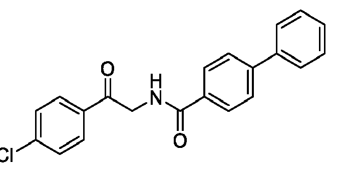 | 349.81 | 1.0mg | 286 |
| 87 | DKC25b44 | 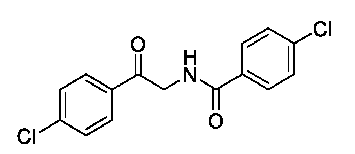 | 308.16 | 1.0mg | 325 |
| 88 | DKC25c01 | 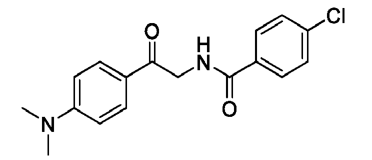 | 316.79 | 1.4mg | 442 |
| 81 | DKC25b38 | 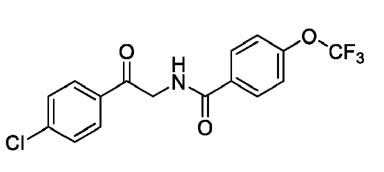 | 357.71 | 1.1mg | 308 |
| 82 | DKC25b39 | 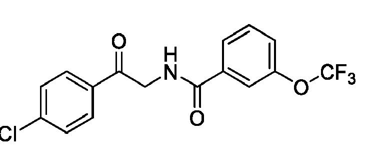 | 357.71 | 1.5mg | 419 |
| 83 | DKC25b40 | 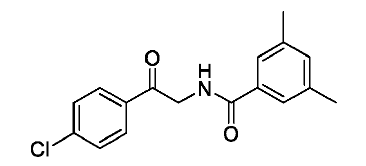 | 301.77 | 1.0mg | 331 |
| 84 | DKC25b41 | 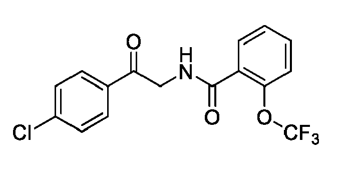 | 357.71 | 1.5mg | 419 |
| 85 | DKC25b42 | 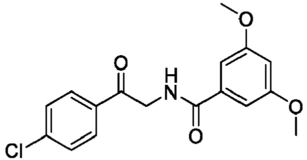 | 333.77 | 0.8mg | 240 |
| 86 | DKC25b43 | 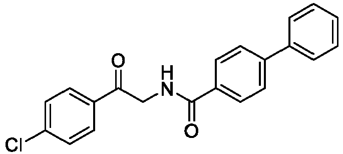 | 349.81 | 1.0mg | 286 |
| 87 | DKC25b44 | 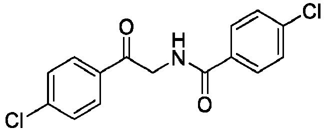 | 308.16 | 1.0mg | 325 |
| 88 | DKC25c01 | 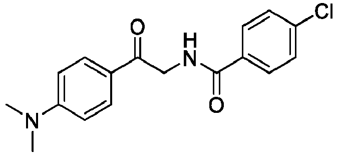 | 316.79 | 1.4mg | 442 |
| 96 | DKC25c09 | 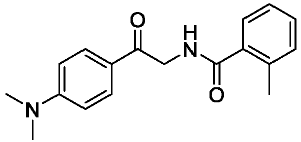 | 296.37 | 0.9mg | 304 |
| 97 | DKC25c10 | 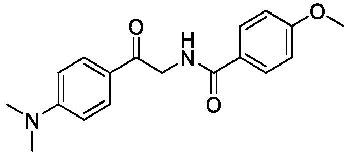 | 312.37 | 0.8mg | 256 |
| 98 | DKC25c11 | 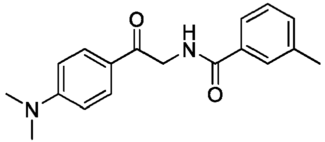 | 296.37 | 1.4mg | 472 |
| 99 | DKC25c12 | 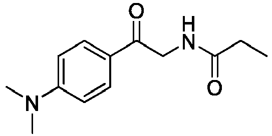 | 234.30 | 1.0mg | 427 |
| 100 | DKC25c13 | 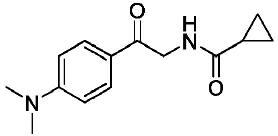 | 246.31 | 0.9mg | 365 |
| 101 | DKC25c14 | 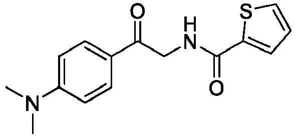 | 288.37 | 1.0mg | 347 |
| 102 | DKC25c15 | 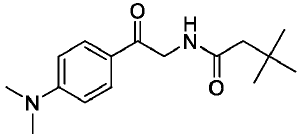 | 276.38 | 1.4mg | 507 |
| 103 | DKC25c16 | 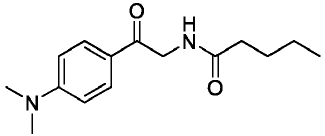 | 262.35 | 1.1mg | 419 |
| 104 | DKC25c17 | 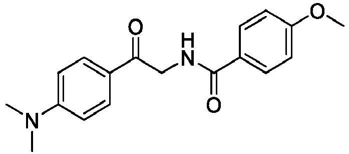 | 312.37 | 1.0mg | 320 |
| 105 | DKC25c18 | 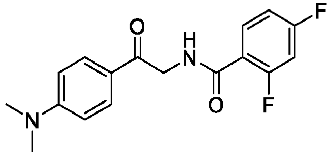 | 318.32 | 1.0mg | 314 |
| 106 | DKC25c19 | 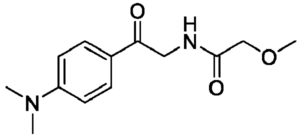 | 250.30 | 1.5mg | 599 |
| 107 | DKC25c20 | 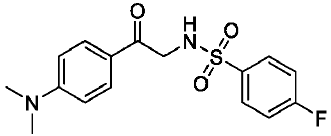 | 336.38 | 1.1mg | 327 |
| 108 | DKC25c21 | 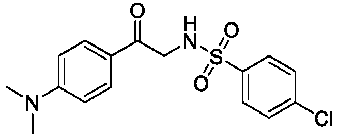 | 352.83 | 1.2mg | 340 |
| 109 | DKC25c22 | 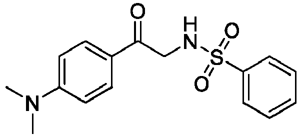 | 318.39 | 0.9mg | 283 |
| 110 | DKC25c23 | 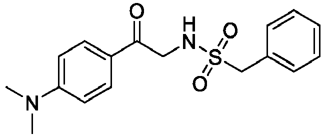 | 332.42 | 0.9mg | 271 |
| 111 | DKC25c24 | 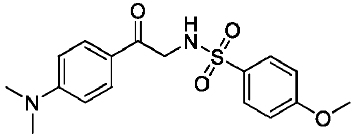 | 348.42 | 1.9mg | 545 |
| 112 | DKC25c25 | 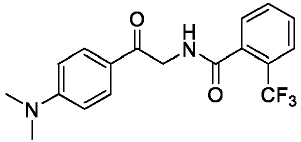 | 350.34 | 1.3mg | 371 |
| 113 | DKC25c26 | 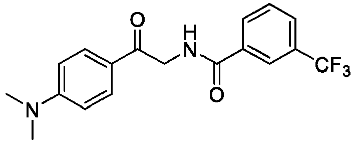 | 350.34 | 1.0mg | 285 |
| 114 | DKC25c27 | 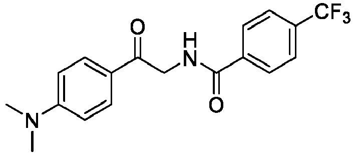 | 350.34 | 1.4mg | 400 |
| 115 | DKC25c28 | 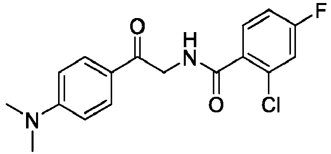 | 334.78 | 1.0mg | 299 |
| 116 | DKC25c29 | 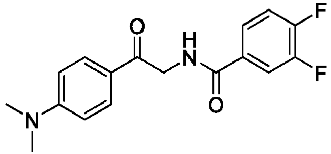 | 318.32 | 1.2mg | 377 |
| 117 | DKC25c30 | 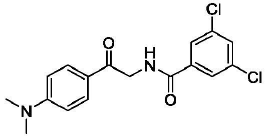 | 351.23 | 0.8mg | 228 |
| 118 | DKC25c31 | 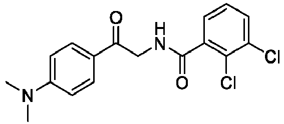 | 351.23 | 1.3mg | 370 |
| 119 | DKC25c32 | 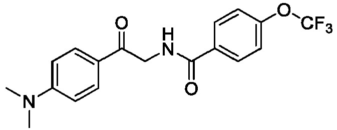 | 366.34 | 1.0mg | 273 |
| 120 | DKC25c33 | 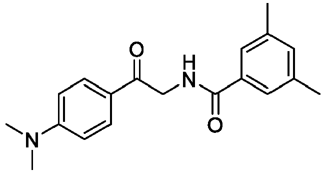 | 310.40 | 1.0mg | 322 |
| 121 | DKC25c34 | 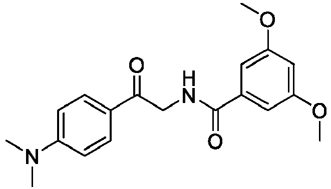 | 342.40 | 1.1mg | 321 |
| 122 | DKC25d01 | 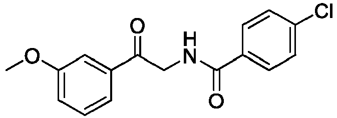 | 303.74 | 1.2mg | 395 |
| 123 | DKC25d02 | 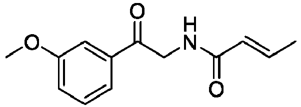 | 233.27 | 0.8mg | 343 |
| 124 | DKC25d03 | 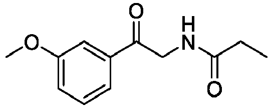 | 221.26 | 1.4mg | 633 |
| 125 | DKC25d04 | 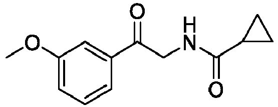 | 233.27 | 1.7mg | 729 |
| 126 | DKC25d05 | 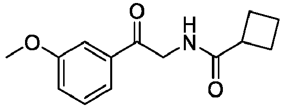 | 247.29 | 1.2mg | 485 |
| 127 | DKC25d06 | 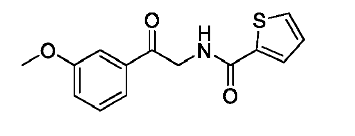 | 275.32 | 1.4mg | 508 |
| 128 | DKC25d07 | 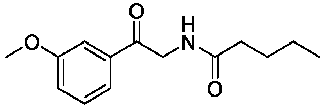 | 249.31 | 1.5mg | 602 |
| 129 | DKC25d08 | 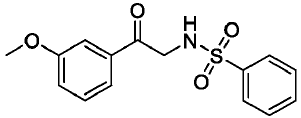 | 305.35 | 1.3mg | 426 |
| 130 | DKC25e01 | 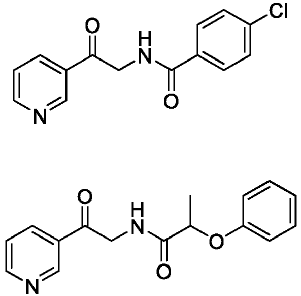 | 274.70 | 1.0mg | 364 |
| 131 | DKC25e02 | 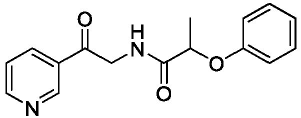 | 284.32 | 0.9mg | 317 |
| 132 | DKC25e03 | 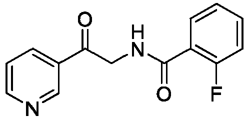 | 258.25 | 1.0mg | 387 |
| 133 | DKC25e04 | 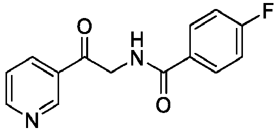 | 258.25 | 0.8mg | 310 |
| 134 | DKC25e05 | 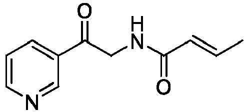 | 204.23 | 1.1mg | 539 |
| 135 | DKC25e06 | 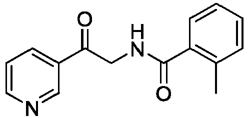 | 254.29 | 1.1mg | 433 |
| 136 | DKC25e07 | 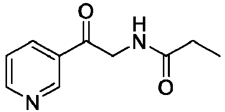 | 192.22 | 1.0mg | 520 |
| 137 | DKC25f01 | 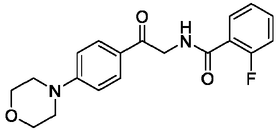 | 342.37 | 1.3mg | 380 |
| 138 | DKC25f02 | 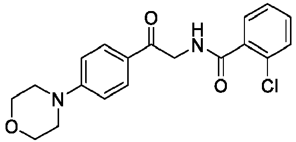 | 358.82 | 1.4mg | 390 |
| 139 | DKC25f03 | 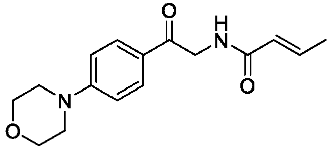 | 288.35 | 0.9mg | 312 |
| 140 | DKC25f04 | 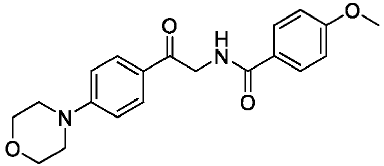 | 354.41 | 0.7mg | 198 |
| 141 | DKC25f05 | 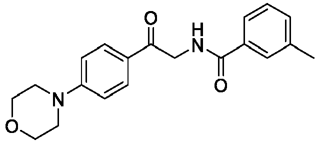 | 338.41 | 1.0mg | 295 |
| 142 | DKC25f06 | 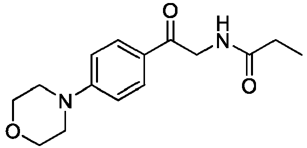 | 376.34 | 0.5mg | 133 |
| 143 | DKC25f07 | 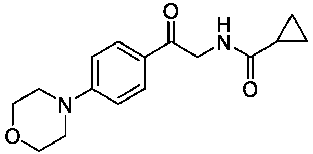 | 288.35 | 0.8mg | 277 |
| 144 | DKC25f08 | 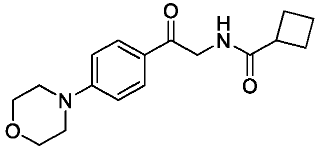 | 302.37 | 0.9mg | 298 |
| 145 | DKC25f09 | 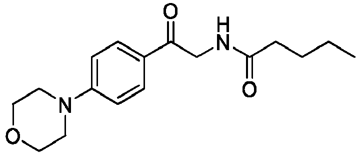 | 304.39 | 0.8mg | 263 |
| 146 | DKC25f10 | 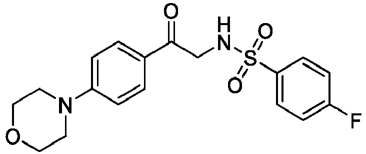 | 378.42 | 0.9mg | 238 |
| 147 | DKC25f11 | 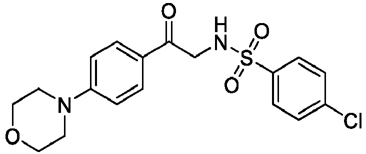 | 394.87 | 1.0mg | 253 |
| 148 | DKC25f12 | 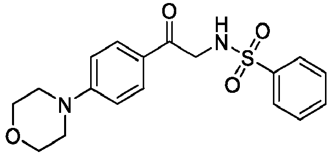 | 360.43 | 0.8mg | 222 |
| 149 | DKC25f13 | 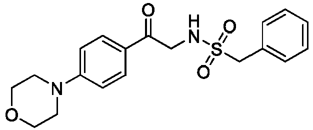 | 374.46 | 1.5mg | 401 |
| 150 | DKC25f14 | 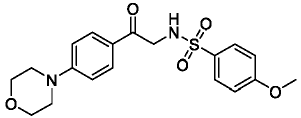 | 390.45 | 1.1mg | 282 |
| 151 | DKC25g01 | 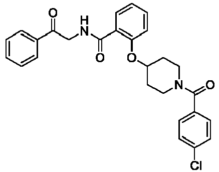 | 476.15 | 1.4mg | 294 |
| 152 | DKC25g02 | 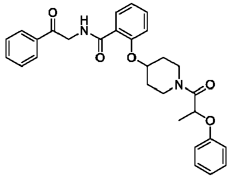 | 486.22 | 1.1mg | 226 |
| 153 | DKC25g03 | 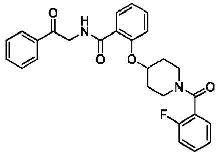 | 460.18 | 1.2mg | 261 |
| 154 | DKC25g04 | 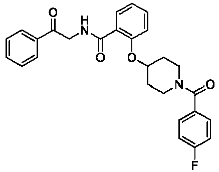 | 460.18 | 1.4mg | 304 |
| 155 | DKC25g05 | 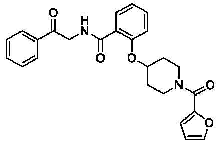 | 432.17 | 0.9mg | 208 |
| 156 | DKC25g06 | 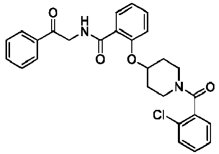 | 476.15 | 0.9mg | 189 |
| 157 | DKC25g07 | 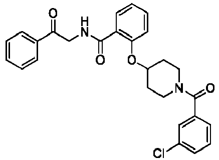 | 476.15 | 1.0mg | 210 |
| 158 | DKC25g08 | 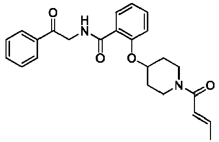 | 406.19 | 1.4mg | 345 |
| 159 | DKC25g09 | 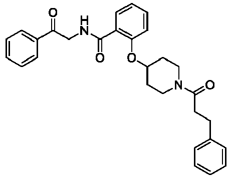 | 470.10 | 0.9mg | 191 |
| 160 | DKC25g10 | 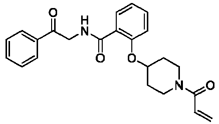 | 392.00 | 1.0mg | 255 |
| 161 | DKC25g11 | 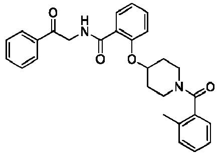 | 456.20 | 0.9mg | 197 |
| 162 | DKC25g12 | 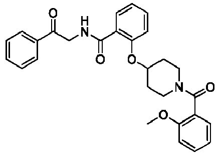 | 472.20 | 1.0mg | 212 |
| 163 | DKC25g13 | 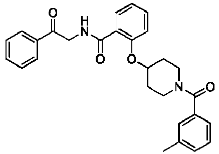 | 456.20 | 1.0mg | 219 |
| 164 | DKC25g14 | 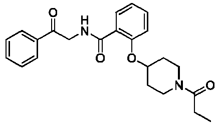 | 394.00 | 1.1mg | 279 |
| 165 | DKC25g15 | 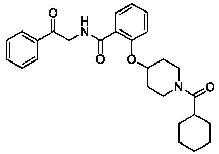 | 448.24 | 0.9mg | 201 |
| 166 | DKC25g16 | 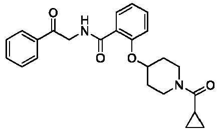 | 406.19 | 1.4mg | 345 |
| 167 | DKC25g17 | 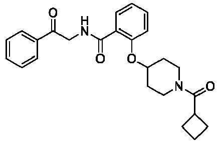 | 420.20 | 1.2mg | 286 |
| 168 | DKC25g18 | 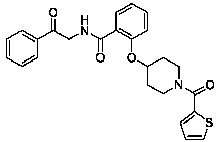 | 448.15 | 1.3mg | 290 |
| 169 | DKC25g19 | 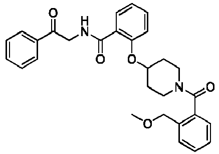 | 486.20 | 1.4mg | 288 |
| 170 | DKC25g20 | 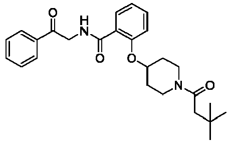 | 436.24 | 1.2mg | 275 |
| 171 | DKC25g21 | 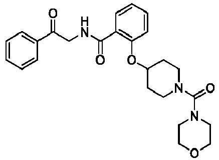 | 451.21 | 1.0mg | 222 |
| 172 | DKC25g22 | 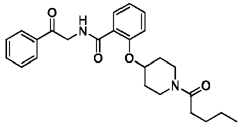 | 522.22 | 1.2mg | 230 |
| 173 | DKC25g23 | 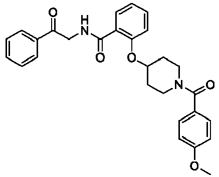 | 572.20 | 1.4mg | 245 |
| 174 | DKC25g24 | 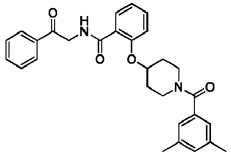 | 470.22 | 1.4mg | 298 |
| 175 | DKC25g25 | 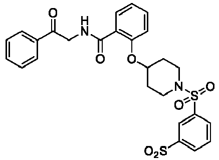 | 541.11 | 1.1mg | 203 |
| 176 | DKC25g26 | 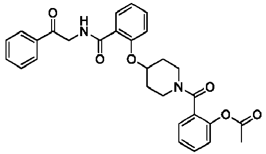 | 519.00 | 1.2mg | 231 |
| 177 | DKC25g27 | 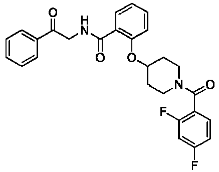 | 478.00 | 0.9mg | 188 |
| 178 | DKC25g28 | 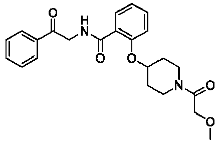 | 410.00 | 1.1mg | 268 |
| 179 | DKC25g29 | 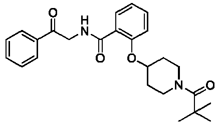 | 422.22 | 1.3mg | 308 |
| 180 | DKC25g30 | 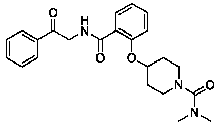 | 409.20 | 0.9mg | 220 |
| 181 | DKC25g31 | 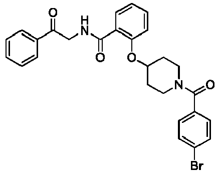 | 520.10 | 0.9mg | 173 |
| 182 | DKC25g32 | 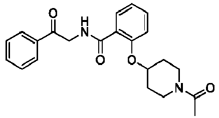 | 380.17 | 1.0mg | 263 |
| 183 | DKC25g33 | 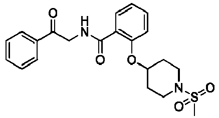 | 416.14 | 1.4mg | 336 |
| 184 | DKC25g34 | 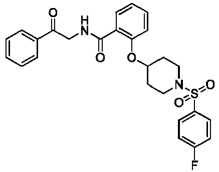 | 496.15 | 1.2mg | 242 |
| 185 | DKC25g35 | 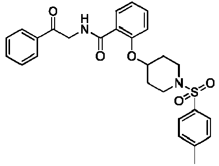 | 492.17 | 1.1mg | 224 |
| 186 | DKC25g36 | 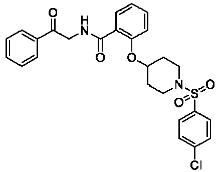 | 512.12 | 1.2mg | 234 |
| 187 | DKC25g37 | 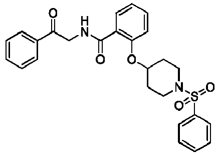 | 478.16 | 1.2mg | 251 |
| 188 | DKC25g38 | 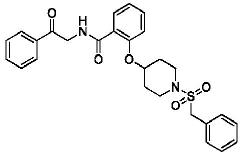 | 492.17 | 0.8mg | 163 |
| 189 | DKC25g39 | 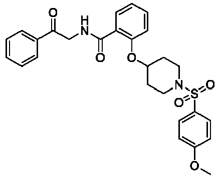 | 408.17 | 1.4mg | 343 |
| 190 | DKC25g40 | 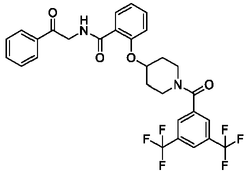 | 578.16 | 1.4mg | 242 |
| 191 | DKC25g41 | 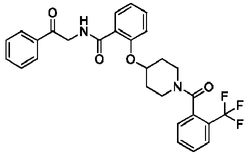 | 510.18 | 1.4mg | 274 |
| 192 | DKC25g42 | 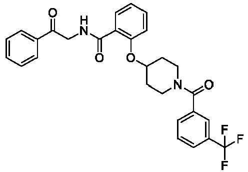 | 510.18 | 1.1mg | 216 |
| 193 | DKC25g43 | 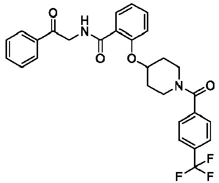 | 510.18 | 1.4mg | 274 |
| 194 | DKC25g44 | 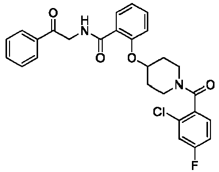 | 494.14 | 1.0mg | 202 |
| 195 | DKC25g45 | 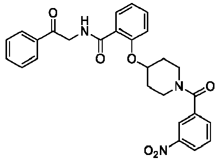 | 487.17 | 1.4mg | 287 |
| 196 | DKC25g46 | 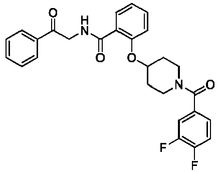 | 478.17 | 1.4mg | 293 |
| 197 | DKC25g47 | 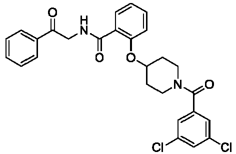 | 510.11 | 1.3mg | 255 |
| 198 | DKC25g48 | 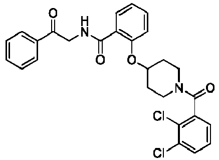 | 510.11 | 1.3mg | 255 |
| 199 | DKC25g49 | 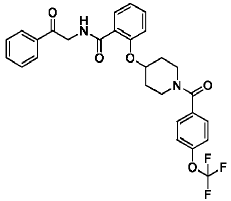 | 526.17 | 1.4mg | 266 |
| 200 | DKC25g50 | 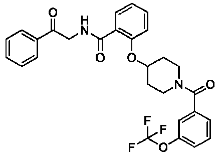 | 526.17 | 1.4mg | 266 |
| 201 | DKC25h01 | 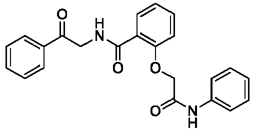 | 388.14 | 1.2mg | 309 |
| 202 | DKC25h02 | 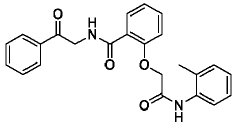 | 402.16 | 1.4mg | 348 |
| 203 | DKC25h03 | 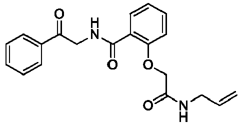 | 352.14 | 0.9mg | 256 |
| 204 | DKC25h04 | 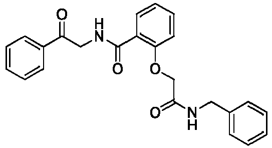 | 402.16 | 1.0mg | 249 |
| 205 | DKC25h05 | 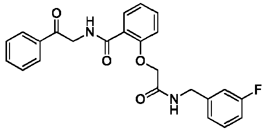 | 420.15 | 0.8mg | 190 |
| 206 | DKC25h06 | 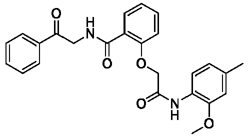 | 432.17 | 0.8mg | 185 |
| 207 | DKC25h07 | 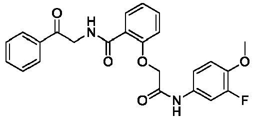 | 436.14 | 1.4mg | 321 |
| 208 | DKC25h08 | 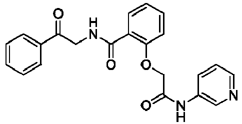 | 389.14 | 0.8mg | 206 |
| 209 | DKC25h09 | 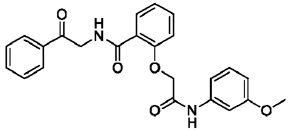 | 418.15 | 1.4mg | 335 |
| 210 | DKC25h10 | 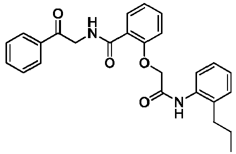 | 430.19 | 1.4mg | 325 |
| 211 | DKC25h11 | 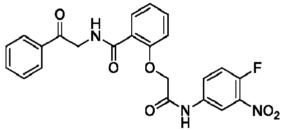 | 451.12 | 1.4mg | 310 |
| 212 | DKC25h12 | 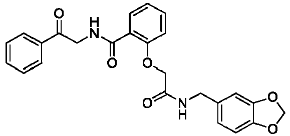 | 446.15 | 1.4mg | 314 |
| 213 | DKC25h13 | 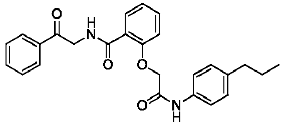 | 430.19 | 1.4mg | 325 |
| 214 | DKC25h14 | 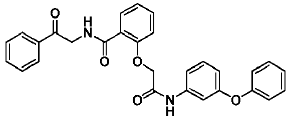 | 480.17 | 1.4mg | 292 |
| 215 | DKC25h15 | 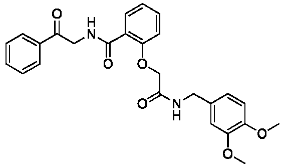 | 462.16 | 1.4mg | 303 |
| 216 | DKC25h16 | 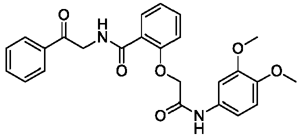 | 448.16 | 1.5mg | 335 |
| 217 | DKC25h17 | 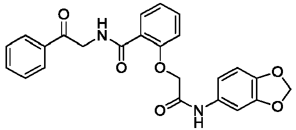 | 432.43 | 1.4mg | 324 |
| 218 | DKC25h18 | 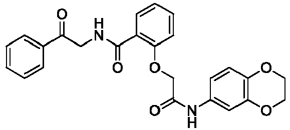 | 446.15 | 1.4mg | 314 |
| 219 | DKC25h19 | 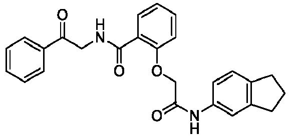 | 428.17 | 0.9mg | 210 |
| 220 | DKC25h20 | 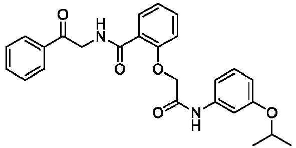 | 446.18 | 1.0mg | 224 |
| 221 | DKC25h21 | 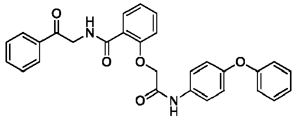 | 480.17 | 1.3mg | 271 |
| 222 | DKC25h22 | 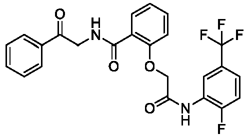 | 474.12 | 0.8mg | 169 |
| 223 | DKC25h23 | 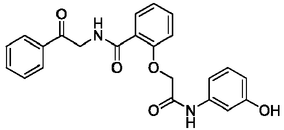 | 404.14 | 0.9mg | 223 |
| 224 | DKC25h24 | 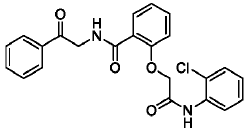 | 422.10 | 1.2mg | 284 |
| 225 | DKC25h25 | 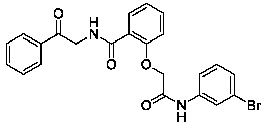 | 466.05 | 0.9mg | 193 |
| 226 | DKC25h26 | 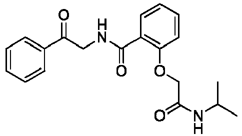 | 354.16 | 1.0mg | 282 |
| 227 | DKC25h27 | 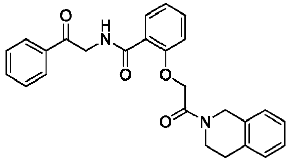 | 428.17 | 1.4mg | 327 |
| 228 | DKC25h28 | 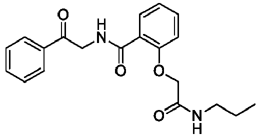 | 354.16 | 1.0mg | 282 |
| 229 | DKC25h29 | 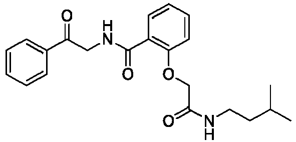 | 382.19 | 0.8mg | 209 |
| 230 | DKC25h30 | 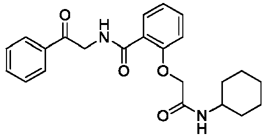 | 394.19 | 1.4mg | 355 |
| 231 | DKC25i01 | 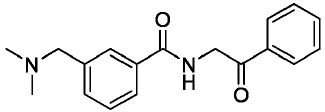 | 296.37 | 1.9mg | 641 |
| 232 | DKC25i02 | 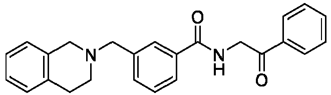 | 384.48 | 2.4mg | 624 |
| 233 | DKC25i03 | 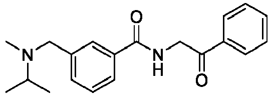 | 324.42 | 1.6mg | 493 |
| 234 | DKC25i04 | 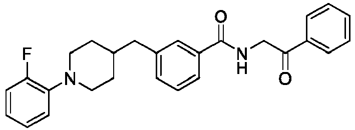 | 430.52 | 1.4mg | 325 |
| 235 | DKC25i05 | 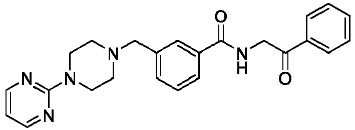 | 415.50 | 1.8mg | 433 |
| 236 | DKC25i06 | 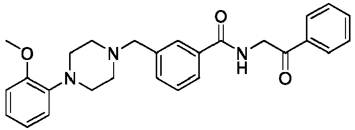 | 443.55 | 2.5mg | 564 |
| 237 | DKC25i07 | 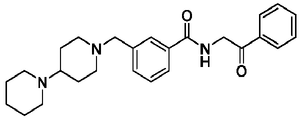 | 419.57 | 2.0mg | 477 |
| 238 | DKC25i08 | 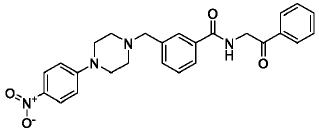 | 458.52 | 1.1mg | 240 |
| 239 | DKC25i09 | 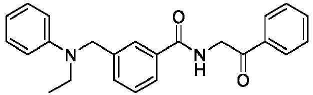 | 372.47 | 2.2mg | 591 |
| 240 | DKC25i10 | 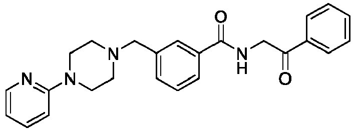 | 414.51 | 1.2mg | 289 |
| 241 | DKC25i11 | 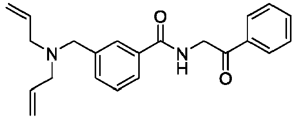 | 348.45 | 1.8mg | 517 |
| 242 | DKC25i12 | 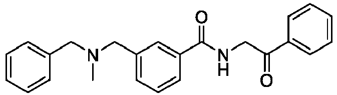 | 372.47 | 1.9mg | 510 |
| 243 | DKC25i13 | 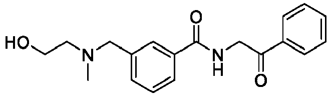 | 326.40 | 1.7mg | 521 |
| 244 | DKC25i14 | 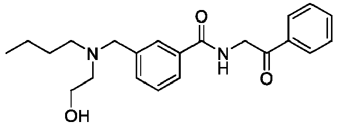 | 368.48 | 2.0mg | 543 |
| 245 | DKC25i15 | 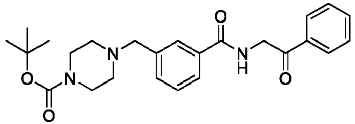 | 437.54 | 2.3mg | 526 |
| 246 | DKC25i16 | 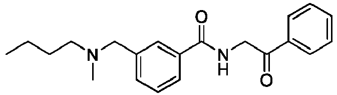 | 338.45 | 1.3mg | 384 |
| 247 | DKC25i17 | 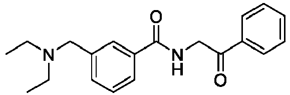 | 324.42 | 1.7mg | 524 |
| 248 | DKC25i18 | 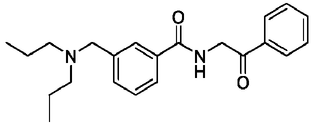 | 352.48 | 1.5mg | 426 |
| 249 | DKC25i19 | 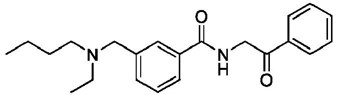 | 352.48 | 1.0mg | 284 |
| 250 | DKC25i20 | 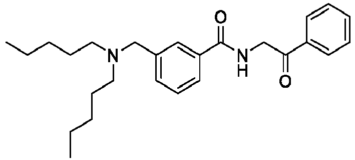 | 408.59 | 1.6mg | 392 |
| 251 | DKC25i21 | 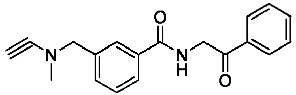 | 306.37 | 1.1mg | 359 |
| 252 | DKC25i22 | 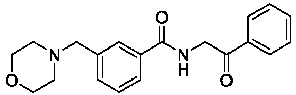 | 338.41 | 1.6mg | 473 |
| 253 | DKC25i23 | 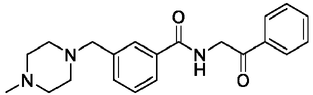 | 351.45 | 1.7mg | 484 |
| 254 | DKC25i24 | 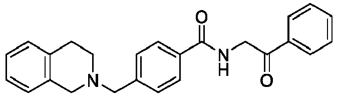 | 384.48 | 1.3mg | 338 |
| 255 | DKC25i25 | 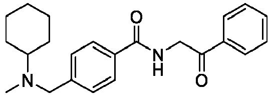 | 364.49 | 1.9mg | 521 |
| 256 | DKC25i26 | 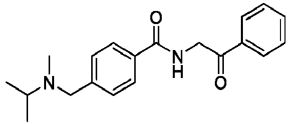 | 324.42 | 2.3mg | 709 |
| 257 | DKC25i27 | 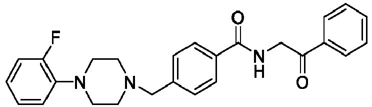 | 431.51 | 2.2mg | 510 |
| 258 | DKC25i28 | 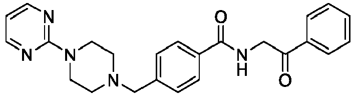 | 415.50 | 1.9mg | 457 |
| 259 | DKC25i29 | 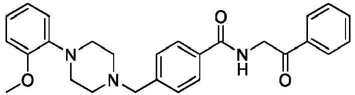 | 443.55 | 1.8mg | 406 |
| 260 | DKC25i30 | 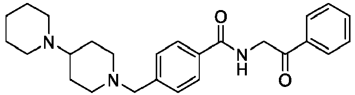 | 419.57 | 1.4mg | 334 |
| 261 | DKC25i31 | 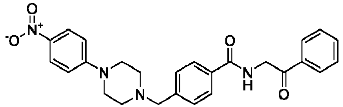 | 458.52 | 2.5mg | 545 |
| 262 | DKC25i32 | 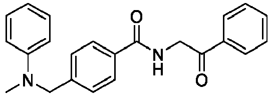 | 358.44 | 1.9mg | 530 |
| 263 | DKC25i33 | 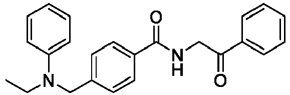 | 372.47 | 1.9mg | 510 |
| 264 | DKC25i34 | 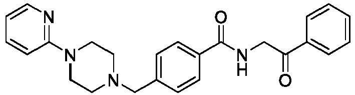 | 414.51 | 1.6mg | 386 |
| 265 | DKC25i35 | 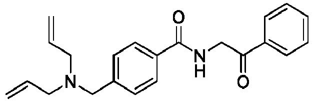 | 348.45 | 1.8mg | 517 |
| 266 | DKC25i36 | 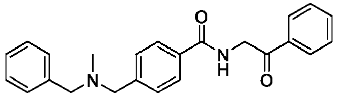 | 372.47 | 1.4mg | 376 |
| 267 | DKC25i37 | 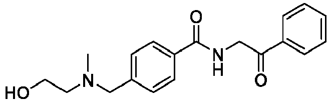 | 326.40 | 2.1mg | 643 |
| 268 | DKC25i38 | 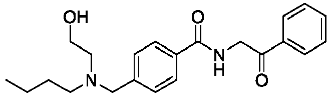 | 368.48 | 2.1mg | 570 |
| 269 | DKC25i39 | 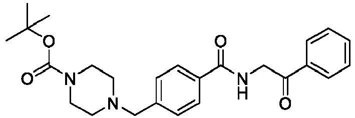 | 437.54 | 1.8mg | 411 |
| 270 | DKC25i40 | 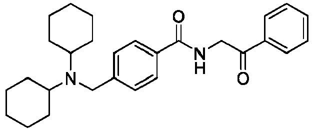 | 432.61 | 1.1mg | 254 |
| 271 | DKC25i41 | 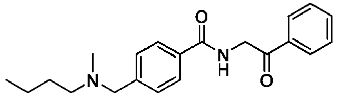 | 338.45 | 1.0mg | 295 |
| 272 | DKC25i42 | 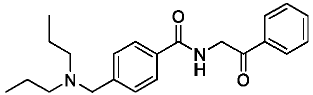 | 352.48 | 2.3mg | 653 |
| 273 | DKC25i43 | 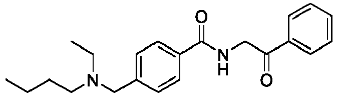 | 352.48 | 2.4mg | 681 |
| 274 | DKC25i44 | 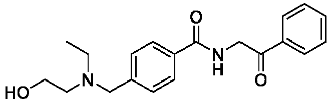 | 340.42 | 1.4mg | 411 |
| 275 | DKC25i45 | 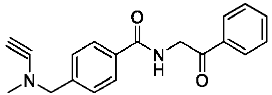 | 306.37 | 1.0mg | 326 |
| 276 | DKC25i46 | 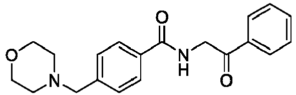 | 338.41 | 2.2mg | 650 |
| 277 | DKC25i47 | 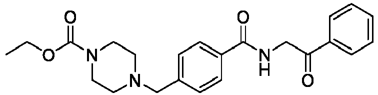 | 409.49 | 2.0mg | 488 |
| 278 | DKC25i48 | 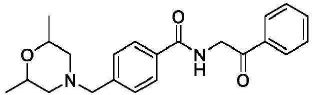 | 366.46 | 1.8mg | 491 |
| 279 | DKC25i49 | 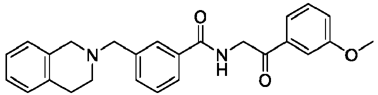 | 414.51 | 1.3mg | 314 |
| 280 | DKC25i50 | 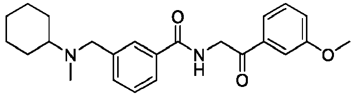 | 394.52 | 2.0mg | 507 |
| 281 | DKC25i51 | 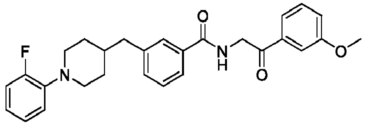 | 460.55 | 1.1mg | 239 |
| 282 | DKC25i52 | 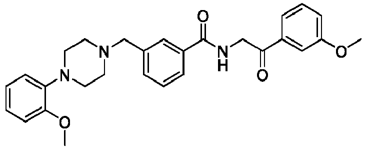 | 473.57 | 2.2mg | 465 |
| 283 | DKC25i53 | 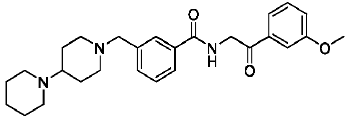 | 449.60 | 1.5mg | 334 |
| 284 | DKC25i54 | 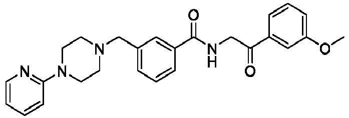 | 444.54 | 2.5mg | 562 |
| 285 | DKC25i55 | 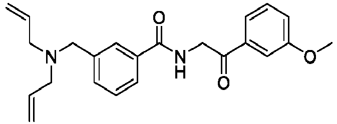 | 378.47 | 1.6mg | 423 |
| 286 | DKC25i56 | 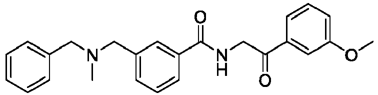 | 402.49 | 1.1mg | 273 |
| 287 | DKC25i57 | 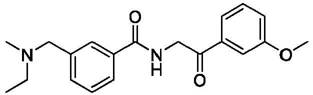 | 340.42 | 1.7mg | 499 |
| 288 | DKC25i58 | 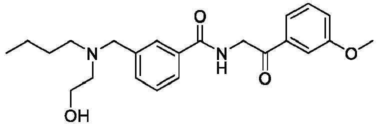 | 398.50 | 2.3mg | 577 |
| 289 | DKC25i59 | 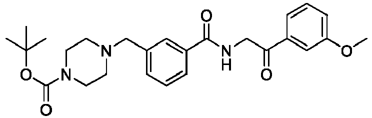 | 467.57 | 1.4mg | 299 |
| 290 | DKC25i60 | 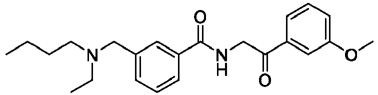 | 382.50 | 2.3mg | 601 |
| 291 | DKC25i61 | 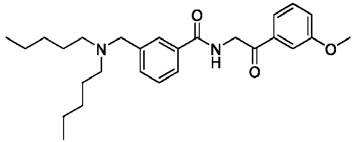 | 370.45 | 1.7mg | 459 |
| 292 | DKC25i62 | 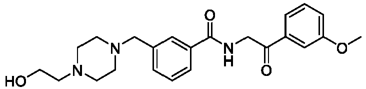 | 438.61 | 1.3mg | 296 |
| 293 | DKC25i63 | 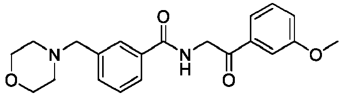 | 368.43 | 1.8mg | 489 |
| 294 | DKC25i64 | 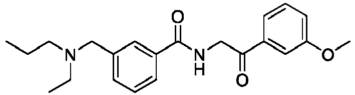 | 368.48 | 1.8mg | 488 |
| 295 | DKC25i65 | 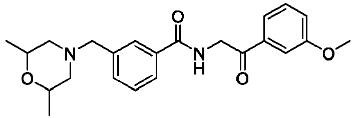 | 396.49 | 1.2mg | 303 |
| 296 | DKC25i66 | 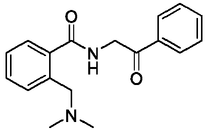 | 296.37 | 1.7mg | 574 |
| 297 | DKC25i67 | 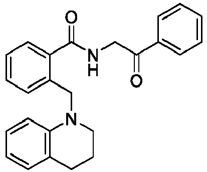 | 384.48 | 2.2mg | 572 |
| 298 | DKC25i68 | 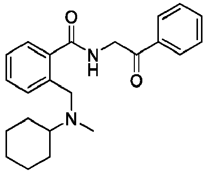 | 364.49 | 1.7mg | 466 |
| 299 | DKC25i69 | 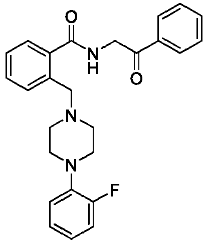 | 431.51 | 1.3mg | 301 |
| 300 | DKC25i70 | 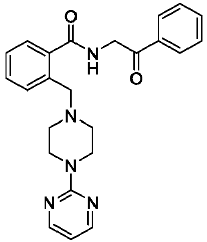 | 415.50 | 2.5mg | 602 |
| 301 | DKC25i71 | 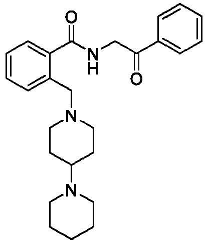 | 419.51 | 2.2mg | 524 |
| 302 | DKC25i72 | 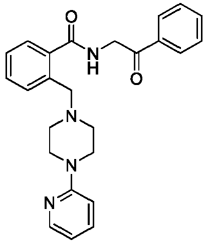 | 414.15 | 2.5mg | 604 |
| 303 | DKC25i73 | 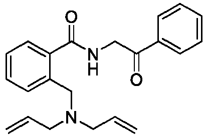 | 348.45 | 1.2mg | 344 |
| 304 | DKC25i74 | 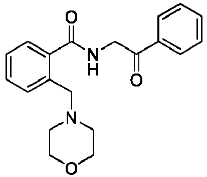 | 338.31 | 1.8mg | 532 |
| 305 | DKC25i75 | 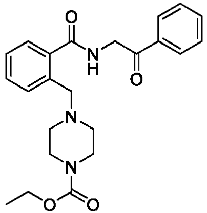 | 409.49 | 1.7mg | 415 |

Meanwhile, the compound of the present invention may exist in the form of a pharmaceutically acceptable salt. As the salt, an acid addition salt formed by a pharmaceutically acceptable free acid is useful. As used herein, the term "pharmaceutically acceptable salt" refers to any organic or inorganic addition salt of each of the compounds represented by Formulas 1 to 3 whose concentration has effective action because it is relatively non-toxic and harmless to a patient and whose side effects do not degrade the beneficial efficacy of the above compounds.

The acid addition salt is prepared by a conventional method, for example, by dissolving the compound in an excess of aqueous acid solution and precipitating the salt using a water-miscible organic solvent such as methanol, ethanol, acetone or acetonitrile. The acid addition salt may also be prepared by heating an equimolar amount of the compound and acid or alcohol (e.g., glycol monomethylether) in water, and then drying the mixture by evaporation or suction-filtering the precipitated salt.

As the free acid, an orgnic acid or inorganic acid may be used. Examples of the inorganic acid include, but are not limited to, hydrochloric acid, phosphoric acid, sulfuric acid, nitric acid, and stannic acid, and examples of the organic acid include, but are not limited to, methanesulfonic acid, p-toluenesulfonic acid, acetic acid, trifluoroacetic acid, maleic acid, succinic acid, oxalic acid, benzoic acid, tartaric acid, fumaric acid, mandelic acid, propionic acid, citric acid, lactic acid, glycolic acid, gluconic acid, galacturonic acid, glutamic acid, glutaric acid, glucuronic acid, aspartic acid, ascorbic acid, carbonic acid, vanillic acid, hydroiodic acid, etc.

In addition, a pharmaceutically acceptable metal salt may be prepared using a base. An alkali metal salt or an alkali earth metal salt is obtained, for example, by dissolving a compound in an excess amount of a solution of an alkali metal hydroxide or an alkali earth metal salt hydroxide, filtering undissolved compound salts, and drying the filtrate by evaporation. In particular, preparing a sodium salt, potassium salt or calcium salt as the salt is suitable from the pharmaceutical point of view, but is not limited thereto. Furthermore, the silver salt corresponding thereto may be obtained by reacting an alkali metal or alkali earth metal salt with an appropriate silver salt (e.g., silver nitrate).

The pharmaceutically acceptable salt of the compound of the present invention may include a salt of the acidic or basic group that can be present in the compound of Formula 1, unless indicated otherwise. Examples of the pharmaceutically acceptable salt includes sodium, potassium and calcium salts of a hydroxyl group, and other pharmaceutically acceptable salts of an amino group include hydrobromide, sulfate, hydrogen sulfate, phosphate, hydrogen phosphate, dihydrogen phosphate, acetate, succinate, citrate, tartrate, lactate, mandelate, methanesulfonate (mesylate), and p-toluenesulfonate (tosylate) salts. These may be prepared through the salt preparation methods known in the related art.

The salt of the compound of Formula 1 of the present invention is a pharmaceutically acceptable salt which exhibits pharmacological activity equivalent to that of the compound of Formula 1. For example, any salt of Formula 1 that delays death caused by metastasis of colorectal cancer cells or treats colorectal cancer by inhibiting the migration and invasion of colorectal cancer cells may be used without limitation.

In addition, the compounds represented by Formula 1 according to the present invention include, without limitation, pharmaceutically acceptable salts thereof, as well as possible solvates such as hydrates and all possible stereoisomers that can be prepared therefrom. Solvates and stereoisomers of the compound represented by Formula 1 may be prepared from the compound represented by Formula 1 using methods known in the art.

Furthermore, the compound represented by Formula 1 according to the present invention may be prepared in a crystalline form or an amorphous form, and when it is prepared in a crystalline form, it may be optionally hydrated or solvated. The present invention may encompass compounds containing various amounts of water as well as stoichiometric hydrates of the compound represented by Formula 1. Solvates of the compound represented by Formula 1 according to the present invention include both stoichiometric solvates and non-stoichiometric solvates.

The compound of Formula 1 according to the present invention may be prepared by the exemplary method to be described later, specific examples of which are shown in the reaction schemes described in the Examples below.

In the preparation method according to the present invention, as reactants used in the above reaction schemes, commercially available compounds may be purchased and used as they are, or compounds synthesized by carrying out one or more reactions known in the art or by performing an appropriate modification of the reactions may be used. For example, the reactants may be synthesized by performing one or more reactions in any order in consideration of the presence, type and/or position of a reactive functional group and/or hetero element included in the skeleton structure, without being limited thereto.

In another aspect, the present invention provides a composition for inhibiting cancer metastasis and invasion containing the compound of the present invention as an active ingredient. The present invention also provides a composition for treating cancer, preferably colorectal cancer, laryngeal cancer, lung cancer, gastric cancer, hepatocellular cancer, prostate cancer, or breast cancer, containing the compound and pharmaceutically acceptable salt thereof according to the present invention as an active ingredient. Preferably, the composition may be a pharmaceutical composition.

In specific examples of the present invention, specific compounds represented by Formula 1 were newly synthesized, and it was confirmed that these compounds had the effect of increasing KAI1 promoter gene expression and inhibiting cancer invasion.

As used herein, the term "treatment" or "treating" refers to any action of alleviating or beneficially changing colorectal cancer symptoms by administration of the pharmaceutical composition of the present invention.

As described above, the compound of the present invention not only increases the expression of the KAI1 promoter gene, but also exhibits an effect of inhibiting cancer metastasis and invasion. Thus, a pharmaceutical composition containing the compound as an active ingredient may be used for the inhibition of cancer metastasis and invasion and the treatment of colorectal cancer.

For example, the composition of the present invention may further contain a pharmaceutically acceptable carrier, diluent or excipient, and may be formulated in various forms such as oral dosage forms, for example, powders, granules, tablets, capsules, suspensions, emulsions, syrups and aerosols, as well as injections such as sterile injectable solutions, according to conventional methods depending on the intended use, and may be administered by various routes, including oral, intravenous, intraperitoneal, subcutaneous, rectal, and topical routes. Examples of a suitable carrier, excipient or diluent that may be contained in the composition include lactose, dextrose, sucrose, sorbitol, mannitol, xylitol, erythritol, maltitol, starch, acacia gum, alginate, gelatin, calcium phosphate, calcium silicate, cellulose, methylcellulose, microcrystalline cellulose, polyvinylpyrrolidone, water, methylhydroxybenzoate, propylhydroxybenzoate, talc, magnesium stearate, and mineral oil. In addition, the composition of the present invention may further contain a filler, an anti-agglomeration agent, a lubricant, a wetting agent, a fragrance, an emulsifier, a preservative, and the like.

Solid formulations for oral administration include tablets, pills, powders, granules, capsules, and the like, and such solid formulations are prepared by mixing the compound with one or more excipients, for example, starch, calcium carbonate, sucrose, lactose, gelatin, etc. In addition to simple excipients, lubricants such as magnesium stearate and talc may also be used.

Liquid formulations for oral administration include, for example, suspensions, solutions, emulsions, syrups, etc., and these formulations may contain various excipients, for example, a wetting agent, a sweetener, a fragrance and a preservative, in addition to commonly used simple diluents such as water and liquid paraffin.

Formulations for parenteral administration include sterile aqueous solutions, non-aqueous solutions, suspensions, emulsions, lyophilized formulations, and suppositories. Non-aqueous solvents and suspending agents include propylene glycol, polyethylene glycol, vegetable oils such as olive oil, and injectable esters such as ethyl oleate. As the base of the suppository, Witepsol, Macrogol, Tween 61, Cacao butter, laurin fat, glycerogelatin, etc. may be used. Meanwhile, injections may contain conventional additives such as a solubilizer, an isotonic agent, a suspending agent, an emulsifier, a stabilizer, and a preservative.

The formulation may be prepared by a conventional mixing, granulation or coating method, and contains the active ingredient in an amount effective for medical treatment, specifically, inhibition of cancer metastasis and invasion, or treatment of colorectal cancer.

The pharmaceutical composition of the present invention is administered in a pharmaceutically effective amount. As used herein, the term "pharmaceutically effective amount" refers to an amount sufficient to treat a disease at a reasonable benefit/risk ratio applicable to any medical treatment. The effective dose level of the pharmaceutical composition may be determined depending on factors, including the patient's health status, the kind and severity of the disease, the activity of the drug, sensitivity to the drug, the mode of administration, the time of administration, the route of administration, excretion rate, and drugs used in combination with the composition, as well as other factors well known in the medical field. The pharmaceutical composition of the present invention may be administered individually or in combination with other therapeutic agents, and may be administered sequentially or simultaneously with conventional therapeutic agents. The pharmaceutical composition may be administered in a single or multiple dosage form. It is important to administer the pharmaceutical composition in the minimum amount that can exhibit the maximum effect without causing side effects, in view of all the above-described factors, and this amount can be easily determined by a person skilled in the art.

For example, the dosage of the pharmaceutical composition may increase or decrease depending on the route of administration, the severity of the disease, the patient's sex, weight and age, etc., and thus the dosage is not intended to limit the scope of the present invention in any sense.

A preferred dosage of the compound of the present invention varies depending on the patient's condition and weight, the severity of the disease, the form of the drug, and the route and duration of administration, but may be appropriately selected by those skilled in the art.

In another aspect, the present invention provides a method for inhibiting cancer metastasis and invasion or treating colorectal cancer comprising a step of administering the pharmaceutical composition of the present invention to a subject in need thereof.

As used herein, the term "subject" refers to all animals, including humans, monkey, cows, horses, sheep, pigs, chicken, turkeys, quails, cats, dogs, mice, rats, rabbits or guinea pigs that are likely to develop cancer metastasis or invasion, or have cancer metastasis or invasion, or have developed colorectal cancer. When the pharmaceutical composition of the present invention is administered to a subject, it is possible to inhibit cancer metastasis and invasion or to effectively treat colorectal cancer. In addition, since the pharmaceutical composition of the present invention exhibits an effect of inhibiting cancer metastasis and invasion and treating colorectal cancer by increasing the expression of the KAI1 promoter gene, it may exhibit a synergistic effect when administered in combination with an existing therapeutic agent.

As used herein, the term "administration" or "administering" means providing a given substance to a patient by any suitable method. The pharmaceutical composition of the present invention may be administered through any general route as long as it can reach the target tissue. The pharmaceutical composition of the present invention may be administered intraperitoneally, intravenously, intramuscularly, subcutaneously, intradermally, orally, topically, intranasally, intrapulmonarily, or intrarectally administration, without being limited thereto. In addition, the composition of the present invention may also be administered using any device capable of delivering the active ingredient to target cells. Preferred administration modes and formulations are intravenous injections, subcutaneous injections, intradermal injections, intramuscular injections, drip injections, and the like. Injections may be prepared using aqueous solvents such as physiological saline and Ringer's solution, or non-aqueous solvents such as vegetable oil, higher fatty acid esters (e.g., ethyl oleate, etc.), or alcohols (e.g., ethanol, benzyl alcohol, propylene glycol, glycerin, etc.), and may contain pharmaceutical carriers, such as stabilizers (e.g., ascorbic acid, sodium hydrogen sulfite, sodium pyrosulfite, BHA, tocopherol, EDTA, etc.) for preventing deterioration, emulsifiers, buffers for pH adjustment, preservatives (e.g., phenylmercuric nitrate, thimerosal, benzalkonium chloride, phenol, cresol, benzyl alcohol, etc.) for inhibiting microbial growth, etc.

Hereinafter, the present invention will be described in more detail with reference to examples. These examples serve to explain the present invention in more detail, and the scope of the present invention is not limited by these examples.

### Examples 1 to 87. Preparation of Compounds 1 to 87 (DKC1125a01 to 43 and DKC1125b01 to 44) according to the Present Invention

Compounds 1 to 87 (DKC1125a01 to 43 and DKC1125b01 to 44) of the present invention were synthesized according to the following Reaction Scheme 1.

### [Reaction Scheme 1]

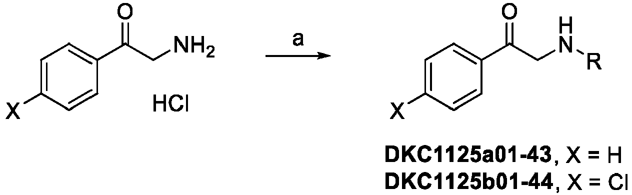

### Reagent and conditions: (a)RCI, NaHCO3, DCM, H2O, 1h

### General Preparation Method for Compounds 1 to 43 (DKC1125a01 to 43) and Compounds 44 to 87 (DKC1125b01 to 44):

CH₂Cl₂ (1 mL) and saturated NaHCO₃ (2 mL) were stirred vigorously and chilled in an ice bath. Each acid chloride (0.29 mmol) shown in Tables 2 and 3 below was added, followed immediately by addition of aminoacetophenone hydrochloride (50 mg, 0.29 mmol). Stirring was continued while warming to room temperature over a period of 1 hour. The organic layer was then separated, and dried with Na₂SO₄, and the solvent was evaporated (80 to 90% yield).

**[Table 2]**

| Acid chlorides used in preparation of Compounds 1 to 43 (DKC1125a01 to 43) | | | | |
|---|---|---|---|---|
| **Compounds** | **Acid chloride** | | **Compounds** | **Acid chloride** |
| **DKC1125a01** | 4-Chlorobenzoyl chloride | | **DKC1125a23** | Methoxyacetyl chloride |
| **DKC1125a02** | 2-Phenoxypropionyl chloride | | **DKC1125a24** | Trimethylacetyl chloride |
| **DKC1125a03** | 2-Fluorobenzoyl chloride | | **DKC1125a25** | Methanesulfonyl chloride |
| **DKC1125a04** | 4-Fluorobenzoyl chloride | | **DKC1125a26** | 4-Fluorobenzenesulfonyl chloride |
| **DKC1125a05** | 2-Furoyl chloride | | **DKC1125a27** | p-Toluenesulfonyl chloride |
| **DKC1125a06** | 2-Chlorobenzoyl Chloride | | **DKC1125a28** | a-Toluenesulfonyl chloride |
| **DKC1125a07** | 3-Chlorobenzoyl chloride | | **DKC1125a29** | 3,5-Bis(trifluoromethyl)benzoyl chloride |
| **DKC1125a08** | Crotonyl chloride | | **DKC1125a30** | 2-(Trifluoromethyl)benzoyl chloride |
| **DKC1125a09** | 3-Phenylpropionyl chloride | | **DKC1125a31** | 3-(Trifluoromethyl)benzoyl chloride |
| **DKC1125a10** | Acryloyl chloride | | **DKC1125a32** | 4-(Trifluoromethyl)benzoyl chloride |
| **DKC1125a11** | o-Toluoyl chloride | | **DKC1125a33** | 2-Chloro-4-fluorobenzoyl chloride |
| **DKC1125a12** | 3-Methoxybenzoyl chloride | | **DKC1125a34** | 3-Nitrobenzoyl chloride |
| **DKC1125a13** | m-Toluoyl chloride | | **DKC1125a35** | 3,4-Difluorobenzoyl chloride |
| **DKC1125a14** | Propionyl chloride | | **DKC1125a36** | 3,5-Dichlorobenzoyl chloride |
| **DKC1125a15** | Cyclohexanecarbonyl chloride | | **DKC1125a37** | 2,3-Dichlorobenzoyl chloride |
| **DKC1125a16** | Cyclopropanecarbonylchloride | | **DKC1125a38** | 4-(Trifluoromethoxy)benzoyl chloride |
| **DKC1125a17** | Cyclobutanecarbonyl chloride | | **DKC1125a39** | 3-(Trifluoromethoxy)benzoyl chloride |
| **DKC1125a18** | Thiophene-2-carbonyl chloride | | **DKC1125a40** | 3,5-Dimethylbenzoyl chloride |
| **DKC1125a19** | tert-butylacetyl chloride | | **DKC1125a41** | 2-(Trifluoromethoxy)benzoyl chloride |
| **DKC1125a20** | Pentanoyl chloride | | **DKC1125a42** | 3,5-Dimethoxybenzoyl chloride |
| **DKC1125a21** | 3-nitrobenzene-1-sulfonyl chloride | | **DKC1125a43** | 4-Biphenylcarbonyl chloride |
| **DKC1125a22** | 2,4-Difluorobenzoyl chloride | | | |

**[Table 3]**

| Acid chlorides used in preparation of Compounds 44 to 87 (DKC1125b01 to 45) | | | | |
|---|---|---|---|---|
| **Compounds** | **Acid chloride** | | **Compounds** | **Acid chloride** |
| **DKC1125b01** | 2-Fluorobenzoyl chloride | | **DKC1125b23** | Pivaloyl chloride |
| **DKC1125b02** | 4-Fluorobenzoyl chloride | | **DKC1125b24** | 4-Fluorobenzenesulfonyl chloride |
| **DKC1125b03** | 2-Furoyl chloride | | **DKC1125b25** | p-Toluenesulfonyl chloride |
| **DKC1125b04** | 3-Chlorobenzoyl chloride | | **DKC1125b26** | 4-Chlorobenzenesulfonyl chloride |
| **DKC1125b05** | Crotonyl chloride | | **DKC1125b27** | a-Toluenesulfonyl chloride |
| **DKC1125b06** | 3-Phenylpropionyl chloride | | **DKC1125b28** | 4-Methoxybenzenesulfonyl chloride |
| **DKC1125b07** | Acryloyl chloride | | **DKC1125b29** | 3,5-Bis(trifluoromethyl)benzoyl chloride |
| **DKC1125b08** | o-Toluoyl chloride | | **DKC1125b30** | 2-(Trifluoromethyl)benzoyl chloride |
| **DKC1125b09** | 3-Methoxybenzoyl chloride | | DKC1125b31 | 3-(Trifluoromethyl)benzoyl chloride |
| **DKC1125b10** | m-Toluoyl chloride | | **DKC1125b32** | 4-(Trifluoromethyl)benzoyl chloride |
| **DKC1125b11** | Propionyl chloride | | **DKC1125b33** | 2-Chloro-4-fluorobenzoyl chloride |
| **DKC1125b12** | Cyclohexanecarbonyl chloride | | **DKC1125b34** | 3-Nitrobenzoyl chloride |
| **DKC1125b13** | Cyclopropanecarbonylchloride | | **DKC1125b35** | 3,4-Difluorobenzoyl chloride |
| **DKC1125b14** | Cyclobutanecarbonyl chloride | | **DKC1125b36** | 3,5-Dichlorobenzoyl chloride |
| **DKC1125b15** | Thiophene-2-carbonyl chloride | | **DKC1125b37** | 2,3-Dichlorobenzoyl chloride |
| **DKC1125b16** | 2-Ethoxybenzoyl chloride | | **DKC1125b38** | 4-(Trifluoromethoxy)benzoyl chloride |
| **DKC1125b17** | tert-butylacetyl chloride | | **DKC1125b39** | 3-(Trifluoromethoxy)benzoyl chloride |
| **DKC1125b18** | Pentanoyl chloride | | **DKC1125b40** | 3,5-Dimethylbenzoyl chloride |
| **DKC1125b19** | 4-Methoxybenzoyl chloride | | **DKC1125b41** | 2-(Trifluoromethoxy)benzoyl chloride |
| **DKC1125b20** | 3-nitrobenzene-1-sulfonyl chloride | | **DKC1125b42** | 3,5-Dimethoxybenzoyl chloride |
| **DKC1125b21** | 2,4-Difluorobenzoyl chloride | | **DKC1125b43** | 4-Biphenylcarbonyl chloride |
| **DKC1125b22** | Methoxyacetyl chloride | | **DKC1125b44** | 4-Chlorobenzoyl chloride |

### Confirmation of Preparation of Compounds 1 to 87 (DKC1125a01 to 43 and DKC1125b01 to 44)

**4-chloro-N-(2-oxo-2-phenylethyl)benzamide (DKC1125a01)** ¹H-NMR (400 MHz, CDCl₃) *δ* 8.01 (dd, *J* = 8.5, 1.1 Hz, 2H), 7.87-7.92 (m, 2H), 7.62-7.66 (m, 1H), 7.50-7.53 (m, 2H), 7.34 (s, 1H), 7.10-7.18 (m, 2H), 4.94 (d, *J =* 4.1 Hz, 2H). MS(ESI): m/z 275 (M+1).

**N-(2-oxo-2-phenylethyl)-2-phenoxypropanamide (DKC1125a02)** ¹H-NMR (400 MHz, CDCl₃) δ 7.95-7.97 (m, 2H), 7.62 (tt, *J* = 7.5, 1.4 Hz, 1H), 7.55 (s, 1H), 7.47-7.51 (m, 2H), 7.29-7.33 (m, 2H), 6.97-7.03 (m, 3H), 4.67-4.87 (m, 2H), 1.63 (d, *J* = 6.9 Hz, 3H). MS(ESI): m/z 284 (M+1).

**2-fluoro-N-(2-oxo-2-phenylethyl)benzamide (DKC1125a03)** ¹H-NMR (400 MHz, CDCl₃) *δ* 8.13 (td, *J* = 7.7, 1.9 Hz, 1H), 8.04 (dd, *J* = 8.5, 1.1 Hz, 2H), 7.90 (d, *J* = 11.4 Hz, 1H), 7.63-7.66 (m, 1H), 7.48-7.55 (m, 3H), 7.26-7.30 (m, 1H), 7.15-7.21 (m, 1H), 5.01 (dd, *J* = 4.1, 0.9 Hz, 2H). MS(ESI): m/z 258 (M+1).

**4-fluoro-N-(2-oxo-2-phenylethyl)benzamide (DKC1125a04)** ¹H-NMR (400 MHz, CDCl₃) *δ* 8.02-8.04 (m, 2H), 7.88-7.93 (m, 2H), 7.63-7.67 (m, 1H), 7.53 (t, *J =* 7.8 Hz, 2H), 7.29 (s, 1H), 7.11-7.17 (m, 2H), 4.95 (d, *J* = 4.1 Hz, 2H). MS(ESI): m/z 258 (M+1).

**N-(2-oxo-2-phenylethyl)furan-2-carboxamide (DKC1125a05)** ¹H-NMR (400 MHz, CDCl₃) *δ* 7.98-8.00 (m, 2H), 7.58-7.62 (m, 1H), 7.47-7.50 (m, 3H), 7.45-7.31 (1H), 7.13-7.13 (m, 1H), 6.49 (q, *J =* 1.8 Hz, 1H), 4.90 (d, *J* = 4.6 Hz, 2H). MS(ESI): m/z 230 (M+1).

**2-chloro-N-(2-oxo-2-phenylethyl)benzamide (DKC1125a06)** ¹H-NMR (400 MHz, CDCl₃) *δ* 8.01 (dt, *J* = 8.2, 1.6 Hz, 2H), 7.72 (dd, *J* = 7.3, 1.8 Hz, 1H), 7.63 (tt, *J* = 7.4, 1.4 Hz, 1H), 7.48-7.53 (m, 3H), 7.30-7.43 (m, 3H), 4.98 (d, *J* = 4.6 Hz, 2H). MS(ESI): m/z 275 (M+1).

**3-chloro-N-(2-oxo-2-phenylethyl)benzamide (DKC1125a07)** ¹H-NMR (400 MHz, CDCl₃) *δ* 8.00-8.03 (m, 2H), 7.88 (t, *J* = 1.8 Hz, 1H), 7.75 (dd, *J* = 7.8, 0.9 Hz, 1H), 7.64 (t, *J* = 7.3 Hz, 1H), 7.48-7.54 (m, 3H), 7.39 (t, *J* = 8.0 Hz, 2H), 4.94 (d, *J* = 4.1 Hz, 2H). MS(ESI): m/z 275 (M+1).

**(E)-N-(2-oxo-2-phenylethyl)but-2-enamide (DKC1125a08)** ¹H-NMR (400 MHz, CDCl₃) *δ* 8.00 (dt, *J* = 8.4, 1.5 Hz, 2H), 7.63 (tt, *J* = 7.5, 1.4 Hz, 1H), 7.49-7.52 (m, 2H), 6.92 (td, *J* = 7.5, 6.4 Hz, 1H), 6.64 (s, 1H), 5.98 (dt, *J* = 15.2, 1.7 Hz, 1H), 4.84 (d, *J* = 4.6 Hz, 2H), 1.89 (dd, *J* = 6.9, 1.8 Hz, 3H). MS(ESI): m/z 204 (M+1).

**N-(2-oxo-2-phenylethyl)-3-phenylpropanamide (DKC1125a09)** ¹H-NMR (400 MHz, CDCl₃) *δ* 7.95 (dt, *J* = 8.4, 1.5 Hz, 2H), 7.58-7.62 (m, 1H), 7.45-7.49 (m, 2H), 7.16-7.29 (m, 5H), 6.69 (s, 1H), 4.74 (d, *J* = 4.1 Hz, 2H), 3.00 (t, *J* = 7.8 Hz, 2H), 2.62 (dd, *J* = 8.7, 6.9 Hz, 2H). MS(ESI): m/z 268 (M+1).

**N-(2-oxo-2-phenylethyl)acrylamide (DKC1125a10)** ¹H-NMR (400 MHz, CDCl₃) *δ* 7.99-8.01 (m, 2H), 7.63 (tt, *J* = 7.5, 1.4 Hz, 1H), 7.49-7.53 (m, 2H), 6.88 (s, 1H), 6.25-6.39 (m, 2H), 5.71 (dd, *J* = 9.6, 1.8 Hz, 1H), 4.87 (d, *J* = 4.6 Hz, 2H). MS(ESI): m/z 190 (M+1).

**2-methyl-N-(2-oxo-2-phenylethyl)benzamide (DKC1125a11)** ¹H-NMR (400 MHz, CDCl₃) *δ* 8.00 (dt, *J* = 8.2, 1.6 Hz, 2H), 7.62 (tt, *J* = 7.4, 1.4 Hz, 1H), 7.47-7.52 (m, 3H), 7.32 (td, *J* = 7.4, 1.7 Hz, 1H), 7.21 (t, *J* = 7.5 Hz, 2H), 6.97 (s, 1H), 4.92 (d, *J* = 4.6 Hz, 2H), 2.48 (s, 3H). MS(ESI): m/z 254 (M+1).

**4-methoxy-N-(2-oxo-2-phenylethyl)benzamide (DKC1125a12)** ¹H-NMR (400 MHz, CDCl₃) *δ* 7.99-8.01 (m, 2H), 7.62 (tt, *J* = 7.4, 1.4 Hz, 1H), 7.48-7.51 (m, 3H), 7.39-7.45 (m, 3H), 7.34 (t, *J =* 7.8 Hz, 1H), 7.04 (dq, *J* = 8.2, 1.2 Hz, 1H), 4.92 (d, *J* = 4.1 Hz, 2H), 3.84 (t, *J* = 4.6 Hz, 3H). MS(ESI): m/z 270 (M+1).

**3-methyl-N-(2-oxo-2-phenylethyl)benzamide (DKC1125a13)** ¹H-NMR (400 MHz, CDCl₃) δ 8.01-8.04 (m, 2H), 7.70 (s, 1H), 7.67 (td, *J* = 4.0, 2.1 Hz, 1H), 7.63 (tt, *J* = 7.4, 1.4 Hz, 1H), 7.49-7.53 (m, 2H), 7.32-7.36 (m, 3H), 4.94 (d, *J* = 4.6 Hz, 2H), 2.38 (d, *J* = 14.2 Hz, 3H). MS(ESI): m/z 254 (M+1).

**N-(2-oxo-2-phenylethyl)propionamide (DKC1125a14)** ¹H-NMR (400 MHz, CDCl₃) *δ* 7.99 (dt, *J* = 8.2, 1.6 Hz, 2H), 7.63 (tt, *J* = 7.4, 1.4 Hz, 1H), 7.49-7.53 (m, 2H), 6.63 (s, 1H), 4.78 (d, *J* = 4.1 Hz, 2H), 2.36 (q, *J =* 7.6 Hz, 2H), 1.22 (t, *J =* 7.5 Hz, 3H). MS(ESI): m/z 192 (M+1).

**N-(2-oxo-2-phenylethyl)cyclohexanecarboxamide (DKC1125a15)** ¹H-NMR (400 MHz, CDCl₃) *δ* 7.97-8.01 (m, 2H), 7.59-7.64 (m, 1H), 7.47-7.51 (m, 2H), 6.68 (s, 1H), 4.58-4.78 (m, 2H), 2.21-2.29 (m, 1H), 1.95 (dd, *J* = 22.2, 12.1 Hz, 2H), 1.76-1.84 (m, 2H), 1.49 (q, *J* = 11.9 Hz, 2H), 1.19-1.35 (m, 4H). MS(ESI): m/z 246 (M+1).

**N-(2-oxo-2-phenylethyl)cyclopropanecarboxamide (DKC1125a16)** ¹H-NMR (400 MHz, CDCl₃) *δ* 7.99 (dt, *J* = 8.2, 1.6 Hz, 2H), 7.63 (tt, *J* = 7.4, 1.4 Hz, 1H), 7.48-7.53 (m, 2H), 6.82 (s, 1H), 4.80 (d, *J* = 4.1 Hz, 2H), 1.57 (tt, *J* = 8.4, 3.8 Hz, 1H), 0.99-1.03 (m, 2H), 0.80 (td, *J* = 7.4, 4.3 Hz, 2H). MS(ESI): m/z 204 (M+1).

**N-(2-oxo-2-phenylethyl)cyclobutanecarboxamide (DKC1125a17)** ¹H-NMR (400 MHz, CDCl₃) *δ* 7.98 (dt, *J* = 8.4, 1.5 Hz, 2H), 7.62 (tt, *J* = 7.3, 1.5 Hz, 1H), 7.48-7.51 (m, 2H), 6.59 (s, 1H), 4.77 (d, *J =* 4.3 Hz, 2H), 3.12-3.21 (m, 1H), 2.29-2.39 (m, 2H), 2.16-2.24 (m, 2H), 1.84-2.05 (m, 2H). MS(ESI): m/z 218 (M+1).

**N-(2-oxo-2-phenylethyl)thiophene-2-carboxamide (DKC1125a18)** ¹H-NMR (400 MHz, CDCl₃) *δ* 8.01-8.04 (m, 2H), 7.62-7.67 (m, 2H), 7.50-7.54 (m, 3H), 7.19 (s, 1H), 7.11 (dd, *J* = 4.8, 3.9 Hz, 1H), 4.95 (d, *J* = 4.6 Hz, 2H). MS(ESI): m/z 246 (M+1).

**3,3-dimethyl-N-(2-oxo-2-phenylethyl)butanamide (DKC1125a19)** ¹H-NMR (400 MHz, CDCl₃) *δ* 7.97-8.00 (m, 2H), 7.60-7.64 (m, 1H), 7.47-7.52 (m, 2H), 6.62 (d, *J* = 13.7 Hz, 1H), 4.79 (dd, *J* = 4.3, 1.1 Hz, 2H), 2.21 (S, 2H), 1.07 (d, *J* = 1.4 Hz, 9H). MS(ESI): m/z 234 (M+1).

**N-(2-oxo-2-phenylethyl)pentanamide (DKC1125a20)** ¹H-NMR (400 MHz, CDCl₃) *δ* 7.99 (dt, *J* = 8.4, 1.5 Hz, 2H), 7.60-7.64 (m, 1H), 7.48-7.52 (m, 2H), 6.68 (s, 1H), 4.78 (d, *J* = 4.1 Hz, 2H), 2.32 (t, *J* = 7.5 Hz, 2H), 1.64-1.71 (m, 2H), 1.34-1.41 (m, 2H), 0.93 (td, *J* = 7.3, 2.3 Hz, 3H). MS(ESI): m/z 220 (M+1).

**3-nitro-N-(2-oxo-2-phenylethyl)benzenesulfonamide (DKC1125a21)** ¹H-NMR (400 MHz, CDCl₃) *δ* 8.76 (t, *J* = 2.1 Hz, 1H), 8.40-8.43 (m, 1H), 8.23-8.25 (m, 1H), 7.85-7.87 (m, 2H), 7.74 (t, *J* = 8.2 Hz, 1H), 7.63 (t, *J* = 7.5 Hz, 1H), 7.49 (t, *J* = 7.8 Hz, 2H), 5.94 (s, 1H), 4.57 (d, *J* = 4.6 Hz, 2H). MS(ESI): m/z 321 (M+1).

**2,4-difluoro-N-(2-oxo-2-phenylethyl)benzamide (DKC1125a22)** ¹H-NMR (400 MHz, CDCl₃) *δ* 8.15 (td, *J* = 8.8, 6.6 Hz, 1H), 8.03 (dd, *J* = 8.5, 1.1 Hz, 2H), 7.83 (d, *J* = 10.5 Hz, 1H), 7.64 (t, *J =* 7.3 Hz, 1H), 7.52 (t, *J* = 7.8 Hz, 2H), 6.89-7.03 (m, 2H), 4.99 (d, *J* = 4.1 Hz, 2H). MS(ESI): m/z 276 (M+1).

**2-methoxy-N-(2-oxo-2-phenylethyl)acetamide (DKC1125a23)** ¹H-NMR (400 MHz, CDCl₃) *δ* 7.97-8.00 (m, 2H), 7.60-7.64 (m, 1H), 7.48-7.55 (m, 3H), 4.80 (d, *J* = 5.0 Hz, 2H), 3.99 (s, 2H), 3.48 (s, 3H). MS(ESI): m/z 208 (M+1).

**N-(2-oxo-2-phenylethyl)pivalamide (DKC1125a24)** ¹H-NMR (400 MHz, CDCl₃) *δ* 7.97-8.07 (m, 2H), 7.62 (t, J= 7.5 Hz, 1H), 7.50 (t, J= 7.8 Hz, 2H), 6.85 (s, 1H), 4.74 (d, *J* = 4.1 Hz, 2H), 1.12-1.34 (m, 9H). MS(ESI): m/z 220 (M+1).

**N-(2-oxo-2-phenylethyl)methanesulfonamide (DKC1125a25)** ¹H-NMR (400 MHz, CDCl₃) *δ* 7.94-7.97 (m, 2H), 7.64 (tt, *J* = 7.4, 1.4 Hz, 1H), 7.49-7.53 (m, 2H), 5.60 (t, *J* = 4.6 Hz, 1H), 4.69 (d, *J* = 5.0 Hz, 2H), 3.03 (s, 3H). MS(ESI): m/z 214 (M+1).

**4-fluoro-N-(2-oxo-2-phenylethyl)benzenesulfonamide (DKC1125a26)** ¹H-NMR (400 MHz, CDCl₃) *δ* 7.93 (tt, *J* = 7.2, 2.4 Hz, 2H), 7.84-7.87 (m, 2H), 7.60-7.64 (m, 1H), 7.46-7.50 (m, 2H), 7.14-7.20 (m, 2H), 5.80 (t, *J* = 4.6 Hz, 1H), 4.49 (d, *J* = 4.6 Hz, 2H). MS(ESI): m/z 294 (M+1).

**4-methyl-N-(2-oxo-2-phenylethyl)benzenesulfonamide (DKC1125a27)** ¹H-NMR (400 MHz, CDCl₃) *δ* 7.85 (dd, *J* = 8.5, 1.1 Hz, 2H), 7.79 (d, *J* = 8.7 Hz, 2H), 7.58-7.62 (m, 1H), 7.46 (t, *J* = 7.8 Hz, 2H), 7.28 (d, *J* = 8.2 Hz, 2H), 5.74 (t, *J* = 4.3 Hz, 1H), 4.47 (d, *J* = 4.6 Hz, 2H), 2.39 (s, 3H). MS(ESI): m/z 290 (M+1).

**N-(2-oxo-2-phenylethyl)-1-phenylmethanesulfonamide (DKC1125a28)** ¹H-NMR (400 MHz, CDCl₃) *δ* 7.78 (d, *J* = 7.3 Hz, 2H), 7.60 (t, *J* = 7.3 Hz, 1H), 7.42-7.47 (m, 4H), 7.29-7.34 (m, 3H), 5.55 (t, *J* = 4.3 Hz, 1H), 4.35 (s, 2H), 4.31 (d, *J* = 4.6 Hz, 2H). MS(ESI): m/z 290 (M+1).

**N-(2-oxo-2-phenylethyl)-3,5-bis(trifluoromethyl)benzamide (DKC1125a29)** ¹H-NMR (400 MHz, CDCl₃) *δ* 8.34 (s, 2H), 8.03-8.06 (m, 3H), 7.66-7.70 (m, 1H), 7.55 (t, *J* = 7.5 Hz, 2H), 7.49 (s, 1H), 5.00 (d, *J* = 4.6 Hz, 2H). MS(ESI): m/z 376 (M+1).

**N-(2-oxo-2-phenylethyl)-2-(trifluoromethyl)benzamide (DKC1125a30)** ¹H-NMR (400 MHz, CDCl₃) *δ* 7.97-7.99 (m, 2H), 7.48-7.71 (m, 7H), 7.07 (s, 1H), 4.95 (d, *J* = 4.1 Hz, 2H). MS(ESI): m/z 308 (M+1).

**N-(2-oxo-2-phenylethyl)-3-(trifluoromethyl)benzamide (DKC1125a31)** ¹H-NMR (400 MHz, CDCl₃) *δ* 8.17 (s, 1H), 8.02-8.07 (m, 3H), 7.79 (d, *J* = 8.2 Hz, 1H), 7.58-7.68 (m, 2H), 7.51-7.55 (m, 2H), 7.43 (s, 1H), 4.98 (d, *J* = 4.6 Hz, 2H). MS(ESI): m/z 308 (M+1).

**N-(2-oxo-2-phenylethyl)-4-(trifluoromethyl)benzamide (DKC1125a32)** ¹H-NMR (400 MHz, CDCl₃) *δ* 8.00-8.06 (m, 4H), 7.75 (d, *J* = 8.2 Hz, 2H), 7.65-7.69 (m, 1H), 7.55 (t, *J* = 7.8 Hz, 2H), 7.37 (s, 1H), 4.98 (d, *J* = 4.1 Hz, 2H). MS(ESI): m/z 308 (M+1).

**2-chloro-4-fluoro-N-(2-oxo-2-phenylethyl)benzamide (DKC1125a33)** ¹H-NMR (400 MHz, CDCl₃) *δ* 7.99-8.01 (m, 2H), 7.77 (dd, *J* = 8.7, 5.9 Hz, 1H), 7.62-7.66 (m, 1H), 7.49-7.55 (m, 3H), 7.15 (dd, *J* = 8.5, 2.5 Hz, 1H), 7.04 (td, *J* = 8.2, 2.7 Hz, 1H), 4.97 (d, *J* = 4.6 Hz, 2H). MS(ESI): m/z 293 (M+1).

**3-nitro-N-(2-oxo-2-phenylethyl)benzamide (DKC1125a34)** ¹H-NMR (400 MHz, CDCl₃) *δ* 8.73 (t, *J* = 2.1 Hz, 1H), 8.37 (dd, *J* = 7.8, 1.8 Hz, 1H), 8.23 (dt, *J* = 7.9, 1.4 Hz, 1H), 8.02-8.04 (m, 2H), 7.64-7.69 (m, 2H), 7.51-7.55 (m, 3H), 5.00 (d, *J* = 4.1 Hz, 2H). MS(ESI): m/z 285 (M+1).

**3,4-difluoro-N-(2-oxo-2-phenylethyl)benzamide (DKC1125a35)** ¹H-NMR (400 MHz, CDCl₃) *δ* 8.02-8.04 (m, 2H), 7.73-7.78 (m, 1H), 7.62-7.68 (m, 2H), 7.52-7.56 (m, 2H), 7.23-7.29 (m, 2H), 4.94-4.95 (m, 2H). MS(ESI): m/z 276 (M+1).

**3,5-dichloro-N-(2-oxo-2-phenylethyl)benzamide (DKC1125a36)** ¹H-NMR (400 MHz, CDCl₃) *δ* 8.01-8.03 (m, 2H), 7.75 (d, *J* = 1.8 Hz, 2H), 7.66 (tt, *J* = 7.4, 1.4 Hz, 1H), 7.50-7.55 (m, 3H), 7.33 (s, 1H), 4.94 (d, *J* = 4.1 Hz, 2H). MS(ESI): m/z 309 (M+1).

**2,3-dichloro-N-(2-oxo-2-phenylethyl)benzamide (DKC1125a37)** ¹H-NMR (400 MHz, CDCl₃) *δ* 7.99-8.01 (m, 2H), 7.63-7.67 (m, 1H), 7.51-7.55 (m, 4H), 7.27 (t, *J =* 8.0 Hz, 2H), 4.98 (d, *J =* 4.1 Hz, 2H). MS(ESI): m/z 309 (M+1).

**N-(2-oxo-2-phenylethyl)-4-(trifluoromethoxy)benzamide (DKC1125a38) (DKC1125a38)** ¹H-NMR (400 MHz, CDCl₃) *δ* 8.01-8.03 (m, 2H), 7.93 (dt, *J* = 9.3, 2.3 Hz, 2H), 7.64 (t, *J* = 7.5 Hz, 1H), 7.52 (t, *J =* 7.8 Hz, 2H), 7.39 (s, 1H), 7.28 (d, *J =* 8.2 Hz, 2H), 4.95 (d, *J =* 4.1 Hz, 2H). MS(ESI): m/z 324 (M+1).

**N-(2-oxo-2-phenylethyl)-3-(trifluoromethoxy)benzamide (DKC1125a39)** ¹H-NMR (400 MHz, CDCl₃) *δ* 8.01 (dd, *J* = 8.5, 1.1 Hz, 2H), 7.77-7.81 (m, 2H), 7.61-7.66 (m, 1H), 7.46-7.53 (m, 4H), 7.37 (dt, *J* = 8.4, 1.1 Hz, 1H), 4.95 (d, *J* = 4.6 Hz, 2H). MS(ESI): m/z 324 (M+1).

**3,5=dimethyl-N-(2-oxo-2-phenylethyl)benzamide (DKC1125a40)** ¹H-NMR (400 MHz, CDCl₃) *δ* 8.00-8.02 (m, 2H), 7.60-7.64 (m, 1H), 7.50 (t, *J =* 7.5 Hz, 4H), 7.34 (s, 1H), 7.13 (s, 1H), 4.93 (d, *J* = 4.1 Hz, 2H), 2.31-2.36 (m, 6H). MS(ESI): m/z 268 (M+1).

**N-(2-oxo-2-phenylethyl)-2-(trifluoromethoxy)benzamide (DKC1125a41)** ¹H-NMR (400 MHz, CDCl₃) *δ* 8.03 (tt, *J* = 5.3, 1.9 Hz, 3H), 7.80 (s, 1H), 7.63 (tt, *J* = 7.4, 1.4 Hz, 1H), 7.49-7.55 (m, 3H), 7.40 (td, *J* = 7.5, 1.1 Hz, 1H), 7.34 (dt, *J* = 8.2, 1.4 Hz, 1H), 4.99 (d, *J =* 4.6 Hz, 2H). MS(ESI): m/z 324 (M+1).

**3,5-dimethoxy-N-(2-oxo-2-phenylethyl)benzamide (DKC1125a42)** ¹H-NMR (400 MHz, CDCl₃) *δ* 7.99-8.02 (m, 2H), 7.63 (tt, *J* = 7.4, 1.4 Hz, 1H), 7.48-7.52 (m, 2H), 7.36 (d, *J* = 3.7 Hz, 1H), 7.00 (d, *J =* 2.3 Hz, 2H), 6.59 (t, *J* = 2.3 Hz, 1H), 4.92 (d, *J* = 4.1 Hz, 2H), 3.80 (d, *J* = 10.1 Hz, 6H). MS(ESI): m/z 300 (M+1).

**N-(2-oxo-2-phenylethyl)-[1,1'-biphenyl]-4-carboxamide (DKC1125a43)** ¹H-NMR (400 MHz, CDCl₃) *δ* 8.04-8.06 (m, 2H), 7.97 (dt, *J* = 8.4, 1.9 Hz, 2H), 7.61-7.71 (m, 5H), 7.53 (t, *J* = 7.8 Hz, 2H), 7.45-7.49 (m, 2H), 7.37-7.41 (m, 2H), 4.99 (d, *J =* 4.1 Hz, 2H). MS(ESI): m/z 316 (M+1).

**N-(2-(4-chlorophenyl)-2-oxoethyl)-2-fluorobenzamide (DKC1125b01)** ¹H-NMR (400 MHz, CDCl₃) *δ* 8.11 (tt, *J* = 7.9, 2.4 Hz, 1H), 7.97 (dt, *J* = 9.0, 2.1 Hz, 2H), 7.84-7.87 (m, 1H), 7.47-7.53 (m, 3H), 7.25-7.29 (m, 1H), 7.10-7.19 (m, 1H), 4.97 (dd, *J* = 4.3, 1.1 Hz, 2H). MS(ESI): m/z 293 (M+1).

**N-(2-(4-chlorophenyl)-2-oxoethyl)-4-fluorobenzamide (DKC1125b02)** ¹H-NMR (400 MHz, CDCl₃) *δ* 7.88-7.97 (m, 4H), 7.50 (dd, *J* = 8.0, 1.6 Hz, 2H), 7.26 (s, 1H), 7.12-7.16 (m, 2H), 4.91 (d, *J* = 2.7 Hz, 2H). MS(ESI): m/z 293 (M+1).

**N-(2-(4-chlorophenyl)-2-oxoethyl)furan-2-carboxamide (DKC1125b03)** ¹H-NMR (400 MHz, CDCl₃) *δ* 7.96-7.98 (m, 2H), 7.48-7.51 (m, 2H), 7.28-7.39 (m, 1H), 7.16 (d, *J* = 1.8 Hz, 1H), 6.52 (s, 1H), 4.90 (t, *J =* 2.1 Hz, 2H). MS(ESI): m/z 265 (M+1).

**3-chloro-N-(2-(4-chlorophenyl)-2-oxoethyl)benzamide (DKC1125b04)** ¹H-NMR (400 MHz, CDCl₃) *δ* 7.94-7.96 (m, 2H), 7.86 (t, *J =* 1.6 Hz, 1H), 7.73 (dd, *J* = 7.8, 0.9 Hz, 1H), 7.47-7.50 (m, 3H), 7.35-7.41 (m, 2H), 4.91 (d, *J =* 4.1 Hz, 2H). MS(ESI): m/z 309 (M+1).

**(E)-N-(2-(4-chlorophenyl)-2-oxoethyl)but-2-enamide (DKC1125b05)** ¹H-NMR (400 MHz, CDCl₃) *δ* 7.93-7.96 (m, 2H), 7.47-7.50 (m, 2H), 6.92 (q, *J* = 7.3 Hz, 1H), 6.59 (s, 1H), 5.97 (dd, *J* = 15.1, 1.8 Hz, 1H), 4.81 (d, *J* = 4.6 Hz, 2H), 1.89 (dd, *J* = 6.9, 1.8 Hz, 3H). MS(ESI): m/z 239 (M+1).

**N-(2-(4-chlorophenyl)-2-oxoethyl)-3-phenylpropanamide (DKC1125b06)** ¹H-NMR (400 MHz, CDCl₃) *δ* 7.89 (dt, *J* = 9.0, 2.3 Hz, 2H), 7.45 (dt, *J* = 8.8, 2.3 Hz, 2H), 7.25-7.30 (m, 2H), 7.17-7.22 (m, 3H), 6.62 (s, 1H), 4.70 (d, *J* = 4.1 Hz, 2H), 3.00 (t, *J* = 7.9 Hz, 2H), 2.62 (dd, *J* = 8.7, 6.9 Hz, 2H). MS(ESI): m/z 303 (M+1).

**N-(2-(4-chlorophenyl)-2-oxoethyl)acrylamide (DKC1125b07)** ¹H-NMR (400 MHz, CDCl₃) *δ* 7.93-7.96 (m, 2H), 7.48-7.51 (m, 2H), 6.78 (s, 1H), 6.23-6.39 (m, 2H), 5.73 (dd, *J* = 10.1, 1.8 Hz, 1H), 4.83 (d, *J =* 4.6 Hz, 2H). MS(ESI): m/z 225 (M+1).

**N-(2-(4-chlorophenyl)-2-oxoethyl)-2-methylbenzamide (DKC1125b08)** ¹H-NMR (400 MHz, CDCl₃) *δ* 7.95 (dt, *J* = 9.0, 2.1 Hz, 2H), 7.46-7.51 (m, 3H), 7.32-7.36 (m, 1H), 7.21-7.27 (m, 2H), 6.90 (s, 1H), 4.90 (d, *J* = 4.6 Hz, 2H), 2.47 (d, *J* = 4.6 Hz, 3H). MS(ESI): m/z 289 (M+1).

**N-(2-(4-chlorophenyl)-2-oxoethyl)-4-methoxybenzamide (DKC1125b09)** ¹H-NMR (400 MHz, CDCl₃) *δ* 7.94 (dt, *J* = 9.0, 2.1 Hz, 2H), 7.48 (dt, *J* = 8.7, 1.9 Hz, 2H), 7.40-7.43 (m, 2H), 7.31-7.37 (m, 2H), 7.04-7.07 (m, 1H), 4.90 (d, *J =* 4.1 Hz, 2H), 3.84 (s, 3H). MS(ESI): m/z 305 (M+1).

**N-(2-(4-chlorophenyl)-2-oxoethyl)-3-methylbenzamide (DKC1125b10)** ¹H-NMR (400 MHz, CDCl₃) *δ* 7.96 (dt, *J* = 9.0, 2.1 Hz, 2H), 7.64-7.69 (m, 2H), 7.48 (dt, *J* = 9.0, 2.1 Hz, 2H), 7.30-7.34 (m, 3H), 4.91 (d, *J =* 4.3 Hz, 2H), 2.39 (d, *J* = 10.1 Hz, 3H). MS(ESI): m/z 289 (M+1).

**N-(2-(4-chlorophenyl)-2-oxoethyl)propionamide (DKC1125b11)** ¹H-NMR (400 MHz, CDCl₃) *δ* 7.92-7.95 (m, 2H), 7.47-7.50 (m, 2H), 6.60 (s, 1H), 4.75 (d, *J* = 4.6 Hz, 2H), 2.35 (q, *J* = 7.6 Hz, 2H), 1.22 (t, *J =* 7.5 Hz, 3H). MS(ESI): m/z 227 (M+1).

**N-(2-(4-chlorophenyl)-2-oxoethyl)cyclohexanecarboxamide (DKC1125b12)** ¹H-NMR (400 MHz, CDCl₃) *δ* 7.92 (dt, *J* = 8.8, 2.2 Hz, 2H), 7.48 (dt, *J* = 9.0, 2.1 Hz, 2H), 6.62 (s, 1H), 4.73 (d, *J* = 4.6 Hz, 2H), 2.24 (tt, *J* = 11.7, 3.6 Hz, 1H), 1.92 (dd, *J* = 13.5, 2.1 Hz, 2H), 1.80-1.84 (m, 2H), 1.67-1.71 (m, 1H), 1.49 (qd, *J =* 12.2, 2.9 Hz, 2H), 1.21-1.36 (m, 3H). MS(ESI): m/z 281 (M+1).

**N-(2-(4-chlorophenyl)-2-oxoethyl)cyclopropanecarboxamide (DKC1125b13)** ¹H-NMR (400 MHz, CDCl₃) *δ* 7.92-7.95 (m, 2H), 7.48-7.50 (m, 2H), 6.70 (s, 1H), 4.76 (d, *J =* 4.6 Hz, 2H), 1.51-1.56 (m, 1H), 1.01 (dt, *J* = 8.2, 3.5 Hz, 2H), 0.81 (td, *J* = 7.4, 4.3 Hz, 2H). MS(ESI): m/z 239 (M+1).

**N-(2-(4-chlorophenyl)-2-oxoethyl)cyclobutanecarboxamide (DKC1125b14)** ¹H-NMR (400 MHz, CDCl₃) *δ* 7.91-7.94 (m, 2H), 7.46-7.49 (m, 2H), 6.50 (s, 1H), 4.74 (d, *J* = 4.1 Hz, 2H), 3.11-3.20 (m, 1H), 1.85-2.38 (m, 6H). MS(ESI): m/z 253 (M+1).

**N-(2-(4-chlorophenyl)-2-oxoethyl)thiophene-2-carboxamide (DKC1125b15)** ¹H-NMR (400 MHz, CDCl₃) *δ* 7.94-7.97 (m, 2H), 7.64 (dd, *J* = 3.7, 0.9 Hz, 1H), 7.47-7.52 (m, 3H), 7.09-7.19 (m, 2H), 4.90 (d, *J =* 4.1 Hz, 2H). MS(ESI): m/z 281 (M+1).

**N-(2-(4-chlorophenyl)-2-oxoethyl)-2-(methoxymethyl)benzamide (DKC1125b16)** ¹H-NMR (400 MHz, CDCl₃) *δ* 9.19 (s, 1H), 8.25 (dd, *J* = 7.8, 1.8 Hz, 1H), 7.97 (dt, *J* = 9.0, 2.1 Hz, 2H), 7.42-7.49 (m, 3H), 6.98-7.08 (m, 2H), 4.97 (d, *J =* 4.1 Hz, 2H), 4.25 (q, *J =* 7.0 Hz, 2H), 1.70 (t, *J* = 7.1 Hz, 3H). MS(ESI): m/z 319 (M+1).

**N-(2-(4-chlorophenyl)-2-oxoethyl)-3,3-dimethylbutanamide (DKC1125b17)** ¹H-NMR (400 MHz, CDCl₃) *δ* 7.93 (dt, *J* = 9.0, 2.1 Hz, 2H), 7.48 (dt, *J* = 9.0, 2.3 Hz, 2H), 6.52 (s, 1H), 4.75 (d, *J* = 4.6 Hz, 2H), 2.20 (s, 2H), 1.07 (d, *J* = 2.3 Hz, 9H). MS(ESI): m/z 269 (M+1).

**N-(2-(4-chlorophenyl)-2-oxoethyl)pentanamide (DKC1125b18)** ¹H-NMR (400 MHz, CDCl₃) *δ* 7.93 (dt, *J* = 9.0, 2.3 Hz, 2H), 7.47-7.50 (m, 2H), 6.63 (s, 1H), 4.75 (d, *J* = 4.6 Hz, 2H), 2.32 (t, *J* = 7.5 Hz, 2H), 1.62-1.71 (m, 2H), 1.33-1.43 (m, 2H), 0.93 (td, *J* = 7.2, 4.3 Hz, 3H). MS(ESI): m/z 255 (M+1).

**N-(2-(4-chlorophenyl)-2-oxoethyl)-4-methoxybenzamide (DKC1125b19)** ¹H-NMR (400 MHz, CDCl₃) *δ* 7.96-7.99 (m, 2H), 7.84-7.87 (m, 2H), 7.49-7.51 (m, 2H), 7.19 (s, 1H), 6.94-6.97 (m, 2H), 4.92 (d, *J* = 4.1 Hz, 2H), 3.86 (d, *J* = 4.1 Hz, 3H). MS(ESI): m/z 305 (M+1).

**N-(2-(4-chlorophenyl)-2-oxoethyl)-3-nitrobenzenesulfonamide (DKC1125b20)** ¹H-NMR (400 MHz, CDCl₃) *δ* 8.75 (t, *J* = 1.8 Hz, 1H), 8.42-8.44 (m, 1H), 8.23 (d, *J* = 7.8 Hz, 1H), 7.81 (d, *J* = 8.2 Hz, 2H), 7.75 (t, *J* = 8.0 Hz, 1H), 7.47 (d, *J* = 8.7 Hz, 2H), 5.81 (s, 1H), 4.53 (d, *J* = 4.1 Hz, 2H). MS(ESI): m/z 356 (M+1).

**N-(2-(4-chlorophenyl)-2-oxoethyl)-2,4ifluorobenzamide (DKC1125b21)** ¹H-NMR (400 MHz, CDCl₃) *δ* 8.11-8.18 (m, 1H), 7.97 (dt, *J* = 9.0, 2.3 Hz, 2H), 7.77-7.80 (m, 1H), 7.50 (dt, *J* = 9.0, 2.3 Hz, 2H), 6.98-7.03 (m, 1H), 6.88-6.95 (m, 1H), 4.96 (dd, *J* = 4.1, 0.9 Hz, 2H). MS(ESI): m/z 311 (M+1).

**N-(2-(4-chlorophenyl)-2-oxoethyl)-2-methoxyacetamide (DKC1125b22)** ¹H-NMR (400 MHz, CDCl₃) *δ* 7.93 (dt, *J* = 8.8, 2.2 Hz, 2H), 7.49 (dt, *J* = 9.0, 2.1 Hz, 3H), 4.77 (d, *J* = 4.6 Hz, 2H), 3.99 (s, 2H), 3.49 (s, 3H). MS(ESI): m/z 243 (M+1).

**N-(2-(4-chlorophenyl)-2-oxoethyl)pivalamide (DKC1125b23)** ¹H-NMR (400 MHz, CDCl₃) *δ* 7.92-7.95 (m, 2H), 7.47-7.50 (m, 2H), 6.79 (s, 1H), 4.71 (d, *J* = 4.1 Hz, 2H), 1.27 (d, *J* = 5.5 Hz, 9H). MS(ESI): m/z 255 (M+1).

**N-(2-(4-chlorophenyl)-2-oxoethyl)-4-fluorobenzenesulfonamide (DKC1125b24)** ¹H-NMR (400 MHz, CDCl₃) *δ* 7.92 (tt, *J* = 7.3, 2.3 Hz, 2H), 7.80 (dt, *J* = 9.0, 2.1 Hz, 2H), 7.45 (dt, *J* = 8.8, 2.3 Hz, 2H), 7.18 (tt, *J* = 8.8, 2.7 Hz, 2H), 5.73 (t, *J* = 4.3 Hz, 1H), 4.46 (d, *J* = 4.6 Hz, 2H). MS(ESI): m/z 329 (M+1).

**N-(2-(4-chlorophenyl)-2-oxoethyl)-4-methylbenzenesulfonamide (DKC1125b25)** ¹H-NMR (400 MHz, CDCl₃) *δ* 7.77-7.81 (m, 4H), 7.44 (dd, *J* = 6.6, 1.6 Hz, 2H), 7.29 (d, *J* = 8.2 Hz, 2H), 5.66 (s, 1H), 4.43 (d, *J* = 4.6 Hz, 2H), 2.40 (s, 3H). MS(ESI): m/z 325 (M+1).

**4-chloro-N-(2-(4-chlorophenyl)-2-oxoethyl)benzenesulfonamide (DKC1125b26)** ¹H-NMR (400 MHz, CDCl₃) *δ* 7.99 (dt, *J* = 9.3, 2.3 Hz, 1H), 7.78-7.85 (m, 3H), 7.61 (dt, *J* = 9.0, 2.3 Hz, 1H), 7.44-7.49 (m, 3H), 5.71 (t, *J* = 4.3 Hz, 1H), 4.45 (d, *J* = 4.6 Hz, 2H). MS(ESI): m/z 345 (M+1).

**N-(2-(4-chlorophenyl)-2-oxoethyl)-1-phenylmethanesulfonamide (DKC1125b27)** ¹H-NMR (400 MHz, CDCl₃) *δ* 7.72 (dt, *J* = 9.0, 2.1 Hz, 2H), 7.41-7.46 (m, 4H), 7.32-7.36 (m, 3H), 5.36 (t, *J* = 4.3 Hz, 1H), 4.35 (s, 2H), 4.24 (d, *J* = 4.6 Hz, 2H). MS(ESI): m/z 325 (M+1).

**N-(2-(4-chlorophenyl)-2-oxoethyl)-4-methoxybenzenesulfonamide (DKC1125b28)** ¹H-NMR (400 MHz, CDCl₃) *δ* 7.78-7.84 (m, 4H), 7.45 (dt, *J* = 9.0, 2.3 Hz, 2H), 6.96 (dt, *J* = 9.6, 2.5 Hz, 2H), 4.42 (d, *J =* 4.6 Hz, 2H), 3.84 (s, 3H). MS(ESI): m/z 341 (M+1).

**N-(2-(4-chlorophenyl)-2-oxoethyl)-3,5-bis(trifluoromethyl)benzamide (DKC1125b29)** ¹H-NMR (400 MHz, CDCl₃) *δ* 8.33 (s, 2H), 8.04 (s, 1H), 7.98 (dd, *J* = 6.9, 1.8 Hz, 2H), 7.53 (dd, *J* = 6.9, 1.8 Hz, 2H), 7.46 (s, 1H), 4.97 (d, *J* = 4.1 Hz, 2H). MS(ESI): m/z 411 (M+1).

**N-(2-(4-chlorophenyl)-2-oxoethyl)-2-(trifluoromethyl)benzamide (DKC1125b30)** ¹H-NMR (400 MHz, CDCl₃) *δ* 7.94 (d, *J* = 8.7 Hz, 2H), 7.72 (d, *J* = 7.3 Hz, 1H), 7.54-7.63 (m, 3H), 7.50 (d, *J* = 8.7 Hz, 2H), 6.98 (s, 1H), 4.93 (d*, J* = 4.6 Hz, 2H). MS(ESI): m/z 343 (M+1).

**N-(2-(4-chlorophenyl)-2-oxoethyl)-3-(trifluoromethyl)benzamide (DKC1125b31)** ¹H-NMR (400 MHz, CDCl₃) *δ* 8.15 (s, 1H), 8.05 (d, *J =* 7.8 Hz, 1H), 7.96 (dt, *J* = 9.0, 2.1 Hz, 2H), 7.76-7.79 (m, 1H), 7.59 (t, *J* = 7.8 Hz, 1H), 7.48-7.51 (m, 2H), 4.94 (d, *J* = 4.6 Hz, 2H). MS(ESI): m/z 343 (M+1).

**N-(2-(4-chlorophenyl)-2-oxoethyl)-4-(trifluoromethyl)benzamide (DKC1125b32)** ¹H-NMR (400 MHz, CDCl₃) *δ* 7.96-8.00 (m, 4H), 7.74 (d, *J* = 8.2 Hz, 2H), 7.52 (dt, *J* = 8.8, 2.2 Hz, 2H), 7.36 (s, 1H), 4.94 (d, *J =* 4.1 Hz, 2H). MS(ESI): m/z 343 (M+1).

**2-chloro-N-(2-(4-chlorophenyl)-2-oxoethyl)-4-fluorobenzamide (DKC1125b33)** ¹H-NMR (400 MHz, CDCl₃) *δ* 7.96 (dt, *J* = 9.0, 2.1 Hz, 2H), 7.80 (dd, *J* = 8.7, 5.9 Hz, 1H), 7.51 (dt, *J* = 9.0, 2.3 Hz, 2H), 7.46 (s, 1H), 7.19 (dd, *J* = 8.5, 2.5 Hz, 1H), 7.05-7.10 (m, 1H), 4.95 (d, *J* = 4.1 Hz, 2H). MS(ESI): m/z 327 (M+1).

**N-(2-(4-chlorophenyl)-2-oxoethyl)-3-nitrobenzamide (DKC1125b34)** ¹H-NMR (400 MHz, CDCl₃) *δ* 8.74 (t, *J* = 1.8 Hz, 1H), 8.41 (dq, *J* = 8.2, 1.1 Hz, 1H), 8.23 (dd, *J* = 7.8, 0.9 Hz, 1H), 7.98-8.01 (m, 2H), 7.70 (t, *J =* 8.0 Hz, 1H), 7.52-7.55 (m, 2H), 7.38 (s, 1H), 4.97 (d, *J =* 4.1 Hz, 2H). MS(ESI): m/z 320 (M+1).

**N-(2-(4-chlorophenyl)-2-oxoethyl)-3,4ifluorobenzamide (DKC1125b35)** ¹H-NMR (400 MHz, CDCl₃) *δ* 7.97 (dt, *J* = 9.0, 2.3 Hz, 2H), 7.75 (ddd, *J* = 10.6, 7.4, 2.2 Hz, 1H), 7.61-7.65 (m, 1H), 7.51 (dt, *J* = 9.0, 2.1 Hz, 2H), 7.23-7.29 (m, 2H), 4.91 (d, *J* = 4.1 Hz, 2H). MS(ESI): m/z 311 (M+1).

**3,5-dichloro-N-(2-(4-chlorophenyl)-2-oxoethyl)benzamide (DKC1125b36)** ¹H-NMR (400 MHz, CDCl₃) *δ* 7.98 (dt, *J* = 9.0, 2.3 Hz, 2H), 7.74 (d, *J* = 1.8 Hz, 2H), 7.53 (td, *J* = 4.5, 2.1 Hz, 3H), 7.21 (s, 1H), 4.91 (d, *J =* 4.1 Hz, 2H). MS(ESI): m/z 344 (M+1).

**2,3-dichloro-N-(2-(4-chlorophenyl)-2-oxoethyl)benzamide (DKC1125b37)** ¹H-NMR (400 MHz, CDCl₃) *δ* 7.95 (d, *J =* 7.8 Hz, 2H), 7.49-7.55 (m, 4H), 7.28 (t, *J =* 7.5 Hz, 2H), 4.94 (d, *J =* 4.1 Hz, 2H). MS(ESI): m/z 344 (M+1).

**N-(2-(4-chlorophenyl)-2-oxoethyl)-4-(trifluoromethoxy)benzamide (DKC1125b38)** ¹H-NMR (400 MHz, CDCl₃) *δ* 7.97 (dt, *J* = 9.0, 2.1 Hz, 2H), 7.93 (dt, *J* = 9.1, 2.4 Hz, 2H), 7.50 (dt, *J* = 9.1, 2.0 Hz, 2H), 7.26-7.32 (m, 3H), 4.92 (d, *J* = 4.1 Hz, 2H). MS(ESI): m/z 359 (M+1).

**N-(2-(4-chlorophenyl)-2-oxoethyl)-3-(trifluoromethoxy)benzamide (DKC1125b39)** ¹H-NMR (400 MHz, CDCl₃) *δ* 7.96 (dt, *J* = 8.8, 2.2 Hz, 2H), 7.77-7.79 (m, 1H), 7.75 (s, 1H), 7.48-7.52 (m, 3H), 7.36-7.40 (m, 2H), 4.92 (d, *J =* 4.1 Hz, 2H). MS(ESI): m/z 359 (M+1).

**N-(2-(4-chlorophenyl)-2-oxoethyl)-3,5-dimethylbenzamide (DKC1125b40)** ¹H-NMR (400 MHz, CDCl₃) *δ* 7.98 (dt, *J* = 9.0, 2.1 Hz, 2H), 7.50 (dt, *J* = 9.0, 2.1 Hz, 2H), 7.47 (s, 2H), 7.23 (s, 1H), 7.16 (s, 1H), 4.92 (d, *J* = 4.1 Hz, 2H), 2.37 (s, 6H). MS(ESI): m/z 303 (M+1).

**N-(2-(4-chlorophenyl)-2-oxoethyl)-2-(trifluoromethoxy)benzamide (DKC1125b41)** ¹H-NMR (400 MHz, CDCl₃) *δ* 8.04 (dd, *J* = 7.8, 1.4 Hz, 1H), 7.97 (dt, *J* = 9.1, 2.1 Hz, 2H), 7.76 (s, 1H), 7.48-7.56 (m, 3H), 7.41 (td, *J* = 7.5, 1.2 Hz, 1H), 7.35 (dd, *J* = 8.2, 1.4 Hz, 1H), 4.96 (d, *J =* 4.6 Hz, 2H). MS(ESI): m/z 359 (M+1).

**N-(2-(4-chlorophenyl)-2-oxoethyl)-3,5imethoxybenzamide (DKC1125b42)** ¹H-NMR (400 MHz, CDCl₃) *δ* 7.95 (dt, *J* = 9.0, 2.1 Hz, 2H), 7.48 (dt, *J* = 9.0, 2.3 Hz, 2H), 7.31 (t, *J* = 3.9 Hz, 1H), 6.99 (d, *J* = 2.3 Hz, 2H), 6.59 (t, *J* = 2.3 Hz, 1H), 4.89 (d, *J* = 4.6 Hz, 2H), 3.82 (s, 6H). MS(ESI): m/z 335 (M+1).

**N-(2-(4-chlorophenyl)-2-oxoethyl)-[1,1'-biphenyl]-4-carboxamide (DKC1125b43)** ¹H-NMR (400 MHz, CDCl₃) *δ* 7.95-8.01 (m, 4H), 7.70 (d, *J =* 8.2 Hz, 2H), 7.62-7.65 (m, 2H), 7.46-7.54 (m, 4H), 7.40-7.42 (m, 1H), 7.31 (s, 1H), 4.96 (d, *J =* 4.1 Hz, 2H). MS(ESI): m/z 351 (M+1).

**4-chloro-N-(2-(4-chlorophenyl)-2-oxoethyl)benzamide (DKC1125b44)** ¹H-NMR (400 MHz, CDCl₃) *δ* 7.90 (dd, *J* = 6.6, 2.1 Hz, 2H), 7.75 (dd, *J* = 8.7, 2.3 Hz, 2H), 7.36-7.45 (m, 4H), 7.23 (s, 1H), 4.85 (d, *J =* 4.6 Hz, 2H). MS(ESI): m/z 309 (M+1).

### Examples 88 to 121. Preparation of Compounds 88 to 122 (DKC1125c01 to 34) according to the Present Invention

Compounds 88 to 122 (DKC1125c01 to 34) of the present invention were synthesized according to the following Reaction Scheme 2.

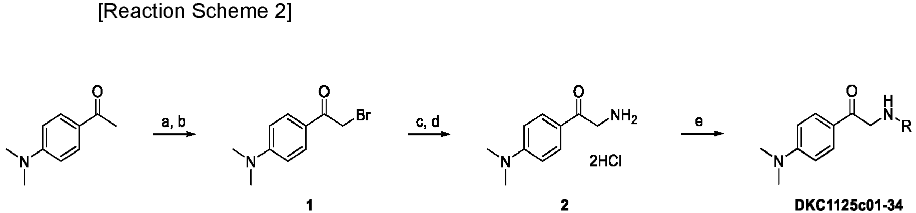

Reagent and conditions: (a) HBr, Br₂, H₂O, 0°C, 1h; (b) NaHCO₃, H₂O; (c) Potassium phthalimide, DMF, room temperature, overnight; (d) HCl, EtOH, 80°C, 2h; (e) RCI, NaHCO₃, DCM, H₂O, 1h

### Synthesis of 2-bromo-1-(4-(dimethv)amino)pheny))ethan-1-one (1):

To a solution of 1-(4-(dimethylamino)phenyl)ethan-1-one (5 g, 30.63 mmol) in HBr (100 mL) at 0°C was added Br₂ (4.9g , 30.63 mmol) dropwise over 1 hour. After the reaction was complete, the product was extracted twice with CH₂Cl₂ and washed with saturated NaHCO₃. The organic layer was dried with MgSO₄, and the solvent was evaporated. The resulting crude product was purified by column chromatography to obtain compound (1) (7.09 g, 95%) of reaction scheme 2.

¹H NMR (400 MHz, CDCl₃) *δ* 7.89 (d, *J* = 9.0 Hz, 2H), 6.66 (d, *J* = 9.0 Hz, 2H), 4.36(s, 2H), 3.08 (s, 6H).

### Synthesis of 2-amino-1-(4-(dimethylamino)phenynethan-1-one hydrochloride (2) :

2-bromo-1-(4-(dimethylamino)phenyl)ethan-1-one (1.6 g, 24.78 mmol) was dissolved in 20 mL of dry potassium phthalimide (5.05 g, 27.26 mmol) solution, and held at 75°C for 18 hrs. The suspension was quenched in 90 mL of water with stirring, which was maintained for 40 min, and the product was collected, washed with 50 mL of water, and recrystallized from 50 mL of acetonitrile at 0°C overnight. (4.58 g, 60%). The solid was dissolved in a solution (90 mL) of 1:2 of concentrated HCl and EtOH. The reaction was boiled under reflux for 2 hrs. The precipitate was filtered out and washed with water and then EtOH (2.61 g, 70%).

¹H NMR (400 MHz, (CD₃)₂SO) *δ* 7.91 (d, *J* = 9.5 Hz, 2H) 6.66 (d, *J* = 9.5 Hz, 2H), 5.06 (s, 2H), 1.22 (s, 6H).

### General Preparation Method for Compounds 88 to 121 (DKC1125c01 to 34)

CH₂Cl₂ (1 mL) and saturated NaHCO₃ (2 mL) were stirred vigorously and chilled in an ice bath. Each acid chloride (0.29 mmol) described in Table 4 below was added, followed immediately by addition of 2-amino-1-(4-(dimethylamino)phenyl)ethan-1-one dihydrochloride (2, 50 mg, 0.29 mmol). Stirring was continued while warming to room temperature over period of 1 hour. The organic layer was then separated, and dried with Na₂SO₄, and the solvent was evaporated. The residue was purified by column chromatography (yield: 70 to 80%).

**[Table 4]**

| Acid chlorides for preparation of compounds 88 to 121 (DKC1125c01 to 34) | | | | |
|---|---|---|---|---|
| **Compounds** | **Acid chloride** | | **Compounds** | **Acid chloride** |
| **DKC1125c01** | 4-Chlorobenzoyl chloride | | **DKC1125c18** | 2,4-Difluorobenzoyl chloride |
| **DKC1125c02** | 2-Phenoxypropionyl chloride | | **DKC1125c19** | Methoxyacetyl chloride |
| **DKC1125c03** | 2-Fluorobenzoyl chloride | | **DKC1125c20** | 4-Fluorobenzenesulfonyl chloride |
| **DKC1125c04** | 4-Fluorobenzoyl chloride | | **DKC1125c21** | 4-Chlorobenzenesulfonyl chloride |
| **DKC1125c05** | 2-Furoyl chloride | | **DKC1125c22** | Benzenesulfonyl chloride |
| **DKC1125c06** | 2-Chlorobenzoyl Chloride | | **DKC1125c23** | a-Toluenesulfonyl chloride |
| **DKC1125c07** | 3-Chlorobenzoyl chloride | | **DKC1125c24** | 4-Methoxybenzenesulfonyl chloride |
| **DKC1125c08** | Acryloyl chloride | | **DKC1125c25** | 2-(Trifluoromethyl)benzoyl chloride |
| **DKC1125c09** | o-Toluoyl chloride | | **DKC1125c26** | 3-(Trifluoromethyl)benzoyl chloride |
| **DKC1125c10** | 3-Methoxybenzoyl chloride | | **DKC1125c27** | 4-(Trifluoromethyl)benzoyl chloride |
| **DKC1125c11** | m-Toluoyl chloride | | **DKC1125c28** | 2-Chloro-4-fluorobenzoyl chloride |
| **DKC1125c12** | Propionyl chloride | | **DKC1125c29** | 3,4-Difluorobenzoyl chloride |
| **DKC1125c13** | Cyclopropanecarbonylchloride | | **DKC1125c30** | 3,5-Dichlorobenzoyl chloride |
| **DKC1125c14** | Thiophene-2-carbonyl chloride | | **DKC1125c31** | 2,3-Dichlorobenzoyl chloride |
| **DKC1125c15** | tert-butylacetyl chloride | | **DKC1125c32** | 4-(Trifluoromethoxy)benzoyl chloride |
| **DKC1125c16** | Pentanoyl chloride | | **DKC1125c33** | 3,5-Dimethylbenzoyl chloride |
| **DKC1125c17** | 4-Methoxybenzoyl chloride | | **DKC1125c34** | 3,5-Dimethoxybenzoyl chloride |

### Confirmation of Preparation of Compounds 88 to 121 (DKC1125c01 to 34)

**4-chloro-N-(2-(4-(dimethylamino)phenyl)-2-oxoethyl)benzamide (DKC1125c01)** ¹H-NMR (400 MHz, CDCl₃) *δ* 7.90-7.92 (m, 2H), 7.83 (dd, *J* = 6.4, 1.8 Hz, 2H), 7.42-7.44 (m, 3H), 6.68 (d, *J =* 9.1 Hz, 2H), 4.83 (d, *J* = 4.1 Hz, 2H), 3.09 (s, 6H). MS(ESI): m/z 318 (M+1).

**N-(2-(4-(dimethylamino)phenyl)-2-oxoethyl)-2-phenoxypropanamide (DKC1125c02)** ¹H-NMR (400 MHz, CDCl₃) *δ* 7.85 (dt, *J* = 9.8, 2.5 Hz, 2H), 7.68 (s, 1H), 7.27-7.33 (m, 2H), 6.97-7.01 (m, 3H), 6.64 (dt, *J* = 9.8, 2.5 Hz, 2H), 4.56-4.80 (m, 2H), 3.06 (d, *J* = 7.3 Hz, 6H), 1.62 (d, *J* = 6.4 Hz, 3H). MS(ESI): m/z 327 (M+1).

**N-(2-(4-(dimethylamino)phenyl)-2-oxoethyl)-2-fluorobenzamide (DKC1125c03)** ¹H-NMR (400 MHz, CDCl₃) *δ* 8.12 (td, *J* = 7.8, 1.8 Hz, 1H), 8.01-8.04 (m, 1H), 7.93 (dt, *J* = 9.6, 2.5 Hz, 2H), 7.46-7.52 (m, 1H), 7.25-7.29 (m, 1H), 7.14-7.19 (m, 1H), 6.66-6.70 (m, 2H), 4.89-4.90 (m, 2H), 3.08 (s, 6H). MS(ESI): m/z 301 (M+1).

**N-(2-(4-(dimethylamino)phenyl)-2-oxoethyl)-4-fluorobenzamide (DKC1125c04)** ¹H-NMR (400 MHz, CDCl₃) *δ* 7.87-7.94 (m, 4H), 7.42 (d, *J =* 11.0 Hz, 1H), 7.11-7.16 (m, 2H), 6.68 (dd, *J =* 12.1, 3.0 Hz, 2H), 4.84 (d, *J* = 4.1 Hz, 2H), 3.11 (d, *J =* 15.1 Hz, 6H). MS(ESI): m/z 301 (M+1).

**N-(2-(4-(dimethylamino)phenyl)-2-oxoethyl)furan-2-carboxamide (DKC1125c05)** ¹H-NMR (400 MHz, CDCl₃) *δ* 7.90-7.93 (m, 2H), 7.56 (s, 1H), 7.50 (t, *J =* 0.9 Hz, 1H), 7.15 (d, *J =* 3.7 Hz, 1H), 6.67-6.70 (m, 2H), 6.51 (q, *J* = 1.7 Hz, 1H), 4.82 (d, *J* = 4.1 Hz, 2H), 3.09 (s, 6H). MS(ESI): m/z 273 (M+1).

**2-chloro-N-(2-(4-(dimethylamino)phenyl)-2-oxoethyl)benzamide (DKC1125c06)** ¹H-NMR (400 MHz, CDCl₃) *δ* 7.90-7.93 (m, 2H), 7.72 (dd, *J* = 7.5, 2.1 Hz, 1H), 7.54 (s, 1H), 7.31-7.45 (m, 3H), 6.67-6.70 (m, 2H), 4.88 (d, *J* = 4.1 Hz, 2H), 3.09 (s, 6H). MS(ESI): m/z 318 (M+1).

**3-chloro-N-(2-(4-(dimethylamino)phenyl)-2-oxoethyl)benzamide (DKC1125c07)** ¹H-NMR (400 MHz, CDCl₃) *δ* 7.88-7.94 (m, 3H), 7.75 (dt, *J* = 7.8, 1.4 Hz, 1H), 7.50 (dq, *J* = 8.1, 1.0 Hz, 1H), 7.38-7.44 (m, 2H), 6.68-6.70 (m, 2H), 4.84 (d, *J* = 4.1 Hz, 2H), 3.10 (s, 6H). MS(ESI): m/z 318 (M+1).

**N-(2-(4-(dimethylamino)phenyl)-2-oxoethyl)acrylamide (DKC1125c08)** ¹H-NMR (400 MHz, CDCl₃) *δ* 7.86-7.91 (m, 2H), 6.86 (s, 1H), 6.67 (dt, *J* = 9.8, 2.5 Hz, 2H), 6.35 (dd, *J* = 16.9, 1.8 Hz, 1H), 6.25 (dd, *J* = 17.2, 9.8 Hz, 1H), 5.70 (dd, *J =* 10.1, 1.8 Hz, 1H), 4.74 (d, *J =* 4.1 Hz, 2H), 3.09 (s, 6H). MS(ESI): m/z 233 (M+1).

**N-(2-(4-(dimethylamino)phenyl)-2-oxoethyl)-2-methylbenzamide (DKC1125c09)** ¹H-NMR (400 MHz, CDCl₃) *δ* 7.91 (dt, *J* = 9.8, 2.5 Hz, 2H), 7.49 (d, *J* = 7.3 Hz, 1H), 7.33 (td, *J* = 7.5, 1.5 Hz, 1H), 7.23-7.26 (m, 2H), 7.04 (s, 1H), 6.66-6.70 (m, 2H), 4.86 (d, *J* = 4.1 Hz, 2H), 3.09 (s, 6H), 2.50 (s, 3H). MS(ESI): m/z 297 (M+1).

**N-(2-(4-(dimethylamino)phenyl)-2-oxoethyl)-4-methoxybenzamide (DKC1125c10)** ¹H-NMR (400 MHz, CDCl₃) *δ* 7.93 (dt, *J* = 9.6, 2.5 Hz, 2H), 7.42-7.46 (m, 3H), 7.37 (t, *J* = 8.0 Hz, 1H), 7.05-7.08 (m, 1H), 6.69 (dt, *J* = 9.8, 2.3 Hz, 2H), 4.85 (d, *J =* 3.7 Hz, 2H), 3.87 (s, 3H), 3.10 (s, 6H). MS(ESI): m/z 313 (M+1).

**N-(2-(4-(dimethylamino)phenyl)-2-oxoethyl)-3-methylbenzamide (DKC1125c11)** ¹H-NMR (400 MHz, CDCl₃) *δ* 7.91-7.94 (m, 2H), 7.69 (dd, *J* = 8.2, 6.4 Hz, 2H), 7.44 (s, 1H), 7.32-7.37 (m, 2H), 6.69 (d, *J* = 9.1 Hz, 2H), 4.85 (d, *J* = 3.7 Hz, 2H), 3.11 (d, *J* = 14.6 Hz, 6H), 2.40 (d, *J* = 14.2 Hz, 3H). MS(ESI): m/z 297 (M+1).

**N-(2-(4-(dimethylamino)phenyl)-2-oxoethyl)propionamide (DKC1125c12)** ¹H-NMR (400 MHz, CDCl₃) *δ* 7.86-7.92 (m, 2H), 6.65-6.71 (m, 3H), 4.66 (d, *J* = 4.1 Hz, 2H), 3.08 (t, *J* = 15.3 Hz, 6H), 2.34 (q, *J* = 7.6 Hz, 2H), 1.18-1.25 (m, 3H). MS(ESI): m/z 235 (M+1).

**N-(2-(4-(dimethylamino)phenyl)-2-oxoethyl)cyclopropanecarboxamide (DKC1125c13)** ¹H-NMR (400 MHz, CDCl₃) *δ* 7.86-7.91 (m, 2H), 6.86 (s, 1H), 6.67 (dt, *J* = 9.8, 2.5 Hz, 2H), 4.68 (d, *J* = 4.3 Hz, 2H), 3.08 (s, 6H), 1.54 (tt, *J* = 8.4, 3.8 Hz, 1H), 1.00 (dt, *J* = 8.1, 3.3 Hz, 2H), 0.78 (dt, *J* = 11.6, 3.4 Hz, 2H). MS(ESI): m/z 247 (M+1).

**N-(2-(4-(dimethylamino)phenyl)-2-oxoethyl)thiophene-2-carboxamide (DKC1125c14)** ¹H-NMR (400 MHz, CDCl₃) *δ* 7.92 (dt, *J* = 9.6, 2.4 Hz, 2H), 7.63 (dd, *J* = 3.9, 1.1 Hz, 1H), 7.50 (dd, *J* = 5.0, 0.9 Hz, 1H), 7.29 (s, 1H), 7.11 (dd, *J* = 5.0, 3.7 Hz, 1H), 6.68 (dt, *J* = 9.8, 2.3 Hz, 2H), 4.83 (d, *J* = 4.1 Hz, 2H), 3.09 (s, 6H). MS(ESI): m/z 289 (M+1).

**N-(2-(4-(dimethylamino)phenyl)-2-oxoethyl)-3,3-dimethylbutanamide (DKC1125c15)** ¹H-NMR (400 MHz, CDCl₃) *δ* 7.87 (dt, *J* = 9.6, 2.5 Hz, 2H), 6.66 (dt, *J* = 9.8, 2.5 Hz, 2H), 6.61 (s, 1H), 4.67 (d, *J* = 4.1 Hz, 2H), 3.07 (d, *J =* 8.7 Hz, 6H), 2.19 (s, 2H), 1.07 (s, 9H). MS(ESI): m/z 277 (M+1).

**N-(2-(4-(dimethylamino)phenyl)-2-oxoethyl)pentanamide (DKC1125c16)** ¹H-NMR (400 MHz, CDCl₃) *δ* 7.87-7.90 (m, 2H), 6.65-6.68 (m, 3H), 4.66 (d, *J* = 4.1 Hz, 2H), 3.08 (s, 6H), 2.30 (t, *J* = 7.5 Hz, 2H), 1.63-1.71 (m, 2H), 1.34-1.41 (m, 3H), 0.93 (t, *J =* 7.3 Hz, 3H). MS(ESI): m/z 263 (M+1).

**N-(2-(4-(dimethylamino)phenyl)-2-oxoethyl)-4-methoxybenzamide (DKC1125c17)** ¹H-NMR (400 MHz, CDCl₃) *δ* 7.93 (dt, *J* = 9.8, 2.5 Hz, 2H), 7.86 (dt, *J* = 9.5, 2.5 Hz, 2H), 7.36 (s, 1H), 6.93-6.97 (m, 2H), 6.68-6.71 (m, 2H), 4.84 (d, *J =* 4.1 Hz, 2H), 3.87 (d, *J* = 4.1 Hz, 3H), 3.09 (s, 6H). MS(ESI): m/z 313 (M+1).

**N-(2-(4-(dimethylamino)phenyl)-2-oxoethyl)-2,4ifluorobenzamide (DKC1125c18)** ¹H-NMR (400 MHz, CDCl₃) *δ* 8.15 (td, *J* = 8.9, 6.7 Hz, 1H), 7.91-7.97 (m, 3H), 6.98-7.02 (m, 1H), 6.89-6.95 (m, 1H), 6.69 (dt, *J* = 9.8, 2.3 Hz, 2H), 4.88-4.89 (m, 2H), 3.10 (s, 6H). MS(ESI): m/z 319 (M+1).

**N-(2-(4-(dimethylamino)phenyl)-2-oxoethyl)-2-methoxyacetamide (DKC1125c19)** ¹H-NMR (400 MHz, CDCl₃) *δ* 7.88 (dt, *J* = 9.8, 2.5 Hz, 2H), 7.64 (s, 1H), 6.67 (dt, *J* = 9.6, 2.4 Hz, 2H), 4.70 (d, *J* = 4.6 Hz, 2H), 3.98 (s, 2H), 3.48 (s, 3H), 3.08 (s, 6H). MS(ESI): m/z 251 (M+1).

**N-(2-(4-(dimethylamino)phenyl)-2-oxoethyl)-4-fluorobenzenesulfonamide (DKC1125c20)** ¹H-NMR (400 MHz, CDCl₃) *δ* 7.88-7.94 (m, 2H), 7.73 (dt, J= 9.8, 2.5 Hz, 2H), 7.13-7.18 (m, 2H), 6.60-6.63 (m, 2H), 5.83 (t, *J* = 4.1 Hz, 1H), 4.35 (d, *J* = 4.1 Hz, 2H), 3.02-3.09 (m, 6H). MS(ESI): m/z 337 (M+1).

**4-chloro-N-(2-(4-(dimethylamino)phenyl)-2-oxoethyl)benzenesulfonamide (DKC1125c21)** ¹H-NMR (400 MHz, CDCl₃) *δ* 7.83 (dt, *J* = 9.0, 2.3 Hz, 2H), 7.72 (dt, *J* = 9.8, 2.5 Hz, 2H), 7.45 (dt, *J* = 9.0, 2.3 Hz, 2H), 6.61 (dt, *J* = 9.7, 2.5 Hz, 2H), 5.85 (t, *J* = 4.1 Hz, 1H), 4.35 (d, *J* = 4.1 Hz, 2H), 3.02-3.09 (m, 6H). MS(ESI): m/z 354 (M+1).

**N-(2-(4-(dimethylamino)phenyl)-2-oxoethyl)benzenesulfonamide (DKC1125c22)** ¹H-NMR (400 MHz, CDCl₃) *δ* 7.88-7.93 (m, 2H), 7.73 (dt, *J* = 9.6, 2.4 Hz, 2H), 7.46-7.57 (m, 3H), 6.59-6.63 (m, 2H), 5.82 (t, *J =* 3.9 Hz, 1H), 4.35 (d, *J* = 4.6 Hz, 2H), 3.02-3.07 (m, 6H). MS(ESI): m/z 319 (M+1).

**N-(2-(4-(dimethylamino)phenyl)-2-oxoethyl)-1-phenylmethanesulfonamide (DKC1125c23)** ¹H-NMR (400 MHz, CDCl₃) *δ* 7.67-7.71 (m, 2H), 7.39-7.43 (m, 2H), 7.32-7.34 (m, 3H), 6.60-6.64 (m, 2H), 5.46 (t, *J* = 4.1 Hz, 1H), 4.33 (d, *J* = 8.7 Hz, 2H), 4.23 (d, *J* = 4.1 Hz, 2H), 3.08 (s, 6H). MS(ESI): m/z 333 (M+1).

**N-(2-(4-(dimethylamino)phenyl)-2-oxoethyl)-4-methoxybenzenesulfonamide (DKC1125c24)** ¹H-NMR (400 MHz, CDCl₃) *δ* 7.80 (dt, *J* = 9.5, 2.5 Hz, 2H), 7.71 (dt, *J* = 9.9, 2.4 Hz, 2H), 6.92 (dt, *J* = 9.5, 2.5 Hz, 2H), 6.58-6.61 (m, 2H), 5.73 (t, *J* = 4.3 Hz, 1H), 4.31 (d, *J* = 4.6 Hz, 2H), 3.81 (s, 3H), 3.02 (d, *J* = 17.4 Hz, 6H). MS(ESI): m/z 349 (M+1).

**N-(2-(4-(dimethylamino)phenyl)-2-oxoethyl)-2-(trifluoromethyl)benzamide (DKC1125c25)** ¹H-NMR (400 MHz, CDCl₃) *δ* 7.91 (dt, J= 9.6, 2.4 Hz, 2H), 7.72-7.75 (m, 1H), 7.61-7.65 (m, 2H), 7.55-7.60 (m, 1H), 7.11 (s, 1H), 6.69 (dt, *J* = 9.8, 2.5 Hz, 2H), 4.87 (d, *J* = 4.1 Hz, 2H), 3.10 (d, *J* = 6.4 Hz, 6H). MS(ESI): m/z 351 (M+1).

**N-(2-(4-(dimethylamino)phenyl)-2-oxoethyl)-3-(trifluoromethyl)benzamide (DKC1125c26)** ¹H-NMR (400 MHz, CDCl₃) *δ* 8.18 (s, 1H), 8.07 (d, *J* = 7.8 Hz, 1H), 7.94 (dt, *J* = 9.8, 2.3 Hz, 2H), 7.79 (d, *J* = 7.8 Hz, 1H), 7.62 (t, *J* = 7.8 Hz, 1H), 7.51 (s, 1H), 6.71 (dt, *J* = 9.8, 2.5 Hz, 2H), 4.87 (d, *J* = 4.1 Hz, 2H), 3.11 (s, 6H). MS(ESI): m/z 351 (M+1).

**N-(2-(4-(dimethylamino)phenyl)-2-oxoethyl)-4-(trifluoromethyl)benzamide (DKC1125c27)** ¹H-NMR (400 MHz, CDCl₃) *δ* 8.00 (d, *J* = 8.2 Hz, 2H), 7.92 (dd, *J* = 7.1, 2.1 Hz, 2H), 7.73 (d, *J* = 7.8 Hz, 2H), 7.52 (s, 1H), 6.68-6.70 (m, 2H), 4.86 (d, *J* = 4.1 Hz, 2H), 3.10 (s, 6H). MS(ESI): m/z 351 (M+1).

**2-chloro-N-(2-(4-(dimethylamino)phenyl)-2-oxoethyl)-4-fluorobenzamide (DKC1125c28)** ¹H-NMR (400 MHz, CDCl₃) *δ* 7.90-7.93 (m, 2H), 7.79 (dd, *J* = 8.5, 6.2 Hz, 1H), 7.58 (s, 1H), 7.19 (dd, *J* = 8.5, 2.5 Hz, 1H), 7.05-7.10 (m, 1H), 6.69 (dd, *J* = 9.4, 2.5 Hz, 2H), 4.88 (d, *J* = 4.1 Hz, 2H), 3.10 (d, *J* = 3.7 Hz, 6H). MS(ESI): m/z 336 (M+1).

**N-(2-(4-(dimethylamino)phenyl)-2-oxoethyl)-3,4ifluorobenzamide (DKC1125c29)** ¹H-NMR (400 MHz, CDCl₃) *δ* 7.90-7.93 (m, 2H), 7.74-7.79 (m, 1H), 7.64 (tt, *J* = 6.1, 2.0 Hz, 1H), 7.42 (s, 1H), 7.22-7.29 (m, 1H), 6.68-6.71 (m, 2H), 4.83-4.89 (m, 2H), 3.13 (d, *J* = 15.1 Hz, 5H). MS(ESI): m/z 319 (M+1).

**3,5-dichloro-N-(2-(4-(dimethylamino)phenyl)-2-oxoethyl)benzamide (DKC1125c30)** ¹H-NMR (400 MHz, CDCl₃) *δ* 7.93 (dt, *J* = 9.8, 2.5 Hz, 2H), 7.75-7.77 (m, 2H), 7.52 (q, *J* = 2.3 Hz, 1H), 7.42 (s, 1H), 6.68-6.72 (m, 2H), 4.83 (d, *J* = 3.7 Hz, 2H), 3.10-3.15 (m, 6H). MS(ESI): m/z 352 (M+1).

**2,3-dichloro-N-(2-(4-(dimethylamino)phenyl)-2-oxoethyl)benzamide (DKC1125c31)** ¹H-NMR (400 MHz, CDCl₃) *δ* 7.91 (dt, *J* = 9.8, 2.5 Hz, 2H), 7.54 (ddd, *J* = 10.6, 7.9, 1.5 Hz, 2H), 7.33 (s, 1H), 7.27-7.31 (m, 1H), 6.69 (dt, *J* = 9.6, 2.5 Hz, 2H), 4.88 (d, *J* = 4.1 Hz, 2H), 3.11 (s, 6H). MS(ESI): m/z 352 (M+1).

**N-(2-(4-(dimethylamino)phenyl)-2-oxoethyl)-4-(trifluoromethoxy)benzamide (DKC1125c32)** ¹H-NMR (400 MHz, CDCl₃) *δ* 7.91-7.97 (m, 4H), 7.45 (s, 1H), 7.30-7.32 (m, 2H), 6.70 (dt, *J* = 9.8, 2.5 Hz, 2H), 4.85 (d, *J* = 4.1 Hz, 2H), 3.11 (s, 6H). MS(ESI): m/z 367 (M+1).

**N-(2-(4-(dimethylamino)phenyl)-2-oxoethyl)-3,5-dimethylbenzamide (DKC1125c33)** ¹H-NMR (400 MHz, CDCl₃) *δ* 7.91-7.95 (m, 2H), 7.49 (s, 2H), 7.42 (d, *J* = 3.7 Hz, 1H), 7.15 (s, 1H), 6.69 (dd, *J =* 11.9, 2.7 Hz, 2H), 4.84 (d, *J* = 4.1 Hz, 2H), 3.11 (d, *J =* 15.1 Hz, 6H), 2.34-2.37 (m, 6H). MS(ESI): m/z 311 (M+1).

**N-(2-(4-(dimethylamino)phenyl)-2-oxoethyl)-3,5imethoxybenzamide (DKC1125c34)** ¹H-NMR (400 MHz, CDCl₃) *δ* 7.93 (dd, *J* = 11.9, 2.7 Hz, 2H), 7.39 (s, 1H), 7.01 (d, *J* = 2.3 Hz, 2H), 6.69 (dd, *J* = 11.9, 2.7 Hz, 2H), 6.60 (t, *J* = 2.3 Hz, 1H), 4.84 (d, *J* = 4.1 Hz, 2H), 3.83-3.89 (m, 6H), 3.10 (s, 6H). MS(ESI): m/z 343 (M+1).

### Examples 122 to 129. Preparation of Compounds 122 to 129 (DKC1125d01 to 08) according to the Present Invention

Compounds 122 to 129 (DKC1125d01 to 08) of the present invention were synthesized according to the following Reaction Scheme 3.

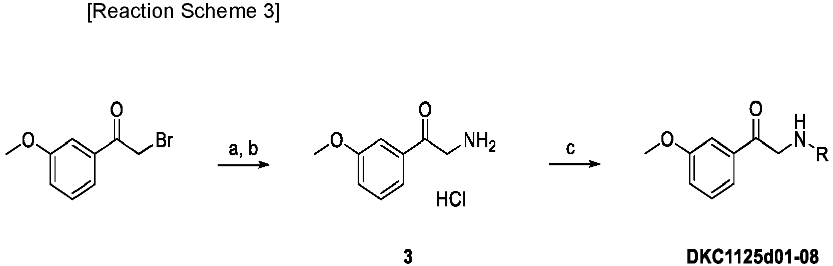

Reagent and conditions: (a) Hexamethylenetetramine, DCM, room temperature, overnight: HCl, EtOH, 80°C, 2h; (c) RCI, NaHCO₃, DCM, H₂O, 1h

### Synthesis of 2-amino-1-(3-methoxyphenyl)ethan-1-one hydrochloride (3)

2-bromo-1-(3-methoxyphenyl)ethan-1-one (5 g, 21.83 mmol) was dissolved in 25 mL of CH₂Cl₂ and treated with hexamethylenetetramine(3.06 g, 21.83 mmol). After overnight at room temperature, the reaction product was cooled with an ice bath and the formed precipitate was washed with CH₂Cl₂ and EtOH to obtain the hexamethylenetetramine salt. The desired amine was obtained by treating the salt with a solution (90 mL) of concentrated HCl and EtOH (1:2). The formed precipitate was filtered out and washed with water and then EtOH (2.95g, 65%).

¹H NMR (400 MHz, (CD₃)₂SO) *δ* 8.50(s, 3H), 7.61 (dd, *J* = 0.9, 7.8 Hz, 1H), 7.50-7.53(m, 2H), 7.31 (m, 1H), 4.58(d, *J* = 4.4 Hz, 2H), 3.85(s, 3H).

### General Preparation Method for Compounds 122 to 129 (DKC1125d01 to 08)

CH₂Cl₂ (1 mL) and saturated NaHCO₃ (2 mL) were stirred vigorously and chilled in an ice bath. Each acid chloride (0.29 mmol) described in Table 5 below was added, followed immediately by addition of 2-amino-1-(3-methoxyphenyl)ethan-1-one hydrochloride (3, 50 mg, 0.29 mmol). Stirring was continued while warming to room temperature over a period of 1 hour. The organic layer was then separated, and dried with Na₂SO₄, and the solvent was evaporated (yield: 80 to 90%).

**[Table 5]**

| Acid chlorides used in preparation of 122 to 129 (DKC1125d01 to 08) | |
|---|---|
| **Compounds** | **Acid chloride** |
| **DKC1125d01** | 4-Chlorobenzoyl chloride |
| **DKC1125d02** | Crotonyl chloride |
| **DKC1125d03** | Propionyl chloride |
| **DKC1125d04** | Cyclopropanecarbonylchloride |
| **DKC1125d05** | Cyclobutanecarbonyl chloride |
| **DKC1125d06** | Thiophene-2-carbonyl chloride |
| **DKC1125d07** | Pentanoyl chloride |
| **DKC1125d08** | Benzenesulfonyl chloride |

### Confirmation of Preparation of Compounds 122 to 129 (DKC1125d01 to 08)

**4-chloro-N-(2-(3-methoxyphenyl)-2-oxoethyl)benzamide (DKC1125d01)** ¹H-NMR (400 MHz, CDCl₃) δ 7.83 (dd, *J* = 8.7, 2.3 Hz, 2H), 7.61 (dd, *J* = 7.7, 0.8 Hz, 1H), 7.52-7.54 (m, 2H), 7.44 (dd, *J* = 8.2, 1.8 Hz, 3H), 7.29 (s, 1H), 7.19 (dd, *J* = 8.2, 2.7 Hz, 1H), 4.94 (d, *J =* 4.1 Hz, 2H). MS(ESI): m/z 305 (M+1).

**(E)-N-(2-(3-methoxyphenyl)-2-oxoethyl)but-2-enamide (DKC1125d02)** ¹H-NMR (400 MHz, CDCl₃) *δ* 7.58 (d, *J =* 7.8 Hz, 1H), 7.50 (t, *J =* 2.1 Hz, 1H), 7.41 (t, *J =* 8.0 Hz, 1H), 7.17 (dd, *J* = 8.0, 2.5 Hz, 1H), 6.92 (q, *J* = 7.3 Hz, 1H), 5.98 (dd, *J* = 15.3, 1.6 Hz, 1H), 4.83 (d, *J* = 4.1 Hz, 2H), 3.87 (s, 3H), 1.89 (dd, *J* = 6.9, 1.4 Hz, 3H), 1.61 (d, *J* = 6.4 Hz, 1H). MS(ESI): m/z 234 (M+1).

**N-(2-(3-methoxyphenyl)-2-oxoethyl)propionamide (DKC1125d03)** ¹H-NMR (400 MHz, CDCl₃) *δ* 7.64-7.50 (1H), 7.50-7.43 (1H), 7.43-7.33 (1H), 7.20-7.07 (1H), 6.72-6.46 (1H), 4.77-4.68 (2H), 3.87-3.77 (3H), 2.38-2.25 (2H), 1.25-1.08 (3H). MS(ESI): m/z 222 (M+1).

**N-(2-(3-methoxyphenyl)-2-oxoethyl)cyclopropanecarboxamide (DKC1125d04)** ¹H-NMR (400 MHz, CDCl₃) *δ* 7.56 (d, *J* = 7.8 Hz, 1H), 7.49 (t, *J* = 2.1 Hz, 1H), 7.41 (q, *J* = 7.8 Hz, 1H), 7.16 (dd, *J* = 8.2, 2.3 Hz, 1H), 6.79 (s, 1H), 4.78 (d, *J* = 4.1 Hz, 2H), 3.86 (s, 3H), 1.53-1.59 (m, 1H), 0.99-1.04 (m, 2H), 0.78-0.84 (m, 2H). MS(ESI): m/z 234 (M+1).

**N-(2-(3-methoxyphenyl)-2-oxoethyl)cyclobutanecarboxamide (DKC1125d05)** ¹H-NMR (400 MHz, CDCl₃) *δ* 7.56 (dd, *J* = 7.8, 1.4 Hz, 1H), 7.49 (q, *J* = 1.4 Hz, 1H), 7.41 (t, *J* = 8.0 Hz, 1H), 7.16 (dt, *J* = 8.2, 1.4 Hz, 1H), 6.51 (s, 1H), 4.75 (d, *J*= 4.1 Hz, 2H), 3.86 (s, 3H), 3.11-3.20 (m, 1H), 2.29-2.39 (m, 2H), 2.17-2.25 (m, 2H), 1.85-2.05 (m, 2H). MS(ESI): m/z 248 (M+1).

**N-(2-(3-methoxyphenyl)-2-oxoethyl)thiophene-2-carboxamide (DKC1125d06)** ¹H-NMR (400 MHz, CDCl₃) *δ* 7.62 (d, *J =* 3.7 Hz, 1H), 7.59 (d, *J =* 7.8 Hz, 1H), 7.49-7.51 (m, 2H), 7.41 (t, *J =* 8.0 Hz, 1H), 7.17 (dd, *J* = 8.2, 2.7 Hz, 1H), 7.13 (s, 1H), 7.10 (t, *J* = 4.3 Hz, 1H), 4.91 (d, *J =* 4.1 Hz, 2H). MS(ESI): m/z 276 (M+1).

N-(2-(3-methoxyphenyl)-2-oxoethyl)pentanamide **(DKC1125d07)** ¹H-NMR (400 MHz, CDCl₃) *δ* 7.56 (dd, *J* = 6.4, 1.4 Hz, 1H), 7.49 (q, *J* = 1.4 Hz, 1H), 7.40 (t, *J* = 8.0 Hz, 1H), 7.16 (dq, *J* = 8.2, 1.2 Hz, 1H), 6.64 (s, 1H), 4.76 (d, *J* = 4.6 Hz, 2H), 3.86 (s, 3H), 2.32 (t, *J* = 7.8 Hz, 2H), 1.64-1.71 (m, 2H), 1.34-1.41 (m, 2H), 0.94 (t, *J* = 7.3 Hz, 3H). MS(ESI): m/z 250 (M+1).

N-(2-(3-methoxyphenyl)-2-oxoethyl)benzenesulfonamide (DKC1125d08) ¹H-NMR (400 MHz, CDCl₃) *δ* 7.89-7.92 (m, 2H), 7.48-7.58 (m, 3H), 7.34-7.42 (m, 3H), 7.14 (dq, *J* = 8.0, 1.2 Hz, 1H), 5.77 (t, *J* = 4.3 Hz, 1H), 4.47 (d, *J* = 4.6 Hz, 2H), 3.83 (s, 3H). MS(ESI): m/z 306 (M+1).

### Examples 130 to 136. Preparation of Compounds 130 to 129 (DKC1125e01 to 07) according to the Present Invention

### Synthesis of Compounds 130 to 136 (DKC1125e01 to 07)

Compounds 130 to 136 (DKC1125e01-07) of the present invention were synthesized according to the following Reaction Scheme 4.

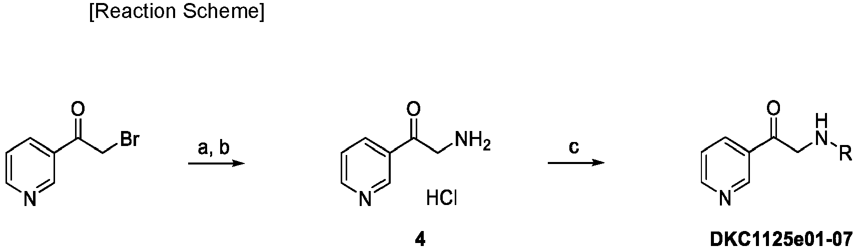

Reagent and conditions: (a) Hexamethylenetetramine, DCM, overnight; (b) HCl, EtOH, 80°C, 2h; (c) RCI, NaHCO₃, DCM, H₂O, 1h

### Synthesis of 2-amino-1-(pyridin-3-yl)ethan-1-one hydrochloride (4)

2-bromo-1-(pyridin-3-yl)ethan-1-one (5 g, 25.00 mmol) was dissolved in 25 mL of CH₂Cl₂ and treated with a solution of hexamethylenetetramine (3.50 g, 25.00 mmol) in 25 mL of CH₂Cl₂. After overnight at room temperature, the reaction product was cooled with an ice bath and the formed precipitate was washed with CH₂Cl₂ and EtOH to obtain the hexamethylenetetramine salt. The desired amine was obtained by treating the salt with a solution (90 mL) of concentrated hydrochloric acid and ethanol (1:2). The formed precipitate was filtered out and washed with water and then ethanol to obtain the title compound (1.30 g, 30%).

¹H-NMR (400 MHz, (CD₃)₂SO) *δ* 9.24 (t, *J* = 1.1 Hz, 1H), 8.93 (q, *J =* 2.3 Hz, 1H), 8.57 (s, 2H), 8.49 (dt, *J* = 8.1, 1.9 Hz, 1H), 7.73-7.76 (m, 1H), 4.67 (d, *J* = 5.5 Hz, 2H).

### General Preparation Method for Compounds 130 to 136 (DKC1125e01 to 07)

CH₂Cl₂ (1 mL) and saturated NaHCO₃ (2 mL) were stirred vigorously and chilled in an ice bath. Each acid chloride (0.29 mmol) described in Table 6 below was added, followed immediately by addition of 2-amino-1-(pyridin-3-yl)ethan-1-one hydrochloride (4, 50 mg, 0.29 mmol). Stirring was continued while warming to room temperature over a period of 1 hour. The organic layer was then separated, and dried with Na₂SO₄, and the solvent was evaporated. The residue was purified by column chromatography (yield: 30 to 40%).

**[Table 6]**

| Acid chlorides used in preparation of compounds 130 to 136 (DKC1125e01 to 07) | |
|---|---|
| **Compounds** | **Acid chloride** |
| **DKC1125e01** | 4-Chlorobenzoyl chloride |
| **DKC1125e02** | 2-Phenoxypropionyl chloride |
| **DKC1125e03** | 2-Fluorobenzoyl chloride |
| **DKC1125e04** | 4-Fluorobenzoyl chloride |
| **DKC1125e05** | Crotonyl chloride |
| **DKC1125e06** | o-Toluoyl chloride |
| **DKC1125e07** | Propionyl chloride |

### Confirmation of Preparation of Compounds 130 to 136 (DKC1125e01 to 07)

**4-chloro-N-(2-oxo-2-(pyridin-3-yl)ethyl)benzamide (DKC1125e01)** ¹H-NMR (400 MHz, CDCl₃) *δ* 9.23 (s, 1H), 8.84 (d, *J* = 3.7 Hz, 1H), 8.27 (dt, *J* = 7.9, 1.9 Hz, 1H), 7.80 (dt, *J* = 9.0, 2.1 Hz, 2H), 7.47 (dd, *J* = 8.0, 4.8 Hz, 1H), 7.42 (dt, *J* = 9.0, 2.1 Hz, 2H), 7.25 (s, 1H), 4.95 (d, *J* = 4.6 Hz, 2H). MS(ESI): m/z 276 (M+1).

**N-(2-oxo-2-(pyridin-3-yl)ethyl)-2-phenoxypropanamide (DKC1125e02)** ¹H-NMR (400 MHz, CDCl₃) *δ* 9.18 (d, *J =* 1.8 Hz, 1H), 8.83 (dd, *J* = 4.8, 1.6 Hz, 1H), 8.24 (dt, *J* = 7.8, 1.8 Hz, 1H), 7.45-7.51 (m, 2H), 7.31 (t, *J* = 8.0 Hz, 2H), 6.96-7.04 (m, 3H), 4.68-4.87 (m, 3H), 1.63 (d, *J* = 6.9 Hz, 3H). MS(ESI): m/z 285 (M+1).

**2-fluoro-N-(2-oxo-2-(pyridin-3-yl)ethyl)benzamide (DKC1125e03)** ¹H-NMR (400 MHz, CDCl₃) *δ* 9.27 (s, 1H), 8.87 (d, *J*= 3.7 Hz, 1H), 8.32 (dt, *J* = 8.2, 1.8 Hz, 1H), 8.11 (td, *J* = 8.0, 1.8 Hz, 1H), 7.84 (d, *J* = 11.9 Hz, 1H), 7.49-7.54 (m, 2H), 7.26-7.30 (m, 1H), 7.18 (ddd, *J* = 11.9, 8.2, 0.9 Hz, 1H), 5.02-5.03 (m, 2H). MS(ESI): m/z 259 (M+1).

**4-fluoro-N-(2-oxo-2-(pyridin-3-yl)ethyl)benzamide (DKC1125e04)** ¹H-NMR (400 MHz, CDCl₃) *δ* 9.22 (s, 1H), 8.83 (d, *J* = 3.7 Hz, 1H), 8.27 (dt, *J* = 8.2, 1.8 Hz, 1H), 7.85-7.88 (m, 2H), 7.46 (q, *J =* 4.1 Hz, 1H), 7.24 (d, *J* = 4.1 Hz, 1H), 7.09-7.13 (m, 2H), 4.94 (d, *J* = 4.6 Hz, 2H). MS(ESI): m/z 259 (M+1).

**(E)-N-(2-oxo-2-(pyridin-3-yl)ethyl)but-2-enamide (DKC1125e05)** ¹H-NMR (400 MHz, CDCl₃) *δ* 9.32-9.09 (1H), 8.92-8.71 (1H), 8.35-8.16 (1H), 7.60-7.35 (1H), 6.96-6.84 (1H), 6.00-5.89 (1H), 4.88-4.81 (2H), 1.92-1.75 (3H). MS(ESI): m/z 205 (M+1).

**2-methyl-N-(2-oxo-2-(pyridin-3-yl)ethyl)benzamide (DKC1125e06)** ¹H-NMR (400 MHz, CDCl₃) *δ* 9.24 (d, *J* = 1.8 Hz, 1H), 8.86 (dd, *J* = 4.8, 1.1 Hz, 1H), 8.30 (dt, *J* = 7.9, 1.9 Hz, 1H), 7.50 (q, *J* = 4.1 Hz, 2H), 7.34-7.38 (m, 1H), 7.23-7.27 (m, 2H), 6.88 (s, 1H), 4.98 (d, *J* = 4.6 Hz, 2H), 2.50 (s, 3H). MS(ESI): m/z 255 (M+1).

**N-(2-oxo-2-(pyridin-3-yl)ethyl)propionamide (DKC1125e07)** ¹H-NMR (400 MHz, CDCl₃) *δ* 9.21 (d, *J* = 2.3 Hz, 1H), 8.85 (dd, *J* = 4.8, 1.6 Hz, 1H), 8.27 (dt, *J* = 8.1, 1.9 Hz, 1H), 7.46-7.50 (m, 1H), 6.57 (s, 1H), 4.80 (d, *J* = 4.1 Hz, 2H), 2.37 (q, *J* = 7.6 Hz, 2H), 1.20-1.26 (m, 3H). MS(ESI): m/z 193 (M+1).

### Examples 137 to 150. Preparation of Compounds 137 to 150 (DKC1125f01 to 14) according to the Present Invention

### Synthesis of Compounds 137-150 (DKC1125f01 to 14)

Compounds 137 to 140 (DKC1125f01 to 14) of the present invention were synthesized according to the following Reaction Scheme 5.

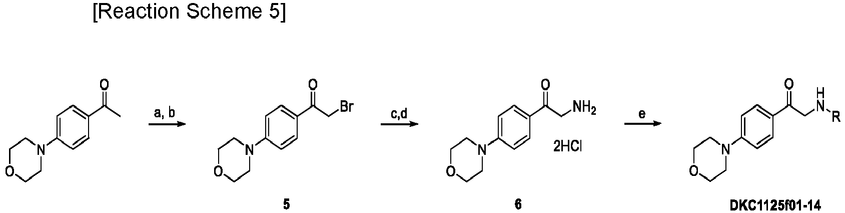

Reagent and conditions: : (a) HBr, Br₂, (b) NaHCO₃, H₂O, (c) Potassium phthalimide, DMF, (d) HCl, MeOH, 140°C, 2h; (e) RCI, NaHCO₃, DCM, H₂O, 1h

### Synthesis of 2-bromo-1 -(4-morpholinophenyl)ethan-1 -one (5)

To a solution of 1-(4-morpholinophenyl)ethan-1-one (5g, 24.36 mmol) in HBr (100 mL) at 0°C was added Br₂ (3.89g, 24.36 mmol) dropwise over 1 hour. After the reaction was complete, the product was extracted twice with CH₂Cl₂ and washed with saturated NaHCO₃. The organic layer was dried with MgSO₄, and the solvent was evaporated. The resulting crude product was purified by column chromatography to give compound (1) of Reaction Scheme 5 (6.23 g, 90%).

¹H NMR (400 MHz, CDCl₃) δ 7.95 (d, *J* = 4.0 Hz, 2H), 7.04 (d, *J* = 4.0 Hz, 2H), 4.60 (s, 2H), 3.83 (m, 4H), 3.65 (m, 4H).

### Synthesis of 2-Amino-1-[4-(morpholin-4-yl)phenyl]ethan-1-one dihydrochloride (6)

The 2-bromo-1-(4-morpholinophenyl)ethan-1-one **(5,** 6 g, 21.11 mmol) was dissolved in 20 mL of dry potassium phthalimide solution (4.30 g, 23.22 mmol), and held at 75°C for 18 hrs. The suspension was quenched in 90 mL of water with stirring, which was maintained for 40 min, and the product was collected, washed with 50 mL of water, and recrystallized from 50 mL of acetonitrile at 0°C overnight. (4.58 g, 60%). The solid was dissolved in a solution (90 mL) of concentrated HCl and EtOH (1:2). The reaction product was boiled under reflux for 2 hrs. The precipitate was filtered out and washed with water and then EtOH (3.79 g, 70%).

¹H-NMR (400 MHz, (CD₃)₂SO) *δ* 1.87(d, *J* = 9.0 Hz, 2H), 7.03(d, *J* = 9.0 Hz, 2H), 4.45(d, *J* = 4.5 Hz, 2H), 3.73(dd, *J* = 4.0, 5.0 Hz, 4H), 3.35(dd, *J* = 4.0, 5.0 Hz, 4H).

### General Preparation Method for Compounds 137 to 150 (DKC1125f01 to 14)

CH₂Cl₂ (1 mL) and saturated NaHCO₃ (2 mL) were stirred vigorously and chilled in an ice bath. Each acid chloride (0.29 mmol) shown in Table 7 below was added, followed immediately by addition of 2-amino-1-(4-morpholinophenyl)ethan-1-one dihydrochloride **(6,** 50 mg, 0.29mmol). Stirring was continued while warming to room temperature over a period of 1 hr. The organic layer was then separated, and dried with Na₂SO₄, and the solvent was evaporated. The residue was purified by column chromatography (yield: 65 to 80%).

**[Table 7]**

| Acid chlorides used in preparation of compounds 137 to 150 (DKC1125f01 to 14) | |
|---|---|
| **Compounds** | **Acid chloride** |
| **DCK1125f01** | 2-Fluorobenzoyl chloride |
| **DCK1125f02** | 2-Chlorobenzoyl Chloride |
| **DCK1125f03** | Crotonyl chloride |
| **DCK1125f04** | 3-Methoxybenzoyl chloride |
| **DCK1125f05** | m-Toluoyl chloride |
| **DCK1125f06** | Propionyl chloride |
| **DCK1125f07** | Cyclopropanecarbonylchloride |
| **DCK1125f08** | Cyclobutanecarbonyl chloride |
| **DCK1125f09** | Pentanoyl chloride |
| **DCK1125f10** | 4-Fluorobenzenesulfonyl chloride |
| **DCK1125f11** | 4-Chlorobenzenesulfonyl chloride |
| **DCK1125f12** | Benzenesulfonyl chloride |
| **DCK1125f13** | a-Toluenesulfonyl chloride |
| **DCK1125f14** | 4-Methoxybenzenesulfonyl chloride |

### Confirmation of Preparation of Compounds 137 to 150 (DKC1125f01 to 14)

**2-fluoro-N-(2-(4-morpholinophenyl)-2-oxoethyl)benzamide (DKC1125f01)** ¹H-NMR (400 MHz, CDCl₃) *δ* 8.13 (td, *J* = 7.8, 1.8 Hz, 1H), 7.95-7.99 (m, 3H), 7.48-7.53 (m, 1H), 7.26-7.30 (m, 1H), 7.16-7.21 (m, 1H), 6.91 (dt, *J* = 9.6, 2.4 Hz, 2H), 4.92 (dd, *J* = 4.1, 0.9 Hz, 2H), 3.88 (t, *J* = 5.0 Hz, 4H), 3.36 (t, *J* = 4.8 Hz, 4H). MS(ESI): m/z 343 (M+1).

**2-chloro-N-(2-(4-morpholinophenyl)-2-oxoethyl)benzamide (DKC1125f02)** ¹H-NMR (400 MHz, CDCl₃) *δ* 7.94 (dt, *J* = 9.6, 2.4 Hz, 2H), 7.73 (dd, *J* = 7.8, 1.8 Hz, 1H), 7.49 (s, 1H), 7.32-7.45 (m, 3H), 6.90 (dd, *J* = 11.9, 2.7 Hz, 2H), 4.90 (d, *J* = 4.6 Hz, 2H), 3.86 (t, *J* = 5.0 Hz, 4H), 3.36 (t, *J* = 5.0 Hz, 4H). MS(ESI): m/z 360 (M+1).

**(E)-N-(2-(4-morpholinophenyl)-2-oxoethyl)but-2-enamide (DKC1125f03)** ¹H-NMR (400 MHz, CDCl₃) *δ* 7.92 (d, *J* = 8.7 Hz, 2H), 6.87-6.95 (m, 3H), 6.65 (s, 1H), 5.96 (dd, *J* = 15.3, 1.6 Hz, 1H), 4.75 (d, *J* = 4.1 Hz, 2H), 3.86 (t, *J* = 4.8 Hz, 4H), 3.35 (t, *J* = 4.8 Hz, 4H), 1.89 (d, *J* = 6.9 Hz, 3H). MS(ESI): m/z 289 (M+1).

**4-methoxy-N-(2-(4-morpholinophenyl)-2-oxoethyl)benzamide (DKC1125f04)** ¹H-NMR (400 MHz, CDCl₃) *δ* 7.96 (d, *J* = 9.1 Hz, 2H), 7.33-7.45 (m, 4H), 7.06-7.08 (m, 1H), 6.91 (d, *J* = 8.7 Hz, 2H), 4.87 (d, *J* = 4.1 Hz, 2H), 3.86-3.88 (m, 8H), 3.36 (t, *J* = 5.0 Hz, 3H). MS(ESI): m/z 355 (M+1).

**3-methyl-N-(2-(4-morpholinophenyl)-2-oxoethyl)benzamide (DKC1125f05)** ¹H-NMR (400 MHz, CDCl₃) *δ* 7.97 (d, *J* = 9.1 Hz, 2H), 7.69 (dd, *J* = 8.9, 6.6 Hz, 2H), 7.35-7.38 (m, 3H), 6.92 (d, *J* = 8.7 Hz, 2H), 4.88 (d, *J* = 4.1 Hz, 2H), 3.88 (t, *J* = 4.8 Hz, 4H), 3.37 (t, *J* = 4.8 Hz, 4H), 2.43 (s, 3H). MS(ESI): m/z 339 (M+1).

**N-(2-(4-morpholinophenyl)-2-oxoethyl)propionamide (DKC1125f06)** ¹H-NMR (400 MHz, CDCl₃) *δ* 7.89-7.93 (m, 2H), 6.89 (dt, *J* = 11.9, 2.3 Hz, 2H), 6.65 (s, 1H), 4.69 (d, *J* = 4.1 Hz, 2H), 3.87 (t, *J* = 5.0 Hz, 4H), 3.35 (t, *J* = 5.0 Hz, 4H), 2.35 (q, *J* = 7.6 Hz, 2H), 1.22 (t, *J* = 7.5 Hz, 4H). MS(ESI): m/z 377 (M+1).

**N-(2-(4-morpholinophenyl)-2-oxoethyl)cyclopropanecarboxamide (DKC1125f07)** ¹H-NMR (400 MHz, CDCl₃) *δ* 7.92 (d, *J* = 9.1 Hz, 2H), 6.89 (d, *J* = 9.1 Hz, 2H), 6.81 (s, 1H), 4.71 (d, *J* = 4.1 Hz, 2H), 3.87 (t, *J* = 5.0 Hz, 4H), 3.35 (t, *J* = 5.0 Hz, 4H), 1.52-1.58 (m, 1H), 0.99-1.03 (m, 2H), 0.80 (dt, *J* = 11.6, 3.4 Hz, 2H). MS(ESI): m/z 289 (M+1).

**N-(2-(4-morpholinophenyl)-2-oxoethyl)cyclobutanecarboxamide (DKC1125f08)** ¹H-NMR (400 MHz, CDCl₃) *δ* 7.90-7.93 (m, 2H), 6.89 (dd, *J* = 11.9, 2.7 Hz, 2H), 6.56 (s, 1H), 4.68 (d, *J* = 4.1 Hz, 2H), 3.87 (t, *J* = 5.0 Hz, 4H), 3.35 (t, *J* = 5.0 Hz, 4H), 3.11-3.19 (m, 1H), 2.29-2.39 (m, 2H), 2.17-2.25 (m, 2H), 1.88-2.06 (m, 2H). MS(ESI): m/z 303 (M+1).

**N-(2-(4-morpholinophenyl)-2-oxoethyl)pentanamide (DKC1125f09)** ¹H-NMR (400 MHz, CDCl₃) *δ* 7.91 (d, *J* = 9.1 Hz, 2H), 6.89 (d, *J* = 9.1 Hz, 2H), 6.64 (s, 1H), 4.69 (d, *J* = 4.1 Hz, 2H), 3.87 (t, *J* = 4.8 Hz, 4H), 3.35 (t, *J* = 5.0 Hz, 4H), 2.32 (t, *J* = 7.5 Hz, 2H), 1.64-1.72 (m, 2H), 1.36-1.44 (m, 2H), 0.94 (t, *J* = 7.3 Hz, 3H). MS(ESI): m/z 305 (M+1).

**4-fluoro-N-(2-(4-morpholinophenyl)-2-oxoethyl)benzenesulfonamide (DKC1125f10)** ¹H-NMR (400 MHz, CDCl₃) *δ* 7.92 (qd, *J* = 4.8, 2.5 Hz, 2H), 7.76-7.79 (m, 2H), 7.15-7.20 (m, 2H), 6.83-6.86 (m, 2H), 4.38 (d, *J* = 4.1 Hz, 2H), 3.86 (t, *J* = 4.8 Hz, 4H), 3.34 (t, *J* = 5.0 Hz, 4H). MS(ESI): m/z 379 (M+1).

**4-chloro-N-(2-(4-morpholinophenyl)-2-oxoethyl)benzenesulfonamide (DKC1125f11)** ¹H-NMR (400 MHz, CDCl₃) *δ* 7.82-7.92 (m, 2H), 7.75-7.78 (m, 2H), 7.47 (dt, *J* = 9.1, 2.3 Hz, 2H), 6.82-6.85 (m, 2H), 5.78 (t, *J* = 4.3 Hz, 1H), 4.38 (d, *J* = 4.6 Hz, 2H), 3.84-3.89 (m, 4H), 3.34 (t, *J* = 5.0 Hz, 4H). MS(ESI): m/z 396 (M+1).

**N-(2-(4-morpholinophenyl)-2-oxoethyl)benzenesulfonamide (DKC1125f12)** ¹H-NMR (400 MHz, CDCl₃) *δ* 7.89-7.94 (m, 2H), 7.77 (dd, *J* = 7.1, 2.1 Hz, 2H), 7.48-7.57 (m, 4H), 6.84 (d, *J* = 9.1 Hz, 2H), 4.39 (d, *J* = 4.6 Hz, 2H), 3.84-3.88 (m, 5H), 3.33 (t, *J* = 5.0 Hz, 4H). MS(ESI): m/z 361 (M+1).

**N-(2-(4-morpholinophenyl)-2-oxoethyl)-1-phenylmethanesulfonamide (DKC1125f13)** ¹H-NMR (400 MHz, CDCl₃) *δ* 7.72 (dt, *J* = 9.6, 2.4 Hz, 2H), 7.40-7.45 (m, 2H), 7.33-7.37 (m, 3H), 6.83-6.86 (m, 2H), 4.33 (s, 2H), 4.25 (d, *J* = 4.6 Hz, 2H), 3.87 (t, *J* = 4.8 Hz, 4H), 3.35 (t, *J* = 5.0 Hz, 4H). MS(ESI): m/z 375 (M+1).

**4-methoxy-N-(2-(4-morpholinophenyl)-2-oxoethyl)benzenesulfonamide (DKC1125f14)** ¹H-NMR (400 MHz, CDCl₃) *δ* 7.82 (dt, *J* = 9.5, 2.4 Hz, 2H), 7.76 (d, *J* = 9.1 Hz, 2H), 6.94 (dt, *J* = 9.6, 2.5 Hz, 2H), 6.83 (d, *J* = 9.1 Hz, 2H), 5.72 (t, *J* = 4.1 Hz, 1H), 4.35 (d, *J* = 4.6 Hz, 2H), 3.85 (dd, *J* = 8.9, 3.9 Hz, 7H), 3.32 (t, *J* = 5.0 Hz, 4H). MS(ESI): m/z 391 (M+1).

### Examples 151 to 200. Preparation of Compounds 151 to 200 (DKC1125g01 to 50) according to the Present Invention

### Synthesis of Compounds 151 to 200 (DKC1125g01 to 50)

Compounds 151 to 200 (DKC1125g01 to 50) of the present invention were synthesized according to the following Reaction Scheme 6.

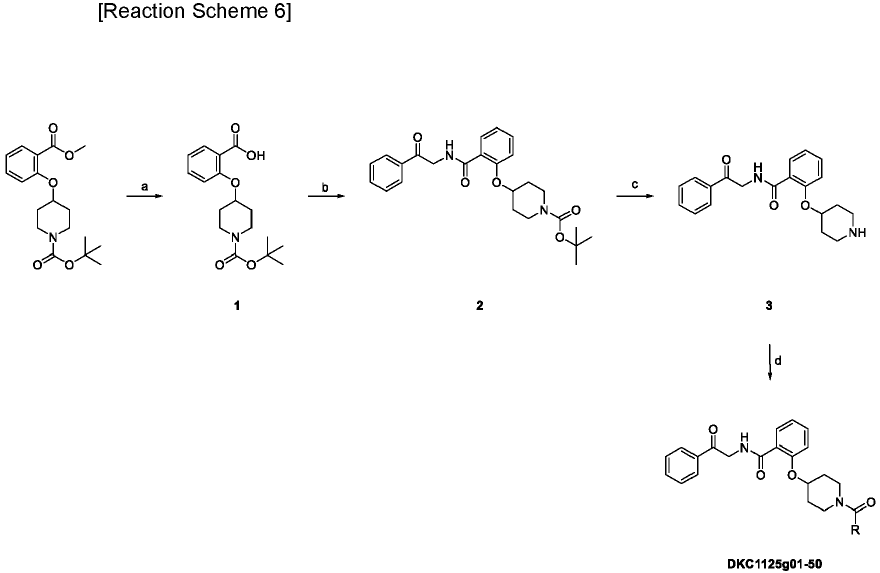

Reagent and conditions. (a)NaOH, H₂O, MeOH, 65°C, 6h; (b)2-amino-1-phenylethan-1-one, HATU, DIEA, rt, 10h; (c)10 % TFA/DCM, rt, 2 h; (d)RCI, Et₃N, CH₂Cl₂, rt, 4h.

### Synthesis of 2-((1-(tert-butoxycarbonyl)) piperidin-4-yl) oxy) benzoic acid (1)

To a solution of tert-butyl 4-(2-(methoxycarbonyl)phenoxy)piperidine-1-carboxylate (2.0 g, 5.69 mol) in methanol (20 mL) was added 4N NaOH (6 mL). The resulting solution was stirred at 65°C for 6 hrs. The reaction monitored by checking TLC. After completion of the reaction, to the resulting mixture added water and HCl to maintain acidic pH, followed by extraction with EA. The organic layer was separated and washed with brine (3 x 100 mL). The washed solution was dried with anhydrous Na₂SO₄, and the solvent was evaporated in vacuum. The residue was purified by column chromatography (EtOAc:Hexane 1:1) to give the title compound (1) as white powder (1.8 g, 90%).

¹H-NMR (400 MHz, CDCl₃) *δ* 8.20 (dd, *J* = 7.8, 1.8 Hz, 1H), 7.52-7.57 (m, 1H), 7.12-7.17 (m, 1H), 7.06 (d, *J* = 8.2 Hz, 1H), 4.73-4.79 (m, 1H), 4.09-4.14 (m, 1H), 3.83 (q, *J* = 4.4 Hz, 2H), 3.29 (tt, *J* = 13.3, 4.4 Hz, 2H), 2.04 (d, *J* = 3.7 Hz, 8H), 1.84 (tt, *J* = 13.0, 4.3 Hz, 2H), 1.47 (s, 9H). MS (ESI): m/z 322 (M +1).

### Synthesis of tert-butyl 4-(2-((2-oxo-2-phenylethyl) carbonyl) phenoxy) piperidine-1-carboxylate (2)

To a solution of 2-((1-(tert-butoxycarbonyl))piperidin-4-yl)oxy)benzoic acid (1, 1.0 g, 3.11 mol) (1.0 g, 3.11 mol) and 2-amino-1-phenylethan-1-one (534 mg, 3.11 mol) in DMF (10 mL) was added DIEA (1.3 mL, 7.78 mol) with stirring, followed by addition of HATU (1.1 g, 3.11 mmol). The resulting solution was stirred at room temperature for 10 hrs. The reaction was monitored by checking TLC. After completions of reaction, to the resulting mixture was added water, followed by extraction with EA. The organic layer was separated and washed with brine (3 x100 mL). The washed solution was dried with anhydrous sodium sulphate, and the solvent was evaporated. The residue was purified by column chromatography (EtOAc : Hexane 2:3) to give the title compound (2) as white powder (1.3 g, 76%).

¹H-NMR (400 MHz, CDCl₃) *δ* 9.00 (s, 1H), 8.24 (dd, *J* = 8.0, 2.1 Hz, 1H), 7.63 (tt, *J* = 7.4, 1.4 Hz, 2H), 7.50-7.54 (m, 2H), 7.44 (dd, *J* = 8.7, 1.4 Hz, 2H), 7.02-7.10 (m, 2H), 5.01 (d, *J* = 3.7 Hz, 2H), 4.66-4.71 (m, 1H), 3.95 (s, 2H), 3.17-3.23 (m, 2H), 2.96 (d, *J* = 5.5 Hz, 4H), 1.47 (s, 9H). MS (ESI): m/z 439 (M +1).

### Synthesis of N-(2-oxo-2-phenylethyl)-2-(piperidin-4-yloxy) benzamine (3)

To a solution of tert-butyl **4**-(2-((2-oxo-2-phenylethyl) carbonyl) phenyl) piperidine-1-carboxylate **(2,** 1.0 g, 3.11 mol) in anhydrous dichloromethane (20 mL) was added trifluoroacetic acid (1.3 mL, 7.78 mol) under nitrogen gas. The resulting solution was stirred at room temperature for 2 hrs. After completion of the reaction, the solvent was evaporated from the mixture, and the residue was purified by column chromatography to give the title compound (3) as a yellow solid (0.9 g, 90%).

¹H-NMR (400 MHz, CDCl₃) *δ* 9.12 (s, 1H), 8.25 (dd, *J* = 7.8, 1.8 Hz, 1H), 8.03-8.05 (m, 2H), 7.61-7.65 (m, 1H), 7.50-7.54 (m, 2H), 7.44 (dd, *J* = 8.7, 1.4 Hz, 1H), 7.03-7.09 (m, 2H), 5.30 (s, 1H), 5.02 (d, *J* = 4.1 Hz, 2H), 4.61-4.68 (m, 1H), 3.26 (td, *J* = 8.7, 4.3 Hz, 2H), 2.78-2.84 (m, 2H), 2.17-2.25 (m, 4H). MS (ESI): m/z 339 (M +1).

### General Preparation Method for Compounds 150 to 200 (DKC1125g01 to 50)

To a solution of N-(2-oxo-2-phenylethyl)-2-(piperidin-4-yloxy)benzamine (3, 10 mg ,0.029 mmol) and each acid chloride (10 µL, 0.07 mmol) shown in Table 8 below in dichloromethane was added triethylamine (3.7 µL, 0.029 mmol). The reaction mixture was stirred at room temperature for 1 hr. After completion of the reaction, the mixture was evaporated and purified by column chromatography to give (EtOAc : Hexane 1:2) to obtain the title compound as a solid (12 mg, 83%).

**[Table 8]**

| Acid chlorides used in preparation of compounds 150 to 200 (DKC1125g01 to 50) | | | | |
|---|---|---|---|---|
| **Compounds** | **Acid Chloride** | | **Compounds** | **Acid Chloride** |
| **DKC1125g01** | 4-Chlorobenzoyl chloride | | **DKC1125g26** | O-Acetylsalicyloyl chloride |
| **DKC1125g02** | 2-Phenoxypropionyl chloride | | **DKC1125g27** | 2,4-Difluorobenzoyl chloride |
| **DKC1125g03** | 2-Fluorobenzoyl chloride | | **DKC1125g28** | Methoxyacetyl chloride |
| **DKC1125g04** | 4-Fluorobenzoyl chloride | | **DKC1125g29** | Pivaloyl chloride |
| **DKC1125g05** | 2-Furoyl chloride | | **DKC1125g30** | Dimethylcarbamyl chloride |
| **DKC1125g06** | 2-Chlorobenzoyl Chloride | | **DKC1125g31** | 4-Bromobenzoyl chloride |
| **DKC1125g07** | 3-Chlorobenzoyl chloride | | **DKC1125g32** | Acetyl chloride |
| **DKC1125g08** | Crotonyl chloride | | **DKC1125g33** | Methanesulfonyl chloride |
| **DKC1125g09** | 3-Phenylpropionyl chloride | | **DKC1125g34** | 4-Fluorobenzenesulfonyl chloride |
| **DKC1125g10** | Acryloyl chloride | | **DKC1125g35** | p-Toluenesulfonyl chloride |
| **DKC1125g11** | o-Toluoyl chloride | | **DKC1125g36** | 4-Chlorobenzenesulfonyl chloride |
| **DKC1125g12** | 3-Methoxybenzoyl chloride | | **DKC1125g37** | Benzenesulfonyl chloride |
| **DKC1125g13** | m-Toluoyl chloride | | **DKC1125g38** | a-Toluenesulfonyl chloride |
| **DKC1125g14** | Propionyl chloride | | **DKC1125g39** | 4-Methoxybenzenesulfonyl chloride |
| **DKC1125g15** | Cyclohexanecarbonyl chloride | | **DKC1125g40** | 3,5-Bis(trifluoromethyl)benzoyl chloride |
| **DKC1125g16** | Cyclopropanecarbonylchloride | | **DKC1125g41** | 2-(Trifluoromethyl)benzoyl chloride |
| **DKC1125g17** | Cyclobutanecarbonyl chloride | | **DKC1125g42** | 3-(Trifluoromethyl)benzoyl chloride |
| **DKC1125g18** | Thiophene-2-carbonyl chloride | | **DKC1125g43** | 4-(Trifluoromethyl)benzoyl chloride |
| **DKC1125g19** | 2-Ethoxybenzoyl chloride | | **DKC1125g44** | 2-Chloro-4-fluorobenzoyl chloride |
| **DKC1125g20** | tert-butylacetyl chloride | | **DKC1125g45** | 3-Nitrobenzoyl chloride |
| **DKC1125g21** | 4-Morpholinecarbonyl chloride | | **DKC1125g46** | 3,4-Difluorobenzoyl chloride |
| **DKC1125g22** | Pentanoyl chloride | | **DKC1125g47** | 3,5-Dichlorobenzoyl chloride |
| **DKC1125g23** | 4-Methoxybenzoyl chloride | | **DKC1125g48** | 2,3-Dichlorobenzoyl chloride |
| **DKC1125g24** | 4-Cyanobenzoyl chloride | | **DKC1125g49** | 4-(Trifluoromethoxy)benzoyl chloride |
| **DKC1125g25** | 3-nitrobenzene-1-sulfonyl chloride | | **DKC1125g50** | 3-(Trifluoromethoxy)benzoyl chloride |

### Confirmation of Preparation of Compounds 150 to 200 (DKC1125g01 to 50)

**2-((1-(4-chlorobenzoyl)piperidin-4-yl)oxy)-N-(2-oxo-2-phenylethyl)benzamide (DKC1125g01)** ¹H-NMR (400 MHz, CDCl₃) *δ* 8.98 (s, 1H), 8.25 (dd, *J* = 7.8, 1.8 Hz, 1H), 8.00-8.08 (m, 2H), 7.63-7.67 (m, 1H), 7.61-7.65 (m, 1H), 7.53-7.57 (m, 2H), 7.36-7.43 (m, 2H), 7.28-7.32 (m, 2H), 7.03-7.12 (m, 2H), 5.02 (d, *J* = 4.1 Hz, 2H), 4.61-4.68 (m, 1H), 3.26 (td, *J* = 8.7, 4.3 Hz, 2H), 2.78-2.84 (m, 2H), 2.17-2.25 (m, 4H). MS (ESI): m/z 477 (M +1).

**N-(2-oxo-2-phenylethyl)-2-((1-(2-phenoxypropanoyl)piperidin-4-yl)oxy)benzamide (DKC1125g02)** ¹H-NMR (400 MHz, CDCl₃) *δ* 8.96 (s, 1H), 8.20 (dd, *J* = 7.8, 1.8 Hz, 1H), 7.80-8.91 (m, 2H), 7.64-7.68 (m, 2H), 7.53-7.57 (m, 2H), 7.36-7.43 (m, 2H), 7.28-7.34 (m, 2H), 7.03-7.12 (m, 2H), 5.01 (d, *J* = 4.1 Hz, 2H), 4.7-4.9 (m, 1H), 4.61-4.68 (m, 1H), 3.26 (td, *J* = 8.7, 4.3 Hz, 2H), 2.87 (s, 3H), 2.78-2.84 (m, 2H), 2.17-2.25 (m, 4H). MS (ESI): m/z 487 (M +1).

**2-((1-(2-fluorobenzoyl)piperidin-4-yl)oxy)-N-(2-oxo-2-phenylethyl)benzamide (DKC1125g03)** ¹H-NMR (400 MHz, CDCl₃) *δ* 8.99 (s, 1H), 8.28 (dd, *J* = 7.8, 1.8 Hz, 1H), 8.30-8.08 (m, 2H), 7.68-7.69 (m, 2H), 7.53-7.57 (m, 2H), 7.36-7.43 (m, 2H), 7.28-7.34 (m, 2H), 7.03-7.12 (m, 2H), 5.02 (d, *J* = 4.1 Hz, 2H), 4.61-4.68 (m, 1H), 3.26 (td, *J* = 8.7, 4.3 Hz, 2H), 2.78-2.84 (m, 2H), 2.17-2.25 (m, 4H). MS (ESI): m/z 461 (M +1).

**2-((1-(4-fluorobenzoyl)piperidin-4-yl)oxy)-N-(2-oxo-2-phenylethyl)benzamide (DKC1125g04)** ¹H-NMR (400 MHz, CDCl₃) *δ* 8.98 (s, 1H), 8.25 (dd, *J* = 7.8, 1.8 Hz, 1H), 8.00-8.08 (m, 2H), 7.63-7.67 (m, 2H), 7.54-7.58 (m, 2H), 7.36-7.43 (m, 2H), 7.28-7.34 (m, 2H), 7.06-7.14 (m, 2H), 5.02 (d, *J* = 4.1 Hz, 2H), 4.61-4.68 (m, 1H), 3.26 (td, *J* = 8.7, 4.3 Hz, 2H), 2.78-2.84 (m, 2H), 2.17-2.25 (m, 4H). MS (ESI): m/z 461 (M +1).

**2-((1-(furan-2-carbonyl)piperidin-4-yl)oxy)-N-(2-oxo-2-phenylethyl)benzamide (DKC1125g05)** ¹H-NMR (400 MHz, CDCl₃) *δ* 8.98 (s, 1H), 8.25 (dd, *J* = 7.8, 1.8 Hz, 1H), 8.10-8.18 (m, 2H), 7.63-7.67 (m, 2H), 7.53-7.57 (m, 2H), 7.36-7.43 (m, 2H), 7.28-7.32 (m, 2H), 7.03-7.12 (m, 1H), 5.02 (d, *J* = 4.1 Hz, 2H), 4.61-4.68 (m, 1H), 3.26 (td, *J* = 8.7, 4.3 Hz, 2H), 2.78-2.84 (m, 2H), 2.17-2.25 (m, 4H). MS (ESI): m/z 433 (M +1).

**2-((1-(2-chlorobenzoyl)piperidin-4-yl)oxy)-N-(2-oxo-2-phenylethyl)benzamide (DKC1125g06)** ¹H-NMR (400 MHz, CDCl₃) *δ* 8.98 (s, 1H), 8.25 (dd, *J* = 7.8, 1.8 Hz, 1H), 8.00-8.08 (m, 2H), 7.61-7.65 (m, 2H), 7.53-7.57 (m, 2H), 7.36-7.43 (m, 2H), 7.28-7.32 (m, 2H), 7.06-7.12 (m, 2H), 5.02 (d, *J* = 4.1 Hz, 2H), 4.61-4.68 (m, 1H), 3.26 (td, *J* = 8.7, 4.3 Hz, 2H), 2.78-2.84 (m, 2H), 2.17-2.25 (m, 4H). MS (ESI): m/z 477 (M +1).

**2-((1-(3-chlorobenzoyl)piperidin-4-yl)oxy)-N-(2-oxo-2-phenylethyl)benzamide (DKC1125g07)** ¹H-NMR (400 MHz, CDCl₃) *δ* 8.98 (s, 1H), 8.25 (dd, *J* = 7.8, 1.8 Hz, 1H), 8.00-8.08 (m, 2H), 7.63-7.67 (m, 1H), 7.61-7.65 (m, 1H), 7.53-7.57 (m, 2H), 7.36-7.43 (m, 2H), 7.20-7.30 (m, 2H), 7.03-7.12 (m, 2H), 5.02 (d, *J* = 4.1 Hz, 2H), 4.61-4.68 (m, 1H), 3.26 (td, *J* = 8.7, 4.3 Hz, 2H), 2.78-2.84 (m, 2H), 2.17-2.25 (m, 4H). MS (ESI): m/z 477 (M +1).

**(E)-2-((1-(but-2-enoyl)piperidin-4-yl)oxy)-N-(2-oxo-2-phenylethyl)benzamide (DKC1125g08)** ¹H-NMR (400 MHz, CDCl₃) *δ* 9.12 (s, 1H), 8.20 (dd, *J* = 7.8, 1.8 Hz, 1H), 8.08-8.12 (m, 2H), 7.61-7.65 (m, 1H), 7.50-7.54 (m, 2H), 7.44 (dd, *J* = 8.7, 1.4 Hz, 1H), 7.03-7.09 (m, 2H), 5.30 (s, 1H), 5.02 (d, *J* = 4.1 Hz, 2H), 4.61-4.68 (m, 1H), 3.26 (td, *J* = 8.7, 4.3 Hz, 2H), 2.88 (s, 1H), 2.78-2.84 (m, 1H), 2.6 (s, 3H), 2.17-2.25 (m, 4H), 2.16 (s, 1 H), 1.94-2.03 (m, 2H). MS (ESI): m/z 407 (M +1).

**N-(2-oxo-2-phenylethyl)-2-((1-(3-phenylpropanoyl)piperidin-4-yl)oxy)benzamide (DKC1125g09)** ¹H-NMR (400 MHz, CDCl₃) *δ* 8.95 (s, 1H), 8.25 (dd, *J* = 7.8, 1.8 Hz, 1H), 8.02-8.08 (m, 2H), 7.63-7.67 (m, 1H), 7.61-7.65 (m, 2H), 7.53-7.57 (m, 2H), 7.36-7.43 (m, 2H), 7.22-7.32 (m, 2H), 7.03-7.12 (m, 2H), 5.01 (d, *J* = 4.1 Hz, 2H), 4.60-4.64 (m, 1H), 3.26 (td, *J* = 8.7, 4.3 Hz, 2H), 2.78-2.84 (m, 2H), 2.58-2.70 (m, 2H), 2.54 (d, *J* = 2.4 Hz, 2H), 2.17-2.25 (m, 4H). MS (ESI): m/z 471 (M +1).

**2-((1-acryloylpiperidin-4-yl)oxy)-N-(2-oxo-2-phenylethyl)benzamide (DKC1125g10)** ¹H-NMR (400 MHz, CDCl₃) *δ* 9.12 (s, 1H), 8.25 (dd, *J* = 7.8, 1.8 Hz, 1H), 8.03-8.05 (m, 2H), 7.61-7.65 (m, 1H), 7.50-7.54 (m, 2H), 7.42 (dd, *J* = 8.6, 1.3 Hz, 1H), 7.03-7.09 (m, 2H), 6.20 (s, 2H), 5.02 (s, 2H), 4.61-4.68 (m, 1H), 3.26 (td, *J* = 8.7, 4.3 Hz, 2H), 2.6 (s, 2H), 2.17-2.25 (m, 4H), 1.94-2.03 (m, 3H). MS (ESI): m/z 393 (M +1).

**2-((1-(2-methylbenzoyl)piperidin-4-yl)oxy)-N-(2-oxo-2-phenylethyl)benzamide (DKC1125g11)** ¹H-NMR (400 MHz, CDCl₃) *δ* 8.93 (s, 1H), 8.20 (dd, *J* = 7.4, 1.8 Hz, 1H), 8.02-8.06 (m, 2H), 7.62-7.64 (m, 1H), 7.60-7.63 (m,, 1H), 7.53-7.57 (m, 2H), 7.36-7.43 (m, 2H), 7.20-7.30 (m, 2H), 7.03-7.12 (m, 2H), 5.02 (d, *J* = 4.1 Hz, 2H), 4.61-4.68 (m, 1H), 3.26 (td, *J* = 8.7, 4.3 Hz, 2H), 2.9 (s, 3H), 2.78-2.84 (m, 2H), 2.17-2.25 (m, 4H). MS (ESI): m/z 457 (M+1).

**2-((1-(2-methoxybenzoyl)piperidin-4-yl)oxy)-N-(2-oxo-2-phenylethyl)benzamide (DKC1125g12)** ¹H-NMR (400 MHz, CDCl₃) *δ* 8.90 (s, 1H), 8.24 (dd, *J* = 7.8, 1.8 Hz, 1H), 8.01-8.07 (m, 2H), 7.64-7.69 (m, 1H), 7.61-7.65 (m, 1H), 7.53-7.57 (m, 2H), 7.36-7.43 (m, 2H), 7.20-7.30 (m, 2H), 7.03-7.12 (m, 2H), 5.02 (d, *J* = 4.1 Hz, 2H), 4.61-4.68 (m, 1H), 3.9 (s, 3H), 3.26 (td, *J* = 8.7, 4.3 Hz, 2H), 2.78-2.84 (m, 2H), 2.17-2.25 (m, 4H). MS (ESI): m/z 473 (M +1).

**2-((1-(3-methylbenzoyl)piperidin-4-yl)oxy)-N-(2-oxo-2-phenylethyl)benzamide (DKC1125g13)** ¹H-NMR (400 MHz, CDCl₃) *δ* 8.98 (s, 1H), 8.25 (dd, *J* = 7.8, 1.8 Hz, 1H), 8.12-8.18 (m, 2H), 7.63-7.67 (m, 1H), 7.61-7.65 (m, 1H), 7.53-7.57 (m, 2H), 7.36-7.43 (m, 2H), 7.22-7.32 (m, 2H), 7.03-7.12 (m, 2H), 5.02 (d, *J* = 4.1 Hz, 2H), 4.61-4.68 (m, 1H), 3.26 (td, *J* = 8.7, 4.3 Hz, 2H), 2.9 (s, 3H), 2.78-2.84 (m, 2H), 2.17-2.25 (m, 4H). MS (ESI): m/z 457 (M +1).

**N-(2-oxo-2-phenylethyl)-2-((1-propionylpiperidin-4-yl)oxy)benzamide (DKC1125g14)** ¹H-NMR (400 MHz, CDCl₃) *δ* 9.12 (s, 1H), 8.25 (dd, *J* = 7.8, 1.8 Hz, 1H), 8.03-8.05 (m, 2H), 7.61-7.65 (m, 1H), 7.50-7.54 (m, 2H), 7.42 (dd, *J* = 8.6, 1.3 Hz, 1H), 7.03-7.09 (m, 2H), 5.30 (s, 2H), 4.61-4.68 (m, 1H), 3.26 (td, *J* = 8.7, 4.3 Hz, 2H), 2.79 (m, 2H), 2.78-2.84 (m, 2H), 2.17-2.25 (m, 4H), 1.94-2.03 (m, 3H). MS (ESI): m/z 395 (M+1).

**2-((1-(cyclohexanecarbonyl)piperidin-4-yl)oxy)-N-(2-oxo-2-phenylethyl)benzamide (DKC1125g15)** ¹H-NMR (400 MHz, CDCl₃) *δ* 9.12 (s, 1H), 8.25 (dd, *J* = 7.8, 1.8 Hz, 1H), 8.03-8.05 (m, 2H), 7.61-7.65 (m, 1H), 7.50-7.54 (m, 2H), 7.44 (dd, *J* = 8.7, 1.4 Hz, 1H), 7.03-7.09 (m, 2H), 5.30 (s, 1H), 4.61-4.68 (m, 1H), 4.20-4.40 (m, 1H), 3.26 (td, *J* = 8.7, 4.3 Hz, 2H), 2.88 (s, 2H), 2.78-2.84 (m, 2H), 2.50-2.68 (m, 4H), 2.22-2.46 (m, 2H), 2.17-2.25 (m, 4H), 2.06 -2.14 (m, 2 H). MS (ESI): m/z 449 (M +1).

**2-((1-(cyclopropanecarbonyl)piperidin-4-yl)oxy)-N-(2-oxo-2-phenylethyl)benzamide (DKC1125g16)** ¹H-NMR (400 MHz, CDCl₃) *δ* 9.12 (s, 1H), 8.25 (dd, *J* = 7.8, 1.8 Hz, 1H), 8.03-8.05 (m, 2H), 7.61-7.65 (m, 1H), 7.50-7.54 (m, 2H), 7.42 (dd, *J* = 8.7, 1.4 Hz, 1H), 7.03-7.09 (m, 2H), 5.30 (s, 2H), 4.61-4.68 (m, 1H), 4.26-4.44 (m, 1H), 3.26-4.10 (m, 2H), 2.78-2.84 (m, 2H), 2.54-2.62 (m, 4H), 2.16-2.24 (m, 4H). MS (ESI): m/z 407 (M +1).

**2-((1-(cyclobutanecarbonyl)piperidin-4-yl)oxy)-N-(2-oxo-2-phenylethyl)benzamide (DKC1125g17)** ¹H-NMR (400 MHz, CDCl₃) *δ* 9.12 (s, 1H), 8.25 (dd, *J* = 7.8, 1.8 Hz, 1H), 8.03-8.05 (m, 2H), 7.61-7.65 (m, 1H), 7.50-7.54 (m, 2H), 7.47 (dd, *J* = 8.7, 1.4 Hz, 1H), 7.03-7.09 (m, 2H), 5.30 (s, 2H), 5.02 (d, *J* = 4.1 Hz, 1H), 4.61-4.68 (m, 1H), 3.26 (td, *J* = 8.7, 4.3 Hz, 2H), 2.78-2.84 (m, 2H), 2.40-2.64 (m, 4H), 2.30-2.34 (m, 2H), 2.06 -2.14 (m, 4H), MS (ESI): m/z 421 (M+1).

**N-(2-oxo-2-phenylethyl)-2-((1-(thiophene-2-carbonyl)piperidin-4-yl)oxy)benzamide (DKC1125g18)** ¹H-NMR (400 MHz, CDCl₃) *δ* 8.98 (s, 1H), 8.25 (dd, *J* = 7.8, 1.8 Hz, 1H), 8.12-8.16 (m, 2H), 7.63-7.67 (m, 1H), 7.61-7.65 (m, 1H), 7.53-7.57 (m, 2H), 7.36-7.43 (m, 2H), 7.22-7.30 (m, 2H), 7.03-7.12 (m, 1H), 5.02 (d, *J* = 4.1 Hz, 2H), 4.61-4.68 (m, 1H), 3.26 (td, *J* = 8.7, 4.3 Hz, 2H), 2.78-2.84 (m, 2H), 2.17-2.25 (m, 4H). MS (ESI): m/z 449 (M +1).

**2-((1-(2-(methoxymethyl)benzoyl)piperidin-4-yl)oxy)-N-(2-oxo-2-phenylethyl)benzamide (DKC1125g19)** ¹H-NMR (400 MHz, CDCl₃) *δ* 8.98 (s, 1H), 8.25 (dd, *J* = 7.8, 1.8 Hz, 1H), 8.12-8.18 (m, 2H), 7.63-7.67 (m, 1H), 7.61-7.65 (m, 1H), 7.53-7.57 (m, 2H), 7.36-7.43 (m, 2H), 7.20-7.29 (m, 2H), 7.03-7.12 (m, 2H), 5.02 (d, *J* = 4.1 Hz, 2H), 4.61-4.68 (m, 1H), 3.92 (s, 3H), 3.26 (td, *J* = 8.7, 4.3 Hz, 2H), 2.96 (s, 2H), 2.78-2.84 (m, 2H), 2.17-2.25 (m, 4H). MS (ESI): m/z 487 (M +1).

**2-((1-(3,3-dimethylbutanoyl)piperidin-4-yl)oxy)-N-(2-oxo-2-phenylethyl)benzamide (DKC1125g20)** ¹H-NMR (400 MHz, CDCl₃) *δ* 9.12 (s, 1H), 8.25 (dd, *J* = 7.8, 1.8 Hz, 1H), 8.10-8.16 (m, 2H), 7.61-7.65 (m, 1H), 7.50-7.54 (m, 2H), 7.46 (dd, *J* = 8.7, 1.4 Hz 1H), 7.03-7.09 (m, 2H), 5.02 (d, *J* = 4.1 Hz, 2H), 4.61-4.68 (m, 1H), 3.26 (td, *J* = 8.7, 4.3 Hz, 2H), 2.9 (m, 2H), 2.78-2.84 (m, 2H), 2.17-2.25 (m, 4H). 1.97 (s, 9H). MS (ESI): m/z 437 (M +1).

**2-((1-(morpholine-4-carbonyl)piperidin-4-yl)oxy)-N-(2-oxo-2-phenylethyl)benzamide (DKC1125g21)** ¹H-NMR (400 MHz, CDCl₃) *δ* 9.12 (s, 1H), 8.25 (dd, *J* = 7.8, 1.8 Hz, 1H), 8.03-8.05 (m, 2H), 7.61-7.65 (m, 1H), 7.50-7.54 (m, 2H), 7.44 (dd, *J* = 8.7, 1.4 Hz, 1H), 7.03-7.09 (m, 2H), 5.30 (s, 2H), 4.61-4.68 (m, 1H), 3.26 (td, *J* = 8.7, 4.3 Hz, 2H), 2.8 (m, 2H), 2.78-2.84 (m, 2H), 2.20-2.27 (m, 4H), 2.14-2.18 (m, 2H), 2.10-2.14 (m, 4H). MS (ESI): m/z 452 (M +1).

**N-(2-oxo-2-phenylethyl)-2-((1-pentanoylpiperidin-4-yl)oxy)benzamide (DKC1125g22)** ¹H-NMR (400 MHz, CDCl₃) *δ* 9.12 (s, 1H), 8.25 (dd, *J* = 7.8, 1.8 Hz, 1H), 8.03-8.05 (m, 2H), 7.61-7.65 (m, 1H), 7.50-7.54 (m, 2H), 7.48 (dd, *J* = 8.7, 1.4 Hz, 1H), 7.03-7.09 (m, 2H), 5.30 (s, 2H), 4.61-4.68 (m, 1H), 3.26 (td, *J* = 8.7, 4.3 Hz, 2H), 2.89 (s, 2 H), 2.78-2.84 (m, 2H), 2.24-2.29 (m, 2H), 2.17-2.25 (m, 4H), 2.16 (s, 2H), 1.79 (s, 3H). MS (ESI): m/z 523 (M +1).

**2-((1-(4-methoxybenzoyl)piperidin-4-yl)oxy)-N-(2-oxo-2-phenylethyl)benzamide (DKC1125g23)** ¹H-NMR (400 MHz, CDCl₃) *δ* 8.98 (s, 1H), 8.25 (dd, *J* = 7.8, 1.8 Hz, 1H), 8.00-8.08 (m, 2H), 7.63-7.67 (m, 1H), 7.61-7.65 (m, 1H), 7.53-7.57 (m, 2H), 7.36-7.43 (m, 2H), 7.22-7.28 (m, 2H), 7.03-7.12 (m, 2H), 5.02 (d, *J* = 4.1 Hz, 2H), 4.61-4.68 (m, 1H), 3.23 (s, 3H), 3.26 (td, *J* = 8.7, 4.3 Hz, 2H), 2.78-2.84 (m, 2H), 2.17-2.25 (m, 4H). MS (ESI): m/z 573 (M +1).

**2-((1-(4-cyanobenzoyl)piperidin-4-yl)oxy)-N-(2-oxo-2-phenylethyl)benzamide (DKC1125g24)** ¹H-NMR (400 MHz, CDCl₃) *δ* 8.98 (s, 1H), 8.25 (dd, *J* = 7.8, 1.8 Hz, 1H), 8.00-8.08 (m, 2H), 7.63-7.67 (m, 1H), 7.61-7.65 (m, 1H), 7.53-7.57 (m, 2H), 7.36-7.43 (m, 2H), 7.22-7.28 (m, 2H), 7.03-7.12 (m, 2H), 5.02 (d, *J* = 4.1 Hz, 2H), 4.61-4.68 (m, 1H), 3.26 (td, *J* = 8.7, 4.3 Hz, 2H), 2.78-2.84 (m, 2H), 2.17-2.25 (m, 4H). MS (ESI): m/z 471 (M +1).

**2-((1-((3-(dioxo-I5-sulfanyl)phenyl)sulfonyl)piperidin-4-yl)oxy)-N-(2-oxo-2-phenylethyl)benzamide (DKC1125g25)** ¹H-NMR (400 MHz, CDCl₃) *δ* 8.98 (s, 1H), 8.25 (dd, *J* = 7.8, 1.8 Hz, 1H), 8.00-8.08 (m, 2H), 7.63-7.67 (m, 1H), 7.61-7.65 (m, 1H), 7.53-7.57 (m, 2H), 7.36-7.43 (m, 2H), 7.24-7.32 (m, 2H), 7.03-7.12 (m, 2H), 5.02 (d, *J* = 4.1 Hz, 2H), 4.61-4.68 (m, 1H), 3.26 (td, *J* = 8.7, 4.3 Hz, 2H), 2.78-2.84 (m, 2H), 2.17-2.25 (m, 4H). MS (ESI): m/z 542 (M +1).

**2-(4-(2-((2-oxo-2-phenylethyl)carbamoyl)phenoxy)piperidine-1-carbonyl)phenylacetate (DKC112526g)** ¹H-NMR (400 MHz, CDCl₃) *δ* 8.98 (s, 1H), 8.25 (dd, *J* = 7.8, 1.8 Hz, 1H), 8.00-8.08 (m, 2H), 7.63-7.67 (m, 1H), 7.61-7.65 (m, 1H), 7.53-7.57 (m, 2H), 7.36-7.43 (m, 2H), 7.10-7.34 (m, 2H), 7.03-7.12 (m, 2H), 5.02 (d, *J* = 4.1 Hz, 2H), 4.61-4.68 (m, 1H), 4.21-4.30 (m, 1H), 3.26 (td, *J* = 8.7, 4.3 Hz, 2H), 2.78-2.84 (m, 2H), 2.17-2.25 (m, 4H). MS (ESI): m/z 520 (M +1).

**2-((1-(2,4-difluorobenzoyl)piperidin-4-yl)oxy)-N-(2-oxo-2-phenylethyl)benzamide (DKC1125g27)** ¹H-NMR (400 MHz, CDCl₃) *δ* 8.98 (s, 1H), 8.25 (dd, *J* = 7.8, 1.8 Hz, 1H), 8.00-8.08 (m, 2H), 7.61-7.65 (m, 1H), 7.53-7.57 (m, 2H), 7.36-7.43 (m, 2H), 7.20-7.36 (m, 2H), 7.03-7.12 (m, 2H), 5.02 (d, *J* = 4.1 Hz, 2H), 4.61-4.68 (m, 1H), 3.26 (td, *J* = 8.7, 4.3 Hz, 2H), 2.78-2.84 (m, 2H), 2.17-2.25 (m, 4H). MS (ESI): m/z 479 (M +1).

**2-((1-(2-methoxyacetyl)piperidin-4-yl)oxy)-N-(2-oxo-2-phenylethyl)benzamide (DKC1125g28)** ¹H-NMR (400 MHz, CDCl₃) *δ* 9.12 (s, 1H), 8.25 (dd, *J* = 7.8, 1.8 Hz, 1H), 8.03-8.05 (m, 2H), 7.61-7.65 (m, 1H), 7.50-7.54 (m, 2H), 7.44 (ddd, *J* = 8.7, 6.9, 1.4 Hz, 1H), 7.03-7.09 (m, 2H), 5.30 (m, 1H), 5.02 (d, *J* = 4.1 Hz, 2H), 4.61-4.68 (m, 2H), 3.97 (s, 3H), 3.26 (td, *J* = 8.7, 4.3 Hz, 2H), 2.78-2.84 (m, 2H), 2.17-2.25 (m, 4H). MS (ESI): m/z 411 (M +1).

**N-(2-oxo-2-phenylethyl)-2-((1-pivaloylpiperidin-4-yl)oxy)benzamide (DKC1125g29)** ¹H-NMR (400 MHz, CDCl₃) *δ* 9.12 (s, 1H), 8.25 (dd, *J* = 7.8, 1.8 Hz, 1H), 8.03-8.05 (m, 2H), 7.61-7.65 (m, 1H), 7.50-7.54 (m, 2H), 7.44 (dd, *J* = 8.7, 1.4 Hz, 1H), 7.03-7.09 (m, 2H), 5.02 (d, *J* = 4.1 Hz, 2H), 4.61-4.68 (m, 1H), 3.26 (td, *J* = 8.7, 4.3 Hz, 2H), 2.78-2.84 (m, 2H), 2.17-2.25 (m, 4H). 1.92 (s, 9H). MS (ESI): m/z 423 (M +1).

**N,N-dimethyl-4-(2-((2-oxo-2-phenylethyl)carbamoyl)phenoxy)piperidine-1-carboxamide (DKC1125g30)** ¹H-NMR (400 MHz, CDCl₃) *δ* 9.12 (s, 1H), 8.25 (dd, *J* = 7.8, 1.8 Hz, 1H), 8.03-8.05 (m, 2H), 7.61-7.65 (m, 1H), 7.50-7.54 (m, 2H), 7.44 (dd, *J* = 8.7, 1.4 Hz, 1H), 7.03-7.09 (m, 2H), 5.30 (s, 2H), 4.61-4.68 (m, 1H), 4.62-4.66 (m, 6H), 3.28 (s, 2H), 2.79 (m, 2H), 2.17-2.25 (m, 4H). MS (ESI): m/z 410 (M +1).

**2-((1-(4-bromobenzoyl)piperidin-4-yl)oxy)-N-(2-oxo-2-phenylethyl)benzamide (DKC1125g31)** ¹H-NMR (400 MHz, CDCl₃) *δ* 8.98 (s, 1H), 8.25 (dd, *J* = 7.8, 1.8 Hz, 1H), 8.00-8.08 (m, 2H), 7.63-7.67 (m, 1H), 7.61-7.65 (m, 1H), 7.53-7.57 (m, 2H), 7.36-7.43 (m, 2H), 7.24-7.34 (m, 2H), 7.03-7.12 (m, 2H), 5.02 (d, *J* = 4.1 Hz, 2H), 4.61-4.68 (m, 1H), 3.26 (td, *J* = 8.7, 4.3 Hz, 2H), 2.78-2.84 (m, 2H), 2.17-2.25 (m, 4H). MS (ESI): m/z 521 (M +1).

**2-((1-acetylpiperidin-4-yl)oxy)-N-(2-oxo-2-phenylethyl)benzamide (DKC1125g32)** ¹H-NMR (400 MHz, CDCl₃) *δ* 9.12 (s, 1H), 8.25 (dd, *J* = 7.8, 1.8 Hz, 1H), 8.03-8.05 (m, 2H), 7.61-7.65 (m, 1H), 7.50-7.54 (m, 2H), 7.44 (dd, *J* = 8.7, 1.4 Hz, 1H), 7.03-7.09 (m, 2H), 5.02 (d, *J* = 4.1 Hz, 2H), 4.61-4.68 (m, 1H), 3.26 (td, *J* = 8.7, 4.3 Hz, 2H), 2.9 (s, 3H), 2.78-2.84 (m, 2H), 2.17-2.25 (m, 4H). MS (ESI): m/z 381 (M +1).

**2-((1-(methylsulfonyl)piperidin-4-yl)oxy)-N-(2-oxo-2-phenylethyl)benzamide** (DKC1125g33) ¹H-NMR (400 MHz, CDCl₃) *δ* 9.12 (s, 1H), 8.25 (dd, *J* = 7.8, 1.8 Hz, 1H), 8.03-8.05 (m, 2H), 7.61-7.65 (m, 1H), 7.50-7.54 (m, 2H), 7.44 (dd, *J* = 8.7, 1.4 Hz, 1H), 7.03-7.09 (m, 2H), 5.02 (d, *J* = 4.1 Hz, 2H), 4.61-4.68 (m, 1H), 3.8 (s, 3H), 3.26 (td, *J* = 8.7, 4.3 Hz, 2H), 2.78-2.84 (m, 2H), 2.17-2.25 (m, 4H). MS (ESI): m/z 417 (M +1).

**2-((1-((4-fluorophenyl)sulfonyl)piperidin-4-yl)oxy)-N-(2-oxo-2-phenylethyl)benzamide (DKC1125g34)** ¹H-NMR (400 MHz, CDCl₃) *δ* 8.98 (s, 1H), 8.25 (dd, *J* = 7.8, 1.8 Hz, 1H), 8.00-8.08 (m, 2H), 7.63-7.67 (m, 1H), 7.61-7.65 (m, 1H), 7.53-7.57 (m, 2H), 7.36-7.43 (m, 2H), 7.24-7.34 (m, 2H), 7.03-7.12 (m, 2H), 5.02 (d, *J* = 4.1 Hz, 2H), 4.61-4.68 (m, 1H), 3.26 (td, *J* = 8.7, 4.3 Hz, 2H), 2.78-2.84 (m, 2H), 2.17-2.25 (m, 4H). MS (ESI): m/z 497 (M +1).

**N-(2-oxo-2-phenylethyl)-2-((1-tosylpiperidin-4-yl)oxy)benzamide (DKC1125g35)** ¹H-NMR (400 MHz, CDCl₃) *δ* 8.98 (s, 1H), 8.25 (dd, *J* = 7.8, 1.8 Hz, 1H), 8.00-8.08 (m, 2H), 7.63-7.67 (m, 2H), 7.53-7.57 (m, 2H), 7.36-7.43 (m, 2H), 7.24-7.34 (m, 2H), 7.03-7.12 (m, 2H), 5.02 (d, *J* = 4.1 Hz, 2H), 4.61-4.68 (m, 1H), 3.26 (td, *J* = 8.7, 4.3 Hz, 2H), 2.96 (s, 3H), 2.78-2.84 (m, 2H), 2.17-2.25 (m, 4H). MS (ESI): m/z 493 (M +1).

**2-((1-((4-chlorophenyl)sulfonyl)piperidin-4-yl)oxy)-N-(2-oxo-2-phenylethyl)benzamide (DKC1125g36)** ¹H-NMR (400 MHz, CDCl₃) *δ* 8.98 (s, 1H), 8.25 (dd, *J* = 7.8, 1.8 Hz, 1H), 8.00-8.08 (m, 2H), 7.63-7.67 (m, 2H), 7.53-7.57 (m, 2H), 7.36-7.43 (m, 2H), 7.24-7.34 (m, 2H), 7.03-7.12 (m, 2H), 5.02 (d, *J* = 4.1 Hz, 2H), 4.61-4.68 (m, 1H), 3.26 (td, *J* = 8.7, 4.3 Hz, 2H), 2.78-2.84 (m, 2H), 2.17-2.25 (m, 4H). MS (ESI): m/z 513 (M +1).

**N-(2-oxo-2-phenylethyl)-2-((1-(phenylsulfonyl)piperidin-4-yl)oxy)benzamide (DKC1125g37)** ¹H-NMR (400 MHz, CDCl₃) *δ* 8.98 (s, 1H), 8.25 (dd, *J* = 7.8, 1.8 Hz, 1H), 8.00-8.08 (m, 2H), 7.63-7.67 (m, 1H), 7.61-7.65 (m, 2H), 7.53-7.57 (m, 2H), 7.36-7.43 (m, 2H), 7.24-7.34 (m, 2H), 7.03-7.12 (m, 2H), 5.02 (d, *J* = 4.1 Hz, 2H), 4.61-4.68 (m, 1H), 3.26 (td, *J* = 8.7, 4.3 Hz, 2H), 2.78-2.84 (m, 2H), 2.17-2.25 (m, 4H). MS (ESI): m/z 479 (M +1).

**2-((1-(benzylsulfonyl)piperidin-4-yl)oxy)-N-(2-oxo-2-phenylethyl)benzamide (DKC1125g38)** ¹H-NMR (400 MHz, CDCl₃) *δ* 8.98 (s, 1H), 8.25 (dd, *J* = 7.8, 1.8 Hz, 1H), 8.00-8.08 (m, 2H), 7.63-7.67 (m, 1H), 7.61-7.65 (m, 2H), 7.53-7.57 (m, 2H), 7.36-7.43 (m, 2H), 7.2-7.3 (m, 2H), 7.03-7.12 (m, 2H), 5.04 (s, 2H), 5.02 (d, *J* = 4.1 Hz, 2H), 4.61-4.68 (m, 1H), 3.26 (td, *J* = 8.7, 4.3 Hz, 2H), 2.78-2.84 (m, 2H), 2.17-2.25 (m, 4H). MS (ESI): m/z 457 (456.18 +1). MS (ESI): m/z 493 (M +1).

**2-((1-((4-methoxyphenyl)sulfonyl)piperidin-4-yl)oxy)-N-(2-oxo-2-phenylethyl)benzamide (DKC1125g39)** ¹H-NMR(400 MHz, CDCl₃) *δ* 8.98 (s, 1H), 8.25 (dd, J = 7.8, 1.8 Hz, 1H), 8.00-8.08 (m, 2H), 7.63-7.67 (m, 1H), 7.61-7.65 (m, 1H), 7.53-7.57 (m, 2H), 7.36-7.43 (m, 2H), 7.28-7.30 (m, 2H), 7.03-7.12 (m, 2H), 5.02 (d, *J* = 4.1 Hz, 2H), 4.61-4.68 (m, 1H), 3.91 (s, 3H), 3.26 (td, *J* = 8.7, 4.3 Hz, 2H), 2.78-2.84 (m, 2H), 2.17-2.25 (m, 4H). MS (ESI): m/z 409 (M +1).

**2-((1-(3,5-bis(trifluoromethyl)benzoyl)piperidin-4-yl)oxy)-N-(2-oxo-2-phenylethyl)benzamide (DKC1125g40)** ¹H-NMR (400 MHz, CDCl₃) *δ* 8.98 (s, 1H), 8.25 (dd, *J* = 7.8, 1.8 Hz, 1H), 8.00-8.08 (m, 2H), 7.63-7.67 (m, 1H), 7.61-7.65 (m, 1H), 7.53-7.57 (m, 2H), 7.36-7.43 (m, 2H), 7.28-7.30 (m, 2H), 7.03-7.12 (m, 1H), 5.02 (d, *J* = 4.1 Hz, 2H), 4.61-4.68 (m, 1H), 3.26 (td, *J* = 8.7, 4.3 Hz, 2H), 2.78-2.84 (m, 2H), 2.17-2.25 (m, 4H). MS (ESI): m/z 579 (M +1).

**N-(2-oxo-2-phenylethyl)-2-((1-(2-(trifluoromethyl)benzoyl)piperidin-4-yl)oxy)benzamide (DKC1125g41)** ¹H-NMR (400 MHz, CDCl₃) *δ* 8.98 (s, 1H), 8.25 (dd, *J* = 7.8, 1.8 Hz, 1H), 8.00-8.08 (m, 2H), 7.63-7.67 (m, 1H), 7.61-7.65 (m, 1H), 7.53-7.57 (m, 2H), 7.36-7.43 (m, 2H), 7.28-7.30 (m, 2H), 7.03-7.12 (m, 2H), 5.02 (d, *J* = 4.1 Hz, 2H), 4.61-4.68 (m, 1H), 3.26 (td, *J* = 8.7, 4.3 Hz, 2H), 2.78-2.84 (m, 2H), 2.17-2.25 (m, 4H). MS (ESI): m/z 511 (M +1).

**N-(2-oxo-2-phenylethyl)-2-((1-(3-(trifluoromethyl)benzoyl)piperidin-4-yl)oxy)benzamide (DKC1125g42)** ¹H-NMR (400 MHz, CDCl₃) *δ* 8.98 (s, 1H), 8.25 (dd, *J* = 7.8, 1.8 Hz, 1H), 8.00-8.08 (m, 2H), 7.63-7.67 (m, 1H), 7.61-7.65 (m, 1H), 7.53-7.57 (m, 2H), 7.36-7.43 (m, 2H), 7.28-7.30 (m, 2H), 7.03-7.12 (m, 2H), 5.40 (d, *J* = 4.2 Hz, 2H), 4.61-4.68 (m, 1H), 3.26 (td, J = 8.7, 4.3 Hz, 2H), 2.78-2.84 (m, 2H), 2.17-2.25 (m, 4H). MS (ESI): m/z 511 (M +1).

**N-(2-oxo-2-phenylethyl)-2-((1-(4-(trifluoromethyl)benzoyl)piperidin-4-yl)oxy)benzamide (DKC1125g43)** ¹H-NMR (400 MHz, CDCl₃) *δ* 8.98 (s, 1H), 8.25 (dd, *J* = 7.8, 1.8 Hz, 1H), 8.00-8.08 (m, 2H), 7.63-7.67 (m, 1H), 7.61-7.65 (m, 1H), 7.53-7.57 (m, 2H), 7.36-7.43 (m, 2H), 7.22-7.30 (m, 2H), 7.08-7.12 (m, 2H), 5.02 (d, *J* = 4.1 Hz, 2H), 4.61-4.68 (m, 1H), 3.26 (td, *J* = 8.7, 4.3 Hz, 2H), 2.78-2.84 (m, 2H), 2.17-2.25 (m, 4H). MS (ESI): m/z 511 (M +1).

**2-((1-(2-chloro-4-fluorobenzoyl)piperidin-4-yl)oxy)-N-(2-oxo-2-phenylethyl)benzamide (DKC1125g44)** ¹H-NMR (400 MHz, CDCl₃) *δ* 8.98 (s, 1H), 8.25 (dd, *J* = 7.8, 1.8 Hz, 1H), 8.00-8.08 (m, 2H), 7.63-7.67 (m, 1H), 7.61-7.65 (m, 1H), 7.53-7.57 (m, 2H), 7.36-7.43 (m, 2H), 7.25-7.32 (m, 2H), 7.03-7.12 (m, 2H), 5.02 (d, *J* = 4.1 Hz, 2H), 4.61-4.68 (m, 1H), 3.26 (td, *J* = 8.7, 4.3 Hz, 2H), 2.78-2.84 (m, 2H), 2.17-2.25 (m, 4H). MS (ESI): m/z 495 (M +1).

**2-((1-(3-nitrobenzoyl)piperidin-4-yl)oxy)-N-(2-oxo-2-phenylethyl)benzamide (DKC1125g45)** ¹H-NMR (400 MHz, CDCl₃) *δ* 8.98 (s, 1H), 8.25 (dd, *J* = 7.8, 1.8 Hz, 1H), 8.00-8.08 (m, 2H), 7.62-7.66 (m, 1H), 7.61-7.65 (m, 1H), 7.53-7.57 (m, 2H), 7.36-7.43 (m, 2H), 7.28-7.30 (m, 2H), 7.03-7.12 (m, 2H), 5.02 (d, *J* = 4.1 Hz, 2H), 4.61-4.68 (m, 1H), 3.26 (td, *J* = 8.7, 4.3 Hz, 2H), 2.78-2.84 (m, 2H), 2.17-2.25 (m, 4H). MS (ESI): m/z 488 (M +1).

**2-((1-(3,4-difluorobenzoyl)piperidin-4-yl)oxy)-N-(2-oxo-2-phenylethyl)benzamide (DKC1125g46)** ¹H-NMR (400 MHz, CDCl₃) *δ* 8.98 (s, 1H), 8.25 (dd, *J* = 7.8, 1.8 Hz, 1H), 8.00-8.08 (m, 2H), 7.63-7.67 (m, 1H), 7.61-7.65 (m, 1H), 7.53-7.57 (m, 2H), 7.38-7.48 (m, 2H), 7.23-7.34 (m, 2H), 7.03-7.12 (m, 1H), 5.02 (d, *J* = 4.1 Hz, 2H), 4.61-4.68 (m, 1H), 3.26 (td, *J* = 8.7, 4.3 Hz, 2H), 2.78-2.84 (m, 2H), 2.17-2.25 (m, 4H). MS (ESI): m/z 479 (M +1).

**2-((1-(3,5-dichlorobenzoyl)piperidin-4-yl)oxy)-N-(2-oxo-2-phenylethyl)benzamide (DKC1125g47)** ¹H-NMR (400 MHz, CDCl₃) *δ* 8.98 (s, 1H), 8.25 (dd, *J* = 7.8, 1.8 Hz, 1H), 8.10-8.18 (m, 2H), 7.64-7.68 (m, 1H), 7.61-7.65 (m, 1H), 7.53-7.57 (m, 2H), 7.36-7.43 (m, 2H), 7.25-7.32 (m, 1H), 7.03-7.12 (m, 2H), 5.02 (d, *J* = 4.1 Hz, 2H), 4.61-4.68 (m, 1H), 3.26 (td, *J* = 8.7, 4.3 Hz, 2H), 2.78-2.84 (m, 2H), 2.17-2.25 (m, 4H). MS (ESI): m/z 511 (M +1).

**2-((1-(2,3-dichlorobenzoyl)piperidin-4-yl)oxy)-N-(2-oxo-2-phenylethyl)benzamide (DKC1125g48)** ¹H-NMR (400 MHz, CDCl₃) *δ* 8.98 (s, 1H), 8.25 (dd, *J* = 7.8, 1.8 Hz, 1H), 8.22-8.26 (m, 1H), 7.63-7.67 (m, 1H), 7.61-7.65 (m, 1H), 7.53-7.57 (m, 2H), 7.36-7.43 (m, 2H), 7.25-7.32 (m, 2H), 7.03-7.12 (m, 2H), 5.02 (d, *J* = 4.1 Hz, 2H), 4.61-4.68 (m, 1H), 3.26 (td, *J* = 8.7, 4.3 Hz, 2H), 2.78-2.84 (m, 2H), 2.17-2.25 (m, 4H). MS (ESI): m/z 511 (M +1).

**N-(2-oxo-2-phenylethyl)-2-((1-(4-(trifluoromethoxy)benzoyl)piperidin-4-yl)oxy)benzamide (DKC1125g49)** ¹H-NMR (400 MHz, CDCl₃) *δ* 8.98 (s, 1H), 8.25 (dd, *J* = 7.8, 1.8 Hz, 1H), 8.10-8.16 (m, 2H), 7.62-7.64 (m, 1H), 7.61-7.65 (m, 1H), 7.53-7.57 (m, 2H), 7.36-7.43 (m, 2H), 7.21-7.30 (m, 1H), 7.03-7.12 (m, 2H), 5.02 (d, *J* = 4.1 Hz, 2H), 4.61-4.68 (m, 1H), 3.26 (td, *J* = 8.7, 4.3 Hz, 2H), 2.78-2.84 (m, 2H), 2.17-2.25 (m, 4H). MS (ESI): m/z 527 (M +1).

**N-(2-oxo-2-phenylethyl)-2-((1-(3-(trifluoromethoxy)benzoyl)piperidin-4-yl)oxy)benzamide (DKC1125g50)** ¹H-NMR (400 MHz, CDCl₃) *δ* 8.98 (s, 1H), 8.25 (dd, *J* = 7.8, 1.8 Hz, 1H), 8.10-8.18 (m, 2H), 7.63-7.67 (m, 1H), 7.61-7.65 (m, 1H), 7.53-7.57 (m, 2H), 7.36-7.43 (m, 2H), 7.21-7.30 (m, 2H), 7.03-7.12 (m, 2H), 5.02 (d, *J* = 4.1 Hz, 2H), 4.61-4.68 (m, 1H), 3.26 (td, *J* = 8.7, 4.3 Hz, 2H), 2.78-2.84 (m, 2H), 2.17-2.25 (m, 4H). MS (ESI): m/z 527 (M +1).

### Examples 201 to 230. Preparation of Compounds 201 to 230 (DKC1125h01 to 30) according to the Present Invention

### Synthesis of Compounds 201 to 230 (DKC1125h01 to 30)

Compounds 201 to 230 (DKC1125h01 to 30) of the present invention were synthesized according to the following Reaction Scheme 7.

### [Reaction Scheme 7]

### Synthesis of compounds 201 to 230 (DKC1125h01 to 30)

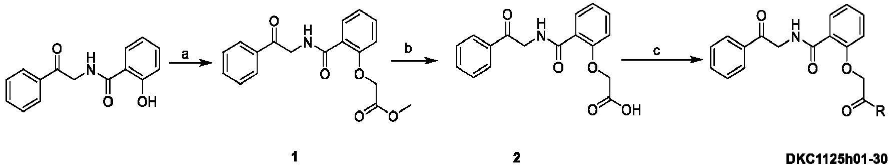

Reagents and conditions: (a)C₃H₅BrO₂, K₂CO₃, DMF, 50°C, 1h; (b)NaOH , EtOH, rt, 4h; (c)RNH₂, HATU, DIEA, DMF, rt 8h.

### Synthesis of methyl 2-(2((-oxo-2-phenylethyl)carbomyl)phenoxy)acetate (1)

To a solution of 2-hydroxy-N-(2-oxo-2-phenylethyl)benzamide (500 mg, 1.97 mmol) and methyl 2-bromoacetate (240 µL, 2.97mol) in DMF (8 mL) was added potassium carbonate (677 mg, 4.97 mol). The resulting solution was stirred at 50°C for 9 hrs. The reaction was monitored by checking TLC. After completion of the reaction, to the mixture were added water and HCl to maintain acidic pH, followed by extraction with EtOAc. The organic layer was washed with brine (3 x 100mL), and dried the solution with Na₂SO₄, and the solvent was evaporated. The residue was purified by column chromatography to give the title compound (1) as a white powder (0.6 g, 84%).

¹H-NMR (400 MHz, CDCl₃) *δ* 9.89 (d, *J* = 10.5 Hz, 1H), 8.14-8.20 (m, 1H), 7.74-7.80 (m, 1H), 7.51-7.58 (m, 1H), 7.38-7.49 (m, 2H), 6.96-6.99 (m, 2H), 6.83 (d, *J* = 7.8 Hz, 1H), 6.75 (d, *J* = 8.2 Hz, 1H), 4.74 (d, *J* = 5.9 Hz, 2H), 4.51 (s, 2H), 3.88 (q, *J* = 7.2 Hz, 3H). MS (ESI): m/z 328 (M +1).

### Synthesis of 2-(2((-oxo-2-phenylethyl)carbomyl)phenoxy)acetic acid (2)

To a solution of methyl 2-(2((-oxo-2-phenylethyl)carbomyl)phenoxy)acetate **(1,** 0.6 g, 1.77 mol) in EtOH (10 mL) was added 4N NaOH (6 mL) with stirring, followed by addition of 2N HCl (1 mL). The resulting solution was stirred at room temperature for 4 hrs. The reaction was monitored by checking TLC. After completion of the reaction, to the mixture were added water and HCl to maintain acidic pH, followed by extraction with EA. The organic layer was separated, washed with brine (3 x100 mL). The washed solution was dried with anhydrous sodium sulphate, and the solvent was evaporated in vaccuo. The residue was purified by column chromatography (EtOAc : Hexane 2:3) to give the title compounds (2) as a white powder (567 mg, 83%).

¹H-NMR (400 MHz, CDCl₃) *δ* 9.01 (t, *J* = 5.3 Hz, 1H), 8.03-8.06 (m, 2H), 7.93 (dd, *J* = 7.3, 1.8 Hz, 1H), 7.67-7.71 (m, 2H), 7.55-7.59 (m, 1H), 7.49-7.53 (m, 1H), 7.09-7.14 (m, 2H), 4.93 (s, 2H), 4.87 (d, *J* = 5.5 Hz, 2H). MS (ESI): m/z 314 (M +1).

### General Preparation Method for Compounds 201 to 230 (DKC1125h01 to 30)

To a solution of 2-(2((-oxo-2-phenylethyl)carbomyl)phenoxy)acetic acid (2, 20 mg ,0.069 mmol) and each amine (10 µL, 0.069 mmol) shown in Table 9 below in DMF was added HATU (18 mg, 0.069 mmol). Finally, DIEA (24 µL, 0.143 mmol) was added thereto, and the reaction mixture was stirred at room temperature for 1 hr. After completion of the reaction, the mixture was evaporated by using Rota vapor and purified by column chromatography to obtain the title compound as a solid (12 mg, 80 to 90%).

**[Table 9]**

| Amines used in preparation of compounds 201 to 230 (DKC1125h01 to 30) | | | | |
|---|---|---|---|---|
| **Compounds** | **Amine** | | **Compounds** | **Amine** |
| **DKC1125h01** | Aniline | | **DKC1125h16** | 3,4-Dimethoxyaniline |
| **DKC1125h02** | o-toluidine | | **DKC1125h17** | 3,4-(methylenedioxy)aniline |
| **DKC1125h03** | Allylamine | | **DKC1125h18** | 1,4-benzodioxan-6-amine |
| **DKC1125h04** | Benzylamine | | **DKC1125h19** | 5-aminoindane |
| **DKC1125h05** | 3-fluorobenzylamine | | **DKC1125h20** | 3-isopropoxyaniline |
| **DKC1125h06** | 2-methoxy-5-methylaniline | | **DKC1125h21** | 4-phenoxyaniline |
| **DKC1125h07** | 3-Fluoro-4-methoxyaniline | | **DKC1125h22** | 3-Amino-4-fluorobenzotrifluoride |
| **DKC1125h08** | 3-aminopyridine | | **DKC1125h23** | 3-aminophenol |
| **DKC1125h09** | m-anisidine | | **DKC1125h24** | 2-chloroaniline |
| **DKC1125h10** | 2-propylaniline | | **DKC1125h25** | 2-aminophenol |
| **DKC1125h11** | 4-fluoro-3-nitroaniline | | **DKC1125h26** | isopropylamine |
| **DKC1125h12** | 3,4-(methylenedioxy)benzylamine | | **DKC1125h27** | 1,2,3,4-tetrahydroisoquinoline |
| **DKC1125h13** | 4-n-propylaniline | | **DKC1125h28** | 1-propylamine |
| **DKC1125h14** | 3-phenoxyaniline | | **DKC1125h29** | isoamylamine |
| **DKC1125h15** | 3,4-Dimethoxybenzyamine | | **DKC1125h30** | cyclohexylamine |

### Confirmation of Preparation of Compounds 201 to 230 (DKC1125h01 to 30)

**2-(2-oxo-2-(phenylamino)ethoxy)-*N*-(2-oxo-2-phenylethyl)benzamide (DKC1125h01)** ¹H-NMR (400 MHz, CDCl₃) *δ* 9.76 (s, 1H), 8.48 (s, 1H), 8.01-8.03 (m, 1H), 7.81-7.84 (m, 1H), 7.74-7.77 (m, 2H), 7.49-7.56 (m, 3H), 7.29 (dd, *J* = 7.2, 1.8 Hz, 3H), 7.10-7.15 (m, 2H), 7.05-7.08 (m, 1H), 5.03 (t, *J* = 3.9 Hz, 2H), 4.83 (s, 2H). MS (ESI): m/z 389 (M +1).

**2-(2-oxo-2-(o-toylamino)ethoxy)-*N*-(2-oxo-2-phenylethyl)benzamide (DKC1125h02)** ¹H-NMR (400 MHz, CDCl₃) *δ* 9.74 (s, 1H), 8.38 (s, 1H), 8.01-8.03 (m, 2H), 7.81-7.84 (m, 1H), 7.74-7.77 (m, 1H), 7.49-7.56 (m, 3H), 7.29 (dd, *J* = 7.2, 1.8 Hz, 3H), 7.10-7.15 (m, 2H), 7.05-7.08 (m, 1H), 5.03 (t, *J* = 3.9 Hz, 2H), 4.83 (s, 2H), 2.9 (s, 3H). MS (ESI): m/z 403 (M +1).

**2-(2-(allylamino)-2-oxoethoxy)-*N*-(2-oxo-2-phenylethyl)benzamide (DKC1125h03)** ¹H-NMR (400 MHz, CDCl₃) *δ* 9.70 (s, 1H), 8.64 (s, 1H), 8.02 (d, *J* = 7.3 Hz, 1H), 7.79 (dd, *J* = 7.8, 1.4 Hz, 1H), 7.71 (d, *J* = 3.2 Hz, 3H), 7.52-7.56 (m, 3H), 6.89-6.94 (m, 1H), 5.03 (t, *J* = 3.9 Hz, 2H), 4.83 (s, 2H), 4.66 (s, 1H), 4.04-4.10 (m, 2H), 3.95 (t, *J* = 5.7 Hz, 2H). MS (ESI): m/z 353(M +1).

**2-(2-(benzylamino)-2-oxoethoxy)-*N*-(2-oxo-2-phenylethyl)benzamide (DKC1125h04)** ¹H-NMR (400 MHz, CDCl₃) *δ* 9.76 (s, 1H), 8.01-8.03 (m, 2H), 7.81-7.84 (m, 1H), 7.74-7.77 (m, 2H), 7.49-7.56 (m, 3H), 7.18 (dd, *J* = 7.2, 1.8 Hz, 3H), 7.10-7.15 (m, 2H), 7.05-7.08 (m, 1H), 5.24 (s, 2H), 5.01 (t, *J* = 3.9 Hz, 2H), 4.67 (s, 2H). MS (ESI): m/z 403(M +1).

**2-(2-((fluorobenzyl)amino)-2-oxoethoxy)-*N*-(2-oxo-2-phenylethyl)benzamide (DKC1125h05)** ¹H-NMR (400 MHz, CDCl₃) *δ* 9.76 (s, 1H), 8.71 (s, 1H), 8.01-8.03 (m, 2H), 7.81-7.84 (m, 1H), 7.74-7.77 (m, 1H), 7.49-7.56 (m, 3H), 7.30 (dd, *J* =7.4, 1.8 Hz, 3H), 7.10-7.15 (m, 2H), 7.05-7.08 (m, 1H), 5.03 (t, *J* = 3.9 Hz, 2H), 4.83 (s, 2H). MS (ESI): m/z 421(M +1).

**2-(2-((2-methoxy-4-methylphenyl)amino)-2-oxoethoxy)-*N*-(2-oxo-2-phenylethyl)benzamide (DKC1125h06) ¹**H-NMR (400 MHz, CDCl₃) *δ* 8.81 (s, 2H), 8.17 (q, *J* = 2.3 Hz, 1H), 8.14 (dd, *J* = 7.8, 1.8 Hz, 1H), 7.90-7.92 (m, 2H), 7.60-7.64 (m, 1H), 7.46-7.53 (m, 3H), 7.15-7.19 (m, 1H), 7.03 (d, *J* = 8.2 Hz, 1H), 6.82 (dd, *J* = 8.2, 1.4 Hz, 1H), 6.57-6.61 (m, 1H), 5.42 (d, *J* = 4.1 Hz, 2H), 4.79 (s, 2H). 3.96 (s, 3H), 3.12 (s, 1H). MS (ESI): m/z 433 (M +1).

**2-(2-((3-fluoro-4-methoxyphenyl)amino)-2-oxoethoxy)-*N*-(2-oxo-2-phenylethyl)benzamide (DKC1125h07)** ¹H-NMR (400 MHz, CDCl₃) *δ* 8.78 (s, 2H), 8.17 (q, *J* = 2.3 Hz, 2H), 8.15 (dd, *J* = 7.8, 1.8 Hz, 2H), 7.90-7.92 (m, 2H), 7.62-7.64 (m, 2H), 7.03 (d, *J* = 8.2 Hz, 2H), 6.82 (dd, *J* = 8.2, 1.4 Hz, 1H), 6.57-6.61 (m, 1H), 5.03 (d, *J* = 4.1 Hz, 2H), 4.89 (s, 2H), 3.68 (d, *J* = 3.2 Hz, 3H). MS (ESI): m/z 437 (M +1).

**2-(2-oxo-2-(pyridin-3-ylamino)ethoxy)-*N*-(2-oxo-2-phenylethyl)benzamide (DKC1125h08)** ¹H-NMR (400 MHz, CDCl₃) *δ* 9.76 (s, 1H), 8.84 (s, 1H), 8.01-8.03 (m, 2H), 7.81-7.84 (m, 1H), 7.74-7.77 (m, 1H), 7.89-7.59 (m, 3H), 7.48-7.42 (dd, *J* = 7.4, 1.8 Hz, 3H), 7.20-7.25 (m, 2H), 7.05-7.08 (m, 1H), 5.47(t, *J* = 3.9 Hz, 2H), 4.87 (s, 2H). MS (ESI): m/z 390 (M +1).

**2-(2-((3-methoxyphenyl)amino)-2-oxoethoxy)-*N*-(2-oxo-2-phenylethyl)benzamide (DKC1125h09)** ¹H-NMR (400 MHz, CDCl₃) *δ* 8.62 (s, 1H), 8.17 (q, J = 2.3 Hz, 1H), 8.14 (dd, *J* = 7.8, 1.8 Hz, 2H), 7.90-7.92 (m, 2H), 7.60-7.64 (m, 1H), 7.46-7.53 (m, 3H), 7.15-7.19 (m, 1H), 7.03 (d, *J* = 8.2 Hz, 1H), 6.82 (dd, *J* = 8.2, 1.4 Hz, 1H), 6.57-6.61 (m, 1H), 5.03 (d, *J* = 4.1 Hz, 2H), 4.89 (s, 2H), 3.61 (d, *J* = 3.2 Hz, 3H). MS (ESI): m/z 419 (M +1).

**2-(2-oxo-2-((2-propylphenyl)amino)ethoxy)-*N*-(2-oxo-2-phenylethyl)benzamide (DKC1125h10)** ¹H-NMR (400 MHz, CDCl₃) *δ* 8.91 (s, 1H), 8.60 (s, 1H), 8.17 (q, *J* = 2.3 Hz, 1H), 8.15 (dd, *J* = 7.8, 1.8 Hz, 2H), 7.90-7.92 (m, 2H), 7.60-7.64 (m, 1H), 7.15-7.19 (m, 1H), 7.03 (d, *J* = 8.2 Hz, 2H), 6.82 (dd, *J* = 8.2, 1.4 Hz, 2H), 6.57-6.61 (m, 2H), 5.03 (d, *J* = 4.1 Hz, 2H), 4.89 (s, 2H), 3.61 (d, *J* = 3.2 Hz, 3H), 2.66-2.72 (m, 2H), 2.42-2.50 (m, 2H). MS (ESI): m/z 431 (M +1).

**2-(2-((1-fluoro-3-nitrophenyl)amino)-2-oxoethoxy)-N-(2-oxo-2-phenylethylbenzamide (DKC1125h11)**¹H-NMR (400 MHz, CDCl₃) δ 8.80 (s, 1H), 8.68 (s, 1H), 8.17 (q, *J* = 2.3 Hz, 2H), 8.14 (dd, *J =* 7.8, 1.8 Hz, 2H), 7.90-7.92 (m, 2H), 7.60-7.64 (m, 1H), 7.05-7.16 (m, 1H), 7.03 (d, *J =* 8.2 Hz, 2H), 6.82 (dd, *J* = 8.2, 1.4 Hz, 1H), 6.57-6.61 (m, 1H), 5.52 (d, *J* = 4.1 Hz, 2H), 4.90 (s, 2H). MS (ESI): m/z 452 (M +1).

**2-(2-((benzo [1,3]dioxol-5-ylmethyl)amino )-2-oxoethoxy)-N-(2-oxo-2-phenylethylbenzamide (DKC1125h12) ¹H-NMR** (400 MHz, CDCl₃) δ 8.90 (s, 1H), 8.71 (s, 1H),8.17 (q, *J* = 2.3 Hz, 1H), 8.14 (dd, *J* = 7.8, 1.8 Hz, 1H), 7.90-7.92 (m, 2H), 7.60-7.64 (m, 1H), 7.46-7.53 (m, 3H), 7.15-7.19 (m, 1H), 7.03 (d, *J =* 8.2 Hz, 1H), 6.82 (dd, *J =* 8.2, 1.4 Hz, 1H), 6.57-6.61 (m, 1H), 6.0 (s, 2H), 5.01 (d, *J =* 4.1 Hz, 2H), 4.68 (s, 2H). MS (ESI): m/z 447 (M +1).

**2-(2-oxo-2-((4-propylphenyl)amino)ethoxy)-N-(2-oxo-2-phenylethyl)benzamide (DKC1125h13) ¹H-NMR** (400 MHz, CDCl₃) δ 9.02 (s, 1H), 8.71 (s, 1H), 8.17 (q, J = 2.3 Hz, 1H), 8.03 (dd, *J = 7.8,* 1.8 Hz, 2H), 7.91-7.92 (m, 2H), 7.62-7.64 (m, 1H), 7.44-7.50 (m, 2H), 7.16-7.19 (m, 1H), 7.03 (d, *J* = 8.2 Hz, 1H), 6.82 (dd, *J* = 8.2, 1.4 Hz, 1H), 6.57-6.61 (m, 2H), 5.20 (d, *J* = 4.1 Hz, 2H), 4.54 (s, 2H), 3.61 (d, *J =* 3.2 Hz, 3H). 2.66 -2.8(m, 2H), 2.42-2.48 (m, 2H). MS (ESI): m/z 431 (M +1).

**2-(2-oxo-2-((3-phenoxyphenyl)amino)ethoxy)-N-(2-oxo-2-phenylethyl)benzamid (DKC1125h14)¹H-NMR** (400 MHz, CDCl₃) δ 8.71 (s, 1H), 8.17 (q, *J* = 2.3 Hz, 1H), 8.14 (dd, *J* = 7.8, 1.8 Hz, 1H), 7.90-7.92 (m, 2H), 7.70-7.80 (m, 2H), 7.61-7.64 (m, 2H), 7.50-7.60 (m, 2H), 7.46-7.53 (m, 3H), 7.15-7.19 (m, 2H), 7.03 (d, *J* = 8.2 Hz, 2H), 6.57-6.61 (m, 1H), 5.02 (d, *J = 4.1* Hz, 2H), 4.67 (s, 2H). MS (ESI): m/z 481 (M +1).

**2-(2-((3,4-dimethoxybenzyl)amino)-2-oxoethoxy)-N-(2-oxo-2-phenylethyl)benzamide (DKC1125h15)** ¹H-NMR (400 MHz, CDCl₃) δ 8.22 (s, 2H), 8.18 (q, *J = 2.3* Hz, 2H), 8.12 (dd, *J =* 7.8, 1.8 Hz, 2H), 7.90-7.91 (m, 2H), 7.60-7.64 (m, 1H), 7.15-7.19 (m, 1H), 7.03 (d, *J* = 8.2 Hz, 2H), 6.82 (dd, *J =* 8.2, 1.4 Hz, 1H), 6.57-6.61 (m, 1H), 5.10 (d, *J*= 4.1 Hz, 2H), 4.02 (s, 2H), 3.61 (d, *J* = 3.2 Hz, 3H), 2.31 (d, *J* = 2.3 Hz, 3H). MS (ESI): m/z 463 (M +1).

**2-(2-((3,4-dimethoxyphenyl)amino)-2-oxoethoxy)-N-(2-oxo-2-phenylethyl)benzamide (DKC1125h16)** ¹H-NMR (400 MHz, CDCl₃) δ 8.20 (s, 2H), 8.17 (q, *J = 2.3* Hz, 2H), 8.14 (dd, *J =* 7.8, 1.8 Hz, 2H), 7.90-7.92 (m, 2H), 7.60-7.64 (m, 1H), 7.15-7.19 (m, 1H), 7.03 (d, *J* = 8.2 Hz, 2H), 6.82 (dd, *J =* 8.2, 1.4 Hz, 1H), 6.57-6.61 (m, 1H), 5.34 (d, *J* = 4.1 Hz, 2H), 4.33 (s, 2H), 3.61 (d, *J* = 3.2 Hz, 3H), 2.31 (d, *J* = 2.3 Hz, 3H). MS (ESI): m/z 449 (M +1).

**2-(2-(benzo[d][1,3]dioxol-5-ylamino)-2-oxoethoxy)-N-(2-oxo-2-phenylethyl)benzamide (DKC1125h17)**¹H-NMR (400 MHz, CDCl₃) δ 8.90 (s, 1H), 8.71 (s, 1H), 8.17 (q, *J* = 2.3 Hz, 1H), 8.14 (dd, *J* = 7.8, 1.8 Hz, 1H), 7.90-7.92 (m, 2H), 7.60-7.64 (m, 1H), 7.46-7.53 (m, 3H), 7.15-7.19 (m, 1H), 7.03 (d, *J* = 8.2 Hz, 1H), 6.82 (dd, *J* = 8.2, 1.4 Hz, 1H), 6.57-6.61 (m, 1H), 6.0 (s, 2H), 5.01 (d, *J* = 4.1 Hz, 2H), 4.68 (s, 2H). MS (ESI): m/z 433 (M +1).

**2-(2-((2, 3-dihydrobenzo[b][1, 4]dioxin-6-yl)amino)-2-oxoethoxy)-N-(2-oxo-2-phenylethyl)benzamide (DKC1125h18)** ¹H-NMR (400 MHz, CDCl₃) δ 9.01 (s, 1H), 8.71 (s, 1H), 8.17 (q, *J =* 2.3 Hz, 1H), 8.14 (dd *J =* 7.8, 1.8 Hz, 1H), 7.90-7.92 (m, 2H), 7.60-7.64 (m, 1H), 7.46-7.53 (m, 3H), 7.15-7.19 (m, 1H), 7.03 (d, *J* = 8.2 Hz, 1H), 6.82 (dd, *J* = 8.2, 1.4 Hz, 1H), 6.57-6.61 (m, 1H), 6.34-6.45 (m, 2H), 6.12- 6.23 (m, 2H), 5.43 (d, *J* = 4.1 Hz, 2H), 4.90 (s, 2H). MS (ESI): m/z 447 (M +1).

**2-(2-((2,3-dihydro-1H-inden-5-yl)amino)-2-oxoethoxy)-N-(2-oxo-2-phenylethyl)benzamide (DKC1125h19)** ¹H-NMR (400 MHz, CDCl₃) δ 8.71 (s, 2H), 8.17 (q, *J* = 2.3 Hz, 1H), 8.14 (dd, *J =* 7.8, 1.8 Hz, 1H), 7.90-7.92 (m, 2H), 7.60-7.64 (m, 1H), 7.46-7.53 (m, 3H), 7.15-7.19 (m, 1H), 7.03 (d, *J =* 8.2 Hz, 1H), 6.82 (dd, *J =* 8.2, 1.4 Hz, 1H), 6.57-6.61 (m, 1H), 5.15 (d, *J* = 4.1 Hz, 2H), 4.78 (s, 2H), 3.60-3.62 (d, *J* = 3.1 Hz, 2H), 3.64-3.66 (d, *J* = 3.4 Hz, 2H), 2.31-2.34 *(d, J* = 2.32 Hz, 2H). MS (ESI): m/z 429 (M +1).

**2-(2-((3-isopropoxyphenyl)amino)-2-oxoethoxy)-N-(2-oxo-2-phenylethyl)benzamide (DKC1125h20)** ¹H-NMR (400 MHz, CDCl₃) δ 9.12 (s, 1H), 8.43(s, 1H), 8.01-8.03 (m, 2H), 7.81-7.84 (m, 1H), 7.74-7.77 (m, 1H), 7.49-7.56 (m, 3H), 7.20 (dd, *J =* 7.4, 1.8 Hz, 3H), 7.10-7.15 (m, 2H), 7.05-7.08 (m, 2H), 5.03 (t, *J* = 3.9 Hz, 2H), 4.83 (s, 2H), 4.76-4.80 (m, 3H), 4.32-4.40 (m, 3H). MS (ESI): m/z 447 (M +1).

**2-(2-oxo-2-((4-phenoxyphenyl)amino)ethoxy)-N-(2-oxo-2-phenylethyl)benzamide (DKC1125h21)¹H-NMR** (400 MHz, CDCl₃) δ 8.31 (s, 1H), 8.17 (q, *J* = 2.3 Hz, 1H), 8.14 (dd, *J* = 7.8, 1.8 Hz, 2H), 7.90-7.92 (m, 2H), 7.80 (dd, *J* = 8.0, 1.8 Hz, 2H), 7.61-7.64 (m, 2H), 7.56 (s, 2H), 7.46-7.53 (m, 3H), 7.32 (d, *J* = 8.2 Hz, 1H), 7.24 (dd, *J* = 6.7, 1.4 Hz, 1H), 6.82 (dd, *J* = 8.2, 1.4 Hz, 1H), 6.57-6.61 (m, 1H), 5.03 (d, *J* = 4.1 Hz, 2H), 4.89 (s, 2H). MS (ESI): m/z 481 (M +1).

**2-(2-((2-fluoro-5-(trifluoromethyl) phenyl)amino)-2-oxoethoxy)-N-(2-oxo-2-phenylethyl)benzamide (DKC1125h22)** ¹H-NMR (400 MHz, CDCl₃) δ 8.71 (s, 2H), 8.37 (q, *J* = 2.3 Hz, 2H), 8.12-8.18 (dd, *J =* 7.8, 1H), 7.90-7.92 (m, 2H), 7.46-7.53 (m, 3H), 7.15-7.19 (m, 1H), 7.03 *(d, J =* 8.2 Hz, 1H), 6.82 (dd, *J* = 8.2, 1.4 Hz, 1H), 6.57-6.61 (m, 1H), 5.13 (d, *J* = 4.1 Hz, 2H), 4.89 (s, 2H). MS (ESI): m/z 475 (M +1).

**2-(2-((3-hydroxyphenyl)amino)-2-oxoethoxy)-N-(2-oxo-2-phenylethyl)benzamide (DKC1125h23)** ¹H-NMR (400 MHz, CDCl₃) δ 9.56 (s, 1H), 8.43 (s, 1H), 8.01-8.03 (dd, *J* = 8.0, 1.4 Hz, 2H), 7.81-7.84 (m, 1H), 7.74-7.77 (m, 1H), 7.49-7.56 (m, 3H), 7.20-7.24 (m, 3H), 7.10-7.15 (m, 2H), 7.05-7.08 (m, 1H), 5.40 (m, 1H), 5.03 (t, *J =* 3.9 Hz, 2H), 4.83 (s, 2H). MS (ESI): m/z 405 (M +1).

**2-(2-((2-chlorophenyl)amino)-2-oxoethoxy)-N-(2-oxo-2-phenylethyl)benzamide (DKC1125h24)** ¹H-NMR (400 MHz, CDCl₃) δ 9.66 (s, 1H), 8.20(s, 1H), 8.11-8.13 (m, 2H), 7.81-7.84 (m, 1H), 7.74-7.77 (m, 1H), 7.49-7.56 (m, 3H), 7.29 (dd, *J* = 6.7, 1.8 Hz, 3H), 7.10-7.15 (m, 2H), 7.05-7.08 (m, 1H), 5.24 (t, *J =* 3.9 Hz, 2H), 4.40 (s, 2H). MS (ESI): m/z 423 (M +1).

**2-(2-((3-bromophenyl)amino)-2-oxoethoxy)-N-(2-oxo-2-phenylethyl)benzamide (DKC1125h25)** ¹H-NMR (400 MHz, CDCl₃) δ 9.76 (s, 1H), 8.39(s, 1H), 8.01-8.03 (m, 2H), 7.81-7.84 (m, 1H), 7.74-7.77 (m, 2H), 7.49-7.56 (m, 3H), 7.24 (dd, *J =* 6.7, 1.4 Hz, 2H), 7.10-7.15 (m, 2H), 7.05-7.08 (m, 1H), 5.12 (t, *J =* 3.9 Hz, 2H), 4.90 (s, 2H). MS (ESI): m/z 467 (M +1).

**2-(2-(isopropylamino)-2-oxoethoxy)-N-(2-oxo-2-phenylethyl)benzamide (DKC1125h26)** ¹H-NMR (400 MHz, CDCl₃) δ 9.76 (s, 1H), 8.01-8.03 (s, 1H), 7.81-7.84 (m, 1H), 7.74-7.77 (m, 2H), 7.30-7.34 (m, 2H), 7.10-7.15 (m, 2H), 7.05-7.08 (m, 1H), 6.01-6.1 (m, 1H), 5.03 (t, *J =* 3.9 Hz, 2H), 4.83 (s, 2H), 4.9 (s, 3H), 4.3 (s, 3H). MS (ESI): m/z 355 (M +1).

**2-(2-(3,4-dihydroisoquinolin-2(1H)-yl)-2-oxoethoxy)-N-(2-oxo-2-phenylethyl)benzamide (DKC1125h27) ¹H-NMR** (400 MHz, CDCl₃) δ 8.71 (s, 1H), 8.17 (d, J = 2.3 Hz, 1H), 8.14 (dd, *J* = 7.8, 1.8 Hz, 1H), 7.90-7.92 (m, 2H), 7.60-7.64 (m, 1H), 7.46-7.53 (m, 3H), 7.15-7.19 (m, 1H), 7.03 (d, *J =* 8.2 Hz, 1H), 6.82 (dd, *J* = 8.2, 1.4 Hz, 2H), 6.57-6.61 (m, 1H), 5.03 (d, *J* = 4.1 Hz, 2H), 4.89 (s, 2H), 3.60-3.62 (d, *J =* 3.1 Hz, 2H), 3.64-3.66 (d, *J =* 3.4 Hz, 2H), 2.31-2.34 (d, *J =* 2.3 Hz, 2H). MS (ESI): m/z 429 (M +1).

**2-(2-oxo-2-propylamino)ethoxy)-N-(2-oxo-2-phenylethyl)benzamide (DKC1125h28)** ¹H-NMR (400 MHz, CDCl₃) δ 8.39(s, 1H), 8.02 (d, *J* = 7.3 Hz, 1H), 7.80 (dd, *J* = 7.8, 1.4 Hz, 2H), 7.62 (d, *J =* 3.2 Hz, 3H), 7.56-7.58 (m, 3H), 6.89-6.96 (m, 1H), 5.03 (t, *J =* 3.9 Hz, 2H), 4.83 (s, 2H), 4.04-4.10 (m, 2H), 3.95 (t, *J =* 5.7 Hz, 2H), 3.7 (s, 3H). MS (ESI): m/z 355 (M +1).

**2-(2-(isopentylamino)-2-oxoethoxy)-N-(2-oxo-2-phenylethyl)benzamide (DKC1125h29)** ¹H-NMR (400 MHz, CDCl₃) δ 8.96 (s, 1H) 8.02 (d, *J* = 7.3 Hz, 1H), 7.72 (dd, *J* = 7.8, 1.4 Hz, 2H), 7.71 (d, *J =* 3.2 Hz, 3H), 7.52-7.56 (m, 3H), 6.89-6.94 (m, 1H), 5.03 (t, *J =* 3.9 Hz, 2H), 4.83 (s, 2H), 4.66 (s, 1H), 4.04-4.10 (m, 2H), 3.95 (t, *J=* 5.7 Hz, 2H), 3.8 (s, 3H), 3.3 (s, 3H). MS (ESI): m/z 383(M +1).

**2-(2-(cyclohexylamino)-2-oxoethoxy)-N-(2-oxo-2-phenylethyl)benzamide (DKC1125h30)** ¹H-NMR (400 MHz, CDCl₃) δ 8.02 (d, *J* = 7.3 Hz, 1H), 7.79 (dd, *J* = 7.8, 1.4 Hz, 1H), 7.71 (d, *J* = 3.2 Hz, 3H), 7.52-7.56 (m, 3H), 6.89-6.94 (m, 1H), 5.03 (t, *J* = 3.9 Hz, 2H), 4.83 (s, 2H), 4.42-4.48 (m, 1H), 3.26 (td, *J =* 8.7, 4.3 Hz, 2H), 2.4 (m, 2H), 2.17-2.25 (m, 4H), 2.06 -2.14 (m, 2H). MS (ESI): m/z 395 (M +1).

### Examples 231 to 305. Preparation of Compounds 231 to 305 (DKC1125i01 to 75) according to the Present Invention

### Synthesis of Compounds 231 to 305 (DKC1125i01 to 75)

Compounds 231 to 305 (DKC1125i01-75) of the present invention were synthesized according to the following Reaction Scheme 8.

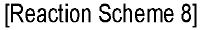

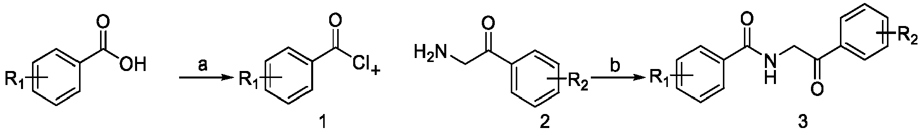

R₁ = CH₂Br
R₂ = H, OCH₃

### Preparation method for bromomethylbenzoyl chloride (1)

SOCl₂ (2 mL, 27.91 mmol) was added dropwise to bromomethylbenzoic acid (1g, 4.651 mmol) under an anhydrous atmosphere at room temperature. The reaction mixture was stirred under reflux for 4.5 hrs. After completion of the reaction, excess of SOCl₂ was removed by evaporation to obtain bromomethylbenzoyl chloride **(1)** (1.62 g, 6.95 mmol, 73%) which was used in the next step without any purification.

### Preparation method for 3-(bromomethyl)-N-(2-oxo-2-phenylethyl)benzamide (R₁=CH₂Br, R₂=H) (3)

To 3-(bromomethyl)benzoyl chloride **(1)** (1 g, 4.29 mmol) in DCM (100 mL) was added 2-amino-1-phenylethan-1-one **(2)** (737 mg, 4.29 mmol) and added NaHCO₃ (50 mL). The reaction mixture was stirred at room temperature for 1 hr. The stirred mixture was extracted with DCM, washed with brine, and then dried over anhydrous Na₂SO₄. Purification using an EA/HEX system column afforded 3-(bromomethyl)-N-(2-oxo-2-phenylethyl)benzamide (R₁=CH₂Br, R₂=H **(3)** (883 mg, 62%).

¹H-NMR (400 MHz, CDCl₃) *δ* 8.03-8.05 (m, 2H), 7.86-7.88 (m, 2H), 7.65 (d, *J* = 7.3 Hz, 1H), 7.49-7.56 (m, 4H), 7.30 (s, 1H), 4.97 (d, *J* = 4.1 Hz, 2H), 4.52 (s, 2H).

### Preparation method for 4-(bromomethyl)-N-(2-oxo-2-phenylethyl)benzamide (R₁=CH₂Br, R₂=H) (3)

To 4-(bromomethyl)benzoyl chloride **(1)** (1 g, 4.29 mmol) in DCM (100 mL) was added 2-amino-1-phenylethan-1-one **(2)** (736 mg, 4.29mmol) and added NaHCO₃ (50 mL). The reaction mixture was stirred at room temperature for 1 hr. The stirred mixture was extracted with DCM, washed brine and then dried over anhydrous Na₂SO₄ Purification using an EA/HEX system column afforded 4-(bromomethyl)-N-(2-oxo-2-phenylethyl)benzamide (R₁=CH₂Br, **R₂=H) (3)** (1.18 g, 3.56 mmol, 83%).

¹H-NMR (400 MHz, CDCl₃) δ 8.05 (dd, *J =* 8.5, 1.1 Hz, 2H), 7.91-7.92 (m, 1H), 7.81 (dd, *J* = 6.2, 1.6 Hz, 1H), 7.64-7.68 (m, 1H), 7.52-7.59 (m, 3H), 7.47 (t, *J =* 7.8 Hz, 1H), 7.31 (s, 1H), 4.98 (d, *J =* 4.1 Hz, 2H), 4.55 (s, 2H).

### Preparation method for 3-(bromomethyl)-N-(2-(3-methoxyphenyl)-2-oxoethyl)benzamide (R₁=CH₂Br, R₂=OCH₃) (3)

To 3-(bromomethyl)benzoyl chloride **(1)** (1 g, 4.29 mmol) in DCM (100 mL) was added 1-amino-3-(3-methoxyphenyl)propan-2-one **(2)** (736 mg, 4.29 mmol) and added NaHCO₃ (50 mL). The reaction mixture was stirred at room temperature for 1 hr. The stirred mixture was extracted with DCM, washed brine and then dried over anhydrous Na₂SO₄ Purification using an r EA/HEX system column afforded 3-(bromomethyl)-N-(2-(3-methoxyphenyl)-2-oxoethyl)benzamide (R₁=CH₂Br, R₂=OCH₃) **(3)** (1.01 g, 65%).

¹H-NMR (400 MHz, CDCl₃) *δ* 7.91-7.92 (m, 1H), 7.81 (dt, *J =* 7.8, 1.4 Hz, 1H), 7.61-7.64 (m, 1H), 7.54-7.59 (m, 2H), 7.46 (q, *J* = 8.1 Hz, 2H), 7.18-7.21 (m, 1H), 4.96 (d, *J* = 4.1 Hz, 2H), 4.55 (s, 2H), 3.89 (s, 3H).

### Preparation method for 2-(bromomethyl)-N-(2-oxo-2-phenylethyl)benzamide (R₁=CH₂Br, R₂=H) (3)

To 2-(bromomethyl)benzoyl chloride **(1)** (1 g, 4.29 mmol) in DCM (100 mL) was added 2-amino-1-phenylethan-1-one **(2)** (736 mg, 4.29 mmol) and added NaHCO₃ (50 mL). The reaction mixture was stirred at room temperature for 1 hr. The stirred mixture was extracted with DCM, washed brine and then dried over anhydrous Na₂SO₄ The resulting crude product was purified by column chromatography to obtain the title compound **(3)** (955 mg, 67%).

¹H-NMR (400 MHz, CDCl₃) δ 8.02-8.05 (m, 2H), 7.66 (tt, J = 7.5, 1.4 Hz, 1H), 7.37-7.59 (m, 6H), 7.10 (s, 1H), 4.99 (d, *J* = 4.6 Hz, 2H), 4.84 (s, 2H).

### General Preparation Method for Compounds 231 to 253 (DKC1125i01 to 23)

The title compounds were prepared. Specifically, to 3-(bromomethyl)-N-(2-oxo-2-phenylethyl)benzamide (R₁=CH₂Br, R₂=H) **(3)** (50 mg, 0.15 mmol) in DCM(1 mL) was added secondary amine described in Table 10 below. Then, DIEA (39 mg, 0.30 mmol) was added thereto. The reaction mixture was stirred at 35°C for 3 hrs. Purification using an EA/HEX system column afforded the final compound (yield: 50 to 60%).

**[Table 10]**

| Secondary amines used in preparation of compounds 231 to 253 (DKC1125i01 to 23) | | | | |
|---|---|---|---|---|
| **Compounds** | **Secondary amine** | | **Compounds** | **Secondary amine** |
| **DKC1125i01** | Dimethylamine hydrochloride | | **DKC1125i13** | 2-(Methylamino)ethanol |
| **DKC1125i02** | 1,2,3,4-tetrahydroisoquinoline | | **DKC1125i14** | 2-(Butylamino)ethanol |
| **DKC1125i03** | N-methylisopropylamine | | **DKC1125i15** | 1-Boc-piperazine |
| **DKC1125i04** | 1-(2-fluorophenyl)piperazine | | **DKC1125i16** | N-methyl-1-butylamine |
| **DKC1125i05** | 2-(1-Piperazinyl)pyrimidine | | **DKC1125i17** | Diethylamine |
| **DKC1125i06** | 1-(2-methoxyphenyl)piperazine | | **DKC1125i18** | Di-n-propylamine |
| **DKC1125i07** | 4-Piperidinopiperidine | | **DKC1125i19** | N-ethylbutylamine |
| **DKC1125i08** | 1-(4-nitrophenyl)piperazine | | **DKC1125i20** | Dipentylamine |
| **DKC1125i09** | N-Ethylaniline | | **DKC1125i21** | N-Methylpropargylamine |
| **DKC1125i10** | 1-(2-pyridyl)piperazine,HCl | | **DKC1125i22** | Morpholine |
| **DKC1125i11** | Diallylamine | | **DKC1125i23** | 1-methylpiperazine |
| **DKC1125i12** | N-Benzylmethylamine | | | |

### General Preparation Method for Compounds 254 to 278 (DKC1125i24 to 48)

To 4-(bromomethyl)-N-(2-oxo-2-phenylethyl)benzamide (R₁=CH₂Br, **R₂=H) (3)** (50 mg, 0.15 mmol) in DCM (1 mL) was added secondary amine shown in Table 11 below. Then, DIEA (39 mg, 0.30 mmol) was added thereto. The reaction mixture was stirred at 35°C for 3 hrs. Purification using an EA/HEX system column afforded to the final compound (50 to 60% yield).

**[Table 11]**

| Secondary amines used in preparation of compounds 254 to 278 (DKC1125i24 to 48) | | | | |
|---|---|---|---|---|
| **Compounds** | **Secondary amine** | | **Compounds** | **Secondary amine** |
| **DKC1125i24** | 1,2,3,4-tetrahydroisoquinoline | | **DKC1125i37** | 2-(Methylamino)ethanol |
| **DKC1125i25** | N-methylcyclohexylamine | | **DKC1125i38** | 2-(Butylamino)ethanol |
| **DKC1125i26** | N-methylisopropylamine | | **DKC1125i39** | 1-Boc-piperazine |
| **DKC1125i27** | 1-(2-fluorophenyl)piperazine | | **DKC1125i40** | dicyclohexylamine |
| **DKC1125i28** | 2-(1-Piperazinyl)pyrimidine | | **DKC1125i41** | N-methyl-1-butylamine |
| **DKC1125i29** | 1-(2-methoxyphenyl)piperazine | | **DKC1125i42** | Di-n-propylamine |
| **DKC1125i30** | 4-Piperidinopiperidine | | **DKC1125i43** | N-ethylbutylamine |
| **DKC1125i31** | 1-(4-nitrophenyl)piperazine | | **DKC1125i44** | 2-(Ethylamino)ethanol |
| **DKC1125i32** | N-Methylaniline | | **DKC1125i45** | N-Methylpropargylamine |
| **DKC1125i33** | N-Ethylaniline | | **DKC1125i46** | N-ethylpropan-1-amine |
| **DKC1125i34** | 1-(2-pyridyl)piperazine,HCl | | **DKC1125i47** | Ethyl piperazine-1-carboxylate |
| **DKC1125i35** | Diallylamine | | **DKC1125i48** | 2,6-dimethylmorpholine |
| **DKC1125i36** | N-Benzylmethylamine | | | |

### General Preparation Method for Compounds 279 to 295 (DKC1125i49 to 65)

To 3-(bromomethyl)-N-(2-(3-methoxyphenyl)-2-oxoethyl)benzamide (R₁=CH₂Br, R₂=OCH₃) **(3)** (50 mg, 0.15 mmol) in DCM (1 mL) was added secondary amine shown in Table 12 below. Then, DIEA (39 mg, 0.30 mmol) was added thereto. The reaction mixture was stirred at 35°C for 3 hrs. Purification using an EA/HEX system column afforded the final compound (50 to 60% yield).

**[Table 12]**

| Secondary amines used in preparation of compounds 279 to 295 (DKC1125i49 to 65) | | | | |
|---|---|---|---|---|
| **Compounds** | **Secondary amine** | | **Compounds** | **Secondary amine** |
| **DKC1125i49** | 1,2,3,4-tetrahydroisoquinoline | | **DKC1125i58** | 2-(Butylamino)ethanol |
| **DKC1125i50** | N-methylcyclohexylamine | | **DKC1125i59** | 1-Boc-piperazine |
| **DKC1125i51** | 1-(2-fluorophenyl)piperazine | | **DKC1125i60** | N-ethylbutylamine |
| **DKC1125i52** | 1-(2-methoxyphenyl)piperazine | | **DKC1125i61** | Dipentylamine |
| **DKC1125i53** | 4-Piperidinopiperidine | | **DKC1125i62** | 1-Piperazineethanol |
| **DKC1125i54** | 1-(2-pyridyl)piperazine,HCl | | **DKC1125i63** | morpholine |
| **DKC1125i55** | Diallylamine | | **DKC1125i64** | N-ethylpropan-1-amine |
| **DKC1125i56** | N-Benzylmethylamine | | **DKC1125i65** | 2,6-dimethylmorpholine |
| **DKC1125i57** | N-Ethylmethylamine | | | |

### General Preparation Method for Compounds 296 to 305 (DKC1125i66 to 75)

To 2-(bromomethyl)-N-(2-oxo-2-phenylethyl)benzamide (R₁=CH₂Br, **R₂=H) (3)** (50 mg, 0.15 mmol) in DCM(1 mL) was added secondary amine shown in Table 13 below. Then, DIEA (39 mg, 0.30 mmol) was added thereto. The reaction mixture was stirred at 35°C for 3 hrs. Purification using an EA/HEX system column afforded the final compound (50 to 60% yield).

**[Table 13]**

| Secondary amines used in preparation of compounds 296 to 305 (DKC1125i66 to 75) | | | | |
|---|---|---|---|---|
| **Compounds** | **Secondary amine** | | **Compounds** | **Secondary amine** |
| **DKC1125i66** | Dimethylamine hydrochloride | | **DKC1125i71** | 4-Piperidinopiperidine |
| **DKC1125i67** | 1,2,3,4-tetrahydroisoquinoline | | **DKC1125i72** | 1-(2-pyridyl)piperazine,HCl |
| **DKC1125i68** | N-methylcyclohexylamine | | **DKC1125i73** | Diallylamine |
| **DKC1125i69** | 1-(2-fluorophenyl)piperazine | | **DKC1125i74** | Morpholine |
| DKC1125i70 | 2-(1-Piperazinyl)pyrimidine | | **DKC1125i75** | Ethyl piperazine-1-carboxylate |

### Confirmation of Preparation of Compounds 231 to 305 (DKC1125i01 to 75)

**3-((dimethylamino)methyl)-N-(2-oxo-2-phenylethyl)benzamide (DKC1125i01)** ¹H-NMR (400 MHz, CDCl₃) δ 8.02-7.29 (m, 10H), 4.94 (d, *J* = 4.0 Hz, 2H), 3.62 (s, 2H), 2.34 (s, 6H). MS(ESI): m/z 297 (M+1).

**3-((3,4-dihydroisoquinolin-2(1H)-yl)methyl)-N-(2-oxo-2-phenylethyl)benzamide (DKC1125i02)** ¹H-NMR (400 MHz, CDCl₃) δ 8.01-7.44 (m, 10H), 7.10 (s, 3H), 6.97 (d, 8.0Hz, 1H), 4.96 (d, *J* = 4 Hz 2H), 3.75 (s, 2H), 3.65 (s, 2H), 2.79 (t, *J* = 6.0 Hz, 2H), 1.26 (t, *J* = 6.8 Hz, 2H). MS(ESI): m/z 385 (M+1).

**3-((isopropyl(methyl)amino)methyl)-N-(2-oxo-2-phenylethyl)benzamide (DKC1125i03)** ¹H-NMR (400 MHz, CDCl₃) δ 8.03-7.40 (m, 10H), 4.95 (d, *J* = 4.0 Hz, 2H), 3.69 (s, 2H), 3.05 (q, *J* = 6.4 Hz, 1H), 2.24 (s, 3H), 1.16 (d, *J* = 6.4 Hz, 6H). MS(ESI): m/z 325 (M+1).

**3-((4-(2-fluorophenyl)piperazin-1-yl)methyl)-N-(2-oxo-2-phenylethyl)benzamide (DKC1125i04)** ¹H-NMR (400 MHz, CDCl₃) δ 8.04-8.06 (m, 2H), 7.89 (s, 1H), 7.79 (d, *J =* 7.8 Hz, 1H), 7.66 (t, *J* = 7.5 Hz, 1H), 7.52-7.56 (m, 3H), 7.44 (t, *J* = 7.8 Hz, 1H), 7.33 (s, 1H), 6.90-7.07 (m, 4H), 4.98 (d, *J* = 4.6 Hz, 2H), 3.65 (s, 2H), 3.13 (t, *J* = 4.6 Hz, 4H), 2.67 (t, *J* = 4.3 Hz, 4H). MS(ESI): m/z 432 (M+1).

**N-(2-oxo-2-phenylethyl)-3-((4-(pyrimidin-2-yl)piperazin-1-yl)methyl)benzamide (DKC1125i05)** ¹H-NMR (400 MHz, CDCl₃) δ 8.27 (d, *J* = 4.8 Hz, 2H), 8.02-7.39 (m, 10H), 6.45 (t, *J* = 4.8 Hz, 1H), 4.95 (d, *J* = 4.4 Hz, 2H), 3.83 (t, *J* = 4.8 Hz, 4H), 3.60 (s, 2H), 2.52 (t, *J* = 4.8 Hz, 4H). MS(ESI): m/z 417 (M+1).

**3-((4-(2-methoxyphenyl)piperazin-1-yl)methyl)-N-(2-oxo-2-phenylethyl)benzamide (DKC1125i06)** ¹H-NMR (400 MHz, CDCl₃) δ 8.09-7.33 (m, 10H), 7.01-6.84 (m, 4H), 4.98 (d, *J* = 4.0 Hz, 2H), 3.85 (s, 3H), 3.65 (s, 2H), 3.10(s, 4H), 2.68 (s, 4H). MS(ESI): m/z 445 (M+1).

**3-([1,4'-bipiperidin]-1'-ylmethyl)-N-(2-oxo-2-phenylethyl)benzamide (DKC1125i07)** ¹H-NMR (400 MHz, CDCl₃) δ 8.01 (d, *J* = 8.7 Hz, 2H), 7.80 (s, 1H), 7.76 (d, *J* = 7.3 Hz, 1H), 7.63 (dd, J = 8.0, 6.6 Hz, 1H), 7.49-7.53 (m, 2H), 7.34-7.45 (m, 3H), 4.94 (d, *J =* 4.1 Hz, 2H), 3.52 (s, 2H), 2.95-3.10 (m, 6H), 2.01-2.13 (m, 5H), 1.75-1.84 (m, 2H), 1.44-1.58 (m, 4H), 1.16-1.30 (m, 2H). MS(ESI): m/z 421 (M+1).

**3-((4-(4-nitrophenyl)piperazin-1-yl)methyl)-N-(2-oxo-2-phenylethyl)benzamide (DKC1125i08)** ¹H-NMR (400 MHz, CDCl₃) δ 8.02-8.14 (m, 4H), 7.88 (s, 1H), 7.79 (d, *J* = 7.8 Hz, 1H), 7.63-7.67 (m, 1H), 7.51-7.54 (m, 3H), 7.41-7.46 (m, 1H), 7.34 (s, 1H), 6.79 (d, *J* = 9.6 Hz, 2H), 4.97 (d, *J* = 4.1 Hz, 2H), 3.62 (s, 2H), 3.42 (t, *J* = 5.0 Hz, 4H), 2.60 (t, *J* = 5.0 Hz, 4H). MS(ESI): m/z 460 (M+1).

**3-((ethyl(phenyl)amino)methyl)-N-(2-oxo-2-phenylethyl)benzamide (DKC1125i09)** ¹H-NMR (400 MHz, CDCl₃) δ 8.01-8.04 (m, 5H), 7.50-7.81 (m, 7H), 6.69 (s, 3H), 4.94 (d, *J* = 4.1 Hz, 2H), 4.53 (s, 2H), 3.49 (brs, 2H), 1.67 (brs, 3H). MS(ESI): m/z 373 (M+1).

**N-(2-oxo-2-phenylethyl)-3-((4-(pyridin-2-yl)piperazin-1-yl)methyl)benzamide (DKC1125i10)** ¹H-NMR (400 MHz, CDCl₃) δ 7.98-8.17 (m, 4H), 7.82 (d, *J=* 7.8 Hz, 1H), 7.43-7.65 (m, 7H), 6.61-6.67 (m, 2H), 4.95 (d, *J* = 4.4 Hz, 2H), 3.75 (s, 2H), 3.67 (t, *J =* 4.8 Hz, 4H), 2.70 (brs, 4H). MS(ESI): m/z 416 (M+1).

**3-((diallylamino)methyl)-N-(2-oxo-2-phenylethyl)benzamide (DKC1125i11)** ¹H-NMR (400 MHz, CDCl₃) δ 8.03-8.06 (m, 2H), 7.30-7.84 (m, 8H), 5.85-5.95 (m, 2H), 5.15-5.24 (m, 4H), 4.97 (d, *J =* 4.1 Hz, 2H), 3.64 (s, 2H), 3.10 (d, *J* = 6.4 Hz, 4H). MS(ESI): m/z 349 (M+1).

**3-((benzyl(methyl)amino)methyl)-N-(2-oxo-2-phenylethyl)benzamide (DKC1125i12)** ¹H-NMR (400 MHz, CDCl₃) δ 8.04-8.07 (m, 2H), 7.88 (s, 1H), 7.27-7.78 (m, 12H), 4.98 (d, *J* = 4.1 Hz, 2H), 3.59 (s, 2H), 3.55 (s, 2H), 2.20 (s, 3H). MS(ESI): m/z 373 (M+1).

**3-(((2-hydroxyethyl)(methyl)amino)methyl)-N-(2-oxo-2-phenylethyl)benzamide (DKC1125i13)** ¹H-NMR (400 MHz, CDCl₃) δ 8.03 (dd, *J* = 8.5, 1.1 Hz, 2H), 7.92 (s, 1H), 7.79-7.82 (m, 1H), 7.62-7.64 (m, 1H), 7.42-7.54 (m, 5H), 4.95 (d, *J =* 4.1 Hz, 2H), 3.76 (s, 2H), 3.71 (t, *J =* 5.2 Hz, 2H), 3.13 (q, *J =* 7.3 Hz, 1H), 2.72 (t, *J =* 5.3 Hz, 2H), 2.35 (s, 3H). MS(ESI): m/z 327 (M+1).

**3-((butyl(2-hydroxyethyl)amino)methyl)-N-(2-oxo-2-phenylethyl)benzamide (DKC1125i14)** ¹H-NMR (400 MHz, CDCl₃) δ 8.16 (s, 1H), 8.00-8.02 (m, 2H), 7.88 (d, *J* = 7.8 Hz, 1H), 7.61-7.69 (m, 3H), 7.45-7.53 (m, 3H), 4.94 (d, *J =* 4.6 Hz, 2H), 4.11 (s, 2H), 3.83 (t, *J =* 5.0 Hz, 2H), 3.13 (q, *J =* 7.5 Hz, 2H), 3.00 (t, *J =* 4.8 Hz, 2H), 2.86 (t, *J =* 7.8 Hz, 2H), 1.58 (t, *J =* 7.3 Hz, 2H), 0.90 (t, *J =* 7.5 Hz, 3H). MS(ESI): m/z 369 (M+1).

**tert-butyl 4-(3-((2-oxo-2-phenylethyl)carbamoyl)benzyl)piperazine-1 -carboxylate (DKC1125i15)** ¹H-NMR (400 MHz, CDCl₃) δ 8.02-8.04 (m, 2H), 7.84 (s, 1H), 7.77 (d, *J =* 7.8 Hz, 1H), 7.65 (t, *J* = 7.5 Hz, 1H), 7.40-7.55 (m, 4H), 7.32 (s, 1H), 4.97 (d, *J* = 4.1 Hz, 2H), 3.56 (s, 2H), 3.43 (t, *J* = 4.8 Hz, 4H), 2.40 (brt, 4H), 1.45 (s, 9H). MS(ESI): m/z 439 (M+1).

**3-((butyl(methyl)amino)methyl)-N-(2-oxo-2-phenylethyl)benzamide (DKC1125i16)** ¹H-NMR (400 MHz, CDCl₃) δ 8.17 (s, 1H), 7.99-8.04 (m, 3H), 7.90 (d, *J =* 7.8 Hz, 1H), 7.60-7.69 (m, 2H), 7.47-7.52 (m, 3H), 4.92 (d, *J* = 5.0 Hz, 2H), 4.03 (s, 2H), 2.81 (t, *J* = 8.0 Hz, 2H), 2.54 (s, 3H), 1.73-1.77 (m, 2H), 1.48-1.58 (m, 2H), 1.32-1.40 (m, 3H). MS(ESI): m/z 339 (M+1).

**3-((diethylamino)methyl)-N-(2-oxo-2-phenylethyl)benzamide (DKC1125i17)** ¹H-NMR (400 MHz, CDCl₃) δ 8.32 (s, 1H), 7.98-8.00 (m, 2H), 7.92 (d, *J* = 7.8 Hz, 1H), 7.84 (s, 1H), 7.71 (d, *J* = 7.8 Hz, 1H), 7.59-7.63 (m, 1H), 7.46-7.51 (m, 3H), 4.91 (d, *J* = 4.6 Hz, 2H), 4.12 (s, 2H), 3.02 (q, *J* = 7.3 Hz, 4H), 1.35 (t, *J =* 7.1 Hz, 6H). MS(ESI): m/z 325 (M+1).

**3-((dipropylamino)methyl)-N-(2-oxo-2-phenylethyl)benzamide (DKC1125i18)** ¹H-NMR (400 MHz, CDCl₃) δ 8.04 (d, *J* = 7.3 Hz, 2H), 7.84 (s, 1H), 7.75 (d, *J* = 7.8 Hz, 1H), 7.64 (t, *J* = 7.3 Hz, 1H), 7.33-7.54 (m, 5H), 4.96 (d, *J* = 4.6 Hz, 2H), 3.62 (s, 2H), 2.35-2.41 (m, 4H), 1.45-1.54 (m, 4H), 0.87 (t, *J* = 7.3 Hz, 6H). MS(ESI): m/z 353 (M+1).

**3-((butyl(ethyl)amino)methyl)-N-(2-oxo-2-phenylethyl)benzamide (DKC1125i19)** ¹H-NMR (400 MHz, CDCl₃) δ 7.37-8.18 (m, 10H), 4.94 (d, *J* = 4.1 Hz, 2H), 3.96 (s, 2H), 2.75-3.05 (m, 4H), 1.25-1.84 (m, 10H). MS(ESI): m/z 353 (M+1).

**3-((dipentylamino)methyl)-N-(2-oxo-2-phenylethyl)benzamide (DKC1125i20)** ¹H-NMR (400 MHz, CDCl₃) δ 7.31-8.05 (m, 10H), 4.97 (d, *J =* 4.1 Hz, 2H), 3.50-3.63 (m, 2H), 2.09-2.40 (m, 8H), 1.01-1.57 (m, 14H). MS(ESI): m/z 410 (M+1).

**3-((ethynyl(methyl)amino)methyl)-N-(2-oxo-2-phenylethyl)benzamide (DKC1125i21)** ¹H-NMR (400 MHz, CDCl₃) δ 8.04 (d, *J* = 7.3 Hz, 2H), 7.79-7.85 (m, 2H), 7.63-7.67 (m, 1H), 7.32-7.55 (m, 5H), 4.97 (d, *J* = 4.1 Hz, 2H), 3.64 (s, 2H), 3.33 (d, *J* = 2.3 Hz, 1H), 2.35 (s, 3H). MS(ESI): m/z 307 (M+1).

**3-(morpholinomethyl)-N-(2-oxo-2-phenylethyl)benzamide (DKC1125i22)** ¹H-NMR (400 MHz, CDCl₃) δ 8.04 (d, *J* = 7.3 Hz, 2H), 7.85 (s, 1H), 7.77 (d, *J* = 7.3 Hz, 1H), 7.62-7.67 (m, 1H), 7.33-7.55 (m, 5H), 4.96 (d, *J* = 4.6 Hz, 2H), 3.71 (t, *J* = 4.6 Hz, 4H), 3.56 (s, 2H), 2.46 (t, *J* = 4.3 Hz, 4H). MS(ESI): m/z 339 (M+1).

**3-((4-methylpiperazin-1-yl)methyl)-N-(2-oxo-2-phenylethyl)benzamide (DKC1125i23)** ¹H-NMR (400 MHz, CDCl₃) δ 7.34-8.04 (m, 10H), 4.96 (d, *J* = 4.1 Hz, 2H), 3.56 (s, 2H), 2.52 (s, 11H). MS(ESI): m/z 352 (M+1).

**4-((3,4-dihydroisoquinolin-2(1H)-yl)methyl)-N-(2-oxo-2-phenylethyl)benzamide (DKC1125i24)** ¹H-NMR (400 MHz, CDCl₃) δ 8.03-8.08 (m, 2H), 7.86 (d, *J =* 8.2 Hz, 2H), 7.63-7.67 (m, 1H), 7.50-7.55 (m, 4H), 7.31 (brs, 1H), 7.08-7.13 (m, 3H), 6.98-7.00 (m, 1H), 4.97 (d, *J =* 4.1 Hz, 2H), 3.75 (s, 2H), 3.65 (s, 2H), 2.92 (t, *J* = 5.7 Hz, 2H), 2.76 (t, *J* = 5.9 Hz, 2H). MS(ESI): m/z 385 (M+1).

**4-((cyclohexyl(methyl)amino)methyl)-N-(2-oxo-2-phenylethyl)benzamide (DKC1125i25)** ¹H-NMR (400 MHz, CDCl₃) δ 8.08 (d, *J* = 7.3 Hz, 2H), 7.92 (d, *J* = 7.8 Hz, 2H), 7.55-7.71 (m, 5H), 7.38 (s, 1H), 5.01 (d, *J* = 4.6 Hz, 2H), 3.91 (s, 2H), 3.72-3.79 (m, 1H), 2.43 (s, 3H), 2.09 (d, *J* = 11.4 Hz, 2H), 1.89 (d, *J* = 12.8 Hz, 2H), 1.61-1.72 (m, 2H), 1.43 (dd, *J =* 12.1, 2.5 Hz, 2H), 1.21-1.33 (m, 2H). MS(ESI): m/z 365 (M+1).

**4-((isopropyl(methyl)amino)methyl)-N-(2-oxo-2-phenylethyl)benzamide (DKC1125i26)** ¹H-NMR (400 MHz, CDCl₃) δ 8.02-8.04 (m, 2H), 7.85-7.87 (m, 2H), 7.63-7.66 (m, 1H), 7.48-7.54 (m, 4H), 7.29 (s, 1H), 4.95 (d, *J =* 4.1 Hz, 2H), 4.50 (s, 2H), 1.60 (s, 3H), 1.24 (s, 6H), 0.81-0.88 (m, 1H). MS(ESI): m/z 325 (M+1).

**4-((4-(2-fluorophenyl)piperazin-1-yl)methyl)-N-(2-oxo-2-phenylethyl)benzamide (DKC1125i27)** ¹H-NMR (400 MHz, CDCl₃) δ 8.03-8.06 (m, 2H), 7.85-7.87 (m, 2H), 7.63-7.68 (m, 1H), 7.47-7.56 (m, 4H), 7.30 (t, *J* = 4.1 Hz, 1H), 6.91-7.08 (m, 4H), 4.98 (d, *J* = 4.6 Hz, 2H), 3.64 (s, 2H), 3.12 (t, *J* = 4.8 Hz, 4H), 2.65 (t, *J =* 4.8 Hz, 4H). MS(ESI): m/z 433 (M+1).

**N-(2-oxo-2-phenylethyl)-4-((4-(pyrimidin-2-yl)piperazin-1-yl)methyl)benzamide (DKC1125i28)** ¹H-NMR (400 MHz, CDCl₃) δ 8.30 (d, *J* = 4.6 Hz, 2H), 8.03-8.05 (m, 2H), 7.86 (d, *J* = 8.2 Hz, 2H), 7.65 (tt, *J* = 7.4, 1.4 Hz, 1H), 7.46-7.55 (m, 4H), 7.30 (t, *J* = 3.9 Hz, 1H), 6.47 (t, *J* = 4.6 Hz, 1H), 4.97 (d, *J* = 4.6 Hz, 2H), 3.84 (t, *J* = 5.0 Hz, 4H), 3.60 (s, 2H), 2.51 (t, *J* = 5.0 Hz, 4H). MS(ESI): m/z 417 (M+1).

**4-((4-(2-methoxyphenyl)piperazin-1-yl)methyl)-N-(2-oxo-2-phenylethyl)benzamide (DKC1125i29)** ¹H-NMR (400 MHz, CDCl₃) δ 8.03-8.05 (m, 2H), 7.86 (d, *J =* 8.2 Hz, 2H), 7.63-7.67 (m, 1H), 7.47-7.55 (m, 4H), 7.30 (t, *J =* 4.1 Hz, 1H), 6.84-7.02 (m, 4H), 4.97 (d = 4.1 Hz, 2H), 3.86 (s, 3H), 3.64 (s, 2H), 3.10 (brs, 4H), 2.67 (brs, 4H). MS(ESI): m/z 445 (M+1).

**4-([1,4'-bipiperidin]-1'-ylmethyl)-N-(2-oxo-2-phenylethyl)benzamide (DKC1125i30)** ¹H-NMR (400 MHz, CDCl₃) δ 7.99-8.02 (m, 2H), 7.82 (d, *J =* 8.2 Hz, 2H), 7.60-7.62 (m, 1H), 7.50 (t, *J =* 7.8 Hz, 2H), 7.34-7.38 (m, 3H), 4.93 (d, *J =* 4.1 Hz, 2H), 3.65-3.72 (m, 3H), 3.54 (s, 2H), 3.11 (q, *J =* 7.5 Hz, 4H), 2.98 (d, *J* = 12.3 Hz, 2H), 2.21 (d, *J* = 11.9 Hz, 2H), 2.09 (t, *J* = 11.2 Hz, 2H), 1.86 (dd, *J* = 11.9, 3.7 Hz, 2H), 1.55 (t, *J =* 7.5 Hz, 4H). MS(ESI): m/z 421 (M+1).

**4-((4-(4-nitrophenyl)piperazin-1-yl)methyl)-N-(2-oxo-2-phenylethyl)benzamide (DKC1125i31)** ¹H-NMR (400 MHz, CDCl₃) δ 8.10-8.14 (m, 2H), 8.03-8.06 (m, 2H), 7.87 (d, *J =* 8.2 Hz, 2H), 7.64-7.68 (m, 1H), 7.43-7.56 (m, 4H), 7.29 (t, *J* = 4.1 Hz, 1H), 6.80-6.84 (m, 2H), 4.98 (d, *J* = 4.6 Hz, 2H), 3.63 (s, 2H), 3.44 (t, *J =* 5.0 Hz, 4H), 2.61 (t, *J =* 5.3 Hz, 4H). MS(ESI): m/z 460 (M+1).

**4-((methyl(phenyl)amino)methyl)-N-(2-oxo-2-phenylethyl)benzamide (DKC1125i32)** ¹H-NMR (400 MHz, CDCl₃) *δ* 8.04 (d, *J =* 7.8 Hz, 2H), 7.82-7.87 (m, 2H), 7.51-7.67 (m, 3H), 7.33-7.40 (m, 2H), 7.22-7.24 (m, 1H), 6.73-6.91 (m, 5H), 4.96 (d, *J* = 4.1 Hz, 2H), 4.59 (s, 2H), 3.05 (s, 3H). MS(ESI): m/z 359 (M+1).

**4-((ethyl(phenyl)amino)methyl)-N-(2-oxo-2-phenylethyl)benzamide (DKC1125i33)** ¹H-NMR (400 MHz, CDCl₃) δ 8.02-8.05 (m, 3H), 7.82-7.88 (m, 3H), 7.49-7.68 (m, 6H), 7.30-7.36 (m, 1H), 7.19 (dd, *J* = 8.9, 7.1 Hz, 1H), 6.69 (t, *J* = 8.0 Hz, 1H), 4.97 (d, *J* = 4.1 Hz, 2H), 4.52 (s, 2H), 3.50 (q, *J* = 7.0 Hz, 2H), 1.22 (t, *J =* 7.1 Hz, 3H). MS(ESI): m/z 373 (M+1).

**N-(2-oxo-2-phenylethyl)-4-((4-(pyridin-2-yl)piperazin-1-yl)methyl)benzamide (DKC1125i34)** ¹H-NMR (400 MHz, CDCl₃) δ 8.18-8.19 (m, 1H), 8.03-8.06 (m, 2H), 7.86 (d, *J =* 8.2 Hz, 2H), 7.63-7.68 (m, 1H), 7.45-7.56 (m, 5H), 7.29 (s, 1H), 6.60-6.65 (m, 2H), 4.98 (d, *J =* 4.1 Hz, 2H), 3.61 (s, 2H), 3.56 (t, *J* = 5.0 Hz, 4H), 2.57 (t, *J =* 5.0 Hz, 4H). MS(ESI): m/z 416 (M+1).

**4-((diallylamino)methyl)-N-(2-oxo-2-phenylethyl)benzamide (DKC1125i35)** ¹H-NMR (400 MHz, CDCl₃) δ 8.04 (d, *J* = 8.2 Hz, 2H), 7.83 (d, *J* = 8.2 Hz, 2H), 7.63-7.67 (m, 1H), 7.53 (t, *J* = 7.5 Hz, 2H), 7.28-7.45 (m, 3H), 5.83-5.93 (m, 2H), 5.13-5.22 (m, 4H), 4.97 (d, *J* = 4.1 Hz, 2H), 3.62 (s, 2H), 3.08 (d, *J* = 6.4 Hz, 4H). MS(ESI): m/z 349 (M+1).

**4-((benzyl(methyl)amino)methyl)-N-(2-oxo-2-phenylethyl)benzamide (DKC1125i36)** ¹H-NMR (400 MHz, CDCl₃) δ 8.04 (d, *J* = 7.3 Hz, 2H), 7.85 (d, *J* = 8.2 Hz, 2H), 7.65 (t, *J* = 7.5 Hz, 1H), 7.47-7.55 (m, 4H), 7.27-7.38 (m, 6H), 4.97 (d, *J =* 4.1 Hz, 2H), 3.57 (s, 2H), 3.54 (s, 2H), 2.20 (s, 3H). MS(ESI): m/z 373 (M+1).

**4-(((2-hydroxyethyl)(methyl)amino)methyl)-N-(2-oxo-2-phenylethyl)benzamide (DKC1125i37)** ¹H-NMR (400 MHz, CDCl₃) δ 8.02 (d, *J* = 7.3 Hz, 2H), 7.86 (d, *J* = 8.2 Hz, 2H), 7.63 (t, *J* = 7.3 Hz, 1H), 7.47-7.53 (m, 4H), 7.36 (s, 1H), 4.95 (d, *J* = 4.6 Hz, 2H), 3.80 (s, 2H), 3.72 (t, *J* = 5.9 Hz, 2H), 2.75 (t, *J =* 5.3 Hz, 2H), 2.37 (s, 3H). MS(ESI): m/z 327 (M+1).

**4-((butyl(2-hydroxyethyl)amino)methyl)-N-(2-oxo-2-phenylethyl)benzamide (DKC1125i38)** ¹H-NMR (400 MHz, CDCl₃) δ 8.02-8.04 (m, 2H), 7.86 (d, *J* = 8.2 Hz, 2H), 7.64 (t, *J* = 7.5 Hz, 1H), 7.46-7.54 (m, 4H), 7.34 (d, *J =* 3.7 Hz, 1H), 4.96 (d, *J =* 4.1 Hz, 2H), 3.80 (s, 2H), 3.65 (t, *J=* 5.0 Hz, 2H), 3.13 (q, *J* = 7.5 Hz, 1H), 2.75 (t, *J* = 5.3 Hz, 2H), 2.59 (t, *J* = 7.5 Hz, 2H), 1.54-1.60 (m, 2H), 1.24-1.34 (m, 2H), 0.89 (q, *J =* 7.3 Hz, 3H). MS(ESI): m/z 369 (M+1).

**tert-butyl 4-(4-((2-oxo-2-phenylethyl)carbamoyl)benzyl)piperazine-1 -carboxylate (DKC1125i39)** ¹H-NMR (400 MHz, CDCl₃) δ 8.03 (d, *J* = 7.3 Hz, 2H), 7.83 (d, *J* = 8.2 Hz, 2H), 7.41-7.66 (m, 5H), 7.30 (d, *J* = 3.7 Hz, 1H), 4.96 (d, *J* = 4.1 Hz, 2H), 3.55 (s, 2H), 3.43 (t, *J* = 4.6 Hz, 4H), 2.38 (brs, 4H), 1.44 (s, 9H). MS(ESI): m/z 439 (M+1).

**4-((dicyclohexylamino)methyl)-N-(2-oxo-2-phenylethyl)benzamide (DKC1125i40)** ¹H-NMR (400 MHz, CDCl₃) δ 8.03-8.05 (m, 2H), 7.80 (d, *J* = 8.2 Hz, 2H), 7.47-7.67 (m, 6H), 4.97 (d, *J* = 4.6 Hz, 2H), 3.80 (s, 2H), 2.49-2.55 (m, 2H), 1.56-1.78 (m, 10H), 1.01-1.30 (m, 10H). MS(ESI): m/z 434 (M+1).

**4-((butyl(methyl)amino)methyl)-N-(2-oxo-2-phenylethyl)benzamide (DKC1125i41)** ¹H-NMR (400 MHz, CDCl₃) δ 7.98-8.00 (m, 2H), 7.85 (d, *J* = 8.2 Hz, 2H), 7.47-7.62 (m, 5H), 7.40 (t, *J* = 4.1 Hz, 1H), 4.92 (d, *J* = 4.1 Hz, 2H), 3.80 (s, 2H), 2.58 (t, *J* = 8.0 Hz, 2H), 2.36 (s, 3H), 1.58-1.64 (m, 2H), 1.26-1.33 (m, 2H), 0.88 (t, *J =* 7.3 Hz, 3H). MS(ESI): m/z 339 (M+1).

**4-((dipropylamino)methyl)-N-(2-oxo-2-phenylethyl)benzamide (DKC1125i42)** ¹H-NMR (400 MHz, CDCl₃) δ 8.01-8.03 (m, 2H), 7.82-7.84 (m, 2H), 7.61-7.65 (m, 1H), 7.40-7.53 (m, 4H), 7.29-7.32 (m, 1H), 4.95 (d, *J* = 4.6 Hz, 2H), 3.66 (s, 2H), 2.42 (t, *J* = 7.5 Hz, 4H), 1.47-1.56 (m, 4H), 0.86 (t, *J* = 7.3 Hz, 6H). MS(ESI): m/z 353 (M+1).

**4-((butyl(ethyl)amino)methyl)-N-(2-oxo-2-phenylethyl)benzamide (DKC1125i43)** ¹H-NMR (400 MHz, CDCl₃) δ 8.01-8.03 (m, 2H), 7.82-7.84 (m, 2H), 7.61-7.65 (m, 1H), 7.40-7.53 (m, 4H), 7.29-7.32 (m, 1H), 4.95 (d, *J* = 4.6 Hz, 2H), 3.66 (s, 2H), 2.42 (t, *J* = 7.5 Hz, 4H), 1.47-1.56 (m, 4H), 0.86 (t, *J* = 7.3 Hz, 6H). MS(ESI): m/z 353 (M+1).

**4-((ethyl(2-hydroxyethyl)amino)methyl)-N-(2-oxo-2-phenylethyl)benzamide (DKC1125i44)** ¹H-NMR (400 MHz, CDCl₃) δ 8.03 (d, *J =* 7.3 Hz, 2H), 7.81-7.84 (m, 2H), 7.64 (t, *J =* 7.5 Hz, 1H), 7.28-7.54 (m, 5H), 4.96 (d, *J* = 4.6 Hz, 2H), 3.47-3.61 (m, 4H), 2.08-2.43 (m, 4H), 1.43-1.48 (m, 3H). MS(ESI): m/z 341 (M+1).

**4-((ethynyl(methyl)amino)methyl)-N-(2-oxo-2-phenylethyl)benzamide (DKC1125i45)** ¹H-NMR (400 MHz, CDCl₃) δ 8.03 (d, *J* = 7.8 Hz, 2H), 7.84 (d, *J* = 8.2 Hz, 2H), 7.30-7.66 (m, 6H), 4.96 (d, *J* = 4.1 Hz, 2H), 3.63 (s, 2H), 3.32 (s, 1H), 2.34 (s, 3H). MS(ESI): m/z 307 (M+1).

**4-(morpholinomethyl)-N-(2-oxo-2-phenylethyl)benzamide (DKC1125i46)** ¹H-NMR (400 MHz, CDCl₃) δ 8.04 (d, *J* = 7.3 Hz, 2H), 7.84 (d, *J* = 8.2 Hz, 2H), 7.65 (t, *J* = 7.3 Hz, 1H), 7.43-7.55 (m, 4H), 7.29 (s, 1H), 4.96 (d, *J* = 4.1 Hz, 2H), 3.72 (t, *J* = 4.8 Hz, 4H), 3.55 (s, 2H), 2.45 (t, *J* = 4.6 Hz, 4H). MS(ESI): m/z 339 (M+1).

**ethyl 4-(4-((2-oxo-2-phenylethyl)carbamoyl)benzyl)piperazine-1-carboxylate (DKC1125i47)** ¹H-NMR (400 MHz, CDCl₃) δ 8.01 (d, *J =* 8.5 Hz, 2H), 7.81-7.83 (m, 2H), 7.60-7.64 (m, 1H), 7.48-7.52 (m, 2H), 7.31-7.42 (m, 3H), 4.94 (d, *J* = 4.1 Hz, 2H), 4.08-4.17 (m, 4H), 3.46 (t, *J* = 5.2 Hz, 4H), 2.38 (s, 4H), 1.23 (t, *J =* 6.8 Hz, 3H). MS(ESI): m/z 410 (M+1).

**4-((2,6-dimethylmorpholino)methyl)-N-(2-oxo-2-phenylethyl)benzamide (DKC1125i48)** ¹H-NMR (400 MHz, CDCl₃) δ 8.03 (d, *J* = 7.8 Hz, 2H), 7.84 (d, *J* = 7.8 Hz, 2H), 7.63-7.66 (m, 1H), 7.29-7.54 (m, 5H), 4.96 (d, *J* = 4.1 Hz, 2H), 3.66-3.72 (m, 2H), 3.52 (s, 2H), 2.66-2.69 (m, 2H), 1.74-1.79 (m, 2H), 1.13 (d, *J =* 5.9 Hz, 6H). MS(ESI): m/z 367 (M+1).

**3-((3,4-dihydroisoquinolin-2(1H)-yl)methyl)-N-(2-(3-methoxyphenyl)-2-oxoethyl)benzamide (DKC1125i49)** ¹H-NMR (400 MHz, CDCl₃) δ 7.79-7.90 (m, 2H), 7.53-7.63 (m, 3H), 7.32-7.46 (m, 3H), 7.17-7.20 (m, 1H), 7.09-7.12 (m, 3H), 6.98 (d, *J* = 7.8 Hz, 1H), 4.95 (d, J = 4.1 Hz, 2H), 3.88 (d, *J* = 3.2 Hz, 3H), 3.75 (s, 2H), 3.65 (s, 2H), 2.92 (t, *J* = 5.7 Hz, 2H), 2.77 (t, *J* = 5.9 Hz, 2H). MS(ESI): m/z 416 (M+1).

**3-((cyclohexyl(methyl)am**i**no)methyl)-N-(2-(3-methoxyphenyl)-2-oxoethyl)benzamide (DKC1125i50)** ¹H-NMR (400 MHz, CDCl₃) δ 7.97 (s, 1H), 7.78 (d, *J =* 7.8 Hz, 1H), 7.37-7.59 (m, 6H), 7.13-7.16 (m, 1H), 4.91 (d, *J =* 4.6 Hz, 2H), 3.84 (s, 3H), 3.75 (s, 2H), 2.61 (s, 1H), 2.28 (s, 3H), 1.99 (d, *J* = 11.0 Hz, 2H), 1.82 (d, *J =* 12.8 Hz, 2H), 1.63 (d, *J* = 12.8 Hz, 2H), 1.19-1.36 (m, 4H). MS(ESI): m/z 396 (M+1).

**3-((1-(2-fluorophenyl)piperidin-4-yl)methyl)-N-(2-(3-methoxyphenyl)-2-oxoethyl)benzamide (DKC1125i51)** ¹H-NMR (400 MHz, CDCl₃) δ 7.77-7.88 (m, 2H), 7.33-7.63 (m, 6H), 6.91-7.19 (m, 5H), 4.95 (d, *J* = 4.1 Hz, 2H), 3.87 (s, 3H), 3.12 (t, *J* = 4.8 Hz, 4H), 2.94 (s, 1H), 2.87 (s, 1H), 2.65 (t, *J =* 4.8 Hz, 5H). MS(ESI): m/z 462 (M+1).

**N-(2-(3-methoxyphenyl)-2-oxoethyl)-3-((4-(2-methoxyphenyl)piperazin-1-yl)methyl)benzamide (DKC1125i52)** ¹H-NMR (400 MHz, CDCl₃) δ 7.88 (s, 1H), 7.77-7.80 (m, 1H), 7.61-7.63 (m, 1H), 7.54-7.56 (m, 2H), 7.41-7.45 (m, 2H), 7.33 (t, *J =* 3.9 Hz, 1H), 7.17-7.20 (m, 1H), 6.88-7.01 (m, 3H), 6.85 (dd, *J* = 8.0, 1.1 Hz, 1H), 4.95 (d, *J* = 4.6 Hz, 2H), 3.88 (s, 3H), 3.85 (s, 3H), 3.65 (s, 2H), 3.10 (brs, 4H), 2.69 (brs, 4H). MS(ESI): m/z 475 (M+1).

**3-([1,4'-bipiperidin]-1'-ylmethyl)-N-(2-(3-methoxyphenyl)-2-oxoethyl)benzamide (DKC1125i53)** ¹H-NMR (400 MHz, CDCl₃) δ 7.75-7.82 (m, 2H), 7.34-7.62 (m, 6H), 7.18 (d, *J =* 8.2 Hz, 1H), 4.94 (d, *J =* 4.1 Hz, 2H), 3.87 (s, 3H), 3.53 (s, 2H), 2.94 (d, *J =* 10.1 Hz, 1H), 2.61 (s, 8H), 1.99 (t, *J =* 11.7 Hz, 2H), 1.83 (d, *J =* 11.0 Hz, 2H), 1.66 (s, 4H), 1.46 (s, 2H). MS(ESI): m/z 451 (M+1).

**N-(2-(3-methoxyphenyl)-2-oxoethyl)-3-((4-(pyridin-2-yl)piperazin-1-yl)methyl)benzamide (DKC1125i54)** ¹H-NMR (400 MHz, CDCl₃) δ 8.17-8.20 (m, 1H), 7.87 (s, 1H), 7.78 (d, *J* = 7.8 Hz, 1H), 7.61-7.63 (m, 1H), 7.41-7.55 (m, 5H), 7.33 (t, *J* = 4.3 Hz, 1H), 7.18 (dd, *J =* 8.0, 3.0 Hz, 1H), 6.59-6.64 (m, 2H), 4.95 (d, *J* = 4.1 Hz, 2H), 3.87 (s, 3H), 3.61 (s, 2H), 3.55 (t, *J* = 5.0 Hz, 4H), 2.57 (t, *J* = 5.0 Hz, 4H). MS(ESI): m/z 446 (M+1).

**3-((diallylamino)methyl)-N-(2-(3-methoxyphenyl)-2-oxoethyl)benzamide (DKC1125i55)** ¹H-NMR (400 MHz, CDCl₃) δ 7.84 (s, 1H), 7.75 (d, *J* = 7.8 Hz, 1H), 7.61-7.63 (m, 1H), 7.51-7.54 (m, 2H), 7.42 (q, *J =* 7.9 Hz, 2H), 7.30 (d, *J = 4.6* Hz, 1H), 7.18 (dd, *J =* 8.0, 3.0 Hz, 1H), 5.84-5.94 (m, 2H), 5.16-5.24 (m, 4H), 4.95 (d, *J* = 4.6 Hz, 2H), 3.88 (s, 3H), 3.64 (s, 2H), 3.10 (d, *J* = 6.4 Hz, 4H). MS(ESI): m/z 379 (M+1).

**3-((benzyl(methyl)amino)methyl)-N-(2-(3-methoxyphenyl)-2-oxoethyl)benzamide (DKC1125i56)** ¹H-NMR (400 MHz, CDCl₃) δ 7.98 (s, 1H), 7.81 (d, *J* = 7.8 Hz, 1H), 7.29-7.62 (m, 11H), 7.16-7.19 (m, 1H), 4.94 (d, *J =* 4.6 Hz, 2H), 3.87 (s, 4H), 3.73 (s, 3H), 2.29 (s, 3H). MS(ESI): m/z 403 (M+1).

3-(**(ethyl(methyl)am**i**no)methyl)-N-(2-(3-methoxyphenyl)-2-oxoethyl)benzamide (DKC1125i57)** ¹H-NMR (400 MHz, CDCl₃) δ 7.30-7.91 (m, 8H), 7.18-7.21 (m, 1H), 4.96 (d, *J =* 4.1 Hz, 2H), 4.65 (s, 1H), 4.54 (s, 1H), 3.85-3.89 (m, 5H), 1.61 (s, 3H), 1.25 (s, 3H). MS(ESI): m/z 341 (M+1).

**3-((butyl(2-hydroxyethyl)amino)methyl)-N-(2-(3-methoxyphenyl)-2-oxoethyl)benzamide (DKC1125i58)** ¹H-NMR (400 MHz, CDCl₃) δ 8.13 (s, 1H), 7.87 (d, *J =* 7.8 Hz, 1H), 7.40-7.63 (m, 6H), 7.15-7.18 (m, 1H), 4.93 (d, *J* = 4.6 Hz, 2H), 4.06 (s, 2H), 3.87 (s, 3H), 3.80 (t, *J* = 5.0 Hz, 2H), 2.96 (t, *J* = 4.8 Hz, 2H), 2.82 (t, *J* = 8.0 Hz, 2H), 1.58 (t, *J* = 7.5 Hz, 3H), 1.30-1.35 (m, 4H). MS(ESI): m/z 400 (M+1).

**tert-butyl 4-(3-((2-(3-methoxyphenyl)-2-oxoethyl)carbamoyl)benzyl)piperazine-1** - **carboxylate (DKC1125i59)** ¹H-NMR (400 MHz, CDCl₃) δ 7.84 (s, 1H), 7.77 (dd, *J =* 7.8, 1.4 Hz, 1H), 7.62 (dd, *J* = 7.8, 0.9 Hz, 1H), 7.29-7.54 (m, 5H), 7.17-7.20 (m, 1H), 4.95 (d, *J* = 4.1 Hz, 2H), 3.88 (s, 3H), 3.57 (s, 2H), 3.44 (t, *J =* 4.6 Hz, 4H), 2.41 (brs, 4H), 1.45 (s, 9H). MS(ESI): m/z 469 (M+1).

**3-((butyl(ethyl)amino)methyl)-N-(2-(3-methoxyphenyl)-2-oxoethyl)benzamide (DKC1125i60)** ¹H-NMR (400 MHz, CDCl₃) δ 8.04 (s, 1H), 7.82 (d, *J* = 7.8 Hz, 1H), 7.61 (d, *J* = 7.8 Hz, 2H), 7.40-7.53 (m, 4H), 7.15-7.18 (m, 1H), 4.93 (d, *J* = 4.6 Hz, 2H), 3.87 (s, 3H), 3.82 (s, 2H), 2.72 (q, *J = 7.0* Hz, 2H), 2.62 (t, *J =* 7.8 Hz, 2H), 1.50 (d, *J* = 6.9 Hz, 3H), 1.27-1.37 (m, 2H), 1.18 (t, *J* = 7.1 Hz, 2H), 0.90 (t, *J =* 7.3 Hz, 3H). MS(ESI): m/z 384 (M+1).

**3-((dipentylamino)methyl)-N-(2-(3-methoxyphenyl)-2-oxoethyl)benzamide (DKC1125i61)** ¹H-NMR (400 MHz, CDCl₃) δ 7.82 (s, 1H), 7.72-7.76 (m, 1H), 7.62 (d, *J =* 7.8 Hz, 1H), 7.52-7.57 (m, 2H), 7.35-7.46 (m, 2H), 7.30 (d, *J* = 3.2 Hz, 1H), 7.17-7.21 (m, 1H), 4.95 (d, *J* = 4.1 Hz, 2H), 3.88 (s, 3H), 3.55 (s, 2H), 2.02-2.43 (m, 8H), 1.39-1.58 (m, 6H), 1.22-1.32 (m, 8H). MS(ESI): m/z 371 (M+1).

**3-((4-(2-hydroxyethyl)piperazin-1-yl)methyl)-N-(2-(3-methoxyphenyl)-2-oxoethyl)benzamide (DKC1125i62)** ¹H-NMR (400 MHz, CDCl₃) δ 7.88 (s, 1H), 7.78 (d, *J* = 7.8 Hz, 1H), 7.69 (s, 1H), 7.60 (d, *J =* 7.8 Hz, 1H), 7.34-7.52 (m, 4H), 7.16-7.19 (m, 1H), 4.93 (d, *J* = 4.6 Hz, 2H), 3.88 (s, 3H), 3.66 (s, 2H), 3.12 (t, *J =* 7.5 Hz, 2H), 2.95 (t, *J = 4.8* Hz, 2H), 2.83 (brs, 4H), 1.50 (brs, 4H). MS(ESI): m/z 440 (M+1).

**N-(2-(3-methoxyphenyl)-2-oxoethyl)-3-(morpholinomethyl)benzamide (DKC1125i63)** ¹H-NMR (400 MHz, CDCl₃) δ 7.91 (s, 1H), 7.77 (d, *J =* 7.8 Hz, 1H), 7.49-7.59 (m, 3H), 7.37-7.42 (m, 3H), 7.13-7.16 (m, 1H), 4.91 (d, *J =* 4.1 Hz, 2H), 3.84 (s, 3H), 3.74 (t, *J =* 4.6 Hz, 4H), 3.64 (s, 2H), 3.11 (q, *J =* 7.3 Hz, 2H), 1.53 (q, *J =* 7.0 Hz, 2H). MS(ESI): m/z 369 (M+1).

**3-((ethyl(propyl)amino)methyl)-N-(2-(3-methoxyphenyl)-2-oxoethyl)benzamide (DKC1125i64)** ¹H-NMR (400 MHz, CDCl₃) δ 8.15 (s, 1H), 7.76-7.79 (m, 1H), 7.48-7.66 (m, 3H), 7.33-7.42 (m, 3H), 7.14-7.18 (m, 1H), 4.90 (d, *J =* 5.0 Hz, 2H), 3.99 (s, 2H), 3.85 (s, 3H), 3.61-3.70 (m, 2H), 1.47 (d, *J =* 6.9 Hz, 2H), 0.94-0.99 (m, 8H). MS(ESI): m/z 369 (M+1).

**3-((2,6-dimethylmorpholino)methyl)-N-(2-(3-methoxyphenyl)-2-oxoethyl)benzamide (DKC1125i65)** ¹H-NMR (400 MHz, CDCl₃) δ 7.84 (s, 1H), 7.77 (d, *J =* 7.8 Hz, 1H), 7.62 (d, *J =* 7.8 Hz, 1H), 7.50-7.54 (m, 2H), 7.43 (td, *J =* 7.8, 4.1 Hz, 2H), 7.30 (d, *J =* 4.1 Hz, 1H), 7.17-7.20 (m, 1H), 4.95 (d, *J =* 4.1 Hz, 2H), 3.88 (s, 3H), 3.67-3.73 (m, 2H), 3.53 (s, 2H), 2.67 (t, *J =* 10.6 Hz, 2H), 1.78 (t, *J =* 10.7 Hz, 2H), 1.14 (d, *J* = 6.4 Hz, 6H). MS(ESI): m/z 397 (M+1).

**2-((dimethylamino)methyl)-N-(2-oxo-2-phenylethyl)benzamide (DKC1125i66)** ¹H-NMR (400 MHz, CDCl₃) δ 7.42-8.05 (m, 10H), 3.67 (d, *J =* 4.1 Hz, 2H), 3.10 (s, 2H), 1.47 (s, 6H). MS(ESI): m/z 297 (M+1).

**2-((3,4-dihydroquinolin-1 (2H)-yl)methyl)-N-(2-oxo-2-phenylethyl)benzamide (DKC1125i67)** ¹H-NMR (400 MHz, CDCl₃) δ 7.93-8.08 (m, 4H), 7.38-7.60 (m, 8H), 7.04-7.10 (m, 1H), 6.92 (d, *J* = 6.9 Hz, 1H), 4.85 (d, *J* = 3.7 Hz, 2H), 3.93 (s, 2H), 2.97-3.09 (m, 2H), 2.90 (t, *J* = 5.7 Hz, 2H), 2.83 (t, *J* = 5.7 Hz, 2H). MS(ESI): m/z 385 (M+1).

**2-((cyclohexyl(methyl)amino)methyl)-N-(2-oxo-2-phenylethyl)benzamide (DKC1125i68)** ¹H-NMR (400 MHz, CDCl₃) δ 7.97-8.01 (m, 3H), 7.83 (d, *J =* 7.3 Hz, 1H), 7.47-7.65 (m, 6H), 4.92 (s, 2H), 4.35 (s, 2H), 3.66-3.72 (m, 1H), 2.63 (s, 3H), 2.23 (d, *J* = 11.0 Hz, 2H), 1.89 (d, *J* = 13.3 Hz, 2H), 1.67 (d, *J =* 13.3 Hz, 2H), 1.28 (m, 4H). MS(ESI): m/z 365 (M+1).

**2-((4-(2-fluorophenyl)piperazin-1-yl)methyl)-N-(2-oxo-2-phenylethyl)benzamide (DKC1125i69)** ¹H-NMR (400 MHz, CDCl₃) δ 7.99-8.07 (m, 3H), 7.42-7.66 (m, 7H), 7.00-7.10 (m, 2H), 6.91-6.97 (m, 2H), 5.02 (d, *J* = 5.5 Hz, 2H), 3.81 (s, 2H), 3.12 (brs, 4H), 2.76 (brs, 4H). MS(ESI): m/z 433 (M+1).

**N-(2-oxo-2-phenylethyl)-2-((4-(pyrimidin-2-yl)piperazin-1-yl)methyl)benzamide (DKC1125i70)** ¹H-NMR (400 MHz, CDCl₃) *δ* 8.28 (d, *J =* 4.4 Hz, 2H), 7.96-8.03 (m, 3H), 7.41-7.63 (m, 6H), 7.20 (d, *J* = 6.8 Hz, 1H), 6.48 (t, *J* = 4.4 Hz, 1H), 5.02 (d, *J =* 5.0 Hz, 2H), 3.84 (brs, 4H), 3.74 (s, 2H), 2.60 (t, *J=* 5.0 Hz, 4H). MS(ESI): m/z 417 (M+1).

**2-([1,4'-bipiperidin]-1 '-ylmethyl)-N-(2-oxo-2-phenylethyl)benzamide (DKC1125i71)** ¹H-NMR (400 MHz, CDCl₃) δ 7.89-7.99 (m, 3H), 7.37-7.64 (m, 5H), 7.20 (d, *J* = 5.9 Hz, 1H), 5.06 (d, *J* = 4.6 Hz, 2H), 3.66-3.73 (m, 3H), 3.06-3.15 (m, 4H), 2.02-2.25 (m, 4H), 1.49-1.58 (m, 10H). MS(ESI): m/z 421 (M+1).

**N-(2-oxo-2-phenylethyl)-2-((4-(pyridin-2-yl)piperazin-1-yl)methyl)benzamide (DKC1125i72)** ¹H-NMR (400 MHz, CDCl₃) δ 8.16-8.17 (m, 1H), 7.97-8.03 (m, 3H), 7.59-7.63 (m, 1H), 7.39-7.54 (m, 6H), 7.22-7.24 (m, 1H), 6.60-6.67 (m, 2H), 5.00 (d, *J* = 5.0 Hz, 2H), 3.77 (s, 2H), 3.54 (t, *J* = 4.8 Hz, 4H), 2.66 (t, *J=* 5.0 Hz, 4H). MS(ESI): m/z 415 (M+1).

**2-((diallylamino)methyl)-N-(2-oxo-2-phenylethyl)benzamide (DKC1125i73)** ¹H-NMR (400 MHz, CDCl₃) δ 8.02-8.04 (m, 2H), 7.92-7.95 (m, 1H), 7.37-7.63 (m, 7H), 5.81-5.92 (m, 2H), 5.14-5.21 (m, 4H), 4.97 (s, 2H), 3.81 (s, 2H), 3.18 (d, *J =* 6.9 Hz, 4H). MS(ESI): m/z 349 (M+1).

**2-(morpholinomethyl)-N-(2-oxo-2-phenylethyl)benzamide (DKC1125i74) ¹H-NMR** (400 MHz, CDCl₃) δ 7.92-8.05 (m, 3H), 7.37-7.69 (m, 6H), 7.20-7.23 (m, 1H), 5.01 (d, *J =* 5.0 Hz, 2H), 3.67-3.73 (m, 6H), 2.55 (brs, 4H). MS(ESI): m/z 339 (M+1).

**ethyl 4-(2-((2-oxo-2-phenylethyl)carbamoyl)benzyl)piperazine-1-carboxylate (DKC1125i75)** ¹H-NMR (400 MHz, CDCl₃) δ 8.01-8.03 (m, 2H), 7.91-7.96 (m, 1H), 7.60-7.64 (m, 1H), 7.37-7.55 (m, 6H), 7.19 (dd, *J* = 6.9, 1.8 Hz, 1H), 5.01 (d, *J* = 4.6 Hz, 2H), 4.12 (q, *J* = 7.2 Hz, 2H), 3.70 (s, 2H), 3.50 (t, *J* = 4.6 Hz, 4H), 2.50 (t, *J*= 5.0 Hz, 4H), 1.24 (t, *J* = 7.1 Hz, 3H). MS(ESI): m/z 410 (M+1).

### Experimental Example 1: Measurement of Cancer Metastasis-Related Promoter Activity-Increasing Effects of Compounds by Luciferase Assay

Experiments were conducted to evaluate the the metastasis-related promoter activity-increasing effects of the compounds according to the present invention. Specifically, the KAI1 promoter reporter vector was used. In this case, the pRL-TK vector, a vector expressing renilla luciferase, was used as an internal control indicating the transfrction efficiency in each transfected well.

First, 24 hours before transfection, 2 × 10⁶ human colon cancer cell line, Caco2) cells were cultured in 10 ml of DMEM containing 10% FBS and 1% penicillin/streptomycin on a 100-mm-diamter plate, and then transformed with 5 µg of a pRL-TK vector mixture containing a transfection reagent and the KAI1 promoter, and then cultured in a 5% CO₂ incubator at 37°C for 48 hours. Thereafter, the adherent cells were detached using trypsin and counted using a hemacytometer, and then 5 × 10³ transfected Caco2 cells in 100 µl of DMEM containing 10% FBS and 1% penicillin/streptomycin were seeded into each well of a 96-well plate and cultured in a 5% CO₂ incubator at 37°C for 24 hours. Next, 1 µM of each of the 305 compounds prepared in Examples 1 to 305 was added to each well, and after 16 hours, 100 µl of a lysis buffer for analysis containing a luciferase substrate was added, and the cells were lysed with stirring for 20 minutes. After completion of lysis, the cells were measured for luciferase activity using a luciferase reporter assay system (Promega, USA). In the experiment, DMSO was used as a control, and PHA-665752 known to increase the KAI1 promoter activity was used as a negative control. In addition, data were analyzed at a significance level of p<0.05 by one-way ANOVA (Tukey's HSD), and SPSS 17.0 (Chicago, USA) statistical program was used. The results are shown in Tables 14 to 20 below. In Table 14 to 20, PHA is the PHA-665752, and KIT is KITENIN.

As a result of the experiment, it was confirmed that compounds 1 to 305 of Examples 1 to 305 had more significant effects on the the KAI1 promoter reporter activity than the control DMSO.

**[Table 14]**

| | Relative KAI promoter activity | SD | *In vivo* relative invation |
|---|---|---|---|
| cont | 1.00 | 0.02 | |
| PHA | 1.43 | 0.27 | |
| Example 1(DKC1125a1) | 1.70 | 0.12 | 0.967212071 |
| Example 2(DKC1125a2) | 1.14 | 0.08 | 0.865139296 |
| Example 3(DKC1125a3) | 2.12 | 0.13 | 1.0000975 |
| Example 4(DKC1125a4) | 1.88 | 0.11 | 1.083507938 |
| Example 5(DKC1125a5) | 1.98 | 0.14 | 0.812007649 |
| Example 6(DKC1125a6) | 1.91 | 0.12 | 1.143814805 |
| Example 7(DKC1125a7) | 1.20 | 0.08 | 1.091112955 |
| Example 8(DKC1125a8) | 1.15 | 0.09 | 1.0773117 |
| Example 9(DKC1125a9) | 1.14 | 0.09 | 0.84188251 |
| Example 10(DKC1125a10) | 1.08 | 0.06 | 0.909895874 |
| Example 11(DKC1125a11) | 1.82 | 0.12 | 1.036035283 |
| Example 12(DKC1125a12) | 1.75 | 0.12 | 1.357212927 |
| Example 13(DKC1125a13) | 1.73 | 0.14 | 0.856537389 |
| Example 14(DKC1125a14) | 1.02 | 0.08 | 0.91063011 |
| Example 15(DKC1125a15) | 1.74 | 0.16 | 1.170012516 |
| Example 16(DKC1125a16) | 0.92 | 0.08 | 0.964495994 |
| Example 17(DKC1125a17) | 0.98 | 0.03 | 0.928830813 |
| Example 18(DKC1125a18) | 2.18 | 0.06 | 1.099903893 |
| Example 19(DKC1125a19) | 0.98 | 0.06 | 1.201337942 |
| Example 20(DKC1125a20) | 0.99 | 0.05 | 1.217248385 |
| Example 21(DKC1125a21) | 0.99 | 0.11 | 1.098839349 |
| Example 22(DKC1125a22) | 1.56 | 0.07 | 1.16319745 |
| Example 23(DKC1125a23) | 1.00 | 0.05 | 1.103863595 |
| Example 24(DKC1125a24) | 0.84 | 0.05 | 1.127960077 |
| Example 25(DKC1125a25) | 1.49 | 0.06 | 1.215411799 |
| Example 26(DKC1125a26) | 1.26 | 0.16 | 1.062762281 |
| Example 27(DKC1125a27) | 1.31 | 0.13 | 1.043524891 |
| Example 28(DKC1125a28) | 1.02 | 0.07 | 1.057274411 |
| Example 29(DKC1125a29) | 0.90 | 0.03 | 1.026118119 |
| Example 30(DKC1125a30) | 1.41 | 0.13 | 0.763132184 |
| Example 31(DKC1125a31) | 1.18 | 0.11 | 0.821148791 |
| Example 32(DKC1125a32) | 1.02 | 0.08 | 1.383289259 |
| Example 33(DKC1125a33) | 1.78 | 0.12 | 0.751253077 |
| Example 34(DKC1125a34) | 1.34 | 0.13 | 1.10271548 |
| Example 35(DKC1125a35) | 1.28 | 0.09 | 0.881117392 |
| Example 36(DKC1125a36) | 1.16 | 0.08 | 1.055670632 |
| Example 37(DKC1125a37) | 1.17 | 0.06 | 0.964820331 |
| Example 38(DKC1125a38) | 1.57 | 0.18 | 1.118590107 |
| Example 39(DKC1125a39) | 1.21 | 0.13 | 0.598418507 |
| Example 40(DKC1125a40) | 1.00 | 0.03 | 1.173484717 |
| Example 41(DKC 1125a41) | 1.19 | 0.14 | 0.756462374 |
| Example 42(DKC1125a42) | 1.12 | 0.07 | 1.525309265 |
| Example 43(DKC1125a43) | 1.91 | 0.17 | 0.977360052 |
| EV | | | 1 |
| KIT | | | 1.418701735 |

**[Table 15]**

| | Relative KAI promoter activity | SD | *In vivo* relative invation |
|---|---|---|---|
| cont | 1.00 | 0.02 | |
| PHA | 1.43 | 0.27 | |
| Example 44(DKC1125b1) | 0.98 | 0.06 | 0.975231046 |
| Example 45(DKC1125b2) | 0.84 | 0.03 | 1.475976967 |
| Example 46(DKC1125b3) | 0.98 | 0.06 | 1.181352768 |
| Example 47(DKC1125b4) | 0.76 | 0.02 | 0.903801892 |
| Example 48(DKC1125b5) | 0.85 | 0.05 | 1.189692419 |
| Example 49(DKC1125b6) | 0.70 | 0.06 | 1.196722292 |
| Example 50(DKC1125b7) | 0.94 | 0.03 | 1.089160084 |
| Example 51(DKC1125b8) | 1.81 | 0.07 | 1.512528499 |
| Example 52(DKC1125b9) | 1.36 | 0.13 | 1.157600115 |
| Example 53(DKC1125b10) | 2.02 | 0.12 | 0.941084399 |
| Example 54(DKC1125b11) | 0.70 | 0.04 | 1.082513363 |
| Example 55(DKC1125b12) | 0.67 | 0.02 | 1.178638102 |
| Example 56(DKC1125b13) | 0.92 | 0.08 | 1.248572694 |
| Example 57(DKC1125b14) | 0.94 | 0.08 | 1.216616259 |
| Example 58(DKC1125b15) | 0.91 | 0.08 | 1.243673251 |
| Example 59(DKC1125b16) | 1.56 | 0.04 | 1.007369545 |
| Example 60(DKC1125b17) | 0.81 | 0.04 | 0.9712691 |
| Example 61(DKC1125b18) | 0.72 | 0.03 | 1.000434781 |
| Example 62(DKC1125b19) | 1.03 | 0.05 | 1.122415428 |
| Example 63(DKC1125b20) | 0.70 | 0.02 | 1.102301352 |
| Example 64(DKC1125b21) | 1.35 | 0.09 | 1.083225317 |
| Example 65(DKC1125b22) | 0.84 | 0.03 | 1.191339154 |
| Example 66(DKC1125b23) | 0.87 | 0.08 | 1.167665305 |
| Example 67(DKC1125b24) | 0.87 | 0.07 | 1.174738656 |
| Example 68(DKC1125b25) | 0.88 | 0.05 | 1.124828465 |
| Example 69(DKC1125b26) | 0.84 | 0.04 | 1.095461697 |
| Example 70(DKC1125b27) | 0.81 | 0.03 | 1.273966647 |
| Example 71(DKC1125b28) | 0.85 | 0.06 | 1.134844742 |
| Example 72(DKC1125b29) | 0.80 | 0.05 | 1.04378249 |
| Example 73(DKC1125b30) | 0.99 | 0.06 | 1.115749685 |
| Example 74(DKC1125b31) | 0.78 | 0.05 | 1.169583778 |
| Example 75(DKC1125b32) | 0.71 | 0.07 | 1.283061731 |
| Example 76(DKC1125b33) | 0.83 | 0.06 | 1.120344782 |
| Example 77(DKC1125b34) | 0.92 | 0.05 | 1.273648713 |
| Example 78(DKC1125b35) | 0.90 | 0.05 | 1.170409863 |
| Example 79(DKC1125b36) | 1.03 | 0.05 | 1.101727984 |
| Example 80(DKC1125b37) | 0.86 | 0.07 | 1.182643525 |
| Example 81(DKC1125b38) | 0.80 | 0.04 | 1.142382765 |
| Example 82(DKC1125b39) | 0.80 | 0.02 | 1.189048399 |
| Example 83(DKC1125b40) | 0.80 | 0.05 | 1.170551167 |
| Example 84(DKC1125b41) | 0.94 | 0.04 | 1.181249507 |
| Example 85(DKC1125b42) | 0.96 | 0.07 | 1.206282048 |
| Example 86(DKC1125b43) | 1.01 | 0.07 | 1.192621759 |
| Example 87(DKC1125b44) | 0.91 | 0.07 | 1.218137994 |
| EV | | | 1 |

**[Table 16]**

| | Relative KAI promoter activity | SD | *In vivo* relative invation |
|---|---|---|---|
| con | 1 | 0.055867817 | |
| PHA | 1.20 | 0.043393675 | |
| Example 88(DKC1125C1) | 1.35 | 0.081249856 | 0.921644707 |
| Example 89(DKC1125C2) | 1.27 | 0.059512199 | 1.07388343 |
| Example 90(DKC1125C3) | 1.88 | 0.219588912 | 1.124246315 |
| Example 91(DKC1125C4) | 2.44 | 0.221626597 | 1.0580728 |
| Example 92(DKC1125C5) | 2.29 | 0.179681535 | 1.064202769 |
| Example 93(DKC1125C6) | 2.48 | 0.140226867 | 1.090545444 |
| Example 94(DKC1125C7) | 1.81 | 0.090850801 | 1.095687249 |
| Example 95(DKC1125C8) | 1.21 | 0.046088014 | 1.160261277 |
| Example 96(DKC1125C9) | 2.23 | 0.129624575 | 1.140757593 |
| Example 97(DKC1125C10) | 1.80 | 0.087958979 | 1.02382481 |
| Example 98(DKC1125C11) | 2.64 | 0.112880624 | 1.120653752 |
| Example 99(DKC1125C12) | 1.71 | 0.049767964 | 1.133689705 |
| Example 100(DKC1125C13) | 1.62 | 0.051742572 | 1.093844908 |
| Example 101(DKC1125C14) | 3.04 | 0.146269026 | 0.881467731 |
| Example 102(DKC1125C15) | 2.53 | 0.156923386 | 1.120589549 |
| Example 103(DKC1125C16) | 1.70 | 0.050962298 | 1.171655873 |
| Example 104(DKC1 125C1 7) | 1.78 | 0.055249295 | 1.115470076 |
| Example 105(DKC1125C18) | 1.25 | 0.039978414 | 1.0791341 |
| Example 106(DKC1125C19) | 2.44 | 0.129482512 | 1.170731912 |
| Example 107(DKC1125C20) | 2.07 | 0.147047337 | 0.986796561 |
| Example 108(DKC1125C21) | 1.36 | 0.071134957 | 0.989004578 |
| Example 109(DKC1125C22) | 2.89 | 0.287084408 | 0.905353953 |
| Example 110(DKC1125C23) | 1.49 | 0.059141858 | 1.009342899 |
| Example 111(DKC1125C24) | 1.44 | 0.058746042 | 1.065364002 |
| Example 112(DKC1125C25) | 2.82 | 0.128566052 | 0.983474766 |
| Example 113(DKC1125C26) | 1.40 | 0.044927755 | 1.166980237 |
| Example 114(DKC1125C27) | 1.38 | 0.04051327 | 1.196784279 |
| Example 115(DKC1125C28) | 2.04 | 0.133934593 | 1.209331733 |
| Example 116(DKC1125C29) | 2.15 | 0.19730863 | 1.164258598 |
| Example 117(DKC1125C30) | 1.32 | 0.062835904 | 1.075661568 |
| Example 118(DKC1125C31) | 1.74 | 0.13810257 | 0.76806331 |
| Example 119(DKC1125C32) | 1.35 | 0.041971277 | 1.202847253 |
| Example 120(DKC1125C33) | 1.38 | 0.049104151 | 1.096641916 |
| Example 121(DKC1125C34) | 0.96 | 0.186314456 | 1.202604399 |
| EV | | | 1 |
| KIT | | | 1.156487271 |

**[Table 17]**

| | Relative KAI promoter activity | SD | *In vivo* relative invation |
|---|---|---|---|
| Cont | 1.00 | 0.04 | |
| PHA | 2.04 | 0.50 | |
| Example 122(DKC1125D1) | 1.65 | 0.08 | 1.373534212 |
| Example 123(DKC1125D2) | 1.14 | 0.02 | 1.312034373 |
| Example 124(DKC1125D3) | 1.04 | 0.04 | 1.266167671 |
| Example 125(DKC1125D4) | 1.48 | 0.07 | 0.89653738 |
| Example 126(DKC1125D5) | 1.08 | 0.02 | 1.351757201 |
| Example 127(DKC1125D6) | 2.46 | 0.07 | 1.350506317 |
| Example 128(DKC1125D7) | 1.10 | 0.04 | 1.179120716 |
| Example 129(DKC1125D8) | 1.30 | 0.04 | 1.298274653 |
| Example 130(DKC1125E1) | 1.32 | 0.05 | 0.987543526 |
| Example 131(DKC1125E2) | 1.19 | 0.05 | 1.292834764 |
| Example 132(DKC1125E3) | 1.18 | 0.02 | 1.226348845 |
| Example 133(DKC1125E4) | 1.15 | 0.07 | 1.319848032 |
| Example 134(DKC1125E5) | 1.09 | 0.04 | 1.296825956 |
| Example 135(DKC1125E6) | 1.30 | 0.06 | 1.484699948 |
| Example 136(DKC1125E7) | 1.17 | 0.03 | 1.296697958 |
| Example 137(DKC1125F1) | 1.19 | 0.04 | 1.231017841 |
| Example 138(DKC1125F2) | 1.15 | 0.04 | 1.258420919 |
| Example 139(DKC1125F3) | 1.01 | 0.03 | 1.313526706 |
| Example 140(DKC1125F4) | 1.15 | 0.03 | 1.552957467 |
| Example 141(DKC1125F5) | 1.06 | 0.03 | 1.392812365 |
| Example 142(DKC1125F6) | 1.12 | 0.03 | 1.800082616 |
| Example 143(DKC1125F7) | 1.16 | 0.03 | 0.802837469 |
| Example 144(DKC1125F8) | 1.08 | 0.02 | 1.284340392 |
| Example 145(DKC1125F9) | 1.10 | 0.03 | 1.432724279 |
| Example 146(DKC1125F10) | 1.16 | 0.05 | 0.747426234 |
| Example 147(DKC1125F11) | 1.10 | 0.04 | 0.952431514 |
| Example 148(DKC1125F12) | 1.44 | 0.04 | 1.84351155 |
| Example 149(DKC1125F13) | 1.07 | 0.03 | 1.260689964 |
| Example 150(DKC1125F14) | 1.14 | 0.03 | 1.071189823 |
| EV | | | 1 |
| KIT | | | 1.580081278 |

**[Table 18]**

| | Relative KAI promoter activity | SD | *In vivo* relative invation |
|---|---|---|---|
| cont | 1.00 | 0.035728039 | |
| #25 | 1.72 | 0.051949457 | |
| Example 151(DKC1125g1) | 1.05 | 0.015406669 | 1.212117692 |
| Example 152(DKC1125g2) | 1.08 | 0.05020165 | 1.160090569 |
| Example 153(DKC1125g3) | 1.05 | 0.022504904 | 1.095142017 |
| Example 154(DKC1125g4) | 1.05 | 0.033408055 | 1.172964579 |
| Example 155(DKC1125g5) | 0.99 | 0.036569054 | 1.150687562 |
| Example 156(DKC1125g6) | 0.99 | 0.040959537 | 1.15752525 |
| Example 157(DKC1125g7) | 1.05 | 0.059800725 | 1.088757172 |
| Example 158(DKC1125g8) | 1.00 | 0.021721992 | 1.170875812 |
| Example 159(DKC1125g9) | 0.99 | 0.05344675 | 1.059322396 |
| Example 160(DKC1125g10) | 1.05 | 0.039047303 | 1.066565508 |
| Example 161(DKC1125g11) | 0.95 | 0.11431494 | 1.121492247 |
| Example 162(DKC1125g12) | 0.99 | 0.04776634 | 1.042903884 |
| Example 163(DKC1125g13) | 1.10 | 0.046391673 | 1.162700934 |
| Example 164(DKC1125g14) | 0.98 | 0.027883891 | 1.088399165 |
| Example 165(DKC1125g15) | 1.07 | 0.031173347 | 1.133081227 |
| Example 166(DKC1125g16) | 1.01 | 0.04132212 | 1.094224477 |
| Example 167(DKC1 125g1 7) | 1.04 | 0.034419555 | 1.159165916 |
| Example 168(DKC1125g18) | 1.05 | 0.051934534 | 1.199229456 |
| Example 169(DKC1125g19) | 0.99 | 0.034417249 | 1.186412348 |
| Example 170(DKC1125g20) | 1.03 | 0.019980382 | 1.244191285 |
| Example 171(DKC1125g21) | 1.07 | 0.030083522 | 1.190841197 |
| Example 172(DKC1125g22) | 0.93 | 0.042731342 | 1.159585566 |
| Example 173(DKC1125g23) | 1.07 | 0.048598895 | 1.120954052 |
| Example 174(DKC1125g24) | 1.07 | 0.045490053 | 1.144233961 |
| Example 175(DKC1125g25) | 0.97 | 0.030587107 | 1.148880933 |
| Example 176(DKC1125g26) | 1.08 | 0.03777825 | 1.260190147 |
| Example 177(DKC1125g27) | 0.95 | 0.025583177 | 1.158698848 |
| Example 178(DKC1125g28) | 0.91 | 0.027068291 | 1.228353644 |
| Example 179(DKC1125g29) | 0.97 | 0.028444174 | 1.081229551 |
| Example 180(DKC1125g30) | 1.09 | 0.030961498 | 1.252928067 |
| Example 181(DKC1125g31) | 1.06 | 0.047857709 | 1.193766893 |
| Example 182(DKC1125g32) | 0.93 | 0.050219774 | 1.094710513 |
| Example 183(DKC1125g33) | 0.96 | 0.034837465 | 1.193745555 |
| Example 184(DKC1125g34) | 0.98 | 0.011433171 | 1.113907724 |
| Example 185(DKC1125g35) | 1.12 | 0.020945265 | 1.113542605 |
| Example 186(DKC1125g36) | 1.06 | 0.023271028 | 1.144717625 |
| Example 187(DKC1125g37) | 1.03 | 0.045390634 | 1.23497558 |
| Example 188(DKC1125g38) | 0.92 | 0.028776265 | 1.123381858 |
| Example 189(DKC1125g39) | 0.90 | 0.024634724 | 1.18502774 |
| Example 190(DKC1125g40) | 0.83 | 0.048934241 | 1.202513159 |
| Example 191(DKC1125g41) | 0.84 | 0.025066468 | 1.105775523 |
| Example 192(DKC1125g42) | 0.92 | 0.024830991 | 1.250301105 |
| Example 193(DKC1125g43) | 0.82 | 0.019545606 | 1.214076059 |
| Example 194(DKC1125g44) | 1.01 | 0.040004484 | 1.259054483 |
| Example 195(DKC1125g45) | 1.08 | 0.037760393 | 1.139454218 |
| Example 196(DKC1125g46) | 0.84 | 0.015353354 | 1.112954621 |
| Example 197(DKC1125g47) | 0.92 | 0.025999083 | 1.213772583 |
| Example 198(DKC1125g48) | 0.87 | 0.033098976 | 1.149841149 |
| Example 199(DKC1125g49) | 0.97 | 0.030843205 | 1.105472047 |
| Example 200(DKC1125g50) | 1.01 | 0.033825902 | 1.073633174 |
| EV | | | 1.047669401 |
| KIT | | | 1.287517189 |

**[Table 19]**

| | Relative KAI promoter activity | SD | *In vivo* relative invasion |
|---|---|---|---|
| cont | 1 | 0.045599246 | |
| #25 | 2.657463873 | 0.063163146 | |
| Example 201(DKC1125h1) | 1.45738535 | 0.079462555 | 0.928329384 |
| Example 202(DKC1125h2) | 1.312848554 | 0.057783663 | 1.13776894 |
| Example 203(DKC1125h3) | 1.053432732 | 0.023471095 | 1.17750128 |
| Example 204(DKC1125h4) | 1.304834442 | 0.036602658 | 1.095031374 |
| Example 205(DKC1125h5) | 1.096349677 | 0.043106182 | 1.165632777 |
| Example 206(DKC1125h6) | 1.050041236 | 0.030215636 | 1.10968126 |
| Example 207(DKC1125h7) | 1.430985662 | 0.052532617 | 1.036274662 |
| Example 208(DKC1125h8) | 1.141502143 | 0.042137772 | 1.18447855 |
| Example 209(DKC1125h9) | 1.124707639 | 0.052152941 | 1.069956065 |
| Example 210(DKC1125h10) | 1.02640633 | 0.023509915 | 0.927379237 |
| Example 211(DKC1125h11) | 1.440195485 | 0.035947539 | 0.869891769 |
| Example 212(DKC1125h12) | 1.012011355 | 0.015036199 | 1.218681463 |
| Example 213(DKC1125h13) | 1.136015792 | 0.03074789 | 1.209439556 |
| Example 214(DKC1125h14) | 1.085757281 | 0.034425293 | 1.244025861 |
| Example 215(DKC1125h15) | 0.975093626 | 0.03535319 | 1.304068487 |
| Example 216(DKC1125h16) | 1.202091224 | 0.03332846 | 1.254991844 |
| Example 217(DKC1125h17) | 1.179071269 | 0.036269836 | 1.265776846 |
| Example 218(DKC1125h18) | 1.333459412 | 0.057451894 | 1.169109505 |
| Example 219(DKC1125h19) | 1.27302544 | 0.040484495 | 1.145489183 |
| Example 220(DKC1125h20) | 1.14776276 | 0.03159924 | 0.932439544 |
| Example 221(DKC1125h21) | 0.961286968 | 0.031480315 | 1.32159739 |
| Example 222(DKC1125h22) | 1.196063971 | 0.044053472 | 1.275056854 |
| Example 223(DKC1125h23) | 1.096834141 | 0.018382377 | 1.194515818 |
| Example 224(DKC1125h24) | 1.133087247 | 0.025384233 | 1.249752938 |
| Example 225(DKC1125h25) | 1.349029621 | 0.045429658 | 1.234879207 |
| Example 226(DKC1125h26) | 1.088612385 | 0.05682518 | 1.348570612 |
| Example 227(DKC1125h27) | 1.012540188 | 0.01932845 | 1.153121316 |
| Example 228(DKC1125h28) | 0.982240102 | 0.013079669 | 1.277757272 |
| Example 229(DKC1125h29) | 1.058902384 | 0.033688612 | 1.244864086 |
| Example 230(DKC1125h30) | 1.075326759 | 0.032648818 | 1.266617453 |
| EV | | | 1 |
| KIT | | | 1.205379404 |

**[Table 20]**

| | Relative KAI promoter activity | SD | *In vivo* relative invation |
|---|---|---|---|
| cont | 1 | 0.129112624 | |
| Example 231(DKC1125i1) | 1.556398647 | 0.356950339 | 1.122056659 |
| Example 232(DKC1125i2) | 1.231242745 | 0.088151665 | 1.125203154 |
| Example 233(DKC1125i3) | 1.151771581 | 0.044393835 | 1.025831359 |
| Example 234(DKC1125i4) | 1.549705281 | 0.215596374 | 1.119107161 |
| Example 235(DKC1125i5) | 1.444493299 | 0.11360543 | 1.13306392 |
| Example 236(DKC1125i6) | 1.301399979 | 0.118292539 | 1.111736154 |
| Example 237(DKC1125i7) | 1.555303186 | 0.075592034 | 1.113930493 |
| Example 238(DKC1125i8) | 1.603237137 | 0.192740023 | 1.119662586 |
| Example 239(DKC1125i9) | 1.080807827 | 0.099370588 | 1.13515976 |
| Example 240(DKC1125i10) | 1.304013005 | 0.126278512 | 1.1003048 |
| Example 241(DKC1125i11) | 1.103382864 | 0.02639089 | 1.126095117 |
| Example 242(DKC1125i12) | 1.17351246 | 0.217832762 | 1.146366755 |
| Example 243(DKC1125i13) | 1.476648593 | 0.246946908 | 1.105040959 |
| Example 244(DKC1125i14) | 1.514132953 | 0.093639982 | 1.100228189 |
| Example 245(DKC1125i15) | 1.34532394 | 0.199290669 | 1.126305796 |
| Example 246(DKC1125i16) | 1.876396336 | 0.278631225 | 1.147773102 |
| Example 247(DKC1125i17) | 1.273772255 | 0.177238928 | 1.124489037 |
| Example 248(DKC1125i18) | 1.300907524 | 0.166614644 | 1.115525629 |
| Example 249(DKC1125i19) | 1.346344025 | 0.217908326 | 1.112617173 |
| Example 250(DKC1125i20) | 1.250584162 | 0.122450664 | 0.978086712 |
| Example 251(DKC1125i21) | 2.365102536 | 0.369710339 | 1.197359133 |
| Example 252(DKC1125i22) | 1.560973955 | 0.242281458 | 1.165185534 |
| Example 253(DKC1125i23) | 0.963357336 | 0.077755496 | 1.421250062 |
| Example 254(DKC1125i24) | 2.076943564 | 0.156698532 | 0.914289467 |
| Example 255(DKC1125i25) | 1.731617111 | 0.258660774 | 1.173719376 |
| Example 256(DKC1125i26) | 2.249993719 | 0.25040892 | 1.266677793 |
| Example 257(DKC1125i27) | 1.360595066 | 0.170128384 | 1.337561493 |
| Example 258(DKC1125i28) | 2.308821978 | 0.32046683 | 1.223037271 |
| Example 259(DKC1125i29) | 1.227092054 | 0.258824364 | 1.251473381 |
| Example 260(DKC1125i30) | 1.328778963 | 0.078885596 | 1.092134856 |
| Example 261(DKC1125i31) | 1.251468571 | 0.187588116 | 1.196759931 |
| Example 262(DKC1125i32) | 1.541190334 | 0.238186218 | 1.089562938 |
| Example 263(DKC1125i33) | 2.229416139 | 0.213378821 | 1.301160646 |
| Example 264(DKC1125i34) | 1.905300423 | 0.18881145 | 1.199189572 |
| Example 265(DKC1125i35) | 1.912227454 | 0.149517135 | 1.342792884 |
| Example 266(DKC1125i36) | 2.212346045 | 0.113129725 | 1.400822467 |
| Example 267(DKC1125i37) | 3.559546841 | 0.38015012 | 0.904641718 |
| Example 268(DKC1125i38) | 1.599719602 | 0.127926085 | 1.220238582 |
| Example 269(DKC1125i39) | 1.37605212 | 0.095531412 | 1.162686287 |
| Example 270(DKC1125i40) | 1.468374849 | 0.151630778 | 1.237535549 |
| Example 271(DKC1125i41) | 1.30625669 | 0.15116153 | 1.227526454 |
| Example 272(DKC1125i42) | 1.589342874 | 0.11944405 | 1.230201962 |
| Example 273(DKC1125i43) | 1.195039773 | 0.196597091 | 1.17105928 |
| Example 274(DKC1125i44) | 5.269907991 | 0.405048334 | 1.21949993 |
| Example 275(DKC1125i45) | 6.848650523 | 1.198566554 | 1.145663608 |
| Example 276(DKC1125i46) | 3.393903609 | 0.21257212 | 1.224121918 |
| Example 277(DKC1125i47) | 1.300399994 | 0.124870762 | 1.126237302 |
| Example 278(DKC1125i48) | 1.380129948 | 0.265090603 | 1.217358561 |
| Example 279(DKC1125i49) | 2.276031015 | 0.299057444 | 1.187429924 |
| Example 280(DKC1125i50) | 1.628018171 | 0.189568898 | 1.19499449 |
| Example 281(DKC1125i51) | 1.518168068 | 0.18664641 | 1.236428774 |
| Example 282(DKC1125i52) | 1.470347181 | 0.166307965 | 1.213865002 |
| Example 283(DKC1125i53) | 1.328550323 | 0.189829759 | 1.235314781 |
| Example 284(DKC1125i54) | 1.443445576 | 0.142080934 | 1.275521989 |
| Example 285(DKC1125i55) | 1.471193902 | 0.109249503 | 1.246500426 |
| Example 286(DKC1125i56) | 1.429571413 | 0.231373226 | 1.232636867 |
| Example 287(DKC1125i57) | 2.059948845 | 0.157138042 | 1.375947375 |
| Example 288(DKC1125i58) | 1.730695015 | 0.22183601 | 1.221513779 |
| Example 289(DKC1125i59) | 1.614154058 | 0.09440281 | 1.262830168 |
| Example 290(DKC1125i60) | 1.231245258 | 0.244002014 | 0.92340877 |
| Example 291(DKC1125i61) | 1.61539022 | 0.243489702 | 1.125527523 |
| Example 292(DKC1125i62) | 1.448628915 | 0.252312759 | 1.238358412 |
| Example 293(DKC1125i63) | 1.246390758 | 0.226601045 | 1.174329078 |
| Example 294(DKC1125i64) | 1.565235197 | 0.14861702 | 1.033340391 |
| Example 295(DKC1125i65) | 1.524549881 | 0.099988167 | 1.310621189 |
| Example 296(DKC1125i66) | 1.531260835 | 0.185676483 | 1.026110264 |
| Example 297(DKC1125i67) | 1.327040296 | 0.133929746 | 1.230702417 |
| Example 298(DKC1125i68) | 1.341102898 | 0.081503164 | 1.213135975 |
| Example 299(DKC1125i69) | 2.166005035 | 0.449875932 | 1.06790064 |
| Example 300(DKC1125i70) | 1.911006367 | 0.368118524 | 0.986889529 |
| Example 301(DKC1125i71) | 1.614450536 | 0.220205424 | 1.145603457 |
| Example 302(DKC1125i72) | 1.412219916 | 0.242568691 | 1.175212092 |
| Example 303(DKC1125i73) | 1.846492264 | 0.176562687 | 1.137812724 |
| Example 304(DKC1125i74) | 1.236122069 | 0.047645221 | 1.193134627 |
| Example 305(DKC1125i75) | 1.14356065 | 0.045375583 | 1.186202848 |
| EV | | | 1 |
| KIT | | | 1.369215237 |

## Claims

1. A compound represented by the following Formula 1 or a pharmaceutically acceptable salt thereof: [Formula 1] 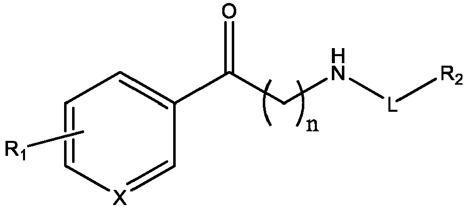 wherein
X is C or N;
L is C1 to C4 alkylene or a bond;
R₁ is hydrogen, halogen, substituted or unsubstituted C1-C4 alkyl, substituted or unsubstituted C1-C4 alkoxy, di(C1-C4 alkyl)amino, mono(C1-C4 alkyl)amino, amino, or substituted or unsubstituted 5- to 12-membered heterocycle, wherein the substituent of the substituted alkyl is amino unsubstituted or substituted with one or two substituents selected from the group consisting of C1-C6 alkyl, C2-C6 alkenyl, C2-C6 alkynyl, C6-C9 aryl, benzyl, hydroxy-C1-C6 alkyl, and C3-C8 cycloalkyl; or a 5- to 12-membered heterocycle or heteroaryl unsubstituted or substituted with one or two substituents selected from the group consisting of C1-C4 alkyl, C1-C4 alkoxycarbonyl, C6-C9 aryl unsubstituted or substituted with one or two substituents selected from the group consisting of C1-C4 alkyl, C1-C4 alkoxy, halogen and nitro, and a 5-to 10-membered heterocycle or heteroaryl;
R₂ is 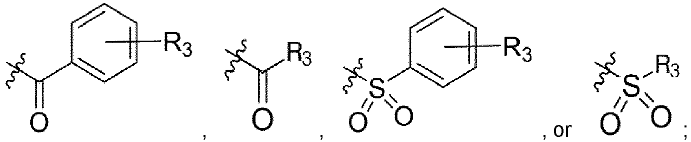
R₃ is at least one selected from the group consisting of hydrogen, halogen, substituted or unsubstituted C1-C6 alkyl, substituted or unsubstituted C2-C6 alkenyl, substituted or unsubstituted C3-C8 cycloalkyl, substituted or unsubstituted C1-C6 alkoxy, substituted or unsubstituted 5- to 9-membered heteroaryl, substituted or unsubstituted C6-C10 aryl, substituted or unsubstituted C6-C10 aryloxy, substituted or unsubstituted 5- to 9-membered heterocyclyl, substituted or unsubstituted 5- to 9-membered heterocyclyloxy, nitro, 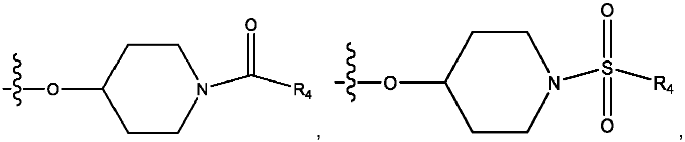 and -O-CH₂-C(O)-NH-R₅;
R₄ hydrogen; halogen; C1-C6 alkyl unsubstituted or substituted with C1-C4 alkoxy, phenoxy or phenyl, wherein the phenyl is unsubstituted or substituted with halogen; C2-C6 alkenyl; amino; di(C1-C4 alkyl)amino; C6-C9 aryl unsubstituted or substituted with at least one selected from the group consisting of halogen, C1-C4 alkoxy, C1-C4 alkyl unsubstituted or substituted with at least one halogen, nitro, C1-C4 alkylcarbonyloxy, and -SO₂; C3-C8 cycloalkyl; or 5- to 9-membered heterocyclyl;
R₅ is at least one (one or two) selected from the group consisting of hydrogen, halogen, hydroxy, substituted or unsubstituted C1-C4 alkyl, nitro, and C6-C10 aryloxy;
n is an integer ranging from 0 to 2.

2. The compound or pharmaceutically acceptable salt thereof according to claim 1, wherein, in the compound of Formula 1,
X is C or N;
L is C1 to C4 alkylene or a bond;
R₁ is hydrogen, F, Cl, Br, substituted or unsubstituted C1-C4 alkyl, methoxy, N(CH₃)₂, NH(CH₃), amino, or a substituted or unsubstituted 5- to 12-membered heterocycle, wherein the substituent of the substituted alkyl is may be unsubstituted or substituted with dimethylamino, diethylamino, dipropylamino, dibutylamino, hydroxyethyl(ethyl)amino, ethyl(butyl)amino, dipentylamino, methyl(ethynyl)amino, ethyl(phenyl)amino, diallylamino, methyl(benzyl)amino, methyl(hydroxyethyl)amino, phenyl(methyl)amino, ethyl(phenyl)amino, butyl(hydroxyethyl)amino, butyl(methyl)amino, dicyclohexylamino, ethoxycarbonyl-piperazine, t-butoxycarbonyl-piperazine, fluorophenyl-piperidine, pyridinyl-piperazine, pyrimidinyl-piperazine, hydroxyethylpiperazine, methoxyphenyl-piperazine, nitrophenyl-piperazine, dimethylmorpholine, morpholine, methylpiperazine, dihydroxyisoquinoline, cyclohexyl(methyl)amino, isopropyl(methyl)amino, fluorophenyl-piperazine, or piperidinylpiperidine;
R₂ is 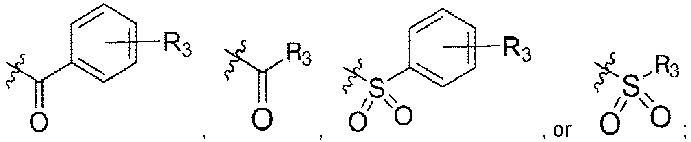
R₃ is at least one selected from the group consisting of hydrogen, F, Cl, Br, substituted or unsubstituted C1-C4 alkyl, substituted or unsubstituted C2-C4 alkenyl, substituted or unsubstituted C3-C8 cycloalkyl, substituted or unsubstituted C1-C4 alkoxy, substituted or unsubstituted 5- to 9-membered heteroaryl, substituted or unsubstituted C6-C10 aryl, substituted or unsubstituted C6-C10 aryloxy, substituted or unsubstituted 5- to 9-membered heterocyclyl, substituted or unsubstituted 5- to 9-membered heterocyclyloxy, nitro, 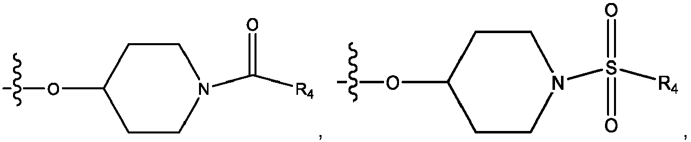 and -O-CH₂-C(O)-NH-R₅;
R₄ is hydrogen; F, Cl, Br; C1-C4 unsubstituted or substituted with methoxy, ethoxy, phenoxy or phenyl, wherein the phenyl may be unsubstituted or substituted with halogen; C2-C4 alkenyl; amino; di(C1-C4 alkyl)amino; C6-C9 aryl unsubstituted or substituted with at least one selected from the group consisting of halogen, C1-C3 alkoxy, C1-C4 alkyl unsubstituted or substituted with at least one halogen, nitro, C1-C3 alkylcarbonyloxy, and -SO₂; C3-C8 cycloalkyl; or 5- to 9-membered heterocyclyl;
R₅ is at least one (one or two) selected from the group consisting of hydrogen, halogen, substituted or unsubstituted C1-C4 alkyl, nitro, and C6-C10 aryloxy;
the substituent of the substituted C1-C4 alkyl, substituted C2-C4 alkenyl, substituted C3-C8 cycloalkyl, substituted C1-C4 alkoxy, substituted 5- to 9-membered heteroaryl, substituted C6-C10 aryl, substituted C6-C10 aryloxy, substituted 5- to 9-membered heterocyclyl, or substituted 5- to 9-membered heterocyclyloxy of R₃ is at least one selected from the group consisting of C1-C4 alkoxy, C6-C9 aryl and C6-C9 aryloxy, more preferably, at least one selected from the group consisting of phenyl, methoxy and phenoxy; and
n is an integer ranging from 0 to 2.

3. The compound or pharmaceutically acceptable salt thereof according to claim 1, which is selected from the group consisting of the following compounds 1 to 305:
4-chloro-N-(2-oxo-2-phenylethyl)benzamide (DKC1125a01);
N-(2-oxo-2-phenylethyl)-2-phenoxypropanamide (DKC1125a02);
2-fluoro-N-(2-oxo-2-phenylethyl)benzamide (DKC1125a03);
4-fluoro-N-(2-oxo-2-phenylethyl)benzamide (DKC1125a04);
N-(2-oxo-2-phenylethyl)furan-2-carboxamide (DKC1125a05);
2-chloro-N-(2-oxo-2-phenylethyl)benzamide (DKC1125a06);
3-chloro-N-(2-oxo-2-phenylethyl)benzamide (DKC1125a07);
(E)-N-(2-oxo-2-phenylethyl)but-2-enamide (DKC1125a08);
N-(2-oxo-2-phenylethyl)-3-phenylpropanamide (DKC1125a09);
N-(2-oxo-2-phenylethyl)acrylamide (DKC1125a10);
2-methyl-N-(2-oxo-2-phenylethyl)benzamide (DKC1125a11);
4-methoxy-N-(2-oxo-2-phenylethyl)benzamide (DKC1125a12);
3-methyl-N-(2-oxo-2-phenylethyl)benzamide (DKC1125a13);
N-(2-oxo-2-phenylethyl)propionamide (DKC1125a14);
N-(2-oxo-2-phenylethyl)cyclohexanecarboxamide (DKC1125a15);
N-(2-oxo-2-phenylethyl)cyclopropanecarboxamide (DKC1125a16);
N-(2-oxo-2-phenylethyl)cyclobutanecarboxamide (DKC1125a17);
N-(2-oxo-2-phenylethyl)thiophene-2-carboxamide (DKC1125a18);
3,3-dimethyl-N-(2-oxo-2-phenylethyl)butanamide (DKC1125a19);
N-(2-oxo-2-phenylethyl)pentanamide (DKC1125a20);
3-nitro-N-(2-oxo-2-phenylethyl)benzenesulfonamide (DKC1125a21);
2,4-difluoro-N-(2-oxo-2-phenylethyl)benzamide (DKC1125a22);
2-methoxy-N-(2-oxo-2-phenylethyl)acetamide (DKC1125a23);
N-(2-oxo-2-phenylethyl)pivalamide (DKC1125a24);
N-(2-oxo-2-phenylethyl)methanesulfonamide (DKC1125a25);
4-fluoro-N-(2-oxo-2-phenylethyl)benzenesulfonamide (DKC1125a26);
4-methyl-N-(2-oxo-2-phenylethyl)benzenesulfonamide (DKC1125a27);
N-(2-oxo-2-phenylethyl)-1-phenylmethanesulfonamide (DKC1125a28);
N-(2-oxo-2-phenylethyl)-3,5-bis(trifluoromethyl)benzamide (DKC1125a29);
N-(2-oxo-2-phenylethyl)-2-(trifluoromethyl)benzamide (DKC1125a30);
N-(2-oxo-2-phenylethyl)-3-(trifluoromethyl)benzamide (DKC1125a31);
N-(2-oxo-2-phenylethyl)-4-(trifluoromethyl)benzamide (DKC1125a32);
2-chloro-4-fluoro-N-(2-oxo-2-phenylethyl)benzamide (DKC1125a33);
3-nitro-N-(2-oxo-2-phenylethyl)benzamide (DKC1125a34);
3,4-difluoro-N-(2-oxo-2-phenylethyl)benzamide (DKC1125a35);
3,5-dichloro-N-(2-oxo-2-phenylethyl)benzamide (DKC1125a36);
2,3-dichloro-N-(2-oxo-2-phenylethyl)benzamide (DKC1125a37);
N-(2-oxo-2-phenylethyl)-4-(trifluoromethoxy)benzamide (DKC1125a38);
N-(2-oxo-2-phenylethyl)-3-(trifluoromethoxy)benzamide (DKC1125a39);
3,5=dimethyl-N-(2-oxo-2-phenylethyl)benzamide (DKC1125a40);
N-(2-oxo-2-phenylethyl)-2-(trifluoromethoxy)benzamide (DKC1125a41);
3,5-dimethoxy-N-(2-oxo-2-phenylethyl)benzamide (DKC1125a42);
N-(2-oxo-2-phenylethyl)-[1,1'-biphenyl]-4-carboxamide (DKC1125a43);
N-(2-(4-chlorophenyl)-2-oxoethyl)-2-fluorobenzamide (DKC1125b01);
N-(2-(4-chlorophenyl)-2-oxoethyl)-4-fluorobenzamide (DKC1125b02);
N-(2-(4-chlorophenyl)-2-oxoethyl)furan-2-carboxamide (DKC1125b03);
3-chloro-N-(2-(4-chlorophenyl)-2-oxoethyl)benzamide (DKC1125b04);
(E)-N-(2-(4-chlorophenyl)-2-oxoethyl)but-2-enamide (DKC1125b05);
N-(2-(4-chlorophenyl)-2-oxoethyl)-3-phenylpropanamide (DKC1125b06);
N-(2-(4-chlorophenyl)-2-oxoethyl)acrylamide (DKC1125b07);
N-(2-(4-chlorophenyl)-2-oxoethyl)-2-methylbenzamide (DKC1125b08);
N-(2-(4-chlorophenyl)-2-oxoethyl)-4-methoxybenzamide (DKC1125b09);
N-(2-(4-chlorophenyl)-2-oxoethyl)-3-methylbenzamide (DKC1125b10);
N-(2-(4-chlorophenyl)-2-oxoethyl)propionamide (DKC1125b11);
N-(2-(4-chlorophenyl)-2-oxoethyl)cyclohexanecarboxamide (DKC1125b12);
N-(2-(4-chlorophenyl)-2-oxoethyl)cyclopropanecarboxamide (DKC1125b13);
N-(2-(4-chlorophenyl)-2-oxoethyl)cyclobutanecarboxamide (DKC1125b14);
N-(2-(4-chlorophenyl)-2-oxoethyl)thiophene-2-carboxamide (DKC1125b15);
N-(2-(4-chlorophenyl)-2-oxoethyl)-2-(methoxymethyl)benzamide (DKC1125b16);
N-(2-(4-chlorophenyl)-2-oxoethyl)-3,3-dimethylbutanamide (DKC1125b17);
N-(2-(4-chlorophenyl)-2-oxoethyl)pentanamide (DKC1125b18);
N-(2-(4-chlorophenyl)-2-oxoethyl)-4-methoxybenzamide (DKC1125b19);
N-(2-(4-chlorophenyl)-2-oxoethyl)-3-nitrobenzenesulfonamide (DKC1125b20);
N-(2-(4-chlorophenyl)-2-oxoethyl)-2,4ifluorobenzamide (DKC1125b21);
N-(2-(4-chlorophenyl)-2-oxoethyl)-2-methoxyacetamide (DKC1125b22);
N-(2-(4-chlorophenyl)-2-oxoethyl)pivalamide (DKC1125b23);
N-(2-(4-chlorophenyl)-2-oxoethyl)-4-fluorobenzenesulfonamide (DKC1125b24);
N-(2-(4-chlorophenyl)-2-oxoethyl)-4-methylbenzenesulfonamide (DKC1125b25);
4-chloro-N-(2-(4-chlorophenyl)-2-oxoethyl)benzenesulfonamide (DKC1125b26);
N-(2-(4-chlorophenyl)-2-oxoethyl)-1-phenylmethanesulfonamide (DKC1125b27);
N-(2-(4-chlorophenyl)-2-oxoethyl)-4-methoxybenzenesulfonamide (DKC1125b28);
N-(2-(4-chlorophenyl)-2-oxoethyl)-3,5-bis(trifluoromethyl)benzamide (DKC1125b29);
N-(2-(4-chlorophenyl)-2-oxoethyl)-2-(trifluoromethyl)benzamide (DKC1125b30);
N-(2-(4-chlorophenyl)-2-oxoethyl)-3-(trifluoromethyl)benzamide (DKC1125b31);
N-(2-(4-chlorophenyl)-2-oxoethyl)-4-(trifluoromethyl)benzamide (DKC1125b32);
2-chloro-N-(2-(4-chlorophenyl)-2-oxoethyl)-4-fluorobenzamide (DKC1125b33);
N-(2-(4-chlorophenyl)-2-oxoethyl)-3-nitrobenzamide (DKC1125b34);
N-(2-(4-chlorophenyl)-2-oxoethyl)-3,4ifluorobenzamide (DKC1125b35);
3,5-dichloro-N-(2-(4-chlorophenyl)-2-oxoethyl)benzamide (DKC1125b36);
2,3-dichloro-N-(2-(4-chlorophenyl)-2-oxoethyl)benzamide (DKC1125b37);
N-(2-(4-chlorophenyl)-2-oxoethyl)-4-(trifluoromethoxy)benzamide (DKC1125b38);
N-(2-(4-chlorophenyl)-2-oxoethyl)-3-(trifluoromethoxy)benzamide (DKC1125b39);
N-(2-(4-chlorophenyl)-2-oxoethyl)-3,5-dimethylbenzamide (DKC1125b40);
N-(2-(4-chlorophenyl)-2-oxoethyl)-2-(trifluoromethoxy)benzamide (DKC1125b41);
N-(2-(4-chlorophenyl)-2-oxoethyl)-3,5imethoxybenzamide (DKC1125b42);
N-(2-(4-chlorophenyl)-2-oxoethyl)-[1,1'-biphenyl]-4-carboxamide (DKC1125b43);
4-chloro-N-(2-(4-chlorophenyl)-2-oxoethyl)benzamide (DKC1125b44);
4-chloro-N-(2-(4-(dimethylamino)phenyl)-2-oxoethyl)benzamide (DKC1125c01);
N-(2-(4-(dimethylamino)phenyl)-2-oxoethyl)-2-phenoxypropanamide (DKC1125c02);
N-(2-(4-(dimethylamino)phenyl)-2-oxoethyl)-2-fluorobenzamide (DKC1125c03);
N-(2-(4-(dimethylamino)phenyl)-2-oxoethyl)-4-fluorobenzamide (DKC1125c04);
N-(2-(4-(dimethylamino)phenyl)-2-oxoethyl)furan-2-carboxamide (DKC1125c05);
2-chloro-N-(2-(4-(dimethylamino)phenyl)-2-oxoethyl)benzamide (DKC1125c06);
3-chloro-N-(2-(4-(dimethylamino)phenyl)-2-oxoethyl)benzamide (DKC1125c07);
N-(2-(4-(dimethylamino)phenyl)-2-oxoethyl)acrylamide (DKC1125c08);
N-(2-(4-(dimethylamino)phenyl)-2-oxoethyl)-2-methylbenzamide (DKC1125c09);
N-(2-(4-(dimethylamino)phenyl)-2-oxoethyl)-4-methoxybenzamide (DKC1125c10);
N-(2-(4-(dimethylamino)phenyl)-2-oxoethyl)-3-methylbenzamide (DKC1125c11);
N-(2-(4-(dimethylamino)phenyl)-2-oxoethyl)propionamide (DKC1125c12);
N-(2-(4-(dimethylamino)phenyl)-2-oxoethyl)cyclopropanecarboxamide (DKC1125c13);
N-(2-(4-(dimethylamino)phenyl)-2-oxoethyl)thiophene-2-carboxamide (DKC1125c14);
N-(2-(4-(dimethylamino)phenyl)-2-oxoethyl)-3,3-dimethylbutanamide (DKC1125c15);
N-(2-(4-(dimethylamino)phenyl)-2-oxoethyl)pentanamide (DKC1125c16);
N-(2-(4-(dimethylamino)phenyl)-2-oxoethyl)-4-methoxybenzamide (DKC1125c17);
N-(2-(4-(dimethylamino)phenyl)-2-oxoethyl)-2,4ifluorobenzamide (DKC1125c18);
N-(2-(4-(dimethylamino)phenyl)-2-oxoethyl)-2-methoxyacetamide (DKC1125c19);
N-(2-(4-(dimethylamino)phenyl)-2-oxoethyl)-4-fluorobenzenesulfonamide (DKC1125c20);
4-chloro-N-(2-(4-(dimethylamino)phenyl)-2-oxoethyl)benzenesulfonamide (DKC1125c21);
N-(2-(4-(dimethylamino)phenyl)-2-oxoethyl)benzenesulfonamide (DKC1125c22);
N-(2-(4-(dimethylamino)phenyl)-2-oxoethyl)-1-phenylmethanesulfonamide (DKC1125c23);
N-(2-(4-(dimethylamino)phenyl)-2-oxoethyl)-4-methoxybenzenesulfonamide (DKC1125c24);
N-(2-(4-(dimethylamino)phenyl)-2-oxoethyl)-2-(trifluoromethyl)benzamide (DKC1125c25);
N-(2-(4-(dimethylamino)phenyl)-2-oxoethyl)-3-(trifluoromethyl)benzamide (DKC1125c26);
N-(2-(4-(dimethylamino)phenyl)-2-oxoethyl)-4-(trifluoromethyl)benzamide (DKC1125c27);
2-chloro-N-(2-(4-(dimethylamino)phenyl)-2-oxoethyl)-4-fluorobenzamide (DKC1125c28);
N-(2-(4-(dimethylamino)phenyl)-2-oxoethyl)-3,4ifluorobenzamide (DKC1125c29);
3,5-dichloro-N-(2-(4-(dimethylamino)phenyl)-2-oxoethyl)benzamide (DKC1125c30);
2,3-dichloro-N-(2-(4-(dimethylamino)phenyl)-2-oxoethyl)benzamide (DKC1125c31);
N-(2-(4-(dimethylamino)phenyl)-2-oxoethyl)-4-(trifluoromethoxy)benzamide (DKC1125c32);
N-(2-(4-(dimethylamino)phenyl)-2-oxoethyl)-3,5imethylbenzamide (DKC1125c33);
N-(2-(4-(dimethylamino)phenyl)-2-oxoethyl)-3,5imethoxybenzamide (DKC1125c34);
4-chloro-N-(2-(3-methoxyphenyl)-2-oxoethyl)benzamide (DKC1125d01);
(E)-N-(2-(3-methoxyphenyl)-2-oxoethyl)but-2-enamide (DKC1125d02);
N-(2-(3-methoxyphenyl)-2-oxoethyl)propionamide (DKC1125d03);
N-(2-(3-methoxyphenyl)-2-oxoethyl)cyclopropanecarboxamide (DKC1125d04);
N-(2-(3-methoxyphenyl)-2-oxoethyl)cyclobutanecarboxamide (DKC1125d05);
N-(2-(3-methoxyphenyl)-2-oxoethyl)thiophene-2-carboxamide (DKC1125d06);
N-(2-(3-methoxyphenyl)-2-oxoethyl)pentanamide (DKC1125d07);
N-(2-(3-methoxyphenyl)-2-oxoethyl)benzenesulfonamide (DKC1125d08);
4-chloro-N-(2-oxo-2-(pyridin-3-yl)ethyl)benzamide (DKC1125e01);
N-(2-oxo-2-(pyridin-3-yl)ethyl)-2-phenoxypropanamide (DKC1125e02);
2-fluoro-N-(2-oxo-2-(pyridin-3-yl)ethyl)benzamide (DKC1125e03);
4-fluoro-N-(2-oxo-2-(pyridin-3-yl)ethyl)benzamide (DKC1125e04);
(E)-N-(2-oxo-2-(pyridin-3-yl)ethyl)but-2-enamide (DKC1125e05);
2-methyl-N-(2-oxo-2-(pyridin-3-yl)ethyl)benzamide (DKC1125e06);
N-(2-oxo-2-(pyridin-3-yl)ethyl)propionamide (DKC1125e07);
2-fluoro-N-(2-(4-morpholinophenyl)-2-oxoethyl)benzamide (DKC1125f01);
2-chloro-N-(2-(4-morpholinophenyl)-2-oxoethyl)benzamide (DKC1125f02);
(E)-N-(2-(4-morpholinophenyl)-2-oxoethyl)but-2-enamide (DKC1125f03);
4-methoxy-N-(2-(4-morpholinophenyl)-2-oxoethyl)benzamide (DKC1125f04);
3-methyl-N-(2-(4-morpholinophenyl)-2-oxoethyl)benzamide (DKC1125f05);
N-(2-(4-morpholinophenyl)-2-oxoethyl)propionamide (DKC1125f06);
N-(2-(4-morpholinophenyl)-2-oxoethyl)cyclopropanecarboxamide (DKC1125f07);
N-(2-(4-morpholinophenyl)-2-oxoethyl)cyclobutanecarboxamide (DKC1125f08);
N-(2-(4-morpholinophenyl)-2-oxoethyl)pentanamide (DKC1125f09);
4-fluoro-N-(2-(4-morpholinophenyl)-2-oxoethyl)benzenesulfonamide (DKC1125f10);
4-chloro-N-(2-(4-morpholinophenyl)-2-oxoethyl)benzenesulfonamide (DKC1125f11);
N-(2-(4-morpholinophenyl)-2-oxoethyl)benzenesulfonamide (DKC1125f12);
N-(2-(4-morpholinophenyl)-2-oxoethyl)-1-phenylmethanesulfonamide (DKC1125f13);
4-methoxy-N-(2-(4-morpholinophenyl)-2-oxoethyl)benzenesulfonamide (DKC1125f14);
2-((1-(4-chlorobenzoyl)piperidin-4-yl)oxy)-N-(2-oxo-2-phenylethyl)benzamide (DKC1125g01);
N-(2-oxo-2-phenylethyl)-2-((1-(2-phenoxypropanoyl)piperidin-4-yl)oxy)benzamide (DKC1125g02);
2-((1-(2-fluorobenzoyl)piperidin-4-yl)oxy)-N-(2-oxo-2-phenylethyl)benzamide (DKC1125g03);
2-((1-(4-fluorobenzoyl)piperidin-4-yl)oxy)-N-(2-oxo-2-phenylethyl)benzamide (DKC1125g04);
2-((1-(furan-2-carbonyl)piperidin-4-yl)oxy)-N-(2-oxo-2-phenylethyl)benzamide (DKC1125g05);
2-((1-(2-chlorobenzoyl)piperidin-4-yl)oxy)-N-(2-oxo-2-phenylethyl)benzamide (DKC1125g06);
2-((1-(3-chlorobenzoyl)piperidin-4-yl)oxy)-N-(2-oxo-2-phenylethyl)benzamide (DKC1125g07);
(E)-2-((1-(but-2-enoyl)piperidin-4-yl)oxy)-N-(2-oxo-2-phenylethyl)benzamide (DKC1125g08);
N-(2-oxo-2-phenylethyl)-2-((1-(3-phenylpropanoyl)piperidin-4-yl)oxy)benzamide (DKC1125g09);
2-((1-acryloylpiperidin-4-yl)oxy)-N-(2-oxo-2-phenylethyl)benzamide (DKC1125g10);
2-((1-(2-methylbenzoyl)piperidin-4-yl)oxy)-N-(2-oxo-2-phenylethyl)benzamide (DKC1125g11);
2-((1-(2-methoxybenzoyl)piperidin-4-yl)oxy)-N-(2-oxo-2-phenylethyl)benzamide (DKC1125g12);
2-((1-(3-methylbenzoyl)piperidin-4-yl)oxy)-N-(2-oxo-2-phenylethyl)benzamide (DKC1125g13);
N-(2-oxo-2-phenylethyl)-2-((1-propionylpiperidin-4-yl)oxy)benzamide (DKC1125g14);
2-((1-(cyclohexanecarbonyl)piperidin-4-yl)oxy)-N-(2-oxo-2-phenylethyl)benzamide (DKC1125g15);
2-((1-(cyclopropanecarbonyl)piperidin-4-yl)oxy)-N-(2-oxo-2-phenylethyl)benzamide (DKC1125g16);
2-((1-(cyclobutanecarbonyl)piperidin-4-yl)oxy)-N-(2-oxo-2-phenylethyl)benzamide (DKC1125g17);
N-(2-oxo-2-phenylethyl)-2-((1-(thiophene-2-carbonyl)piperidin-4-yl)oxy)benzamide (DKC1125g18);
2-((1-(2-(methoxymethyl)benzoyl)piperidin-4-yl)oxy)-N-(2-oxo-2-phenylethyl)benzamide (DKC1125g19);
2-((1-(3,3-dimethylbutanoyl)piperidin-4-yl)oxy)-N-(2-oxo-2-phenylethyl)benzamide (DKC1125g20);
2-((1-(morpholine-4-carbonyl)piperidin-4-yl)oxy)-N-(2-oxo-2-phenylethyl)benzamide (DKC1125g21);
N-(2-oxo-2-phenylethyl)-2-((1-pentanoylpiperidin-4-yl)oxy)benzamide (DKC1125g22);
2-((1-(4-methoxybenzoyl)piperidin-4-yl)oxy)-N-(2-oxo-2-phenylethyl)benzamide (DKC1125g23);
2-((1-(4-cyanobenzoyl)piperidin-4-yl)oxy)-N-(2-oxo-2-phenylethyl)benzamide (DKC1125g24);
2-((1-((3-(dioxo-l5-sulfanyl)phenyl)sulfonyl)piperidin-4-yl)oxy)-N-(2-oxo-2-phenylethyl)benzamide (DKC1125g25);
2-(4-(2-((2-oxo-2-phenylethyl)carbamoyl)phenoxy)piperidine-1-carbonyl)phenylacetate (DKC1125g26);
2-((1-(2,4-difluorobenzoyl)piperidin-4-yl)oxy)-N-(2-oxo-2-phenylethyl)benzamide (DKC1125g27);
2-((1-(2-methoxyacetyl)piperidin-4-yl)oxy)-N-(2-oxo-2-phenylethyl)benzamide (DKC1125g28);
N-(2-oxo-2-phenylethyl)-2-((1-pivaloylpiperidin-4-yl)oxy)benzamide (DKC1125g29);
N,N-dimethyl-4-(2-((2-oxo-2-phenylethyl)carbamoyl)phenoxy)piperidine-1-carboxamide (DKC1125g30);
2-((1-(4-bromobenzoyl)piperidin-4-yl)oxy)-N-(2-oxo-2-phenylethyl)benzamide (DKC1125g31);
2-((1-acetylpiperidin-4-yl)oxy)-N-(2-oxo-2-phenylethyl)benzamide (DKC1125g32);
2-((1-(methylsulfonyl)piperidin-4-yl)oxy)-N-(2-oxo-2-phenylethyl)benzamide (DKC1125g33);
2-((1-((4-fluorophenyl)sulfonyl)piperidin-4-yl)oxy)-N-(2-oxo-2-phenylethyl)benzamide (DKC1125g34);
N-(2-oxo-2-phenylethyl)-2-((1-tosylpiperidin-4-yl)oxy)benzamide (DKC1125g35);
2-((1-((4-chlorophenyl)sulfonyl)piperidin-4-yl)oxy)-N-(2-oxo-2-phenylethyl)benzamide (DKC1125g36);
N-(2-oxo-2-phenylethyl)-2-((1-(phenylsulfonyl)piperidin-4-yl)oxy)benzamide (DKC1125g37);
2-((1-(benzylsulfonyl)piperidin-4-yl)oxy)-N-(2-oxo-2-phenylethyl)benzamide (DKC1125g38);
2-((1-((4-methoxyphenyl)sulfonyl)piperidin-4-yl)oxy)-N-(2-oxo-2-phenylethyl)benzamide (DKC1125g39);
2-((1-(3,5-bis(trifluoromethyl)benzoyl)piperidin-4-yl)oxy)-N-(2-oxo-2-phenylethyl)benzamide (DKC1125g40);
N-(2-oxo-2-phenylethyl)-2-((1-(2-(trifluoromethyl)benzoyl)piperidin-4-yl)oxy)benzamide (DKC1125g41);
N-(2-oxo-2-phenylethyl)-2-((1-(3-(trifluoromethyl)benzoyl)piperidin-4-yl)oxy)benzamide (DKC1125g42);
N-(2-oxo-2-phenylethyl)-2-((1-(4-(trifluoromethyl)benzoyl)piperidin-4-yl)oxy)benzamide (DKC1125g43);
2-((1-(2-chloro-4-fluorobenzoyl)piperidin-4-yl)oxy)-N-(2-oxo-2-phenylethyl)benzamide (DKC1125g44);
2-((1-(3-nitrobenzoyl)piperidin-4-yl)oxy)-N-(2-oxo-2-phenylethyl)benzamide (DKC1125g45);
2-((1-(3,4-difluorobenzoyl)piperidin-4-yl)oxy)-N-(2-oxo-2-phenylethyl)benzamide (DKC1125g46);
2-((1-(3,5-dichlorobenzoyl)piperidin-4-yl)oxy)-N-(2-oxo-2-phenylethyl)benzamide (DKC1125g47);
2-((1-(2,3-dichlorobenzoyl)piperidin-4-yl)oxy)-N-(2-oxo-2-phenylethyl)benzamide (DKC1125g48);
N-(2-oxo-2-phenylethyl)-2-((1-(4-(trifluoromethoxy)benzoyl)piperidin-4-yl)oxy)benzamide (DKC1125g49);
N-(2-oxo-2-phenylethyl)-2-((1-(3-(trifluoromethoxy)benzoyl)piperidin-4-yl)oxy)benzamide (DKC1125g50);
2-(2-oxo-2-(phenylamino)ethoxy)-*N*-(2-oxo-2-phenylethyl)benzamide (DKC1125h01);
2-(2-oxo-2-(o-toylamino)ethoxy)-*N*-(2-oxo-2-phenylethyl)benzamide (DKC1125h02);
2-(2-(allylamino)-2-oxoethoxy)-*N*-(2-oxo-2-phenylethyl)benzamide (DKC1125h03);
2-(2-(benzylamino)-2-oxoethoxy)-*N*-(2-oxo-2-phenylethyl)benzamide (DKC1125h04);
2-(2-((fluorobenzyl)amino)-2-oxoethoxy)-*N*-(2-oxo-2-phenylethyl)benzamide (DKC1125h05);
2-(2-((2-methoxy-4-methylphenyl)amino)-2-oxoethoxy)-*N*-(2-oxo-2-phenylethyl)benzamide (DKC1125h06);
2-(2-((3-fluoro-4-methoxyphenyl)amino)-2-oxoethoxy)-*N*(2-oxo-2-phenylethyl)benzamide (DKC1125h07);
2-(2-oxo-2-(pyridin-3-ylamino)ethoxy)-*N*-(2-oxo-2-phenylethyl)benzamide (DKC1125h08);
2-(2-((3-methoxyphenyl)amino)-2-oxoethoxy)-*N*-(2-oxo-2-phenylethyl)benzamide (DKC1125h09);
2-(2-oxo-2-((2-propylphenyl)amino)ethoxy)-*N*-(2-oxo-2-phenylethyl)benzamide (DKC1125h10);
2-(2-((1-fluoro-3-nitrophenyl)amino)-2-oxoethoxy)-*N*-(2-oxo-2-phenylethylbenzamide (DKC1125h11);
2-(2-((benzo [1,3]dioxol-5-ylmethyl)amino )-2-oxoethoxy)-*N*-(2-oxo-2-phenylethylbenzamide (DKC1125h12);
2-(2-oxo-2-((4-propylphenyl)amino)ethoxy)-*N*-(2-oxo-2-phenylethyl)benzamide (DKC1125h13);
2-(2-oxo-2-((3-phenoxyphenyl)amino)ethoxy)-*N*-(2-oxo-2-phenylethyl)benzamid (DKC1125h14);
2-(2-((3,4-dimethoxybenzyl)amino)-2-oxoethoxy)-*N*-(2-oxo-2-phenylethyl)benzamide (DKC1125h15);
2-(2-((3,4-dimethoxyphenyl)amino)-2-oxoethoxy)-*N*-(2-oxo-2-phenylethyl)benzamide (DKC1125h16);
2-(2-(benzo[*d*][1,3]dioxol-5-ylamino)-2-oxoethoxy)-*N*-(2-oxo-2-phenylethyl)benzamide (DKC1125h17);
2-(2-((2, 3-dihydrobenzo[*b*][1, 4]dioxin-6-yl)amino)-2-oxoethoxy)-*N*-(2-oxo-2-phenylethyl)benzamide (DKC1125h18);
2-(2-((2,3-dihydro-1*H*-inden-5-yl)amino)-2-oxoethoxy)-*N*-(2-oxo-2-phenylethyl)benzamide (DKC1125h19);
2-(2-((3-isopropoxyphenyl)amino)-2-oxoethoxy)-*N*-(2-oxo-2-phenylethyl)benzamide (DKC1125h20);
2-(2-oxo-2-((4-phenoxyphenyl)amino)ethoxy)-*N*-(2-oxo-2-phenylethyl)benzamide (DKC1125h21);
2-(2-((2-fluoro-5-(trifluoromethyl) phenyl)amino)-2-oxoethoxy)-*N*-(2-oxo-2-phenylethyl)benzamide (DKC1125h22);
2-(2-((3-hydroxyphenyl)amino)-2-oxoethoxy)-*N*-(2-oxo-2-phenylethyl)benzamide (DKC1125h23);
2-(2-((2-chlorophenyl)amino)-2-oxoethoxy)-*N*-(2-oxo-2-phenylethyl)benzamide (DKC1125h24);
2-(2-((3-bromophenyl)amino)-2-oxoethoxy)-*N*-(2-oxo-2-phenylethyl)benzamide (DKC1125h25);
2-(2-(isopropylamino)-2-oxoethoxy)-*N*-(2-oxo-2-phenylethyl)benzamide (DKC1125h26);
2-(2-(3,4-dihydroisoquinolin-2(1*H*)-yl)-2-oxoethoxy)-*N*-(2-oxo-2-phenylethyl)benzamide (DKC1125h27);
2-(2-oxo-2-propylamino)ethoxy)-*N*-(2-oxo-2-phenylethyl)benzamide (DKC1125h28);
2-(2-(isopentylamino)-2-oxoethoxy)-*N*-(2-oxo-2-phenylethyl)benzamide (DKC1125h29);
2-(2-(cyclohexylamino)-2-oxoethoxy)-*N*-(2-oxo-2-phenylethyl)benzamide (DKC1125h30);
3-((dimethylamino)methyl)-N-(2-oxo-2-phenylethyl)benzamide (DKC1125i01);
3-((3,4-dihydroisoquinolin-2(1H)-yl)methyl)-N-(2-oxo-2-phenylethyl)benzamide (DKC1125i02);
3-((isopropyl(methyl)amino)methyl)-N-(2-oxo-2-phenylethyl)benzamide (DKC1125i03);
3-((4-(2-fluorophenyl)piperazin-1-yl)methyl)-N-(2-oxo-2-phenylethyl)benzamide (DKC1125i04);
N-(2-oxo-2-phenylethyl)-3-((4-(pyrimidin-2-yl)piperazin-1-yl)methyl)benzamide (DKC1125i05);
3-((4-(2-methoxyphenyl)piperazin-1-yl)methyl)-N-(2-oxo-2-phenylethyl)benzamide (DKC1125i06);
3-([1,4'-bipiperidin]-1'-ylmethyl)-N-(2-oxo-2-phenylethyl)benzamide (DKC1125i07);
3-((4-(4-nitrophenyl)piperazin-1-yl)methyl)-N-(2-oxo-2-phenylethyl)benzamide (DKC1125i08);
3-((ethyl(phenyl)amino)methyl)-N-(2-oxo-2-phenylethyl)benzamide (DKC1125i09);
N-(2-oxo-2-phenylethyl)-3-((4-(pyridin-2-yl)piperazin-1-yl)methyl)benzamide (DKC1125i10);
3-((diallylamino)methyl)-N-(2-oxo-2-phenylethyl)benzamide (DKC1125i11);
3-((benzyl(methyl)amino)methyl)-N-(2-oxo-2-phenylethyl)benzamide (DKC1125i12);
3-(((2-hydroxyethyl)(methyl)amino)methyl)-N-(2-oxo-2-phenylethyl)benzamide (DKC1125i13);
3-((butyl(2-hydroxyethyl)amino)methyl)-N-(2-oxo-2-phenylethyl)benzamide (DKC1125i14);
tert-butyl 4-(3-((2-oxo-2-phenylethyl)carbamoyl)benzyl)piperazine-1-carboxylate (DKC1125i15);
3-((butyl(methyl)amino)methyl)-N-(2-oxo-2-phenylethyl)benzamide (DKC1125i16);
3-((diethylamino)methyl)-N-(2-oxo-2-phenylethyl)benzamide (DKC1125i17);
3-((dipropylamino)methyl)-N-(2-oxo-2-phenylethyl)benzamide (DKC1125i18);
3-((butyl(ethyl)amino)methyl)-N-(2-oxo-2-phenylethyl)benzamide (DKC1125i19);
3-((dipentylamino)methyl)-N-(2-oxo-2-phenylethyl)benzamide (DKC1125i20);
3-((ethynyl(methyl)amino)methyl)-N-(2-oxo-2-phenylethyl)benzamide (DKC1125i21);
3-(morpholinomethyl)-N-(2-oxo-2-phenylethyl)benzamide (DKC1125i22);
3-((4-methylpiperazin-1-yl)methyl)-N-(2-oxo-2-phenylethyl)benzamide (DKC1125i23);
4-((3,4-dihydroisoquinolin-2(1H)-yl)methyl)-N-(2-oxo-2-phenylethyl)benzamide (DKC1125i24);
4-((cyclohexyl(methyl)amino)methyl)-N-(2-oxo-2-phenylethyl)benzamide (DKC1125i25);
4-((isopropyl(methyl)amino)methyl)-N-(2-oxo-2-phenylethyl)benzamide (DKC1125i26);
4-((4-(2-fluorophenyl)piperazin-1-yl)methyl)-N-(2-oxo-2-phenylethyl)benzamide (DKC1125i27);
N-(2-oxo-2-phenylethyl)-4-((4-(pyrimidin-2-yl)piperazin-1-yl)methyl)benzamide (DKC1125i28);
4-((4-(2-methoxyphenyl)piperazin-1-yl)methyl)-N-(2-oxo-2-phenylethyl)benzamide (DKC1125i29);
4-([1,4'-bipiperidin]-1'-ylmethyl)-N-(2-oxo-2-phenylethyl)benzamide (DKC1125i30);
4-((4-(4-nitrophenyl)piperazin-1-yl)methyl)-N-(2-oxo-2-phenylethyl)benzamide (DKC1125i31);
4-((methyl(phenyl)amino)methyl)-N-(2-oxo-2-phenylethyl)benzamide (DKC1125i32);
4-((ethyl(phenyl)amino)methyl)-N-(2-oxo-2-phenylethyl)benzamide (DKC1125i33);
N-(2-oxo-2-phenylethyl)-4-((4-(pyridin-2-yl)piperazin-1-yl)methyl)benzamide (DKC1125i34);
4-((diallylamino)methyl)-N-(2-oxo-2-phenylethyl)benzamide (DKC1125i35);
4-((benzyl(methyl)amino)methyl)-N-(2-oxo-2-phenylethyl)benzamide (DKC1125i36);
4-(((2-hydroxyethyl)(methyl)amino)methyl)-N-(2-oxo-2-phenylethyl)benzamide (DKC1125i37);
4-((butyl(2-hydroxyethyl)amino)methyl)-N-(2-oxo-2-phenylethyl)benzamide (DKC1125i38);
tert-butyl 4-(4-((2-oxo-2-phenylethyl)carbamoyl)benzyl)piperazine-1-carboxylate (DKC1125i39);
4-((dicyclohexylamino)methyl)-N-(2-oxo-2-phenylethyl)benzamide (DKC1125i40);
4-((butyl(methyl)amino)methyl)-N-(2-oxo-2-phenylethyl)benzamide (DKC1125i41);
4-((dipropylamino)methyl)-N-(2-oxo-2-phenylethyl)benzamide (DKC1125i42);
4-((butyl(ethyl)amino)methyl)-N-(2-oxo-2-phenylethyl)benzamide (DKC1125i43);
4-((ethyl(2-hydroxyethyl)amino)methyl)-N-(2-oxo-2-phenylethyl)benzamide (DKC1125i44);
4-((ethynyl(methyl)amino)methyl)-N-(2-oxo-2-phenylethyl)benzamide (DKC1125i45);
4-(morpholinomethyl)-N-(2-oxo-2-phenylethyl)benzamide (DKC1125i46);
ethyl 4-(4-((2-oxo-2-phenylethyl)carbamoyl)benzyl)piperazine-1-carboxylate (DKC1125i47);
4-((2,6-dimethylmorpholino)methyl)-N-(2-oxo-2-phenylethyl)benzamide (DKC1125i48);
3-((3,4-dihydroisoquinolin-2(1H)-yl)methyl)-N-(2-(3-methoxyphenyl)-2-oxoethyl)benzamide (DKC1125i49);
3-((cyclohexyl(methyl)amino)methyl)-N-(2-(3-methoxyphenyl)-2-oxoethyl)benzamide (DKC1125i50);
3-((1-(2-fluorophenyl)piperidin-4-yl)methyl)-N-(2-(3-methoxyphenyl)-2-oxoethyl)benzamide (DKC1125i51);
N-(2-(3-methoxyphenyl)-2-oxoethyl)-3-((4-(2-methoxyphenyl)piperazin-1-yl)methyl)benzamide (DKC1125i52);
3-([1,4'-bipiperidin]-1'-ylmethyl)-N-(2-(3-methoxyphenyl)-2-oxoethyl)benzamide (DKC1125i53);
N-(2-(3-methoxyphenyl)-2-oxoethyl)-3-((4-(pyridin-2-yl)piperazin-1-yl)methyl)benzamide (DKC1125i54);
3-((diallylamino)methyl)-N-(2-(3-methoxyphenyl)-2-oxoethyl)benzamide (DKC1125i55);
3-((benzyl(methyl)amino)methyl)-N-(2-(3-methoxyphenyl)-2-oxoethyl)benzamide (DKC1125i56);
3-((ethyl(methyl)amino)methyl)-N-(2-(3-methoxyphenyl)-2-oxoethyl)benzamide (DKC1125i57);
3-((butyl(2-hydroxyethyl)amino)methyl)-N-(2-(3-methoxyphenyl)-2-oxoethyl)benzamide (DKC1125i58);
tert-butyl 4-(3-((2-(3-methoxyphenyl)-2-oxoethyl)carbamoyl)benzyl)piperazine-1-carboxylate (DKC1125i59);
3-((butyl(ethyl)amino)methyl)-N-(2-(3-methoxyphenyl)-2-oxoethyl)benzamide (DKC1125i60);
3-((dipentylamino)methyl)-N-(2-(3-methoxyphenyl)-2-oxoethyl)benzamide (DKC1125i61);
3-((4-(2-hydroxyethyl)piperazin-1-yl)methyl)-N-(2-(3-methoxyphenyl)-2-oxoethyl)benzamide (DKC1125i62);
N-(2-(3-methoxyphenyl)-2-oxoethyl)-3-(morpholinomethyl)benzamide (DKC1125i63);
3-((ethyl(propyl)amino)methyl)-N-(2-(3-methoxyphenyl)-2-oxoethyl)benzamide (DKC1125i64);
3-((2,6-dimethylmorpholino)methyl)-N-(2-(3-methoxyphenyl)-2-oxoethyl)benzamide (DKC1125i65);
2-((dimethylamino)methyl)-N-(2-oxo-2-phenylethyl)benzamide (DKC1125i66);
2-((3,4-dihydroquinolin-1(2H)-yl)methyl)-N-(2-oxo-2-phenylethyl)benzamide (DKC1125i67);
2-((cyclohexyl(methyl)amino)methyl)-N-(2-oxo-2-phenylethyl)benzamide (DKC1125i68);
2-((4-(2-fluorophenyl)piperazin-1-yl)methyl)-N-(2-oxo-2-phenylethyl)benzamide (DKC1125i69);
N-(2-oxo-2-phenylethyl)-2-((4-(pyrimidin-2-yl)piperazin-1-yl)methyl)benzamide (DKC1125i70);
2-([1,4'-bipiperidin]-1'-ylmethyl)-N-(2-oxo-2-phenylethyl)benzamide (DKC1125i71);
N-(2-oxo-2-phenylethyl)-2-((4-(pyridin-2-yl)piperazin-1-yl)methyl)benzamide (DKC1125i72);
2-((diallylamino)methyl)-N-(2-oxo-2-phenylethyl)benzamide (DKC1125i73);
2-(morpholinomethyl)-N-(2-oxo-2-phenylethyl)benzamide (DKC1125i74); and
ethyl 4-(2-((2-oxo-2-phenylethyl)carbamoyl)benzyl)piperazine-1-carboxylate (DKC1125i75).

4. A composition for inhibiting cancer metastasis and invasion containing the compound of any one of claims 1 to 3.

5. The composition of claim 4, wherein the cancer is selected from the group consisting of colon cancer, laryngeal cancer, lung cancer, prostate cancer, breast cancer, gastric cancer, and hepatocellular carcinoma.

6. A composition for treating cancer containing the compound of any one of claims 1 to 3.

7. The composition of claim 6, wherein the cancer is selected from the group consisting of colon cancer, laryngeal cancer, lung cancer, prostate cancer, breast cancer, gastric cancer, and hepatocellular carcinoma.
